(19) Europäisches Patentamt
European Patent Office
Office européen des brevets

(11) Publication number: **0 284 180 B1**

(12) **EUROPEAN PATENT SPECIFICATION**

(45) Date of publication of patent specification: **19.08.92**

(51) Int. Cl.$^5$: **C07C 405/00**, A61K 31/557

(21) Application number: **88300709.8**

(22) Date of filing: **28.01.88**

(54) **Prostaglandins E and anti ulcers containing same.**

(30) Priority: **28.01.87 JP 18820/87**
**18.03.87 JP 65352/87**

(43) Date of publication of application:
**28.09.88 Bulletin 88/39**

(45) Publication of the grant of the patent:
**19.08.92 Bulletin 92/34**

(84) Designated Contracting States:
**AT BE CH DE ES FR GB GR IT LI LU NL SE**

(56) References cited:
**FR-A- 2 272 641**
**US-A- 3 836 578**

(73) Proprietor: **Kabushiki Kaisha Ueno Seiyaku Oyo Kenkyujo**
**2-31, Koraibashi**
**Higashi-ku Osaka-shi Osaka-fu(JP)**

(72) Inventor: **Ueno, Ryuzo**
**10-27 Nango-cho**
**Nishinomiya-shi Hyogo-ken(JP)**
Inventor: **Ueno, Ryuji**
**2-23 Shimogamomiyazaki-cho Sakyo-ku**
**Kyoto-shi Kyoto-fu(JP)**
Inventor: **Kato, Ichie**
**1-16 Jizoyama, Aza Higashiunero**
**Kawanishi-shi Hyogo-ken(JP)**
Inventor: **Oda, Tomio**
**1-116-1-202, Midorigaoka**
**Itami-shi Hyogo-ken(JP)**

(74) Representative: **Atkinson, Peter Birch et al**
**Marks & Clerk Suite 301 Sunlight House**
**Ouay Street**
**Manchester M3 3JY(GB)**

**EP 0 284 180 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel type of prostaglandin E and ulcer preventive agents containing the same.

Prostaglandin is a generic term for various prostanoic acids and is classified into various groups, such as E, F, A, B, C, D, and H, according to the manner in which keto and/or hydroxyl groups are introduced in five- membered ring portions. Prostaglandins will stimulate the uterine muscle and, in addition, they have various physiological and pharmacological actions, such as vasodilation, inhibition of platelet aggregation, and inflammatory action.

Prostaglandin E (hereinafter referred to as PGE), as a substance with a five-membered ring structure, as a group represented by:

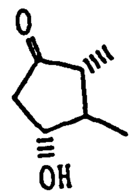

Broadly, there are known two types of PGE, namely, $PGE_1$ in which the carbon-carbon bond at the 5- and 6-positions ($C_5$-$C_6$ bond) is a single bond:

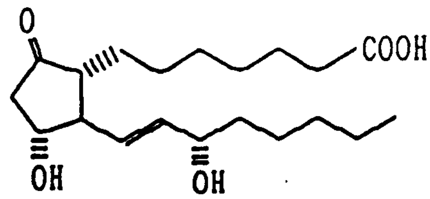

and $PGE_2$ in which the $C_5$-$C_6$ bond is a double bond:

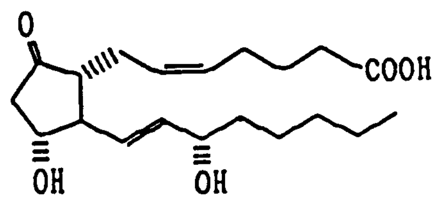

$PGE_2$, for example, is known as having antiulcer activity on one hand, but on the other hand it has such actions as uterine contraction, intestine contraction, and vasodilation; further it is recognized as having side effects, such as severe alvine flux. Therefore, it is unsuitable or impossible to use $PGE_2$ as antiulcers.

Whilst, it has been recognized that in human or animal metabolites there are present free substances similar to prostaglandin E in which $C_{13}$-$C_{14}$ bond is saturated and in which the carbon at the 15-position forms a carbonyl group. These substances, or species of 13,14-dihydro-15-keto prostaglandin E are:

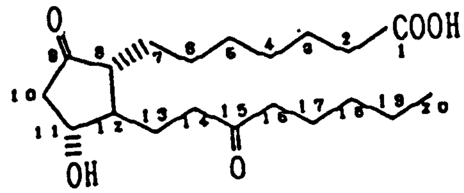

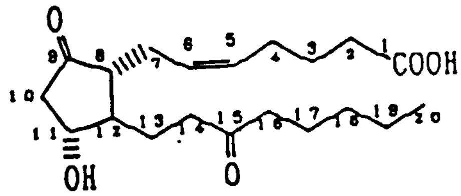

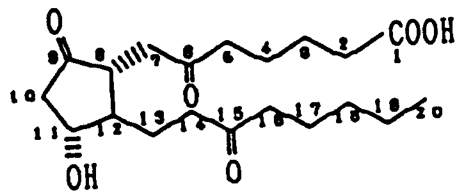

These correspond to $PGE_1$, $PGE_2$, and 6-keto $PGE_1$ respectively, and they are known as substances whech are naturally metabolically produced in vivo through enzymic metabolic reaction. These species of 13, 14-dihydro-15-keto PGE have been reported as physiologically and pharmacologically inactive metabolic products which exhibit little of the various physiological activities of PGE (Acta Physiologica Scandinavica, Vol 66, p. 509 ~ , 1966), and has been regarded as such. Therefore, little has been expected of the pharmacological effect of these metabolic products and compounds similar to them.

## SUMMARY OF THE INVENTION

While evaluating the pharmacological activities of derivatives of the aforesaid metabolic products, the present inventor found the the derivatives, such as esters salts, one having a protective group on the carboxyl group as well as one having free carboxyl group, one having substituent groups at the 16-, 17-, 19-, and/or 20-positions, one in which the carbon at the 11- position has a hydroxymethyl group, and one having an alkoxy group at the terminal of a ω chain, exhibited antiulcer activities, and that they showed no trace or a significantly reduced degree of such central and peripheral physiological effects as were simultaneously appeared as a side effect and were inherent to known or common PGE which had been recognized as having antiulcer activities.

## BRIEF DESCRIPTION OF DRAWING

Fig. 1 - 57 show n.m.r. spectra of the prostagrandins obtained in the present invention.

## DETAILED DESCRIPTION OF THE INVENTION

According to a first aspect of the present invention there is provided 13,14-dihydro-15-keto prostaglandins E represented by the general formula:

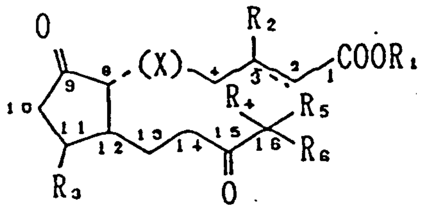

(I)

(in which X represents :

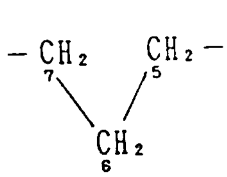 , 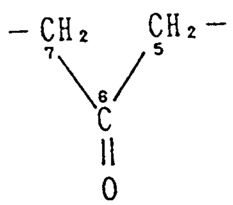 , 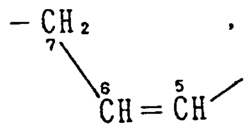 , 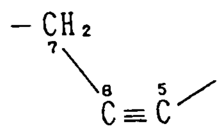

$R_1$ represents : hydrogen atom, physiologically acceptable salts, physiologically acceptably protective group $C_1$-$C_4$ alkyl, benzyl, hydroxyalkyl, or alkoxyalkyl group;

$R_2$ represents: hydrogen atom or a methyl group;

$R_3$ represents: a hydroxyl, or hydroxymethyl group;

$R_4$ and $R_5$ are the same or different, and signify a hydrogen atom, a methyl group, hydroxy group, or halogen atom, subject to the provisos that

(I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or

(II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond.

According to a second aspect of the present invention there is provided the use of prostaglandins E expressed by a general formula:

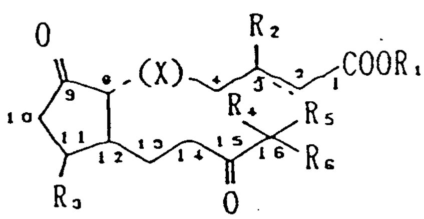

(I)

in which X represents:

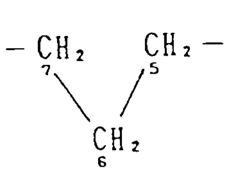 . 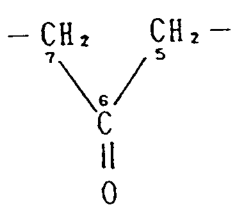 . 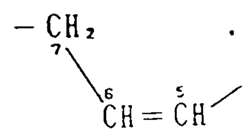 . 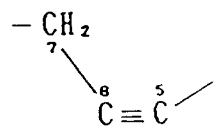

$R_1$ represents: hydrogen atom, physiologically acceptable salts, physiologically acceptable

protective group, $C_1$-$C_4$ alkyl, benzyl, hydroxyalkyl;

$R_2$ represents: hydrogen atom or a methyl group;

$R_3$ represents: a hydroxyl, or hydroxymethyl group;

$R_4$ and $R_5$, are the same or different, and signify a hydrogen atom, a methyl group, hydroxy group, or halogen atom, subject to the provisos that

(I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or

(II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent groups in which $C_2$-$C_3$ bond may be a double bond,

for the manufacture of an anti-ulcer composition.

In the general formula (I), (X) - has any of the above shown structures.

A compound where - (X) - is

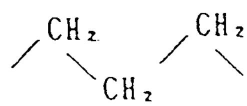

is a prostaglandin belonging to the $PGE_1$ group, and a compound where - (X) - is

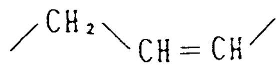

is a prostaglandin to the $PGE_2$. Therefore, a compound where -(X)- is

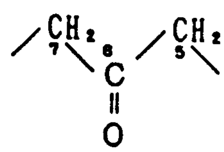

is a prostaglandin to the 6-keto $PGE_1$.

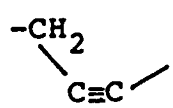

is a prostaglandins E belonging to 5,6-dehydro-$PGE_2$.

$R_1$ in the general formula (I) represents hydrogen atom an alkyl, benzyl, hydroxyalkyl, alkoxyalkyl group having 1 - 4 carbon atoms, a physiologically acceptable salt residue, or a physiologically acceptable protective group.

The alkyl group may be a cycloalkyl group, e.g., a cyclopropyl group, a cyclopentyl group, or an alkyl group having a side chain or a double bond structure, such as, for example, isopropyl group, tert-butyl group, or allyl group. Preferably, however, it is a straight chain saturated alkyl group, or more specifically a methyl or ethyl group. Examples of the hydroxyalkyl group are hydroxyethyl and hydroxyisopropyl groups. Or, it may be an alkoxyalkyl group, such as methoxyethyl group or alkoxyalkyl group.

$R_2$ represents hydrogen or a methyl group, in which the carbons at the 2- and 3-positions may have a double bond.

The carboxyl group may be free, a salt residue, or a protective group. As the salt may be a physiologically acceptable salt, for example, alkaline metal salt such as sodium salt, potassium salt and the

like; alkaline earth metal salt such as calcium, magnesium salt; ammonium salt; a physiologically acceptable amine salt such as salt of methylamine, dimethylamin, cyclopentylamine, benzylamine, pyperidine, monoethanolamine, diethanolamine, monomethylmonoethanolamine, tromethamine, lysine, tetraalkylammonium and the like. The protective group may include alkylsilicon such as trimethylsilicon, triethylsilicon and the like; tetrahydroxypyran and the like.

$R_3$ represents a hydroxyl, or hydroxymethyl group, in which the steric configuration relating to the carbon at the 11-position may take the form of $\alpha$, $\beta$, or a mixture thereof. Especially, one in which such steric configuration takes the $\alpha$-position.

$R_4$ and $R_5$ are independently hydrogen, methyl or hydroxyl groups, or halogens. $R_4$ and $R_5$ may be identical or different, but preferably at least one of them is a methyl group or a halogen, or more particularly fluorine atom.

$R_6$ is a saturated or unsaturated $C_2$ ~ $C_9$ alkyl group, or a $C_1$ ~ $C_9$ alkyl group having an alkoxy-substituent group. For the alkyl group, one having $C_4$ ~ $C_9$ is particularly preferred. For such $C_4$ ~ $C_9$ alkyl group, a straight-chain alkyl group or an alkyl group having one methyl group branch is particularly preferred. In the alkyl group having an alkoxy substituent, the alkoxy group is preferably methoxy or ethoxy, and for the alkyl group, one having $C_2$ ~ $C_6$ is suitable.

Typical examples of the compounds according to, and/or used in, the invention are:

13,14-dihydro-15-keto-$PGE_2$ cycloalkyl ester;
13,14-dihydro-15-keto-$PGE_2$ hydroxy alkyl ester;
13,14-dihydro-15-keto-$PGE_2$ benzyl ester;
13,14-dihydro-15-keto-$PGE_1$ alkyl ester;
13,14-dihydro-15-keto-18-methoxy-19, 20-dinor-$PGE_2$ or alkylester;
13,14-dihydro-15-keto-18-methoxy-$PGE_2$ or alkylester;
13,14-dihydro-15-keto-20-methoxy-$\Delta^2$-$PGE_2$ or alkyl ester;
13,14-dihydro-15-keto-3R,S-methyl-20-methoxy-$PGE_2$ or alkyl ester;
13,14-dihydro-15-keto-16R,S-hydroxy-$PGE_2$ or alkyl ester;
13,14-dihydro-15-keto-16R,S-fluoro-$PGE_2$ or alkyl ester;
13,14-dihydro-15-keto-16R,S-methyl-$PGE_2$ or alkyl ester;
13,14-dihydro-15-keto-16,16-dimethyl-$PGE_2$ or alkyl ester;
13,14-dihydro-15-keto-16,16-dimethyl-20-methoxy-$PGE_2$ or alkyl ester;
13,14-dihydro-15-keto-17S-methyl-$PGE_2$ or alkyl ester;
13,14-dihydro-15-keto-19-methy-$PGE_2$ or alkyl ester;
13,14-dihydro-15-keto-20-isopropropylidene $PGE_2$ or alkyl ester;
13,14-dihydro-15-keto-20-ethyl-$PGE_2$ or alkyl ester;
13,14-dihydro-15-keto-20-n-propyl-$PGE_2$ or alkyl ester;
13,14-dihydro-15-keto-20-ethyl-$PGE_1$ or alkyl ester;
13,14-dihydro-6,15-diketo-16R,S-fluoro-$PGE_1$
13,14-dihydro-6,15-diketo-16R,S-fluoro-11-dehydroxy-11R-methyl-$PGE_1$ or alkyl ester;
13,14-dihydro-6,15-diketo-16R,S-methyl-$PGE_1$ or alkyl ester;
13,14-dihydro-6,15-diketo-16,16-dimethyl-$PGE_1$ or alkyl ester;
13,14-dihydro-6,15-diketo-19-methyl-$PGE_1$ or alkyl ester;
13,14-dihydro-6,15-diketo-20-methyl-$PGE_1$ or alkyl ester;
13, 14-dihydro-6,15-diketo-11-dehydroxy-11R-hydroxymethyl $PGE_1$ alkyl ester.
13,14-dihydro-15-keto-20-methyl-$PGE_1$ or alkyl ester;
13,14-dihydro-15-keto-16R,S-fluoro-20-methyl-$PGE_2$ or alkyl ester,
13,14-dihydro-15-keto-16,16-difluoro-$PGE_2$ or alkyl ester,
13,14-dihydro-15-keto-5,6-dehydro-20-methoxy-$PGE_2$or alkyl ester.

The protaglandins E of the present invention can be synthesized in such way as shown illustrated in Examples and the accompanying synthesis charts (I) ~ (XXI). That is, a commercially available (-) or (±) Corey lactone (1) may be used as the starting material, and then collins-oxidized to give an aldehyde (2); the aldehyde (2) may be reacted with dimethyl (2-oxoalkyl) phosphonate to give an $\alpha$, $\beta$-unsaturated ketone (3), which is then reducted. The resulting unsaturated ketone (4) is protected with respect to its carbonyl group. A hydroxyl group after protective group, p-phenylbenzoate being removed is protected with THP. After lactone (7) is reduced to lactol (8), an $\alpha$ chain is introduced by Wittig reaction.

The $PGE_2$ in which - (X) - is

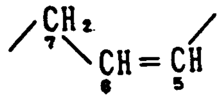

can be obtained by reducing the lactone (7) to lactol (8), then subjecting the lactol (8) to reaction with (4-carboxybutyl) triphenylphosphonium bromide.

The $PGE_1$ in which - (X) - is

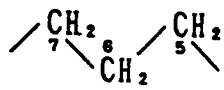

can be obtained through reduction of the $PGE_2$.

The 6-keto $PGE_1$ in which - (X) - is

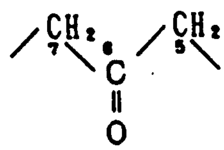

can be obtained by adding bromine or iodine atom on $C_5$-$C_6$ double bond of

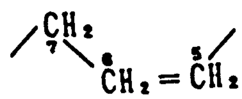

using N-bromosuccinimide or iodine atom, and simultaneously cyclizing the $C_6$-carbon and the hydroxyl group at the 9-position to give a bromide or a iodide, and then the bromide or iodide is treated with DBU to ketonize the carbon at the 6-position.

5,6-Dehydro-$PGE_2$s in which (X) is:

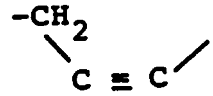

may be prepared by treating copper enolate which can be prepared by adding a monoalkyl-copper complex or a dialkylcopper complex of following formula on 1,4-position of 4R-t-butyldimethylsilyloxy-2-cyclopentene-1-on (167):

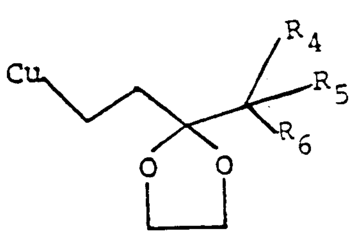

or

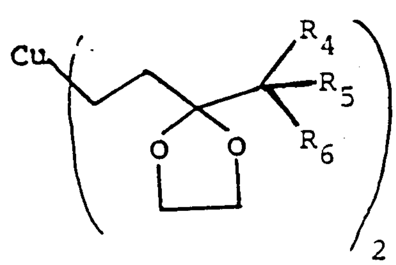

with 6-carboalkoxy-1-iodo-2-hexine or its derivatives.

The compound in which $R_3$ is hydroxymethyl group can be obtained by applying benzophenone as a photo-sensitizer to the A-type prostaglandin (PGA) obtained in manner as above described, then adding methanol.

For the synthesis of the PGE in which either $R_4$ or $R_5$ is a group other than hydrogen atom, and of the PGE in which $R_6$ is other than n-butyl, the compound used in obtaining the $\alpha$, $\beta$-unsaturated ketone (3), namely, dimethyl (2-oxoalkyl) phosphonate should be correspondingly replaced by other suitable compound. For example, where $R_4$ is fluorine atom, $R_6$ is n-butyl, and $R_5$ is hydrogen atom, dimethyl (3-fluoro-2-oxopeptyl) phosphonate may be used. Where $R_4$ and $R_5$ are hydrogen atom, and $R_6$ is an isopentyl group, dimethyl (6-methyl-2-oxoheptyl) phosphonate may be used.

The synthesis of the compounds of the invention is not limited to the foregoing. For protection of individual functional groups and for oxidation-reduction, suitable procedures may be applied as required.

The prostaglandins E of the present invention may be used as medicines for animal and human. Usually, they are used systemically or locally in various ways, such as oral administration, intravenous injection, and subcutaneous injection. The dosage varies according to the subject for administration, animal or human, age, weight, symptoms, efficacy of treatment, method of administration, and time of treatment.

Where the compounds of the invention are used in the form of solid compositions for oral administration, they include tablets, powder, and granules. In such solid composition, one or more active substances are mixed with at least one kind of inactive diluent, for example, lactose, mannitol, grape sugar, hydroxypropyl cellulose, crystallite cellulose, starch, polycinyl pyrrolidone, or magnesium metasilicoaluminate. Such composition may, according to the conventional procedure, contain some additive other than said inactive diluent, e.g., lubricant, such as magnesium stearate, decomposer, such as calcium fibrogluconate, etherified cyclodextrin, such as $\alpha$, $\beta$- or $\gamma$-cyclodextrin, dimethyl-$\alpha$-, dimethyl-$\beta$-, or hydroxypropyl-$\beta$-cyclodextrin, branched cyclodextrin, such as glucosyl-, or maltosylcyclodextrin, or stabilizer, such as formylated cyclodextrin, sulfur-containing cyclodextrin, misoprotol, or phospholipid. The aforesaid cyclodextrins may provide increased stability. The stability may be improve by forming liposome with a phospholipid.

Tablets or pills may be coated or covered with a gastrically soluble material, such as refined sugar, gelatin, hydroxypropyl cellulose, or hydroxypropyl methyl cellulose phthalate, or a film of such matereial in one or more layers. Also, they may be encapsulized with an absorbable material, such as gelatin.

In the form of liquid compositions for oral administration, the compounds of the invention include medically allowable emulsions, solutions, suspensions, syrups, and elixers. They may contain inactive diluents conventionally used, such as, for example, refined water, ethanol, and coconut oil. In addition to such inactive diluent, the compositions may contain wetting agents, auxiliary agents, such as suspensions, edulcorants, flavors, aromatics, and preservatives. The liquid compositions may be encapsulated as such in soft capsules and the like.

Other forms of compositions for oral administration include sprays prepared per se according to the usual known procedures which may contain one or more kinds of active substances.

The compounds of the invention in the form of injections for non-oral administration include sterile aqueous and non-aqueous solvents, suspensions, emulsions, and detergents.

The aqueous solutions and suspensions include, for example, distilled water and physiologic salt solution. The non- aqueous solutions and suspensions include, for example, vegetable oils, such as polyethylene glycol and olive oil, alcohols, such as ethanol, and Polysorbate. Such composition may contain auxiliaries, such as presevatives, wetting agents, emulsions, and dispersions. These compositions are sterilized by being passed through bacteria retaining filters or by incorporation of bactericides, or by light iradiation. It is also possible to first prepare a germ-free solid composition and dissolve same in a germ-free injection solvent before using it as an injection.

Example 1

1) Preparation of Dimethyl (3R,S-Fluoro-z-oxoheptyl)phosphonate:

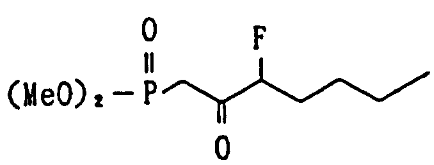

1 - 1 Methyl 2-R,S-Fluorocaproate:

Methyl 2R,S-bromocaproate (40g) was added to anhydrous potassium fluoride (23g) in acetoamide (23g) kept at 105° C. The mixture was vigorously stirred at 105° C for 6h. A crude product obtained after the usual work-up, was distilled under reduced pressure. Yield 20g (71%), b.p. 66° C/20 mmHg.

1 - 2 Dimethyl (3R,S-Fluoro-2-oxoheptyl)phosphonate:

Dimethyl methylphosphonate (8.38g) was dissolved in dry THF (200 ml), and the resulting solution was cooled to -78° C. n-Butyl lithium (1.6-M, 42ml) was added dropwise to the solution, and 10 min later 10 ml of the THF solution of methyl 2R, S-fluorocaproate (20g) was added dropwise. After the addition, the reaction solution was stirred at -78° C for 45 min, and then at room temperature for 45 min. A crude product obtained after the usual work-up was chromatographed (hexane : ethyl acetate = 1 : 1). Yield 5.04 g (62%).

2) Preparation of Dimethyl (3R,S-Methyl-2-oxoheptyl)phosphonate:

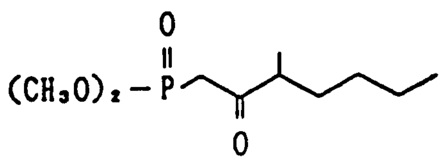

2 - 1 Methyl 2R,S-Methylcaproate :

A THF (50 ml) solution of dissopropylamine (12.9 ml) was cooled to -78° C and n-BuLi (1.6-M, 57,6 ml) was added dropwise over 1.5 h (preparation of LDA). A solution of methyl caproate (10g) in THE (50 ml) was added dropwise to the prepared LDA over 50 min. After stirring for 2 h, a solution of methyl iodide (6.2 ml) in THF (20 ml) was added dropwise over 40 min. The reaction solution was stirred at -78° C for 1 h, and then at room temperature overnight.

After the usual work-up, the resulting residue was distilled under reduced pressure, and thus 3.15 g of methyl 2R,S-methylcaproate (b.p. 44° C/10 mmHg) was obtained.

2 - 2 Dimethyl (3-Methyl-2-oxoheptyl)phosphonate:

To a THF (120 ml) solution of dimethyl methylphosphonate (5.04 g) at -60° C was added dropwise n-BuLi (1.6-M, 25.4 ml), and the mixture was stirred for 30 min. A THF (50 ml) solution of methyl 2R,S-methylcaproate (3.15 g) was added dropwise. The mixture was stirred at -60° C for 1 h, then at room temperature for 1.5 h, and thereafter acetic acid (2 ml) was added at 0° C. A crude product obtained after the usual work-up was chromatographed (hexane : ethyl acetate = 1 : 5). Yeild : 2,85g (58%).

3) Preparation of Dimethyl (6-Methyl-2-oxoheptyl)phosphonate:

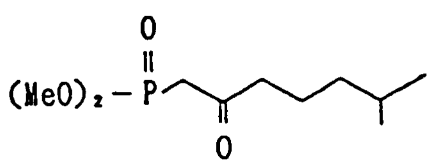

3 - 1 Methyl 5-methylcaproate:

Sodium ethoxide was prepared from sodium metal (9.1 g) and freshly distilled absolute ethanol (250 ml). Diethyl malonate (63.5 g) was added to sodium ethoxide in ethanol, and the mixture was stirred at 60 - 70° C for 50 min. Isoamyl bromide (60 g) was added, and the reaction mixture was heated under reflux overnight. After the usual work-up, the resulting crude product was distilled under reduced pressure to give

diethyl isoamylmalonate. Yield : 71.7 g (78%).

Diethyl isoamylmalonate (71.7 g) was added to a 50% aqueous solution of sodium hydroxide (60 ml), and the mixture was heated under reflux for 6 h. After cooling, the mixture was extracted with ether; the water layer was acidified with hydrochloric acid and, after saturation with sodium chloride, was extracted with ether. The extracts from the acidic aqueous layer were concentrated under reduced pressure to give isoamylmalonic acid. The obtained dicaboxylic acid was heated at 180° C for 2 h. After distillation under reduced pressure, 5-mehyl-caproic acid was obtained. Yield: 30 g (75%), b.p. 107 - 108° C/11 mmHg.

The 5-methyl-caproic acid (30 g) was treated with methanol (600 ml) and sulfuric acid (3 ml), and thus methyl 5-methylcaproate was obtained. Yield : 27 g (81%).

3 - 2 Dimethyl (6-Methyl-2-oxoheptyl)phosphonate:

Dimethyl (6-methyl-2-oxoheptyl)phosphonate was prepared from methyl 5-methylcaproate and dimethyl methylphosphonate according to the known method.

4) Preparation of Dimethyl (3,3-Dimethyl-7-methoxy-2-oxoheptyl)phosphonate:

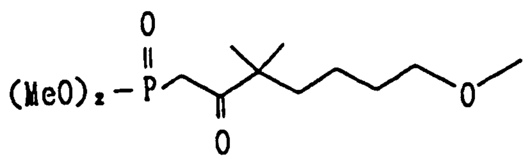

4 - 1 Methyl 2,2-Dimethyl-6-methoxy caproate:

1,4-Butanediol (50 g) was treated with sodium hydride (NaH) (60%, 26,6g) and methyl iodide (250g) in THF (150 ml) to give 4-methoxy-1-butanol. Yield : 21.8g (38%), b.p. 135/760 mmHg.

4-Methoxyl-1-butanol (8.49g) was treated with p-toluenesulfonyl chloride and 4-dimethylaminopyridine in methylene chloride (150 ml) to give 4-methoxy-butyl-1-tosylate. Yield : 16.1 g (77%).

4-Methoxy-butyl-1-tosylate (16.1g), together with NaI (18.7g) was agitated in acetone (80 ml) at room temperature for 3 h to give 1-iodo-4-methoxy-butane (9.05g, 68%).

To N-isopropylcyclohexylamine (5.96 ml) in THF (30 ml) was added dropwise n-BuLi (1,6-M, 22.7 ml) at -78° C, and the mixture was stirred for 30 min, to which a THF (5 ml) solution of methyl isobutyrate (3.43g) was added, and stirred at -78° C for 45 min. Then, a HMPA (6.3 ml) solution of 1-iodo-4-methoxy-butane (9.05g) was added to the mixture, and stirred at room temperature for 1 h to give methyl 2,2-dimethyl-6-methoxycaproate (4.81g, 85%) after usual work-up.

4 - 2 Dimethyl (3,3-Dimethyl-7-methoxy-2-oxoheptyl)phosphonate:

Prepared from methyl 2,2-dimethyl-6-methoxycaproate and dimethyl methylphosphonate according to the known method.

5) Preparation of Dimethyl (3-(2-Tetrahydropyranyl)oxy-2-oxoheptyl)phosphonate:

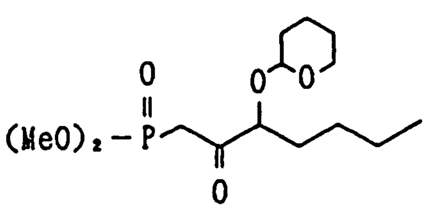

5 - 1 Methyl 2-(2-Tetrahydropyranyl)oxycaproate:

A tetrahydropyranyl ether was prepared from commercially available methyl 2R,S-hydroxycaproate according to the usual method. (Yield 71%).

5 - 2 Dimethyl (3-(2-Tetrahydropyranyl)oxy-2-oxoheplyl)phosphonate:

Prepared from methyl 2-(2-tetrahydropyranyl)oxycaproate and dimethyl methylphosphonate according to the known method. (Yield 48%).

6) Preparation of Dimethyl(4S-methyl-2-oxoheptyl)phosphonate:

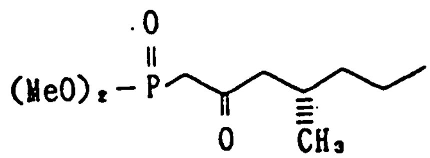

6 - 1 Ethyl 3S-Methyl-caproate :

Sodium ethoxide was prepared from sodium metal (7.61g) and absolute ethanol (200 ml). Diethyl malonate (50.3 ml) was added dropwise to the ethanol containing sodium ethoxide. After heating to 80 ° C, 2-bromopentane (50g) was added and the mixture was refluxed for 24 h. Diethyl (2-pentyl)malonate (62.7g) was obtained after the usual work-up. Diethyl (2-pentyl)malonate was added to a 50% potassium hydroxide solution and the mixture was heated for 3 h while water/ethanol being distilled off. After cooling, the solution was acidified with concentrated hydrochloric acid. Then, the solution was extracted with ethyl acetate. The extract was concentrated under reduced pressure, and the resulting product was heated to 180 ° C until bubbling ceased. After distillation, colorless 3R,S-methy-caproic acid was obtained. Yield : 27.7 g (35%), b.p. 200 ° C/760 mmHg.

3R,S-Methyl-caproic acid was dissolved in ehanol (160 ml) and cinchonidine (64g) was added and dissolved under heating.

The solution was concentrated under reduced pressure, and the resulting salt was recrystallized from 60% methanol six times to give needle crystals. Yield: 14.4g, $(\alpha)_D^{31}$ = -3.3 (C = 13.6 (benzene) literature value -3.1)

3S-Methyl-caproic acid (3.94g) was converted to the corresponding ethyl ester with using ethanol and catalytic amount of sulfuric acid. Yield : 4.04g (84%).

6 - 2 Dimethyl(4S-Methyl-2-oxoheptyl)phosphonate:

This compound was prepared from ethyl 3S-methyl-caproate and dimethyl methylphosphonate according to the known method.

7) Preparation of Dimethyl (3,3-Dimethyl-2-oxoheptyl)phosphonate :

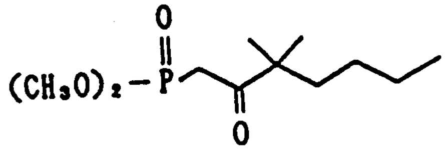

7 - 1 Ethyl 2,2-Dimethyl-caproate:

To LDA prepared at -78 ° C in the usual manner was added ethyl isobutyrate (45g) in THF, and stirred for 1 h. A dry HMPA solution of butyl iodide (107g) was added, and the mixture was stirred at -78 ° C for 1 h and then at room temperature for additional 1 h.

A crude product obtained after the usual work-up was distilled. Yield : 50g (75%), b.p. 68 ° C / 25

mmHg.

7 - 2 Dimethyl(3,3-Dimethyl-2-oxoheptyl) phosphonate:

Prepared from ethyl 2,2-dimethyl-caproate and dimethyl methylphosphonate according to the usual method.

8) Preparation of (3R,S-Methyl-4-carboxybutyl)triphenylphosphonium bromide:

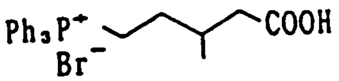

In ether (300 ml), 3-methyl-1,5-pentanediol (23.3g) was converted to 5-acetoxy-3-methyl-1-pentanol with pyridine (16 ml) and acetyl chloride (14 ml) at 0 ° C. Yield : 18.4 g.

5-Acetoxy-3-methyl-1-pentanol was oxidized with Jones reagent in acetone (200 mℓ) at -20 ° C to give 5-acetoxy-3R,S-methyl valeric acid. Yield : 8.2g (24%).

To 5-acetoxy-3R,S-methyl valeric acid (8.2g) was added hydrobromic acid (40 mℓ) and concentrated sulfuric acid (10 mℓ), and the mixture was agitated at 90 ° C overnight. Thereafter, the solution was poured into iced water. A crude product obtained after the usual work-up was chromatographed (ethyl acetate : hexane = 1.5), and thus 8.0g of 5-bromo-3R,S-methyl valeric acid (87%) was obtained.

5-Bromo-3R,S-methyl valeric acid with triphenyl phosphine (21.5g) was reflued in acetonitrile (100 mℓ) for 2 days. The reaction solution was poured into ether and the resulting precepitate was separated by filtration. Thus, (3-R,S-methyl-4-carboxybutyl)triphenylphosphonium bromide was obtained. Yield : 9.78g (52%).

Example 2 (See Chart I)

Preparation of 13,14-Dihydro-6,15-diketo-PGE$_1$ ethyl ester (15), R : Et

2 - 1 Preparation of 1S-2-Oxa-3-oxo-6R-(3-oxo-1-trans-octenyl)-7R-(4-phenylbenzoyl)oxy-cis-bicychlo(3, 3, 0) octane (3):

To the suspension of sodium hydride (NaH) (60%, 250 mg) in THF (40 mℓ) was added dropwise dimethyl(2-oxoheptyl)phosphonate, and the reaction solution was stirred for 30 min. A THF solution (40 mℓ) of the aldehyde (2) previously prepared by Collins oxidization of (-)-Corey lactone (1) (2g) was added. Reaction was maintained at room temperature overnight, and then acetic acid was added. After the usual work-up, an $\alpha,\beta$-unsaturated ketone (3) was obtained. Yield : 1.95g (50%).

2 - 2 Preparation of 1S-2-Oxa-3-oxo-6R-(3,3-ethylenedioxyoctanyl)-7R-(4-phenylbenzoyl)oxy-cis-bicyclo (3,3,0) octane (5) :

The unsaturated ketone (3) was hydrogenated in ethyl acetate (100 mℓ) with using 5% paradium-carbon (100 mg) and hydrogen to give the corresponding saturated ketone (4).

The ketone (4) (1.95g) was dissolved in toluene (150 mℓ), and ethylene glycol and p-toluenesulfonic acid (catalytic amount) were added. The solution was heated under reflux overnight while water produced was distilled off. After the usual work-up, ketal (5) was obtained. Yield : 1.8g (84%).

2 - 3 Preparation of 1S-2-Oxa-3-oxo-6R-(3,3-ethylenedioxy-1-octanyl)-7R-hydroxy-cis-bicyclo (3,3,0) octane (6) :

The compound (5) (1.8g) was dissolved in methanol (80 mℓ) and THF (20 mℓ), and after addition of potassium carbonate (0.563g), the solution was stirred at room temperature for 7 h. A crude product obtained by a usual manner was chromatographed (ethyl acetatehexane = 1 : 3 → 1 : 1) to give alcohol (6). Yield : 0.95g (82%).

2 - 4 Preparation of tetrahydropyranyl ether (7):

The compound (6) (0.95g) was dissolved in dichloromethane (100 mℓ) and then dihydropyran (0.76g) and p-toluene sulfonate (catalytic amount) were added. The resulting solution was stirred overnight. After the usual work-up and purification, tetrahydropyranyl ether (7) was obtained. Yield : 1.06g (88%).

2 - 5 Preparation of lactol (8)

To the tetrahydropyranyl ether (7) (1.06g) in dry toluene (30 mℓ) at -78°C was added dropwise diisobutylaluminum hydride (DIBAL-H) (1.5 M, 2.3 mℓ) and stirred for 60 min. Lactol (8) was obtained after the usual work-up.

2 - 6 Preparation of 13,14-Dihydro-11-(2-tetrahydropyranyl)oxy-15,15-ethylenedioxy $PGF_{2\alpha}$(9):

Sodium hydride (60%, 0.86g), washed with pentane, was suspended in DMSO (50 mℓ), and stirred for 90 min at 60 ~ 70°C. After the reaction solution was cooled to room temperature, (4-carboxybutyl)-triphenylphosphonium bromide in DMSO was added, and agitated for 30 min, to which lactol (8) in DMSO (10ml) was added. After stirred overnight, the reaction solution was poured into ice-water, made basic with addition of 20% sodium hydroxide solution, and extracted with ether. The aqueous layer was adjusted to pH 4 ~ 5 with 4N-hydrochloric acid and extracted with ethyl acetate. The ethyl acetate layer was washed with water, then with saturated sodium chloride solution, and was dried over magnesium sulfate. Thereafter, the solvent was distilled off. Ether was added and insolubles were separated by filtration. The filtrate was concentrated under reduced pressure to give the compound (9).

2 - 7 Esterification of the compound (9);

Preparation of the compound (10), R = Et:

The carboxylic acid (9) was dissolved in dry acetonitrile (50 mℓ) and then DBU (0.48g) and ethyl iodide (1.76g) were added. the solution was stirred at room temperature overnight. A crude product was obtained after the usual work-up, and was column-chromatographed (ethyl acetate-hexane 1 : 3). Thus, 1.04g of ethylester (10) was obtained. (Yield : 76% from (7))

2 - 8 Preparation of the compound (11)

The alcohol (10) (1.04g) was dissolved in dry tetrahydrofuran (3.4 mℓ) and dry methylene chloride (26.4 mℓ), and after addition of NBS (0.364g) at 0°C, the reaction solution was stirred for 5 min. A crude product was obtained after the usual work-up, and chromatographed (ethyl acetate-hexane = 1 : 3) to give the compound (11). Yield : 0.61g (51%).

2 - 9 Preparation of 13,14-Dihydro-15,15-ethylenedioxy-6-keto-11-(2-tetrahydropyranyl)oxy-$PGF_{1\alpha}$ ethyl ester (13) :

The bromoether (11) (0.61g) was dissolved in dry toluene (30 mℓ), and then DBU (25 mmℓ) was added. The solution was agitated at 40°C overnight. After the end of the period, the solution was cooled with ice and 1 N-hydrochloric acid was added to acidify the solution, and agitated for 10 minutes. Subsequently, the solution was extracted with ethyl acetate. A crude product was obtained after the usual work-up, and then chromatographed (ethyl acetate-hexane = 1 : 3 → 1 : 1) to give the compound (13). Yield : 0.332g (61%).

2 - 10 Preparation of 13,14-Dihydro-15,15-ethylenedioxy-6-keto-11-(2-tetrapyranyl)oxy-$PGE_1$ ethyl ester (14) :

The alcohol (13) (0.332g) was oxidized in acetone (20 mmℓ) at - 20°C with Jones reagent (2.67 M, 0.36 mmℓ). A crude product obtained after the usual work-up was chromatographed (ethyl acetate-hexane = 1 : 3) to give the compound (14). Yield : 0.198g (58%).

2 - 11 Preparation of 13,14-Dihydro-6,15-diketo-$PGE_1$ ethyl ester (15) :

The tetrahydropyranyl ether (14) (0.198g) was dissolved in a mixed solvent (14 mℓ) of acetic acid : water : THF (4 : 2 : 1), and the solution was stirred for 1 h at 45° C. Benzene was added, and the solvent was removed under reduced pressure. The resulting crude product was chromatographed (ethyl acetate-hexane = 1 : 3) to give 13,14-dihydro-6,15-diketo-$PGE_1$ ethyl ester (15). Yield : 0.098g (65%).

The n. m. r. spectrum of 13,14-dihydro-6,15-diketo-$PGE_1$ ethyl ester (15) is shown in Figure 1.

Mass (SIMS) m/z: 397 $(M + H)^+$, 379 ($(M + H)^+$ -18), 287, 157, 111, 99.

Example 3 (See Chart I)

Preparation of (±) 13,14-Dihydro-6,15-diketo-$PGE_1$ ethyl ester (15), R : Et :

Preparation of the title compound was carried out using (±)-Corey lactone (1) and a similar manner to the Example 1.

The n. m. r. spectrum of (±)-13,14-dihydro-6,15-diketo-$PGE_1$ ethyl ester (15) is shown in Figure 2.

Mass (SIMS) m/z: 397 $(M + H)^+$, 379 ($(M + H)^+$ -18), 287, 157, 111, 99.

Example 4 (See Chart I)

Preparation of 13,14-Dihydro-6,15-diketo-$PGE_1$ methyl ester (15), R: Me :

Preparation of the title compound was carried out in the same way as in Examples 2 and 3, except that (-)-Corey lactone (1) was used, and that the carboxylic acid (9) was methylated with diazomethane to give the compound (10) (R = $CH_3$).

The n. m. r. spectrum of the 13,14-dihydro-6,15-diketo-$PGE_1$ methyl ester (15) is shown in Figure 2.

Mass (SIMS) m/z: 405 $(M + H)^+$, 383 ($(M + H)^+$ -18), 365, 287, 143, 121, 111, 99.

Example 5 (See Charts I and II)

Preparation of13,14-Dihydro-15-keto-3R,S-methyl-$PGE_2$ methyl ester (19) :

Sodium hydride (60%, 1.72g), washed with pentane, was suspended in dry DMSO, and the suspension was agitated for 45 min at 70° C. After the reaction solution was ice-cooled, a DMSO solution of (3R,S-methyl-4-carboxybutyl)triphenylphosphonium bromide was added. The reaction was stood at room temperature. Then, a DMSO solution of lactol (8) produced from (-)-Corey lactone with the procedure shown in Examples 2 to 4 was added, and agitated for 2 h. The resultant was diluted with a mixed solvent of ether and ethyl acetate (1 : 1), and poured into 5% potassium carbonate solution. After vigorous stirring, separated organic layer was extracted with aqueous potassium carbonate solution twice. The combined basic aqueous layers were acidified with hydrochloric acid at 0° C, and then were extracted with ethyl acetate three times. The combined ethyl acetate layers were washed with sodium chloride solution, and then concentrated under reduce pressure. The residue thus obtained was dissolved in ether and insolubles were filtered off. The filtrate was partially concentrated and was treated with diazomethane. After subseqent concentration, a crude product was obtained, and was chromatographed (ethyl acetate-hexane = 2 : 5) to give a colorless oily substance (17) (2.15g, 56%).

The alcoholic substance (17) (2.15g) was oxidized in acetone (60 mℓ) at - 30° C with Jones reagent (2.67-M) (2.20 mℓ).

A residue obtained after the usual work-up was chromatographed (ethyl acetate : hexane = 1 : 3) to give a colorless oily substance (18) (1.64g, 77%).

The tetrahydropyranyl ether (18) (1.64g) was dissolved into a mixed solvent (50 mℓ) of acetic acid : water : THF (4 : 2 : 1), and agitated for 3 h at 45° C. The reaction solution was concentrated under reduced pressure, and the resulting crude product was chromatographed (ethyl acetate : benzene = 4 : 5) to give a colorless oily substance, 13,14-dihydro-15-keto-3R,S-methyl-$PGE_2$ methyl ester (19). Yield: 0.98g (80%).

The n. m. r. spectrum of 13,14-dihydro-15-keto-3R,S-methyl-$PGE_2$ methyl ester (19) is shown in Figure 3.

Mass (D I) m/z: 380 ($M^+$), 362 ($M^+$ - 18), 208, 109, 94, 81.

Example 6 (See Chart III)

Preparation of 13,14-Dihydro-15-keto-16R,S-methyl-$PGE_2$ ethyl ester (29), R = Et :

6 - 1 Preparation of 1S-2-Oxa-3-oxo-6R-(4R,S-methyl-3-oxo-1-trans-octenyl)-7R-(4-phenyl)benzoyloxy-cis-bicyclo (3, 3, 0) octane (20) :

Sodium hydride (60%, 0.228g) was suspended in anhydrous THR (40 mℓ), and a THF (30 mℓ) solution of dimethyl (3R,S-methyl-2-oxoheptyl)phosphonate (1.4g) was added with agitation for 30 min. To the resultant was added a THF solution (30 mℓ) of the aldehyde (2) obtained after collins oxidation of (-)- Corey lactone. The reaction was kept at room temperature for 2 h, and then acetic acid was added to neutralize the reaction. An $\alpha,\beta$-unsaturated ketone (20) was obtained after the usual work-up and the purification. Yield : 1.606g (61%).

6 - 2 Preparation of 1S-2-Oxa-3-oxo-6R-(3,3-ethylenedioxy-4R,S-methyl-1-octanyl)-7R-(4-phenyl)-benzoyloxy-cis-bicyclo (3, 3, 0) octane (22) :

The $\alpha,\beta$-unsaturated ketone (20) was hydrogenated in ethyl acetate with 5% palladium-carbon (0.150g), and hydrogen. The saturated ketone (21) thus obtained was dissolved in anhydrous benzene (150 mℓ), to which p-toluenesulfonic acid (in catalytic amount) and ethylene glycol (10 mℓ) were added, and refluxed overnight while water was distilled off. Ketal (22) was obtained after the usual work-up. Yield : 1.538g (87%).

6 - 3 Transesterification of the ketal (22): Synthesis of alcohol (23):

The ketal (22) (1.538g) was dissolved in absolute methanol (100 mℓ), and $K_2CO_3$ (0.503g) was added, the reaction was stirred for 5 h.

The reaction solution was neutralized with addition of acetic acid.

A crude product obtained after the usual work-up was chromatographed (ethyl acetate : hexane = 1 : 2) to give the alcohol (23). Yield : 0.8682g (88%).

6 - 4 Preparation of Tetrahydropyranyl ether (24):

The compound (23) (0.8682g) was dissolved in dry $CH_2C\ell_2$ (100 mℓ), and dihydropyran (5 mℓ) and p-toluenesulfonic acid (catalytic amount) were added. The reaction solution was stirred for 20 min. A crude product obtained after the usual work-up was chromatographed (hexane : ethyl acetate = 5 : 1) to give the tetrahydropyranyl ether (24). Yield: 1.040g (94%).

6 - 5 Preparation of lactol (25):

The tetrahydropyranyl ether (24) was treated with DIBAL-H (1.5-M, 5 mℓ) in dry toluene (30 mℓ) at -78 ° C to give the lactol (25). Yield : 1.030g.

6 - 6 Preparation of 13,14-Dihydro-15,15-ethylenedioxy-16R,S-methyl-11-(2-tetrahydropyranyl)oxy-PGF$_{2\alpha}$ - (26):

Sodium hydride (50%, 0.600g) washed with dry ether was suspended in DMSO (8 mℓ), and the suspension was heated at 60 ° C for 1 h with agitation. A DMSO (10 mℓ) solution of (4-carboxybutyl)-triphenylphosphonium bromide (3.3g) was added dropwise. Deep red ylide was obtained, to which the above lactol (25) in DMSO (8 ml) was added. The reaction was kept overnight at room temperature with stirring, and then poured into an ice-water, the aqueous solution was adjusted to pH 12 with 10% sodium hydroxide solution. The basic aqueous solution was extracted with ethyl acetate. The aqueous layer was adjusted to pH 6 with 1 N hydrochloric acid at 0 ° C, and was extracted with ethyl acetate, and the combined organic extract were washed with brine. After drying, the extract was concentrated under reduced pressure to give the carboxylic acid (26). Yield: 1.299g.

6 - 7 Preparation of ethyl ester (27), R = Et :

Esterification of the compound (26):

The carboxylic acid (26) (1.299g) was dissolved in dry acetonitrile (50 mℓ). To the solution were added ethyl iodide (0.6g) and DBU (0.4750g). The mixture was kept at 60 ° C for 2 h. A crude product obtained after the usual work-up, was chromatographed (hexane : ethyl acetate = 2 : 1) to give 0.6226g of the ethyl

ester (27). (Yield: 48%, from (24)).

6 - 8 Preparation of ketone (28):

The ethyl ester (27) (0.6226g) was oxidized with Jones reagent (2.67 - M, 0.45 ml) in acetone (40 mℓ) at -40° C.

A crude product obtained after the usual work-up was chromatographed (hexane-ethyl acetate = 3 : 1). Yield: 0.3942g (63%).

6 - 9 Preparation of 13,14-Dihydro-15-keto-16R,S-methyl-$PGE_2$ ethyl ester (29):

The ketone (28) (0.3942g) was dissolved in a mixted solvent (10 mℓ) of acetic acid : water : THF (3 : 1 : 1), and the solution was kept at 40° C for 4 h. A crude product obtained after the usual work-up was chromatographed (hexane-ethyl acetate = 4 : 1) to give 13,14-dihydro-15-keto-16R,S-methyl-$PGE_2$ ethyl ester (29). Yield: 0.1559g (53%).

The n. m. r. spectrum of the 13,14-Dihydro-15-keto-16R,S-methyl-$PGE_2$ ethyl ester (29) is shown in Figure 4.

Mass (SIMS) m/z: 395 $(M + H)^+$, 377 ($(M + H)^+$ -18), 331, 203, 109, 85.

Example 7 (See Chart III)

Synthesis of 13,14-Dihydro-15-keto-16R,S-methyl-$PGE_2$ methyl ester (29), R = Me :

The title compound (29) was prepared in the same manner as in Example 6 except that the carboxylic acid (26) was methylated with diazomethane.

The n. m. r. spectrum of the 13,14-dihydro-15-keto-16R,S-methyl-$PGE_2$ methyl ester (29) is shown in Figure 5.

Mass (D I) m/z: 380 ($M^+$), 362 (($M^+$ - 18), 331, 249, 234, 222, 137, 109.

Example 8 (See Chart IV)

Synthesis of 13,14-Dihydro-6,15-diketo-16R,S-methyl-$PGE_1$ ethyl ester (33), R = Et :

8 - 1 Preparation of bromide (30) R = Et :

$PGF_2$-ethyl ester derivative (27) (1.405g) was dissolved in a mixted solvent (50 mℓ) of THF-$CH_2C\ell_2$ (2 : 5). To the solution was added a THF-$CH_2C\ell_2$ (2 : 5; 20 mℓ) solution of NBS (0.5250g) at 0° C, which was agitated for 20 min. A crude product obtained after the usual work-up was chromatographed (hexane : ethyl acetate = 3 : 1) to give the bromide (30). Yield: 1.592g (98%).

8 - 2 Preparation of 13,14-Dihydro-15,15-ethylenedioxy-6-keto-16R,S-methyl-11-(2-tetrahydropyranyl)oxy-$PGF_{2\alpha}$ ethyl ester (31):

The bromide (30) (1.592g) was dissolved in toluene (4 mℓ) and DBU (3.5 mℓ), and the solution was stirred at 50° C overnight. After cooled, the solution was diluted with ether, and washed with sodium hydrogensulfite solution. A crude product obtained after the usual work-up was chromatographed (hexane : ethyl acetate = 1.5 : 1) to give the compound (31). Yield : 1.031g (72%).

8 - 3 Preparation of ketone (32)

The 6-keto-PGF derivative (31) (0.5012g) was oxidized with Jones reagent (2.67 - M : 1.2 ml) in acetone (35 mℓ) at - 25° C. A crude product obtained after the usual work-up was chromatographed (hexane : ethyl acetate = 1 : 1) to give the ketone (32). Yield : 0.3907g (78%).

8 - 4 Preparation of 13,14-Dihydro-6,15-diketo-16R,S-methyl-$PGE_1$ ethyl ester (33):

The 6-keto-PGF derivative (32) (0.3907g) was dissolved in a mixed solvent (24 mℓ) of acetic acid : water : THF (3 : 1 : 1), and the solution was kept at 50° C for 3.5 h. After cooled, the solution was

concentrated under reduced pressure. The resulting crude product was chromatographed (hexane : ethyl acetate = 1 : 1) to give 13,14-dihydro-6,15-diketo-16R,S-methyl-$PGE_1$ ethyl ester (33). Yield : 0.2100g (71%).

The n. m. r. spctrum of 13,14-dihydro-6,15-diketo-16R,S-methyl-$PGE_1$ ethyl ester (33) R ; Et, is shown in Figure 6.

Mass (SIMS) m/z: 411 $(M + H)^+$, 393 ($(M + H)^+$ -18), 375, 347, 301, 149, 130.

Example 9 (See Chart IV)

Synthesis of 13,14-Dihydro-6,15-diketo-16R, S-methyl-$PGE_1$ methyl ester (33), R = Me :

The title compound (33) was prepared from the methyl ester (27) following the same manner as the preparation of 13,14-dihydro-6,15-diketo-16R,S-methyl-$PGE_1$ ethyl ester (33).

The n. m. r. spctrum of the 13,14-dihydro-6,15-diketo-16R,S-methyl-$PGE_1$ methyl ester (33) is shown in Figure 7.

Mass (SIMS) m/z: 397 $(M + H)^+$, 379 ($(M + H)^+$ -18), 365, 347, 301, 143, 121, 111.

Example 10 (See Chart V)

Preparation of 13,14-Dihydro-15-keto-3R,S,16R,S-dimethyl-$PGE_2$ methyl ester (36):

10 - 1 Preparation of 13,14-Dihydro-15,15-ethylene dioxy-3R,S,16R,S-dimethyl-11-(2-tetrahydropyranyl)oxy-$PGF_{2\alpha}$ methyl ester (34):

Sodium hydride (60%, 0.4660g), washed with dry ether, was suspended in dry DMSO (8 mℓ), and the suspension was stirred at 60°C for 1 h. A DMSO solution of (3R,S-methyl-4-carboxybutyl)-triphenylphosphonium bromide (2.66g) was added to sodium methylsulfinyl carbonion to give deep red ylide. After addition, the reaction solution was stirred for 15 minutes. A DMSO solution (10 mℓ) of lactol (25) (0.8g) was added dropwise, and the mixture was agitated overnight. The reaction solution was poured in ice-water and adjusted to pH 12 with 10% sodium hydroxide solution, and then extracted with ether. The aqueous layer was adjusted to pH 5-6 with 1-N hydrochrolic acid and then extracted with ether. The organic extract of the acidic aqueous solution was dried, and concentrated under reduced pressure. The crude product thus obtained was esterified with diazomethane and then was chromatographed to give 13,14-dihydro-3R,S, 16R,S-dimethyl-15,15-ethylenedioxy-11-(2-tetrahydropyranyl)oxy-$PGE_2$ methyl ester (34). Yield: 0.7483g.

10 - 2 Preparation of 13,14-Dihydro-15-keto-3R,S, 16R,S-dimethyl-$PGE_2$ methyl ester (36):

According to the manner analogous to the Examples 2 to 9 with using the $PGF_2$ derivative (34), 13,14-dihydro-15-keto-3R,S,16R,S-dimethyl-$PGE_2$methyl ester (36) was produced.

The n. m. r. spectrum of 13,14-dihydro-15-keto-3R,S,16R,S-dimethyl-$PGE_2$ methyl ester (36) is shown in Figure 8.

Mass (SIMS) m/z: 395 $(M + H)^+$, 377 ($(M + H)^+$ -18), 345, 121, 109, 95.

Example 11 (See Chart VI)

Preparation of 13,14-Dihydro-6,15-diketo-16R,S-fluoro-$PGE_1$ ethyl ester (50):

11 - 1 Preparation of 1S-2-Oxa-3-oxo-6R-(4R,S-fluoro-3-oxo-1-trans-octenyl)-7R-(4-phenylbenzoyl)oxy-cis-bicyclo (3, 3, 0) octane (37):

Sodium hydride (60%, 1.70g) was suspended in THF, and a THF solution of dimethyl(3R,S-fluoro-2-oxoheptyl)phosphonate (4) (10.23g) was added to the suspension, and agitated at room temperature for 20 min. To the mixture was added a THF solution of aldehyde (2) which was obtained after Collins-oxidation of the (-)-lactone (1) (15.00 g).

After 2 h agitation at room temperature, the reaction solution was neutralized with acetic acid (15 mℓ). Thereafter, a residue obtained after the usual work-up was purified by column-chromatography (ethyl acetate : hexane = 1 : 2) to give a colorless oily enone (37) Yield: 10.45g (53%).

11 - 2 Preparation of 1S-2-Oxa-3-oxo-6R-(4R, S-fluoro-3R,S-hydroxy-1-octyl)-7R-(4-phenylbenzoyl)oxy-cis-bicyclo (3, 3, 0) octane (39):

The enone (37) (10.45g) was hydrogenated with 5% palladium or carbon (1.0g) and hydrogen in ethyl acetate (50 mℓ) to give ketone (38). Yield : 9.35g (89%).

The ketone (38) (9.35g) was reduced with sodium borohydride (1.15%) in absolute methanol (200 mℓ) to give a colorless oily substance (39).
Yield : 6.50g (69%).

11 - 3 Preparation of 1S-2-Oxa-3-oxo-6R-(4R,S-fluoro-3R,S-t-butyldimethylsilyloxy-1-octyl)-7R-hydroxy-cis-bicyclo(3, 3, 0)octane (41):

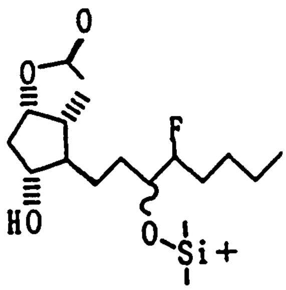

(41)

The alcohol (39) (6.50g) was converted with t-butyldimethylsilyl chloride (6.27g) and imidazole (5.67g) in dry DMF (30 mℓ) to the corresponding t-butyldimethylsilyl ether (40).
Yield: 8.80g (100%).

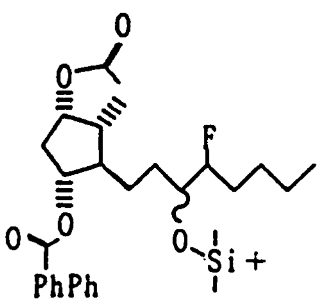

(40)

The t-butyldimethylsilyl ether (40) (8.80g) was dissolved in methanol (80 mℓ), and anhydrous potassium carbonate (2.09g) was added to the solution. The reaction was stirred for 4 h at room temperature. A colorless oily alcohol (41) was obtained after the usual work-up, and purification. Yield : 4.11g (67%).

11 - 4 Preparation of 13,14-Dihydro-16R,S-fluoro-15R,S-t-butyldimethylsilyloxy-11-(2-tetrahydropyranyl)oxy-$PGF_{2\alpha}$ (44):

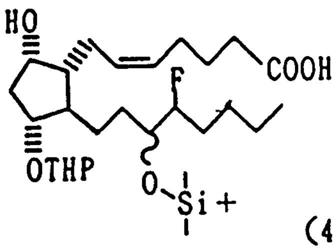

(44)

The alcohol (41) (4.11g) was dissolved in dry dichloromethane (50 mℓ), and dihydropyran (4.10 mℓ) and p-toluenesulufonic acid (catalytic amount) were added to the solution. The reaction solution was stirred at room temperature for 10 min. The residue obtained after usual work-up was chromatographed (ethyl acetate : hexane = 1 : 4 ~ 1 : 3) to give a colorless oily tetrahydropyranyl ether (42). Yield : 5.08g (100%).

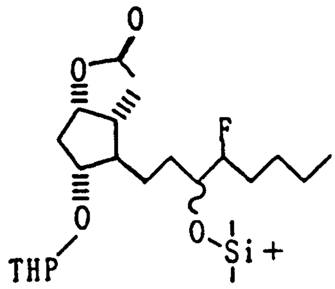

(42)

The tetrahydropyranyl ether (42) (5.08g) was reduced with DIBAL-H (1.5 M, 20 mℓ) in dry toluene (60 mℓ) at -78 °C, and a colorless oily lactol (43) was obtained.

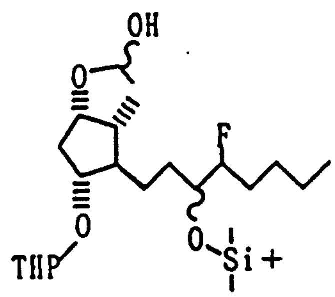

(43)

Ylide was prepared from (4-carboxybutyl) triphenylphosphonium bromide (18.51g) according to the usual procedure, and to this ylide was added a DMSO solution of the previously prepared lactol (43). The reaction solution was stirred at room temperature for 2h. The residue obtained after the usual work-up was dissolved in ether. After insoluble material was separated by filtration, the filtrate was concentrated under reduced pressure, and a crude carboxylic acid (44) was obtained. Yield: 8.0g.

11 - 5 Preparation of 13,14-Dihydro-16R,S-fluoro-15R,S-hydroxy-11-(2-tetrahydropyranyl)oxy-PGF$_{2\alpha}$ ethyl ester (46) :

The crude carboxylic acid (44) (8.0g) was dissolved in dry acetonitrile (40 mℓ), and DBU (3.0 mℓ) and ethyl iodide (6.0 mℓ) were added, and agitated at 60 °C for 60 min. The residue obtained after usual work-up was chromatographed (with ethylacetate : hexane = 1 : 4 ~ 1 : 2) to give a colorless oily ester (45). Yield: 1.84g.

The ester (45) (1.84g) was dissolved in dry THF (30 mℓ), and tetrabutylammonium fluoride (1.0-M, 45 mℓ) was added. The reaction solution was stirred at room temperature for 3.5 h. The reside obtained after the usual work-up was chromatographed (ethyl acetate : hexane = 1 : 2 ~ 1 : 3) to give a colorless oily alcohol (46). Yield : 1.34g (90%).

11 - 6 Preparation of 13,14-Dihydro-16R,S-fluoro-15R,S-hydroxy-6-keto-11-(2-tetrahydropyranyl)oxy-PGF$_2$ ethyl ester (48):

The alcohol (46) (0.6254g) was dissolved in dry dichloromethane (30 mℓ) and dry THF (3 mℓ), and N - bromosuccinimide (0.229g) were added. The reaction solution was stirred for 10 min. The residue obtained after the usual work-up was chromatographed (ethyl acetate : hexane = 2 : 3) to give a colorless oily bromo-ether (47). Yield : 0.6837g (94%).

The bromo-ether (47) (0.8243g) was dissolved in dry toluene (30 mℓ) and DBU (2.20 mℓ). The mixture was stirred at 65 °C overnight. After addition of water to the reaction solution, the mixture was acidified with

dilute hydrochloric acid under ice cooling, and was extracted with ethyl acetate. The residue obtained after the usual work-up was chromatographed (ethyl acetate : hexane = 1 : 1 ~ 2 : 1) to give a colorless oily 6-keto substance (48). Yield : 0.482g (66%).

11 - 7 Preparation of 13,14-Dihydro-6,15-diketo-16R,S-fluoro-$PGE_1$ ethyl ester (50) :

The dialcoholic substance (48) (0.230g) was oxidized in acetone (20 mℓ) at -10°C to -8°C with Jones reagent (2.67 M, 1.5 mℓ).

The residue obtained after the usual work-up was chromatographed (with ethyl acetate : hexane = 1 : 2) to give a colorless oily keto substance (49). Yield : 0.100g (44%)

The tetrahydropyranyl ether (49) (0.200g) was dissolved in a mixed solvent (20 mℓ) of acetic acid : water : THF (4 : 2 : 1), and the solution was stirred at 47°C for 3 hours.

The reaction solution was concentrated under reduced pressure, and the resulting residue was chromatographed (ethyl acetate : hexane = 1 : 1) to give 13,14-dihydro-6,15-diketo-16R,S-fluoro-$PGE_1$ ethyl ester (50). Yield: 0.153g (92%).

The n. m. r. spectrum of 13,14-dihydro-6,15-diketo-16R,S-fluoro-$PGE_1$ ethyl ester (50) is shown in Figure 9.

Mass (SIMS) m/z: 415 $(M + H)^+$, 397 $((M + H)^+ -18)$, 377, 351, 305, 157, 111.

Example 12 (See Chart VII)

Preparation of 13,14-Dihydro-15-keto-16R,S-fluoro-11R-dihydroxy-11R-methyl-$PGE_2$ ethyl ester (54):

12 - 1 Preparation of 13,14-Dihydro-15R,S-t-butyldimethylsilyloxy-16R,S-fluoro-11-(2-tetrahydropyranyl)oxy-$PGE_2$ ethyl ester (51):

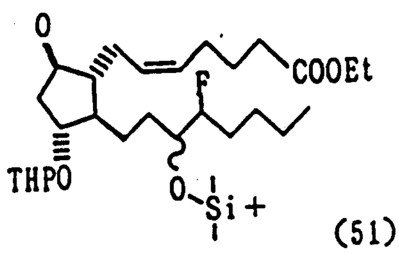

(51)

The alcohol (45) (0.506g) was oxidized (2.67 M) in acetone at -30°C with Jones reagent. The crude product obtained after the usual work-up was chromatographed (ethyl actate : hexane = 2 : 9) to give a ketonic substance (51). Yield : 0.380g (75%).

12 - 2 Preparation of 13,14-Dihydro-16R,S-fluoro-15R,S-hydroxy-$PGA_2$ ethyl ester (52) :

The tetrahydropyranyl ether (51) was dissolved in 23 mℓ of a mixed solvent of acetic acid and water (20 : 3), and the solution was stirred at 70°C. The reactant was concentrated under reduced pressure, and then was chromatographed (ethyl acetate : hexane = 1 : 3 ~ 1 : 1) to give a colorless oily enone (52). Yield: 0.078g (32%).

12 - 3 Preparation of 13,14-Dihydro-15-keto-16R,Sfluoro-11R-dehydroxy-11R-methyl-$PGE_2$ ethyl ester (54) :

Cuprous iodide (0.318g) was suspended in anhydrous ether (30 m ℓ), and methyl lithium (15-M; 2.23 mℓ) was added dropwise to the suspension at - 13°C to give a clear solution, to which the enone (52) (0.080g) in ether (20 ml) was added. The reaction solution was stirred for 45 min. Then, acetic acid (0.84 ml) was added. The mixture was poured into an aqueous ammonium chloride, and extracted with ether.

The extract was washed, dried, and then concentrated under reduced pressure. The resulting crude product was chromatographed (ethyl acetate : hexane = 2 : 5) to give a colorless oily alcoholic substance (53). Yield : 0.075g (90%).

The alcoholic substance (53) (0.136g) was oxidized with Jones reagent (2.67 M) in acetone (20 mℓ) at -

10°C to - 8°C. A crude product obtained after the usual work-up was chromatographed (ethyl acetate : hexane = 1 : 4) to give colorless oily 13,14-dihydro-15-keto-16R,S-fluoro-11R-dehydroxy-11R-methyl-$PGE_2$ ethyl ester (54). Yield: 0.122g (90%).

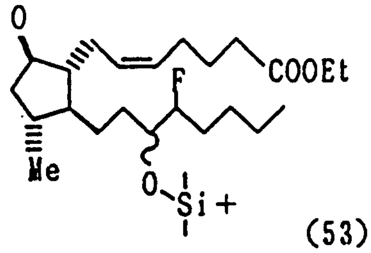

(53)

The n. m. r. spectrum of 13,14-dihydro-15-keto-16R,S-fluoro-11R-dehydroxy-11R-methyl-$PGE_2$ ethyl ester (54) is shown in Figure 10.

Mass (SIMS) m/z: 397 $(M + H)^+$, 379 ($(M + H)^+$ -18), 329, 301, 258, 237, 207, 167, 132.

Example 13

Preparation of 13,14-Ddihydro-15-keto-16R,S-fluoro-$PGE_2$ ethyl ester (56):

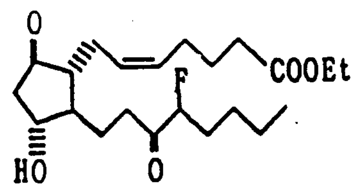

(56)

Diol (46) (Chart VI) (0.501g) was dissolved in acetone (35 mℓ) and was oxidized with Jones reagent at -35°C (2.67-M; 1 mℓ).

The crude product obtained after the usual work-up was chromatographed to give a tetrahydropyranyl ether (55). Yield: 0.347g (70%).

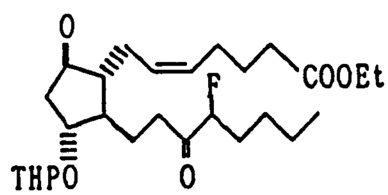

(55)

The tetrahydropyranyl ether (5) (0.347g) was dissolved in 25mℓ of a mixed solvent of acetic acid: THF : water (3 : 1 : 1), and the solution was stirred at 40°C for 12 h.

A crude product obtained after the usual work-up was chromatographed to give 13,14-dihydro-15-keto-16R,S-fluoro-$PGE_2$ ethyl ester (56). Yield: 0.204g (71%).

The n. m. r. spectrum of 13,14-dihydro-15-keto-16R,S-fluoro-$PGE_2$ ethyl ester (56) is shown in Figure 11.

Mass (D I) m/z: 398 $(M + H)^+$, 380 ($M^+$ - 18), 226, 109, 95, 81.

Example 14

Preparation of 13,14-Dihydro-6,15-diketo-16,16-dimethyl-$PGE_1$ ethyl ester (57) :

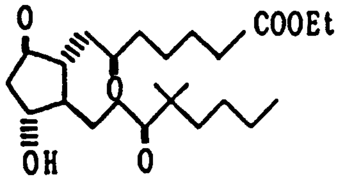

(57)

The title compound (57) was prepared following the procedure analogous to that in Example 2 to 13 with using (-)-Corey lactone (1) and dimethyl (3,3-dimethyl-2-oxoheptyl)phosphonate.

The n. m. r. spectrum of 13,14-dihydro-6,15-diketo-16,16-dimethyl-$PGE_1$ ethyl ester (57) is shown in Figure 12.

Mass (D I) m/z : 398 $(M + H)^+$, 380 $(M^+ - 18)$, 226, 109, 95, 81.

Example 15

Preparation of 13,14-Dihydro-15-keto-17S-methyl-$PGE_2$ ethyl ester (58) :

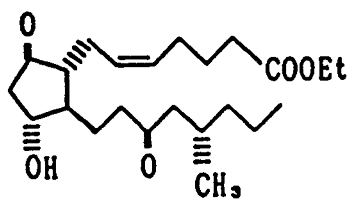

(58)

The same procedure as in Examples 1 to 14 was followed using dimethyl (4S-methyl-2-oxoheptyl)-phosphonate and (-)- Corey lactone (1), and thus 13,14-Dihydro-15-keto-17S-methyl-$PGE_2$ ethyl ester (58) was synthesized.

The n. m. r. spectrum of 13,14-dihydro-15-keto-17S-methyl-$PGE_2$ ethyl ester (58) is shown in Figure 13.

Mass (D I) m/z : 394 $(M^+)$, 376 $(M^+ - 18)$, 222, 109, 94.

Example 16 (See Chart VIII)

Preparation of 13,14-Dihydro-15-keto-19-methyl-$PGE_2$ ethyl ester (60), R = Et :

Same procedure as in Examples 2 to 15 was followed using the unsaturated ketone (59) obtained from dimethyl (6-methyl-2-oxoheptyl)phosphonate and (-)-Corey lactone (1), and thus 13, 14-dihydro-15-keto-19-methyl-$PGE_2$ ethyl ester (60) was synthesized.

The n. m. r. spectrum of 13,14-dihydro-15-keto-19-methyl-$PGE_2$ ethyl ester (60) is shown in Figure 14.

Mass (D I) m/z : 394 $(M^+)$, 376 $(M^+ - 18)$, 331, 222, 109, 95, 94.

Example 17 (See Chart VIII)

Preparation of 13,14-dihydro-15-keto-19-methyl-$PGE_2$ methyl ester (61), R = Me :

Preparation was carried out using the unsaturated ketone (59) and the same way as in Examples 2 to 16.

The n. m. r. spectrum of 13,14-dihydro-15-keto-19-methyl-$PGE_2$ methyl ester (61) is shown in Figure 15.

Mass (D I) m/z : 380 $(M^+)$, 362 $(M^+ - 18)$, 331, 222, 109, 95, 94.

Example 18 (See Chart VIII)

Preparation of 13,14-Dihydro-6,15-diketo-19-methyl-$PGE_1$ ethyl ester (62), R = Et :

Preparation was carried out using the unsaturated ketone (59) and the same way as in Examples 2 to 17.

The n. m. r. spectrum of 13,14-dihydro-6,15-diketo-19-methyl-PGE$_1$ ethyl ester (62) is shown in Figure 16.

Mass (SIMS) m/z : 411 (M + M)$^+$, 393 ((M + H)$^+$ -18), 323, 292, 291, 217, 201, 109.

Example 19 (See Chart VIII)

Preparation of 13,14-Dihydro-6,15-diketo-19-methyl-PGE$_1$ methyl ester (63), R = Me :

Preparation was carried out using the unsaturated ketone (59) and the same way as in Examples 2 to 18.

The n. m. r. spectrum of 13,14-dihydro-6,15-diketo-19-methyl-PGE$_1$ methyl ester (63) is shown in Figure 17.

Mass (D I) m/z : 369 (M$^+$), 378 ((M$^+$ - 18), 265, 235, 143, 111.

Example 20

Preparation of 13,14-Dihydro-15-keto-16,16-dimethyl-20-methoxy-PGE$_2$ methyl ester (64) :

The same procedure as in Examples 2 to 19 was followed using dimethyl (3,3-dimethyl-7-methoxy-2-oxoheptyl)phosphonate and (-)-Corey lactone (1), and thus 13,14-dihydro-15-keto-16,16-dimethyl-20-methoxy-PGE$_2$ methyl ester (64) was prepared.

The n. m. r. spectrum of 13,14-dihydro-15-keto-16,16-dimethyl-20-methoxy-PGE$_2$ methyl ester (64) is shown in Figure 18.

Mass (E I) m/z : 424 (M$^+$), 406 ((M$^+$ - 18), 375, 266, 375, 266, 245, 217, 129.

Example 21

Preparation of 13,14-Dihydro-15-keto-16R,S-hydroxy-PGE$_2$ ethyl ester (65) :

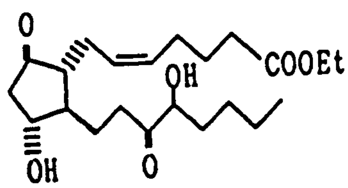

(65)

The same procedure as in Examples 2 to 20 was followed using (-)-Corey lactone (1) and dimethyl (3-(2-tetrahydropyranyl)oxy-2-oxoheptyl)phosphonate, and thus 13,14-dihydro-15 keto-16R,S-hydroxy PGE$_2$ ethyl ester (65) was synthesized.

The n. m. r. spectrum of 13,14-dihydro-15 keto-16R,S-hydroxy-PGE$_2$ ethyl ester (65) is shown in Figure 19.

Mass (D I) m/z : 396 (M$^+$), 378 ((M$^+$ - 18), 333, 309, 96, 81.

Example 22 (See Chart IX)

Preparation of 13,14-Dihydro-15-keto-PGE$_1$ ethyl ester(66), R: Et :

22 - 1) Preparation of 13,14-Dihydro-15,15-ethylenedioxy-11-(2-tetrahydropyranyloxy)-PGF$_{1\alpha}$ ethyl ester (64), R : Et :

13,14-dihydro-15,15-ethylenedioxy-11-(2-tetrahydropyranyl)oxy-PGF$_{2\alpha}$ ethyl ester (10), R = Et, (3.56g) was hydrogenated with platinum oxide and hydrogen in ethanol (150 mℓ). After the usual work-up, there was obtained 3.50g of 13,14-dihydro-15,15-ethylenedioxy-11-(2-tetrahydropyranyl)oxy-PGF$_{1\alpha}$ ethyl ester

(64).

22 - 2) Preparation of 13,14-Dihydro-15,15-ethylenedioxy-11-(2-tetrapyranyl)oxy-$PGE_1$ ethyl ester (65) :

13,14-dihydro-15,15-ethylenedioxy-11-(2-tetrahydropyranyl)oxy-$PGF_{1\alpha}$ ethyl ester (64) (3.25g) was oxidized with Jones reagent (2.67- M; 3.2 mℓ) in acetone (100 ml) at - 30°C. The crude product obtained after the usual work-up was chromatographed (hexane : ethyl acetate = 5 : 2) to give 13,14-dihydro-15,15-ethylenedioxy-11-(2-tetrahydropyranyl) oxy-$PGE_1$ ethyl ester (65). Yield 2.72 g.

22 - 3) Preparation of 13,14-Dihydro-15-keto-$PGE_1$ ethyl ester (66):

13,14-Dihydro-15,15-ethylenedioxy-11-(2-tetrahydropyranyl)oxy-$PGE_1$ ethyl ester (65) (2.72g) was dissolved in a mixed solvent (90 mℓ) of acetic acid : water : THF (4 : 2 : 1), and the solution was agitated for 3 h at 40 ~ 45°C. The solvent was distilled off under reduced pressure, and the resulting crude product was chromatographed twice (hexane : ethyl acetate = 1 : 1, and ethyl acetate : benzene = 1 : 1) to give 13,14-dihydro-15-keto-$PGE_1$ ethyl ester (66).

The n. m. r. spectrum of 13,14-Dihydro-15-keto-$PGE_1$ ethyl ester (66) is shown in Figure 20..

Example 23 (See Chart IX)

Preparation of 13, 14-Dihydro-15-keto-$PGE_1$ methyl ester (66), R : Me:

The same procedure as in Example 22 was followed using 13,14-dihydro-15,15-ethylenedioxy-11-(2-tetrahydropyranyl)oxy-$PGF_{2\alpha}$ methyl ester (10), the compound obtained from the carboxylic acid (9) with diazomethane, and thus 13,14-dihydro-15-keto-$PGE_1$ methyl ester (66) was synthesized.

The n. m. r. spectrum of 13,14-dihydro-15-keto-$PGE_1$ methyl ester (66) is shown in Figure 21..

Example 24 (See Chart X)

Preparation of 13,14-Dihydro-15-keto-$PGE_2$ ethyl ester (68), R : Et:

24 - 1) Preparation of 13,14-Dihydro-15,15-ethylenedioxy-11-(2-tetrahydropyranyl)-oxy-$PGE_2$ ethyl ester (67):

The ethyl ester (10) (3.4 g) was oxidized with Jones reagent acetone (150 ml) at -40°C, and ketone (67) was obtained. Yield : 2.6g.

24 - 2) Preparation of 13,14-Dihydro-15-keto-$PGE_2$ ethyl ester (68) :

Ketone (67) (2.6g) was dissolved in a mixed solvent (20 mℓ) of acetic acid : water : THF (4 : 2 : 1), and the solution was kept at 40 - 50°C for 3 h. Following the usual procedures, there was obtained 1.4g of 13,14-dihydro-15-keto-$PGE_2$ ethyl ester (68).

The n. m. r. spectrum of 13,14-dihydro-15-keto-$PGE_2$ ethyl ester (68) is shown in Figure 22.

Example 25 (See Chart X)

Preparation of 13,14-Dihydro-15-keto-$PGE_2$ methyl ester (68), R = Me:

The procedure of Example 24 was repeated, except that the carboxylic acid (9) was converted to the corresponding methyl ester (10) with diazomethane, and thus 13,14-dihydro-15-keto-$PGE_2$ methyl ester (68), R = Me, was obtained.

The n. m. r. spectrum of 13,14-dihydro-15-keto-$PGE_2$ methyl ester (68) is shown in Figure 23.

Example 26 (See Chart X)

preparation of 13,14-Dihydro-15-keto-$PGE_2$ n-propyl ester (68), R = n-Pro:

The same procedure as in Examples 24 and 25 was followed, except that the carboxylic acid (9) was

converted to the corresponding n-propyl ester (10) with DBU and n-propyl iodide in acetonitrile, and thus 13,14-dihydro-15-keto-$PGE_2$ n-propyl ester (68) was obtained.

The n. m. r. spectrum of 13,14-dihydro-15-keto-$PGE_2$ n-propyl ester (68) is shown in Figure 24.

Example 27 (See Chart X)

Preparation of 13,14-Dihydro-15-keto-$PGE_2$ isopropyl ester (68), R = iso-Pro:

The same procedure as in Examples 24, 25, and 26 was followed, except that the carboxylic acid (9) was converted to the corresponding isopropyl ester (10) with DBU and isopropyl iodide in acetonitrile, and thus 13,14-dihydro-15-keto-$PGE_2$ isopropyl ester (68) was obtained.

The n. m. r. spectrum of the 13,14-dihydro-15-keto-$PGE_2$ isopropyl ester (68) is shown in Figure 25.

Example 28 (See Chart X)

Preparation of 13,14-Dihydro-15-keto-$PGE_2$-n-butyl ester (68), R = n-Bu:

The same procedure as in Examples 24, 25, 26, and 27 was followed, except that the carboxylic acid (9) was converted to the corresponding n-butyl-ester (10) with DBU and n-butyl iodide in acetonitrile, and thus 13,14-dihydro-15-keto-$PGE_2$-n-butyl ester (68) was obtained.

The n. m. r. spectrum of 13,14-dihydro-15-keto-$PGE_2$ n-butyl ester (68) is shown in Figure 26.

Example 29 (See Chart X)

Preparation of 13, 14-Dihydro-15-keto-$PGE_2$ cyclopentyl ester (68), R = cyclopentyl:

The same procedure as in Examples 24, 25, 26, 27, and 28 was followed, except that the carboxylic acid (9) was converted to the corresponding cyclopentyl-ester (10) with DBU and cyclopentyl iodide in acetonitrile, and thus 13,14-dihydro-15-keto-$PGE_2$ cyclopentyl ester (68) was obtained.

The n. m. r. spectrum of 13,14-dihydro-15-keto-$PGE_2$ cyclopentyl ester (68) is shown in Figure 27.

Example 30 (See Chart X)

Preparation of 13,14-Dihydro-15-keto-$PGE_2$ benzyl ester (68), R = Benzyl:

The same procedure as in Examples 24, 25, 26, 27, 28, and 29 was followed, except that the carboxylic acid (9) was converted to the corresponding benzyl ester (10) with DBU and benzyl bromide in acetonitrile, and thus 13,14-dihydro-15-keto-$PGE_2$-benzyl ester (68) was obtained.

The n. m. r. spectrum of 13,14-Dihydro-15-keto-$PGE_2$ benzyl ester (68) is shown in Figure 28.

Example 31

Preparation of 13,14-Dihydro-15-keto-16,16-dimethyl-$PGE_2$ methyl ester (69), R = Me:

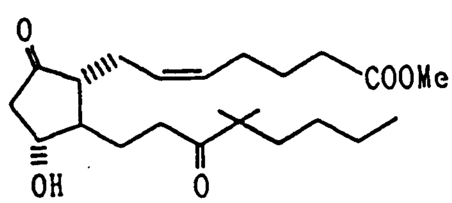

(69)

The same procedure as in Examples 24 to 30 was followed using (-)-Corey lactone (1) and a dimethyl (3,3-dimethyl-2-oxoheptyl)phosphonate obtained in the ordinary method, to produce 13,14-dihydro-15-keto-16,16-dimethyl-$PGE_2$ methyl ester (69).

The n. m. r. spectrum of 13,14-dihydro-15-keto-16,16-dimethyl-$PGE_2$ methyl ester (69) is shown in

Figure 29.

Example 32

Preparation of 13,14-Dihydro-15-keto-16,16-dimethyl-$PGE_2$ ethyl ester (70), R = Et:

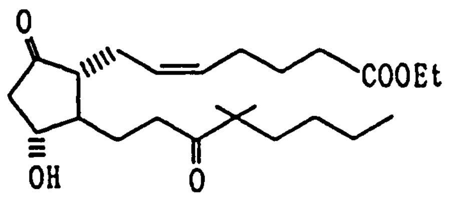

(70)

The same procedure as in Examples 24 to 31 was followed using (-)-Corey lactone (1) and dimethyl (3,3-dimethyl-2-oxoheptyl)phosphonate to produce 13,14-dihydro-15-keto-16,16-dimethyl-$PGE_2$ ethyl ester (70).

The n. m. r. spectrum of 13,14-Dihydro-15-keto-16,16-dimethyl-$PGE_2$ ethyl ester (70) is shown in Figure 30.

Example 33 (See Charts X and XI)

Preparation of 13,14-Dihydro-15-keto-3R,S-methyl-$PGE_2$ ethyl ester (74):

13,14-Dihydro-15-keto-3R,S-methyl-$PGE_2$ ethyl ester (74) was obtained by following the same procedure as in Examples 24 to 30 except that ylide prepared from (3-methyl-4-carboxybutyl)-triphenylphosphonium bromide, and that the lactol (8), were used to produce 13,14-dihydro-15,15-ethylenedioxy-3-methyl-11-(2-tetrahydropyranyl)oxy-$PGF_{2\alpha}$ (71).

The n. m. r. spectrum of 13,14-dihydro-15-keto-3 R,S-methyl-$PGE_2$ ethyl ester (74) is shown in Figure 31.

Example 34 (See Charts X and XII)

Preparation of 13,14-Dihydro-15-keto-20-methoxy-$PGE_2$ methyl ester (79):

The same procedure as in Examples 24 to 30 was followed using (-)-Corey lactone (1) and dimethyl (7-methoxy-2-oxoheptyl)phosphonate produced in the ordinary method, and thus 13,14-dihydro-15-keto-20-methoxy-$PGE_2$ methyl ester (79) was obtained.

The n. m. r. spectrum of 13,14-dihydro-15-keto-20-methoxy-$PGE_2$ methyl ester (79) is shown in Figure 32.

Example 35 (See Chart XII)

Preparation of 13,14-Dihydro-15-keto-3R,S-methyl-20-methoxy-$PGE_2$ methyl ester (80):

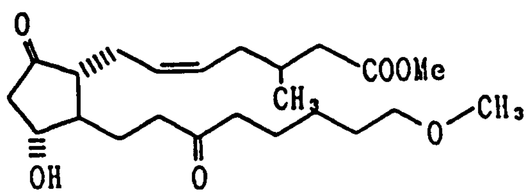

(80)

The same procedure as in Examples 24 to 30, 33 and 34 was followed using lactol (75) and (3-methyl-4-carboxybutyl) triphenylphosphonium bromide produced in the usual manner, and thus 13,14-Dihydro-15-

keto-3R,S-methyl-20- methoxy-$PGE_2$ methyl ester (80) was obtained.

The n. m. r. spectrum of 13,14-Dihydro-15-keto-3R,S-methyl-20-methoxy-$PGE_2$ methyl ester (80) is shown in Figure 33.

Example 36 (See Chart XIII)

Preparation of 13,14-Dihydro-15-keto-$\Delta^2$-$PGE_2$ methyl ester (85), R = H:

36 - 1) 11-(t-Butyldimethylsilyl)oxy-13,14-Dihydro-15,15-ethylenedioxy-2-phenylselecyl-$PGF_{2\alpha}$ methyl ester (82):

LDA was prepared from diisopropyl- amine (0.13 mℓ) in dry THF (3 ml), and n-butyl lithium (1.6 - M; 0.58 mℓ), at - 78° C. To LDA was added 0.1850g of (81) in THF, and stirred for 1.5 h. A dry THF solution (2 mℓ) of diphenyl diselenide (0.18g) was added, and the reaction solution was stirred at -78° C for 30 min, then at room temperature for 1 h. Following usual procedure, there was obtained 0.1366g of 2-phenylselenyl-$PGF_{2\alpha}$ methyl ester (82).

36 - 2) Preparation of 11-(t-Butyldimethylsilyl)oxy-13,14-dihydro-15,15-ethylenedioxy-$\Delta^2$-$PGF_{2\alpha}$ methyl ester (83) :

The 2-Phenylselenyl-$PGF_{2\alpha}$ methyl ester (82) (0.1366g) was dissolved in a mixed solvent (4 mℓ) of ethyl acetate- THF (1 : 1), and sodium hydrogen carbonate (0.1g) and 30% hydrogen pereoxide (0.3 mℓ) were added. The reaction solution was stirred at room temperature for 15 min. Following the usual procedures, there was obtained 0.0850g of 11-(t-butyldimethylsilyl)oxy-13,14-dihydro-15,15-ethylene dioxy-$\Delta^2$-$PGF_{2\alpha}$ methyl ester (83). Yield : 0.0850 g.

36 -3) Preparation of 11-(t-butyldimethylsilyl)oxy-13,14-Dihydro-15,15-ethylenedioxy-$\Delta^2$-$PGE_2$ methyl ester (84).

The $\Delta^2$-$PGF_{2\alpha}$ methyl ester (83) (0.0717g) was oxidized with PCC on aluminum oxide (1g) in benzene (2 mℓ). Following the usual procedures, there was obtained 0.0554g of $\Delta^2$ - $PGE_2$ methyl ester (84). Yield : 0.0554 g.

36 - 4) Preparation of 13,14-Dihydro-15-keto-$\Delta^2$-$PGE_2$ methyl ester (85) :

$\Delta^2$-$PGE_2$ methyl ester (84) (0.0554g) was dissolved in acetonitrile (2 mℓ), and a mixture (1.5 ml) of 46%-aqueous hydrogen fluoride and acetonitrile (1 : 2) was added. The reaction solution was stirred at room temperature for 50 min. Following the usual procedures, there was obtained 13,14-Dihydro-15-keto-$\Delta^2$-$PGE_2$ methyl ester (85). Yield : 0.0312 g.

The n. m. r. spectrum of 13,14-dihydro-15 keto-$\Delta^2$-$PGE_2$ methyl ester (85) is shown in Figure 34.

Example 37 (See Chart XIII)

Preparation of 13,14-Dihydro-15-keto-20-methoxy-$\Delta^2$-$PGE_2$ methyl ester (85), R = -OMe:

The same procedure as in Examples 24 to 30, 34 and 36 was followed with using (-)-Corey lactone (1) and dimethyl-(7-methoxy-2-oxoheptyl)phosphonate, and thus 13, 14-dihydro-15-keto-20-methoxy-$\Delta^2$-$PGE_2$ methyl ester (85), R = - OMe, was obtained.

The n. m. r. spectrum of 13,14-Dihydro-15-keto-20-methoxy-$\Delta^2$-$PGE_2$ methyl ester (85) is shown in Figure 35.

Example 38

Preparation of 13,14-Dihydro-15-keto-18-methoxy-19, 20-bisnor-$PGE_2$ methyl ester (86):

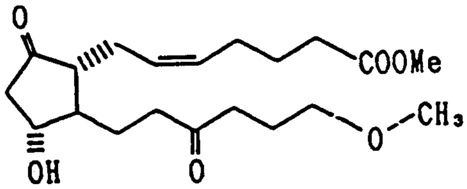

(86)

The same procedure as in Examples 24 to 30, and 34 was followed with using(-)-Corey lactone (1) and dimethyl (5-methoxy-2-oxopentyl)phosphonate, and thus 13,14-dihydro-15-keto-18-methoxy-19,20-bisnor-$PGE_2$ methyl ester (86) was obtained.

The n. m. r. spectrum of 13,14-Dihydro-15-keto-18-methoxy-19,20-bisnor-$PGE_2$ methyl ester (86) is shown in Figure 36.

Example 39

Preparation of 13,14-Dihydro-15-keto-20-ethyl-$PGE_2$ methyl ester (87), R = Me:

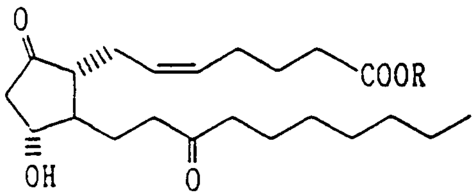

(87)

R =-Me

The same procedure as in Examples 24 to 30 was followed with using(-)-Corey lactone (1) and dimethyl(2-oxononyl)phosphonate, and thus 13,14-dihydro-15-keto-20-ethyl-$PGE_2$ methyl ester (87) was obtained.

The n. m. r. spectrum of 13,14-Dihydro-15-keto-20-ethyl-$PGE_2$-methyl ester (87) is shown in Figure 37.

Example 40

Preparation of 13,14-Dihydro-15-keto-20-ethyl-$PGE_2$ ethyl ester (87), R = Et:

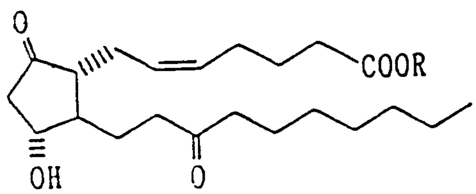

(87)

R =-Et

The same procedure as in Examples 24 to 28 was followed with using(-)-Corey lactone (1) and dimethyl(2-oxononyl)phosphonate produced with the known method, and thus 13,14-Dihydro-15-keto-20-ethyl-$PGE_2$ ethyl ester (87) was obtained.

The n. m. r. spectrum of 13,14-Dihydro-15-keto-20-ethyl-$PGE_2$-ethyl ester (87), R = Et, is shown in Figure 38.

Mass(DI) m/z: 408, 390, 345.

Example 41 (See the structural formula (87) shown in Example 39)

Preparation of 13,14-Dihydro-15-keto-20-ethyl-$PGE_1$ methyl ester (88):

13,14-Dihydro-15-keto-20-ethyl-$PGE_2$ methyl ester (87), R = Me, obtained in the same way as in Example 39 was hydrogenated with plutinum oxide and hydrogen in ethanol, and thus 13,14-Dihydro-15-keto-20-ethyl-$PGE_1$ methyl ester (88) was prepared.

The n. m. r. spectrum of 13,14-Dihydro-15-keto-20-ethyl-$PGE_1$ methyl ester (88), R = Me, is shown in Figure 39.

Example 42

Preparation of 13,14-Dihydro-15-keto-20-n-propyl-$PGE_2$ methyl ester (89):

The same procedure as in Examples 24 to 30 and 39 was followed using(-)-Corey lactone (1) and dimethyl(2-oxodecyl)phosphonate produced according to the known method, and thus 13,14-Dihydro-15-keto-20-n-propyl-$PGE_2$ methyl ester (89) was synthesized.

The n. m. r. spectrum of 13,14-Dihydro-15-keto-20-n-propyl-$PGE_2$ methyl ester (89) is shown in the Figure 40.

Mass (SIMS) : 409, 391, 369.

Example 43 (See Chart XIV)

Preparation of 13,14-Dihydro-15-keto-20-ethyl-11R-dehydroxy-11R-methyl-$PGE_2$ methyl ester (98):

43 - 1) Tosylation of 1S-2-Oxa-3-oxo-6R-(3,3-ethylenedioxy-1-decyl)-7R-hydroxy-cis-bicyclo (3,3,0)octane (90) :

Preparation of tosylate (91) :

Alcohol (90) (1.723g) was treated with p-toluenesulfonyl chloride (2.893g) in pyridine (5 mℓ) to give the tosylate(91). Yield : 1.812 g (74%).

43 - 2) Preparation of 1S-2-Oxa-3-oxo-6S-(3,3-ethylenedioxy-1-decyl)-cis-bicyclo(3,3,0)oct-7-ene (92) :

DBU (5.6 mℓ) was added to a toluene solution (1.9 mℓ) of the tosylate (91) (1.812g), and the reaction solution was kept at 60 °C for 7 h. A crude product obtained after the usual work-up was chromatographed (hexane-ethyl acetate = 3 : 1) to give the olefin (92).

Yield : 0.7594g (63%).

43 - 3) DIBAL-H reduction of 1S-2-Oxa-3-oxo-6S-(3,3-ethylenedioxy-1-decyl)-cis-bicyclo(3,3,0)-oct-7-ene (92):

Preparation of lactol (93):

The olefin (92) (0.7594g) was treated with DIBAL - H (1.5 - M; 6.2 mℓ) to produce the lactol (93).

43 - 4) Preparation of methyl 15,15-Ethylenedioxy-20-ethyl-9S-hydroxy-cis-$\Delta^5$-$\Delta^{10}$-prostanoate (95):

The lactol (93) was added to a ylide generated from (4-carboxybutyl)triphenylphosphonium bromide and sodium methylsulfinyl carbanion, in DMSO, whereby prostanoic acid (94) was obtained. The acid (94) was esterified with diazomethane, and thus methyl 20-ethyl-prostanoate (95) was obtained.

Yield : 0.6600g (67%).

43 - 5) Preparation of 15,15-Ethylenedioxy-20-ethyl-13,14-dihydro-$PGA_2$ methyl ester (96):

The methyl 20-ethyl-prostanoate (95) (0.6600g) was oxidized with Jones reagent in acetone (400 mℓ) at - 20 °C. The crude material obtained after the usual work-up was chromatographed (hexane-ethyl acetate = 3 : 1) to give 15,15-ethylenedioxy-20-ethyl-13,14-dihydro-$PGA_2$ methyl ester (96).

Yield : 0.6182g (99%).

43 - 6) Preparation of 15,15-Ethylenedioxy-20-ethyl-13,14-dihydro-11R-dehydroxy-11R-methyl $PGE_2$ methyl ester (97) :

The enone (96) (0.6100g) was treated with lithium dimethylcuprate obtained from copper (I) iodide (0.8380g) and methyl lithium (1.5 - M; 5.8 mℓ), in ether (15 mℓ), and there was obtained 15,15-ethylenedioxy-20-ethyl-13,14-dehydroxy-11R-dihydroxy-11R-methyl $PGE_2$ methyl ester (97).

Yield : 0.5720g (94%).

43 - 7) Preparation of 13,14-Dihydro-15-keto-20-ethyl-11R-dehydroxy-11R-methyl-$PGE_2$ methyl ester (98):

15,15-Ethylenedioxy-20-ethyl-13,14-dihydro-11R-dehydroxy-11R-methyl-$PGE_2$ methyl ester (97) (0.2300 g) was dissolved in 25 mℓ of a mixed solvent of acetic acid : water : THF (3 : 1 : 1), and the solution was kept at 50 ° C for 2 h. A crude product obtained after the usual work-up was chromatographed to give 13,14-dihydro-15-keto-20-ethyl-11R-dehydroxy-11R-methyl-$PGE_2$ methyl ester (98).

Yield : 0.200g.

The n. m. r. spectrum of 13,14-dihydro-15-keto-20-ethyl-11R-dehydroxy-11R-methyl-$PGE_2$ methyl ester (98) is shown in Figure 41.

Mass (DI) m / z : 392, 374, 361, 343.

Example 44

Preparation of 13,14-Dihydro-15-keto-11R-dehydroxy-11R-methyl-$PGE_2$ ethyl ester (99):

The same procedure as in Example 43 was followed with using (-)-Corey lactone (1), dimethyl(2-oxoheptyl)phosphonate, and (4-carboxybutyl)triphenylphosphonium bromide, and 13,14-dihydro-15-keto-11R-dehydroxy-11R-methyl-$PGE_2$ethyl ester (99) was produced.

The n. m. r. spectrum of 13,14-dihydro-15-keto-11R-dehydroxy-11R-methyl-$PGE_2$ ethyl ester (99) is shown in Figure 42.

Mass (SIMS) m / z : 387, 360, 333, 315.

Example 45 (See Chart XV)

Preparation of 13,14-Dihydro-15-keto-20-isopropylidene-$PGE_2$ methyl ester (103):

1S-2-Oxa-3-oxo-6R-(8-isopropylidene-3-keto-1-trans-octenyl)-7R-(4-phenylbenzoyl)oxy-cis-bicyclo(3, 3, 0)-octane (100), a compound produced from(-)-Corey lactone (1) and dimethyl (2-oxo-7-isopropylideneheptyl)phosphonate, was converted to the corresponding silylenolether (101) with dimethylphenyl silane (0.9 mℓ) and Wilkinson catalyst (50 mg) in THF (40 mℓ). The silylenolether (101) was ketalized in benzene in the usual manner, and thus there was obtained 1S-2-oxa-3-oxo-6R-(8-isopropylidene-3,3-ethylenedioxy-1-octyl)-7R-(4-phenylbenzoyl)oxy-cis-bicyclo(3, 3, 0) octane (102).

Yield: 2.32g (82%).

Subsequently, the same procedure as in Examples 24 to 30, 40, 41, and 42 was followed to produce 13,14-dihydro-15-keto-20-isopropylidene-$PGE_2$ methyl ester (103).

The n. m. r. spectrum of 13,14-dihydro-15-keto-20-isopropylidene-$PGE_2$ methyl ester (103) is shown in Figure 43.

Example 46 (See Chart XVI)

Preparation of 13,14-Dihydro-6,15-diketo-$PGE_1$ n-butyl ester (107), R = n - Bu:

46 - 1) Preparation of the bromo-ether (104) :

Bromo-ether formation from 15,15-ethylenedioxy-13,14-dihidro-11-(2-tetrahydropyranyl)oxy-$PGF_{2\alpha}$ n-butyl ester (10):

The butyl ester (10) (1.165g) was dissolved in a THF-dichloromethane mixture (3 mℓ + 30 mℓ), and the solution was ice-cooled. After addition of N-bromosuccinimide (0.405g), the solution was stirred for 1 h. The reaction solution was poured in aqueous dilute sodium sulfite, and extracted with dichloromethane. The extract was dried, then concentrated under reduced pressure. The resulting crude product was chromatographed and thus bromo-ether (104) was obtained.

46 - 2) Preparation of 15,15-Ethylenedioxy-13,14-dihydro-6-keto-11-(2-tetrahydropyranyl)oxy-$PGF_{2\alpha}$ n-butyl ester (105):

Bromo-ether (104) (1.057g) was dissolved in dry toluene (6 mℓ) and DBU (2.6 mℓ), and then agitated at 55 ° C for 18 h. After being diluted with ethyl acetate, the mixture was adjusted to pH 3. Then, the organic layer of the solution was processed in the usual way.

Yield : 0.7132g (75%).

46 - 3) Preparation of 15,15-Ethylenedioxy-13,14-dihydro-6-keto-11-(2-tetrahydropyranyl)oxy-$PGE_2$ n-butyl ester (106):

In acetone (40 mℓ), 6-keto-$PGF_{2\alpha}$ (105) (0.7132g) was oxidized at -40 ° C with Jones reagent, whereby 13,14-dihydro-15,15-ethylenedioxy-6-keto-11-(2-tetrahydropyranyl)oxy-$PGE_2$ n-butyl ester (106) (0.4404g) was obtained.

Yield : 0.4404g (62%).

46 - 4) Preparation of 13,14-Dihydro-6,15-diketo-$PGE_2$ n-butyl ester (107):

13, 14-Dihydro-15,15-ethylenedioxy-6-keto-11-(2-tetrahydropyranyloxy)-$PGE_2$ n-butyl ester (106) (0.4404g) was kept at 55 ° C in a mixed solvent of acetic acid : THF : water (3 : 1 : 1) for 3.5 h, whereby there was obtained 13,14-dihydro-6,15-diketo-$PGE_2$ n-butyl ester (107).

Yield : 0.200g (59%).

The n. m. r. spectrum of 13,14-dihydro-6,15-diketo-$PGE_2$ n-butyl ester (107) is shown in the Figure 44.

## Example 47

### Preparation of 13,14-Dihydro-6,15-diketo-20-methyl-$PGE_2$ ethyl ester (108):

The procedure of Example 46 was repeated with using(-)-Corey lactone (1) and dimethyl(2-oxooctyl)-phosphonate, and thus 13,14-dihydro-6,15-diketo-20-methyl-$PGE_2$ ethyl ester (108) was obtained.

The n. m. r. spectrum of 13,14-dihydro-6,15-diketo-20-methyl-$PGE_2$ethyl ester (108) is shown in Figure 45.

## Example 48 (See Chart XVII)

### Preparation of 13,14-Dihydro-6,15-diketo-11R-dehydroxy-11 R-methyl $PGE_1$ ethyl ester (115), R = Et:

48 - 1) Preparation of 15,15-Ethylenedioxy-13,14-dihydro-11R-dehydroxy-11R-methyl-$PGF_{2\alpha}$ ethyl ester (110) :

15,15-Ethylenedioxy-13,14-dihydro-11R-dehydroxy-11R-methyl $PGE_2$ ethyl ester (109) (1.775 g), the compound obtained in the same way as in Example 43, was dissolved in a THF-methanol mixed solvent, and 0.1600g of $NaBH_4$ was added. The solution was kept at - 18 ° C overnight. A crude product obtained after the usual work-up was chromatographed (hexane-ethyl acetate = 3.5 : 1) to give.

9α-hydroxy substance (110) : 0.9464g;

9β-hydroxy substance (111) : 0.5867g.

The 9β-hydroxy substance (111) was oxidized with Jones reagent whereby 15,15-ethylenedioxy-13,14-dihydro-11R-dehydroxy-11R-methyl $PGE_2$ ethyl ester (109) was recovered, which was again reduced with $NaBH_4$. These reaction were repeated to amount to 1.446g of 13,14-dihydro-15,15-ethylenedioxy-11R-dehydroxy-11 R-methyl-$PGF_{2\alpha}$ ethyl ester (110).

48 - 2) Preparation of Bromo-ether (112) :

13,14-Dihydro-15,15-ethylenedioxy-11R-dehydroxy-11R-methyl-$PGF_{2\alpha}$ ethyl ester (110) (1.446g) was dissolved in a mixed solvent of THF (12 mℓ) and dichloromethane (3.5 mℓ), and NBS (0.6453g) was added at -18 ° C. Following the usual procedure, there was obtained 1.932g of bromo-ether (112).

48 - 3) Preparation of 15,15-Ethylenedioxy-13,14-dihydro-11R-dehydroxy-6-keto-11R-methyl-$PGF_{2\alpha}$ ethyl ester (113) :

The bromo-ether (112) (1.932g) was dissolved in DBU (6 mℓ) and toluene (3 mℓ), and the solution was kept at 75 °C. A crude product obtained after the usual work-up was chromatographed (hexane-ethyl acetate = 3 : 1) to give the title compound (113).

Yield : 1.230g.

48 - 4) Preparation of 15,15-Ethylenedioxy-13,14-dihydro-11R-dehydroxy-6-keto-11R-methyl-$PGE_1$ ethyl ester (114) :

6-Keto-11R-methyl-$PGF_{2\alpha}$ ethyl ester (113) (1.230g) was oxidized with Jones reagent in acetone, whereby 15,15-ethylenedioxy-13,14-dihydro-11R-dehydroxy-6-keto-11R-methyl-$PGE_1$ ethyl ester (114) was obtained.

Yield : 0.7614g (62%).

48 - 5) Preparation of 13,14-Dihydro-6,15-diketo-11R-dehydroxy-11R-methyl-$PGE_1$ ethyl ester (115) :

15,15-Ethylenedioxy-13,14-dihydro-11R-dehydroxy-11R-methyl-$PGE_1$ ethyl ester (114) (0.7614g) was dissolved in a mixed solvent of acetic acid : THF : water (3 : 1 : 1), and the solution was kept at 50 °C for 5. Following the usual procedure, there was obtained 0.6290g of 13,14-dihydro-6,15-diketo-11R-dehydroxy-11R-methyl-$PGE_1$ ethyl ester (115), R = Et.

The n. m. r. spectrum of 13,14-dihydro-6,15-diketo-11R-dehydroxy-11R-methyl-$PGE_1$ ethyl ester (115) is shown in Figure 46.

Mass (SIMS) : 395 $(M + 1)^+$, 377, 349.

Example 49 (See Chart XVII)

Preparation of 13,14-Dihydro-6,15-diketo-11R-dehydroxy-11R-methyl-$PGE_1$ methyl ester (115), R = Me:

The same procedure as in Example 48 was followed except that diazomethane was used for methyl-esterification, and thus there was obtained 13,14-dihydro-6,15-diketo-11R-dehydroxy-11R-methyl-$PGE_1$ methyl ester (115), R = Me.

The n. m. r. spectrum of 13,14-dihydro-6,15-diketo-11R-dehydroxy-11R-methyl-$PGE_1$ methyl ester (115), R = Me, is shown in Figure 47.

Mass (DI): 380, 362, 349, 331.

Example 50 (See Chart XVIII)

Preparation of 13,14-Dihydro-15-keto-16R,S-fluoro-$PGE_2$ methyl ester (125), R = Me:

50 - 1) Preparation of 1S-2-Oxa-3-oxo-6R-(4-fluoro-3-keto-1-octyl-7R-hydroxy-cis-bicyclo(3, 3, 0)octane (118) :

A saturated ketone (117) (5.20g) obtained after catalytic hydrogenation of the unsaturated ketone (116) produced from(-)-Corey lactone (1) and dimethyl(3-fluoro-2-oxoheptyl)phosphonate was dissolved in a mixed solvent (18 mℓ) of THF and methanol (3 : 1), and potassium carbonate (1.54g) was added. The solution was stirred for 3 h.

The crude product obtained after the usual work-up was chromatographed (hexane : ethyl acetate = 1 : 1) to yield an alcohol (118).

Yield : 1.81g (57%).

50 - 2) Preparation of 1S-2-Oxa-3-oxo-6R-(4R,S-fluoro-3-oxo-1-octyl)-7R-(2-tetrahydropyranyl)oxy-cis-bicyclo-(3, 3, 0) octane (119):

The alcohol (118) (1.81g) was converted to the corresponding tetrahydropyranyl ether (119) with dihydrapyran and p-toluenesulfonic acid in dichloromethane.

Yield : 2.33g.

50 - 3) Preparation of 1S-2-Oxa-3-oxo-6R-(4R,S-fluoro-3R,S-hydroxy-1-octyl)-7R-(2-tetrahydropyranyl) oxy-cis-bicyclo-(3, 3, 0)octane (120):

The tetrahydropyranyl ether (119) (2.33g) was reduced with $NaBH_4$ in methanol. Alcoholic-lactone (120) was thus obtained.

50 - 4) Preparation of Lactol (121):

The alcoholic-lactone (120) (0.84g) was reduced with DIBAL-H (1.5 - M, 6 mℓ) in toluene (20 mℓ) to the corresponding lactol (121).

50 - 5) Preparation of 16R,S-fluoro-13,14-dihydro-15R,S-hydroxy-11R-(2-tetrahydropyranyl)oxy-$PGF_{2\alpha}$ methyl ester (123), R = Me :

Ylide produced from (4-carboxybutyl)triphenyl~ phosphonium bromide (3.50g) in the ordinary method was let to react with the previously synthesized lactol (121) in DMSO. A carboxylic acid (122) obtained according to the ordinary procedure was treated with diazomethane. Methyl ester 9123) was thus obtained.

Yield : 0.470g (44%).

50 - 6) Preparation of 13,14-Dihydro-15-keto-16R,S-fluoro-$PGE_2$ methyl ester (125), R = Me :

The methyl ester (123) (0.470) was oxidized with Jones reagent in acetone (25 mℓ) at -30°C. After the usual work-up, the crude product was charomatographed (hexane : ethyl acetate = 5 : 2) to yield 0.298g of 13,14-dihydro-15-keto-16R,S-fluoro-11R-(2-tetrahydropyranyl)oxy-$PGE_2$ methyl ester (124).

The methyl ester (124) (0.298g) was dissolved in a mixed solvent (25 mℓ) of acetic acid, THF, and water (4 : 1 : 2), and the solution was kept at 45°C for 3 h. Then, a crude product obtained after the usual work-up was chromatographed (benzene - ethyl acetate = 2 : 3) to give 13,14-dihydro-15-keto-16R,S-fluoro-$PGE_2$ methyl ester (125), R = Me.

Yield : 0.202g.

The n. m. r. spectrum of 13,14-dihydro-15-keto-16R,S-fluoro-$PGE_2$methyl ester (125) is shown in Figure 48.

Mass (DI) 384, 366, 346, 335

Example 51 (See Chart XIX)

Preparation of 13,14-Dihydro-6,15-diketo-16R,S-fluoro-11R-dehydroxy-11R-methyl-$PGE_1$ ethyl ester (135):

51 - 1) Tosylation of 16R,S-Fluoro-13,14-dihydro-15R,S-(t-butyldimethylsilyl)oxy-$PGF_{2\alpha}$ ethyle ster (126) :

Preparation of tosylate (127) : 16R,S-Fluoro-13,14-dihydro-15R,S-(t-butyldimethylsilyl)oxy-$PGF_{2\alpha}$ ethyl ester (126) (1.00 g) produced from(-)-Corey lactone (1) and dimethyl(3-fluoro-2-oxoheptyl)phosphonate according to the known method was tosylated with tosyl chloride (4.00g) in pyridine (10 mℓ) at 0°C.

Yield : 1.04g.

51 - 2) Preparation of 16R,S-fluoro-13,14-dihydro-15R,S-(t-butyldimethylsilyl)oxy-$PGA_2$ ethyl ester (128) :

The tosylate (127) (1.04g) was oxidized with Jones reagent (2.67- M, 2 mℓ) in acetone (30 mℓ) at -20°C. A crude product obtained after usual processing was chromatographed (hexane-ethyl acetate = 5 : 1) to give 16R,S-fluoro-13,14-dihydro-15R,S-(t-butyldimethylsilyl)oxy-$PGA_2$ ethyl ester (128).

Yield : 0.627g.

51 - 3) Preparation of 16R,S-Fluoro-13,14-dihydro-11R-dehydroxy-11R-methyl-15R,S-(t- butyldimethylsilyl)-oxy-$PGE_2$ ethyl ester (129):

To lithium dimethylcuprate, prepared in ether (70 ml) from copper (I) iodide (1.28g) and methyl lithium (1.5-M; 9.0 mℓ) was added an ether solution (40 mℓ) of the enone (128) (1.114 g). The mixture was stirred for 30 min. Then, after usual processing, there was obtained 16R,S-fluoro-13,14-dihydro-11R-dehydroxy-11R-methyl-15R,S-t-butyldimethylsilyl)oxy-$PGE_2$ ethyl ester (129).

Yield: 0.931g.

51 - 4) Preparation of 16R,S-Fluoro-13,14-dihydro-11R-dehydroxy-11R-methyl-15R,S-(t-butyl dimethylsilyl)-oxy-$PGF_{2\alpha}$ ethyl ester (130):

The ketone (129) (0.931g) was reduced with $NaBH_4$ (0.688g) in methanol (40 mℓ), and thus 9α-hydroxy-PGF derivative (130) and 9β-hydroxy-PGF derivative (131) were obtained.

The 9β-hydroxy-PGF derivative (131) was oxidized by Jones reagent to the ketone (129), and then reduction of the ketone (129) with $NaBH_4$ was carried out again. A total yield of 0.677g of 16R,S-fluoro-13,14-dihydro-11R-dehydroxy-11R-methyl-15R,S-(t-butyldimethylsilyl)oxy-$PFG_{2\alpha}$ ethyl ester (130) was obtained.

51 - 5) Preparation of 16R,S-Fluoro-13,14-dihydro-11R-dehydroxy-15R,S-hydroxy-11R-methyl-$PGF_{2\alpha}$ ethyl ester (132):

Tetrabutylammonium fluoride (1.0 - M ; 8 mℓ) was added to a THF solution of 15R,S-(t-butyldimethylsilyl)oxy-$PGF_{2\alpha}$ ethyl ester (130) (0.677g), and the mixture was stirred at room temperature overnight. A crude product obtained after the usual processing was chromatographed (hexane-ethyl acetate = 3 : 1) to give 16R,S-fluoro-13,14-dihydro-11R-dehydroxy-15R,S-hydroxy-11R-methyl-$PGF_{2\alpha}$ ethyl ester (132) (0.503g).

51 - 6) Preparation of 13,14-Dihydro-6,15-diketo-16R,S-fluoro-11R-dehydroxy-11R-methyl-$PGE_1$ ethyl ester (135) :

The same procedure as in Examples 48 and 49 was followed with using 16R,S-fluoro-13,14-dihydro-11R-dehydroxy-15R,S- hydroxy-11R-methyl-$PGF_{2\alpha}$ ethyl ester (132), and thus there was obtained 13,14-dihydro-6,15-diketo-16R,S-fluoro-11R-dehydroxy-11R-methyl-$PGE_1$ ethyl ester (135).

The n. m. r. spectrum of 13,14-dihydro-6,15-diketo-16R,S-fluoro-11R-dehydroxy-11R-methyl-$PGE_1$ ethyl ester (135) is shown in Figure 49.

Mass (DI) : 412, 394, 367.

<u>Example 52</u> (See Chart XX)

<u>Preparation of 13,14-Dihydro-6,15-diketo-11R-dehydroxy-11R-hydroxymethyl-19-methyl-$PGE_1$ methyl ester (138):</u>

52 - 1) Preparation of 15,15-Ethylenedioxy-13,14-dihidro-11R-dehydroxy-11R-hydroxymethyl-$PGE_2$ methyl ester (137):

15,15-Ethylenedioxy-13,14-dihydro-19-methyl-$PGA_2$ methyl ester (136) (0.410 g) produced from (-)-Corey lactone (1) and dimethyl(6-methyl-2-oxoheptyl)phosphonate, and 0.255g of benzophenone were dissolved in 80 mℓ of methanol. The solution was irradiated through a pyrex filter with a 300 W high pressure mercury lamp. After the ordinary work-up and purification, there was obtained 15,15-ethylene dioxy-13,14-dihidro-11R-dehydroxy-11R-hydroxymethyl-19-methyl-$PGE_2$ methyl ester (137).

52 - 2) Preparation of 13,14-Dihydro-6,15-diketo-11R-dehydroxy-11R-hydroxymethyl-19-methyl-$PGE_1$ methyl ester (138):

The same procedure as in Examples 47, 48, and 49 was applied on the compound (137), and thus 13,14-dihydro-6,15-diketo-11R-dehydroxy-11R-hydroxymethyl-19-methyl-$PGE_1$ methyl ester (138) was obtained.

In Figure 51 there is shown the n. m. r. spectrum of 13,14-dihydro-6,15-diketo-11R-dehydroxy-11R-hydroxymethyl-19-methyl-$PGE_1$ methyl ester (138) is shown in Figure 50

Mass m/z 410 ($M^+$), 392($M^+$ - 18), 379, 361.

<u>Example 53</u> (See Chart XXI)

<u>Preparation of 13,14-Dihydro-15-keto-16R,S-fluoro-$PGE_2$ (140):</u>

53-1) Preparation of 13,14-Dihydro-15-keto-16R,S-fluoro-11R-(2-tetrahydropyranyl)oxy-$PGE_2$ (139):

The carboxylic acid (122) was oxidized in aceton (25 ml) with Jones reagent (2.67-M, 1.1 ml) at -15 °C. A crude product obtained after the usual work-up was chromatographed to give 13,14-dihydro-15-keto-16R,S-fluoro-11R-(2-tetrahydropyranyl)oxy-$PGE_2$ (139). Yield: 0.247 g.

53-2) Preparation of 13,14-Dihydro-15-keto-16R,S-fluoro-$PGE_2$ (140):

13,14-Dihydro-15-keto-16R,S-fluoro-11R-(2-tetrahydropyranyl)oxy-$PGE_2$ (139) (0.247 g) was dissolved in a mixture (25 ml) of acetic acid - water - THF (4 : 2 : 1) to be kept at 45 °C for 3 h. A crude product obtained after the usual work-up was chromatographed to give 13,14-dihydro-15-keto 16R,S-fluoro-$PGE_2$ - (140). Yield: 0.148 g.

The n. m. r. spectrum of 13,14-dihydro-15-keto-16R,S-fluoro-$PGE_2$ (140) is shown. in Figure 51.

Mass 352 ($M^+$ -18) 282, 281, 226.

Example 54

Preparation of 13,14-Dihydro-15-keto-20-methyl-$PGE_1$ methyl ester (141):

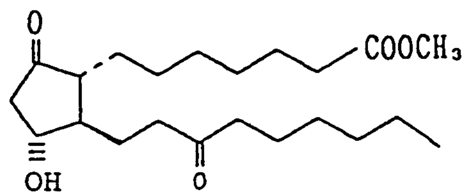

(141)

13,14-Dihydro-15-keto-20-methyl-$PGE_1$ methyl ester (141) was prepared using (-)-Corey lactone together with dimethyl(2-oxooctyl)phosphonate according to the procedure as in Example 41.

The n.m.r. spectrum of the titled compound (141) was shown in Fig. 52.

Mass (DI) m/z 382($M^+$), 364, 333.

Example 55 (See Chart XXII)

Preparation of 13,14-Dihydro-15-keto-$\Delta^2$-$PGE_1$ methyl ester (146):

According to the same manner as in Example 36 13,14-dihydro-15-keto-$\Delta^2$-$PGE_1$ methyl ester (146) was prepared using 13,14-dihydro-15,15-ethylenedioxy-11-(2-tetrahydropyranyl)oxy-$PGF_{1\alpha}$ methyl ester (142) which can be obtained by catalitic hydrogenation of the compound (10).

The n.m.r. spectrum of the titled compound (146) is shown in Figure 53.

Mass (DI) z/m 366, 348, 316.

Example 56 (See Chart XXII)

Preparation of 13,14-Dihydro-15-keto-$\Delta^2$-$PGE_1$ (149):

56-1 Preparation of 13,14-Dihydro-15,15-ethylenedioxy-11-(2-tetrahydropyranyl)oxy-$\Delta^2$-$PGF_{1\alpha}$ (147):

To the solution of 13,14-dihydro-15,15-ethylenedioxy-11-(2-tetrahydropyranyl)oxy-$\Delta^2$-$PGF_{1\alpha}$ methyl ester (144) (0.7687 g) in THF (15 ml) 0.5-M aqueous solution of litium hydroxide (20 ml) was added, and stirred at room temperature over night. A crude carboxylic acid (147) was obtained after a usual work-up. Yield: 0.8779 g.

56-2 Preparation of 13,14-Dihydro-15,15-ethylenedioxy-11-(2-tetrahydropyranyl)oxy-$\Delta^2$-$PGE_{1\alpha}$ (148):

Carboxylic acid (147) (0.8779 g) was oxidized with Jones reagent (2.67-M, 1.7 ml) at -35 °C in acetone

(50 ml). A crude product obtained after a usual work-up was chromatographed (3 - 5% isopropanol-hexane) to give 13,14-dihydro-15,15-ethylenedioxy-11-(2-tetrahydropyranyl)oxy-$\Delta^2$-$PGE_1$ (148). Yield: 0.5972 g.

56-3 Preparation of 13,14-Dihydro-15-keto-$\Delta^2$-$PGE_1$ (149):

In a mixed solvent of acetic acid:THF:water (3:1:1) (15 ml) $\Delta^2$-$PGE_1$ (148) (0.5972 g) was dissolved and maintained at 40 °C for 3.5 hours. A crude compound obtained by a usual work-up was chromatographed twice (acid washed Mallincklodt silica-gel, hexane:ethyl acetate = 3:1 - 1:1, and then 8% isopropanol-hexane) to give 13,14-dihydro-15-keto-$\Delta^2$-$PGE_1$ (149). Yield: 0.2473.

The n.m.r. of the titled compound (149) was shown in Figure 54.

Mass (DI) z/m 352($M^+$), 334, 316.

Example 57

Preparation of 13,14-Dihydro-15-keto-16R,S-fluoro-20-methyl-$PGE_2$ methyl ester (150):

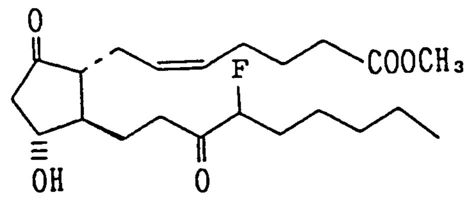

(150)

Using (-)-Corey lactone and dimethyl(3R,S-fluoro-2-oxooctyl)phosphonate, 13,14-dihydro-15-keto-16R,S-fluoro-20-methyl-$PGE_2$ methyl ester (150) was prepared according to the same manner as in Example 50.

The n.m.r. spectrum of the titled compound (150) was shown in Figure 55.

Mass (DI) m/z 398($M^+$), 380.

Example 58 (See Chart XXIII)

Preparation of 13,14-Dihydro-15-keto-16,16-difluoro-$PGE_2$ methyl ester (160):

58-1 Preparation of 1S-2-Oxa-3-oxo-6R-(4,4-difluoro-3-oxo-trans-1-octenyl)-7R-(4-phenylbenzoyl)oxy-cis-bicyclo(3,3,0)octane (151):

Aldehyde (2) was obtained by the oxidation of (-)-Corey lactone (1) (6.33 g) with Collins reagent. Separately thallium ethoxide (4.26 g) was dissolved in benzene, to which the solution of dimethyl(3,3-difluoro-2-oxoheptyl)phosphonate (4.64 g) in benzene was added at cool temperature, and the mixture was stirred for 30 min. To the resultant the solution of the aldehyde (2) in benzene as prepared above was added, and stirred at room temperature for 3 h. After the mixture was neutralized with acetic acid, a saturated aqueous solution of potassium iodide was added and passed through a celite column. After a usual work-up the desired unsaturated ketone (151) was obtained. Yield: 3.88 g.

58-2 Preparation of 1S-2-Oxa-3-oxo-6R-(4,4-difluoro-3R,S-hydroxy-1-octyl)-7R-(4-phenylbenzoyl)oxy-cis-bicyclo(3,3,0)octane (153):

The unsaturated ketone (151) (3.88 g) was hydrogenated with palladium on carbon (5%) in ethyl acetate (40 ml) to give the saturated ketone (152). The saturated ketone (152) was reduced with $NaBH_4$ in a mixed solvent of methanol-THF (70:30) to give the alcohol (153). Yield: 4.02 g.

58-3 Preparation of 1S-2-Oxa-3-oxo-6R-(4,4-difluoro-15R,S-t-butyldimethylsilyloxy-1-octyl)-7R-hydroxy-cis-bicyclo(3,3,0)octane (155):

The alcohol (153) was treated with imidazol and t-butyldimethylsilyl chloride in DMF to give 1S-2-oxa-3-oxo-6R-(4,4-difluoro-15R,S-t-butyldimethylsilyloxy-1-octyl)-7R-(4-phenylbenzoyl)oxy-cis-bicyclo(3,3,0) octane

(154). The resultant (154) was methonolysised with potassium carbonate (1.14 g) in methanol (20 ml) to give 1S-2-oxa-3-oxo-6R-(4,4-difluoro-15R,S-t-butyldimethylsilyloxy-1-octyl)-7R-hydroxy-cis-bicyclo(3,3,0)octane (155). Yield: 2.89 g.

58-4 Preparation of 1S-2-Oxa-3-oxo-6R-(4,4-difluoro-15R,S-t-butyldimethylsilyloxy-1-octyl)-7R-(2-tetrahydropyranyl)oxy-cis-bicyclo(3,3,0)octane (156):

The alcohol (155) was converted to the tetrahydropyranyl ether (156) according to a known method. Yield: 3.38 g.

58-5 Preparation of 16,16-difluoro-13,14-dihydro-15R,S-t-butyldimethylsilyloxy-11-(2-tetrahydropyranyl)oxy-$PGF_{2\alpha}$ methyl ester (157):

The desired silylether (157) was obtained from the tetrahydropyranyl ether (156) (3.38 g) according to the procedure in Examples 50 and 51. Yield: 3.02 g.

58-6 Preparation of 16,16-Difluoro-13,14-dihydro-15R,S-hydroxy-11R-(2-tetrahydropyranyl)oxy-$PFG_{2\alpha}$ methyl ester (158):

The silyl ether (157) (0.882 g) was treated with tetrabutylammonium fluoride (1.1-M, 10.6 ml) in THF (25 ml) to give the desired diol (158). Yield: 0.710 g.

58-7 Preparation of 13,14-Dihydro-15-keto-16,16-difluoro-$PGE_2$ methyl ester (160):

Collins reagent was prepared from chromic anhydride (2.57 g) and pyridine (4.15 ml) in dichloromethane (40 ml). To the resultant was added the solution of the diol (158) (0.360 g) in dichloromethane (15 ml). After the usual work-up and purification, 13,14-dihydro-15-keto-16,16-difluoro-11-(2-tetrahydropyranyl)oxy-$PGE_2$ methyl ester (159) was obtained. Yield: 0.277 g. The obtained compound (159) (0.208 g) was dissolved in a mixed solvent of acetic acid : THF:water (4:2:1) (30 ml) and maintained at 45 ° C for 3.5 h. A crude compound obtained after a usual work-up was chromatographed to give 13,14-dihydro-15-keto-16,16-difluoro-$PGE_2$ methyl ester (160). Yield: 0.208 g.

The n.m.r. spectrum of the titled compound (160) is shown in Fig 56.

Mass (DI) z/m 402($M^+$), 384($M^+$ - 18), 364.

Example 59 (See Charts XXIV and XXV)

Preparation of 13,14-dihydro-15-keto-5,6-dehydro-20-methoxy-$PGE_2$ methyl ester (171):

To a solution of 8-methoxy-3,3-ethylenedioxy-1-iodooctane (167) (0.985 g) in ether (15 ml) t-butyllitium (2.3-M, 2.87 ml) was added dropwise at -78 ° C, and the resultant mixture was stirred for 3 h, to which an ether solution of copper (I) iodide and tributylphosphine was added all at once, and stirred for 20 min. To the reaction mixture was added a solution of 4R-t-butyldimethylsilyloxy-2-cyclopentene-1-on (168) (0.637 g) in THF (21 ml) dropwise over 15 min. After 15 min HMPA (2.61 ml) was added to the resultant followed by the addition of triphenyltin chloride (1.217 g) in THF (6 ml) after 30 min, and then stirred for 15 min. The reaction mixture was cooled at -30 ° C, to which a solution of 6-carboxymethoxy-1-iodo-2-hexyne (169) (3.19 g) in HPMA (2.61 ml) was added, and stirred for 4.5 h and then at room temperature for 12 h. The reaction mixture was poured into a saturated ammonium chloride solution with vigorous agitation. The organic layer was collected. The aqueous layer was extracted with ether, and the extracted layer was put together with the organic layer, which was then washed with a saturated aqueous solution of sodium chloride. After dried the organic layer was concentrated under reduced pressure to give a crude product. The crude product was chromatographed to give 11-t-butyldimethylsilyloxy-15,15-ethylenedioxy-13,14-dihydro-5,6-dihydro-20-methoxy-$PGE_2$ methyl ester (170). Yield: 0.3700 g.

n.m.r.: 0.08(3H,s), 0.10(3H,s), 1.3 - 2.8(24H,m), 3.30 (3H,s), 3.32(2H,t), 3.74(3H,s), 3.90(4H,s), 4.10-(1H,m).

59-2 Preparation of 13,14-dihydro-15-keto-5,6-dehydro-20-methoxy-$PGE_2$ methyl ester (171):

A mixture (3ml) of hydrofluoric acid (46 %) : acetonitrile (1 : 2) cooled at 0 °C was added to 11-t-butyldimethylsilyloxy-15,15-ethylenedioxy-13,14-dihydro-5,6-dehydro-20-methoxy-$PGE_2$ methyl ester (170) (0.035 g), and stirred at room temperature for 25 min, to which water was poured, and the reaction product was extracted with ethyl acetate. The obtained organic layer was neutralized with a saturated aqueous solution of sodium bicarbonate, and concentrated under reduced pressure to give a crude product, which was chromatographed to give 13,14-dihydro-15-keto-5,6-dehydro-20-methoxy-$PGE_2$ methylester (171). Yield: 0.0081 g.

The n.m.r. spectrum of the obtained compound (171) was shown in Figure 57.

The n.m.r. data of compounds in the above Examples are shown as follows, wherein the compounds number in brackets.

(6) δ: 0.88 (3H, 6Hz), 1.1 - 3.0(19H, m), 3.8 - 4.1(1H, m), 3.90(4H, s), 4.93(1H, dt, J = 6Hz, J = 3Hz)

(7) 0.88(3H, 6Hz), 1.0 - 2.9(24H, m), 350(1H, m), 3.88(4H, s), 3.6 - 4.1(2H, m), 4.63(1H, bs), 4.8 - 5.06-(1H, m)

(11) 0.88(3H, t, J = 6Hz), 1.24(3H, t, J = 7.5Hz), 1.0 - 2.7(30H, m), 3.3 - 3.6(1H, m), 3.89(4H, s), 3.6 - 4.35(5H, m), 4.10(2H, q, 8.75Hz), 4.35 - 4.7(1H, m)

(23) 0.7 - 1.0(6H, m), 1.0 - 3.0(18H, m), 3.8 - 4.1(1H), 3.90(4H, s), 4.92(1H, dt, J = 6Hz, J = 3Hz)

(30) 0.73 - 1.0(6H, m), 1.24(3H, t, J = 7Hz), 1.0 - 2.5(29H, m), 3.3 - 4.7(7H, m), 3.88(4H, s), 4.11(2H, q, J = 7Hz)

(38) 0.88(3H, t, J = 6Hz), 1.1 - 3.6(16H, m), 4.43(0.5H, t, J = 6Hz), 4.9 - 5.3(2.5H, m), 7.3 - 8.2(9H, m)

(39) 0.90(3H, t, J = 6Hz), 1.1 - 3.2(17H, m), 3.3 - 3.8(1H, m), 3.8 - 4.15(0.5H, m), 4.33 - 4.75(0.5H, m), 4.9 - 5.16(1H, bs), 5.16 - 5.33 (1H, m), 7.3 - 8.2(9H, m)

(40) 0.07(6H, S), 0.87(9H, S), 0.7 - 1.05(3H), 1.05 - 3.2(16H, m), 3.5 - 3.85(1H, m), 3.85 - 4.15(0.5H, m), 4.3 - 4.6(0.5H, m), 4.05 - 5.15(1H, m) 5.15 - 5.33(1H, m), 7.3 - 8.2(9H, m)

(41) 0.07(6H, S), 0.88(9H, S), 0.75 - 1.05(3H), 1.05 - 3.0(17H, m), 3.45 - 3.85(1H, m), 3.85 - 4.15(1.5H, m), 4.4 - 4.65(0.5H, m), 4.93(1H, dd, J = 6Hz, J = 3Hz)

(42) 0.05(6H,s), 0.88(9H, S), 0.75 - 1.05(3H), 1.05 - 3.0(22H, m), 3.3 - 5.1(7H, m)

(45) 0.07(6H, S), 0.88(9H, S), 0.75 - 1.0(3H), 1.23(3H, t, J = 7Hz), 1.05 - 2.6(29H, m), 3.2 - 4.7(7H, m), 4.07(2H, q, J = 7Hz), 5.1 - 5.65(2H, m)

(46) 0.88(3H, t, J = 6Hz), 1.23(3H, t, J = 7Hz), 1.1 - 2.6(30H, m), 3.3 - 4.2(6H, m), 4.10(2H, q, J = 7Hz), 4.60(1H, bS), 5.1 - 5.7(2H, m)

(47) 0.90(3H, t, J = 6Hz), 1.25(3H, t, J = 6Hz), 1.03 - 2.70(29H, m), 3.25 - 4,70(9H, m), 4.07(2H, q, J = 6Hz)

(52) 0.92(3H, t, J = 6Hz), 1.24(3H, t, J = 6Hz), 1.05 - 2.75(21H, m), 3.3 - 3.8(1H, m), 4.10(2H, q, 6Hz), 4.10(0.5H), 4.4 - 4.7(0.5H, m), 5.67(2H, m), 6.10(1H, dd, J = 6Hz, J = 3Hz), 7.57(1H, dd, J = 6Hz, J = 3Hz)

(92) 0.88(3H, t, J = 6Hz), 1.1 - 1.8(16H, m), 2.2 - 3.0(4H, m), 3.88(4H, s), 5.4 - 5.57(1H, m), 5.80(1H, dd, J = 6Hz, J = 3Hz), 6.02(1H, dd, J = 6Hz, 3Hz)

(95) 0.88(3H, t, J = 6Hz), 1.0 - 2.6(27H, m), 3.62(2H, s), 3.88(4H, S), 4.5 - 4.7(1H, m), 5.1 - 5.6(2H, m), 5.6 - 6.0(2H, m)

(96) 0.87(3H, t, J = 6Hz), 1.1 - 2.7(26H, m), 3.62(3H, S), 3.87(4H, S), 5.15 - 5.60(2H, m), 6.07(1H, dd, J = 6Hz, J = 3Hz), 7.53(1H, dd, J = 6Hz, J = 3Hz)

(97) 0.87(3H, t, J = 6Hz), 1.10(3H, d, J = 5Hz), 1.0 - 2.7(29H, m), 3.62(3H, S), 3.7 - 4.0(4H), 5.1 - 5.6(2H, m)

(104) 0.7 - 1.03(6H, m), 1.03 - 2.6(34H, m), 3.3 - 4.3(6H, m), 3.88(4H, S), 4.08(2H, q, J = 7Hz), 4.60(1H, m)

(112) 0.88(3H, t, J = 6Hz), 0.97(3H, d, J = 6Hz), 1.23(3H, t, J = 7Hz), 1.1 - 2.5(25H, m) 3.90(4H, s), 4.10-(2H, q, J = 7Hz), 3.8 - 4.7(3H, m)

(118) 0.90(3H, t, J = 6Hz), 1.1 - 3.1(17H, m), 3.93(1H, q, J = 6Hz), 4.41(0.5H, t, J = 6Hz), 4.7 - 5.1(1.5H, m)

(127) 0.05(6H, s), 0.88(9H, s), 0.75 - 1.0(3H), 1.23(3H, t, J = 7Hz), 1.05 - 2,4(23H, m), 2.42(3H, s), 4.08-(2H, q, J = 7Hz), 3.9 - 4.7(4H, m), 5.35(2H, m), 7.27(2H, d, J = 9Hz), 7.75(2H, d, J = 9Hz)

(129) 0.05(6H, s), 0.88(9H, s), 0.7 - 1.0(3H), 1.23(3H, t, J = 7Hz), 1.05 - 2.65(20H, m), 3.4 - 3.85(1H, m), 4.07(2H, q, J = 7Hz), 3.85 - 4.15(0.5H), 4.35 - 4.65(0.5H, m) 5.35(2H, m), 6.08(1H, dd, J = 6Hz, J = 3Hz), 7.53-(1H, dd, J = 6Hz, J = 3Hz)

(137) 0.85(6H, d, J = 7Hz), 1.0 - 2.7(25H, m), 3.62(2H, S), 3.5 - 3.75(2H), 3.88(4H, s), 5.1 - 5.6(2H, m)

The above data were determined by n.m.r. measuring apparatus R-90H available from Hitachi Seisakusho.

Test Example 1

Antiulcer activity:

As test samples, we used the PGE as obtained in Examples 2 to 52 as described herein, 13,14-dihydro-15-keto-$PGE_2$ (produced by Funakoshi & Co.) being employed as a control reference.

Each group of test animals used consisted of 8 to 10 male rats of the Crj : Wistar strain, weighing 180 to 230 g. Test animals were fasted for 24 hours before the oral administration of the test samples; in the case of development of confinement-stress induced ulcers through immersion in water, 10 minutes after oral administration of the test specimens, the aminals were confined in a stress cage developed by Univ. of Tokyo, then immersed up to the ensiform process of sternum in water at 23 ° C for 4 hours and sacrificed; in the case of formation of indomethacin-induced ulcers, shortly after the materials were given, animals were given indomethacin orally at a dose of 10 mg/kg and sacrificed after 5 hours.

The stomacks were taken out, followed by fixation with 1 % formalin, and incised along the greater curvature to carry out investigation under an illuminated magnifier for ulceration. The degree and extent of lesion and ulcer were rated based on the ulceration index being classified into the following five numerical categories:

0: normal, with no lesion detected;
1: bleeding or erosion of mucosa;
2: development of less than 5 small ulcers (not greater than 2 mm in diameter);
3: generation of not less than 5 small ulcers or a large ulcer (not less than 2 mm in diameter);
4: generation of not less than 2 large ulcers.

On the basis of the criteria that the rats with the ulceration index of not less than "2", the ulcer inhibition rate ($ED_{50}$) was calculated from ulcer-generation ratio in the control and the ratio of the test specimens.

The results are shown in Table 1 (confinement-stress induced ulcers through immersion in water) and Table 2 (indomethacin-induced ulcers).

Table-1 (Hydrorestraint Stress Ulcer Preventing Effect)

| Material tested | Dosage (mg/kg) | Animal used (no. of heads) | Ulcer index (aver. ± SE) | Inhibition factor (%) | $ED_{50}$ (mg/kg) |
|---|---|---|---|---|---|
| Control | - | 10 | 3.0±0.2 | - | - |
| (1) | 20 | 8 | 2.5±0.3 | 6.3 | >20 |
| (2) | 15 | 8 | 0.5±0.3 | 87.5 | 4.0 |
| | 5 | 8 | 1.4±0.4 | 58.3 | |
| (3) | 10 | 8 | 0.3±0.1 | 100.0 | |
| | 3 | 8 | 1.1±0.3 | 86.1 | 1.5 |
| | 1 | 8 | 2.1±0.4 | 25.0 | |
| (4) | 5 | 8 | 1.6±0.3 | 44.4 | 7.0 |
| | 1 | 8 | 2.1±0.4 | 16.7 | |
| (5) | 5 | 8 | 1.5±0.3 | 44.0 | 6.5 |
| | 1 | 8 | 2.2±0.4 | 14.3 | |
| (6) | 10 | 8 | 1.4±0.2 | 72.2 | 5.5 |
| | 3 | 8 | 2.4±0.3 | 28.0 | |
| (7) | 10 | 8 | 1.1±0.2 | 75.0 | 4.5 |
| | 3 | 8 | 1.9±0.3 | 37.5 | |
| (8) | 1 | 10 | 1.5±0.5 | 62.5 | 0.60 |
| | 0.3 | 10 | 2.0±0.3 | 30.6 | |
| (9) | 1 | 10 | 1.5±0.3 | 75.0 | 0.45 |
| | 0.3 | 10 | 1.9±0.2 | 37.5 | |
| (10) | 3 | 10 | 0.9±0.4 | 78.4 | 1.5 |
| | 1 | 10 | 2.0±0.4 | 25.5 | |
| (11) | 10 | 10 | 1.1±0.2 | 85.7 | |
| | 3 | 10 | 1.4±0.2 | 57.1 | 2.4 |
| | 1 | 10 | 2.0±0.4 | 25.0 | |
| (12) | 10 | 10 | 1.1±0.2 | 75.3 | 6.2 |
| | 3 | 10 | 2.6±0.3 | 13.6 | |
| (13) | 10 | 8 | 1.5±0.4 | 50.0 | 10 |
| | 3 | 8 | 2.6±0.3 | 13.2 | |

| Material tested | Dosage (mg/kg) | Animal used (no. of heads) | Ulcer index (aver. ± SE) | Inhibition factor (%) | $ED_{50}$ (mg/kg) |
|---|---|---|---|---|---|
| (14) | 1 | 10 | 1.3±0.2 | 77.8 | |
| | 0.3 | 10 | 1.7±0.3 | 44.4 | 0.35 |
| | 0.1 | 10 | 1.9±0.4 | 22.2 | |
| (15) | 6 | 10 | 0.8±0.3 | 79.9 | 3.5 |
| | 3 | 10 | 1.8±0.3 | 37.5 | |
| (16) | 1 | 10 | 1.7±0.3 | 44.4 | 2.0 |
| | 0.3 | 10 | 2.5±0.3 | 0 | |
| (17) | 0.1 | 10 | 0.5±0.2 | 95.7 | |
| | 0.03 | 10 | 1.5±0.3 | 81.4 | 0.005 |
| | 0.01 | 10 | 1.7±0.2 | 67.1 | |
| | 0.003 | 10 | 2.2±0.4 | 39.0 | |
| | 0.3 | 10 | 0.5±0.2 | 95.9 | |
| (18) | 0.1 | 10 | 0.7±0.2 | 89.0 | 0.03 |
| | 0.03 | 10 | 1.7±0.3 | 49.3 | |
| | 0.3 | 10 | 1.1±0.2 | 83.3 | |
| (19) | 0.1 | 10 | 1.6±0.3 | 63.0 | 0.06 |
| | 0.03 | 10 | 2.5±0.4 | 33.3 | |
| | 3 | 10 | 0.9±0.2 | 87.7 | |
| (20) | 1 | 10 | 1.7±0.2 | 58.3 | 0.80 |
| | 0.3 | 10 | 2.4±0.3 | 22.2 | |
| (21) | 3 | 10 | 0.9±0.2 | 87.5 | 0.80 |
| | 1 | 10 | 1.6±0.3 | 52.4 | |
| (22) | 3 | 10 | 1.2±0.3 | 70.0 | 1.8 |
| | 1 | 10 | 2.0±0.4 | 30.0 | |
| (23) | 3 | 10 | 2.0±0.2 | 50.0 | 3.0 |
| | 1 | 10 | 2.9±0.3 | 12.5 | |
| (24) | 10 | 10 | 1.4±0.2 | 87.1 | 2.0 |
| | 3 | 10 | 1.7±0.2 | 61.4 | |
| (25) | 10 | 8 | 1.4±0.2 | 62.5 | 8.0 |
| | 3 | 8 | 2.3±0.4 | 12.5 | |
| (26) | 10 | 8 | 1.1±0.2 | 72.2 | 4.0 |
| | 3 | 8 | 1.6±0.3 | 44.4 | |

| Material tested | Dosage (mg/kg) | Animal used (no. of heads) | Ulcer index (aver. ± SE) | Inhibition factor (%) | $ED_{50}$ (mg/kg) |
|---|---|---|---|---|---|
| (27) | 6 | 8 | 1.6±0.2 | 56.0 | 5.0 |
| | 3 | 8 | 2.1±0.3 | 31.8 | |
| (28) | 6 | 8 | 1.3±0.3 | 70.2 | 4.0 |
| | 3 | 8 | 1.9±0.2 | 36.0 | |
| (29) | 6 | 10 | 2.0±0.4 | 42.2 | >6 |
| | 3 | 10 | 2.5±0.3 | 30.0 | |
| (30) | 6 | 8 | 1.1±0.2 | 57.8 | 5.0 |
| | 3 | 8 | 2.3±0.4 | 29.7 | |
| (31) | 0.3 | 10 | 1.0±0.2 | 75.0 | |
| | 0.1 | 10 | 2.2±0.3 | 37.5 | 0.14 |
| | 0.03 | 10 | 2.6±0.4 | 12.5 | |
| (32) | 1 | 10 | 0.8±0.2 | 82.3 | |
| | 0.3 | 10 | 1.5±0.3 | 57.0 | 0.2 |
| | 0.1 | 10 | 2.0±0.4 | 27.9 | |
| (33) | 5 | 10 | 1.2±0.3 | 55.0 | 3.9 |
| | 1 | 10 | 2.6±0.3 | 25.0 | |
| (34) | 10 | 10 | 0.8±0.3 | 90.0 | |
| | 3 | 10 | 1.4±0.4 | 60.0 | 1.3 |
| | 1 | 10 | 2.0±0.4 | 40.0 | |
| (35) | 3 | 10 | 1.2±0.3 | 77.5 | |
| | 1 | 10 | 1.5±0.3 | 55.0 | 0.9 |
| | 0.3 | 10 | 2.3±0.3 | 21.3 | |
| (36) | 1 | 10 | 1.5±0.3 | 57.0 | 0.8 |
| | 0.3 | 10 | 2.4±0.4 | 22.6 | |
| (37) | 6 | 10 | 1.0±0.2 | 79.7 | |
| | 3 | 10 | 1.9±0.2 | 45.9 | 3.0 |
| | 1 | 10 | 2.7±0.4 | 8.1 | |

| Material tested | Dosage (mg/kg) | Animal used (no. of heads) | Ulcer index (aver. ± SE) | Inhibition factor (%) | $ED_{50}$ (mg/kg) |
|---|---|---|---|---|---|
| (38) | 3 | 10 | 1.0±0.2 | 79.7 | 1.5 |
| | 1 | 10 | 1.9±0.2 | 45.9 | |
| | 0.3 | 10 | 2.7±0.4 | 8.1 | |
| (39) | 3 | 10 | 0.7±0.2 | 85.3 | 0.5 |
| | 1 | 10 | 1.0±0.2 | 77.5 | |
| | 0.3 | 10 | 2.0±0.3 | 30.2 | |

Table-2 (Indomethacin Ulcer Preventing Effect)

| Material tested | Dosage (mg/kg) | Animal used (no. of heads) | Ulcer index (aver. ± SE) | Inhibition factor (%) | $ED_{50}$ (mg/kg) |
|---|---|---|---|---|---|
| Control | - | 10 | 2.5±0.3 | - | - |
| (1) | 20 | 8 | 2.4±0.4 | 4.0 | >20 |
| (2) | 20 | 9 | 0.4±0.2 | 100.0 | 6.0 |
| | 6 | 8 | 1.4±0.3 | 50.0 | |
| | 3 | 8 | 1.9±0.4 | 30.0 | |
| (3) | 10 | 9 | 1.0±0.3 | 71.4 | 4.4 |
| | 3 | 9 | 1.7±0.4 | 42.9 | |
| (8) | 3 | 10 | 1.7±0.3 | 42.9 | 3.8 |
| | 1 | 10 | 2.3±0.4 | 14.3 | |
| (9) | 3 | 10 | 1.5±0.3 | 50.5 | 3.0 |
| | 1 | 10 | 2.1±0.3 | 37.5 | |
| (11) | 10 | 10 | 2.0±0.2 | 50.0 | 10.0 |
| | 3 | 10 | 2.6±0.3 | 0 | |
| (12) | 10 | 10 | 0.8±0.2 | 71.4 | 7.4 |
| | 3 | 10 | 1.6±0.3 | 48.0 | |
| (14) | 3 | 10 | 0.6±0.1 | 80.0 | 1.5 |
| | 1 | 10 | 2.0±0.3 | 32.0 | |
| (16) | 10 | 10 | 1.0±0.2 | 60.0 | 8.2 |
| | 3 | 10 | 2.3±0.3 | 10.0 | |
| (18) | 1 | 10 | 0.2±0.02 | 100.0 | 0.17 |
| | 0.3 | 10 | 1.4±0.1 | 62.5 | |
| | 0.1 | 10 | 1.8±0.2 | 38.3 | |

| Material tested | Dosage (mg/kg) | Animal used (no. of heads) | Ulcer index (aver. ± SE) | Inhibition factor (%) | $ED_{50}$ (mg/kg) |
|---|---|---|---|---|---|
| (19) | 10 | 10 | 0.4±0.1 | 85.7 | 3.6 |
| | 3 | 10 | 1.9±0.2 | 42.9 | |
| (20) | 10 | 10 | 0.6±0.1 | 87.5 | 3.5 |
| | 3 | 10 | 1.3±0.2 | 44.4 | |
| (21) | 10 | 10 | 1.5±0.3 | 80.0 | 6.0 |
| | 3 | 10 | 2.5±0.4 | 0 | |
| (23) | 10 | 10 | 1.5±0.3 | 50.0 | 10.0 |
| | 3 | 10 | 2.0±0.3 | 25.0 | |
| (30) | 10 | 10 | 0.6±0.1 | 71.5 | |
| | 3 | 10 | 2.1±0.4 | 24.0 | 0.9 |
| | 1 | 10 | 2.4±0.5 | 10.0 | |
| (33) | 10 | 10 | 1.6±0.2 | 62.0 | 6.3 |
| | 3 | 10 | 2.3±0.3 | 30.0 | |
| (34) | 10 | 10 | 1.9±0.2 | 70.0 | 6.0 |
| | 3 | 10 | 2.2±0.4 | 20.0 | |
| (35) | 10 | 10 | 1.0±0.2 | 72.5 | 4.8 |
| | 3 | 10 | 2.3±0.4 | 29.3 | |

Materials tested in Table 1 is shown hereinafter:

(1) 13, 14-dihydro-15-keto-$PGE_2$,
(2) 13, 14-dihydro-15-keto-$PGE_2$ methyl ester,
(3) 13, 14-dihydro-15-keto-$PGE_2$ ethyl ester,
(4) 13, 14-dihydro-15-keto-$PGE_2$-n-propyl ester,
(5) 13, 14-dihydro-15-keto-$PGE_2$ isopropyl ester,
(6) 13, 14-dihydro-15-keto-$PGE_1$ methyl ester,
(7) 13, 14-dihydro-15-keto-$PGE_1$ ethyle ester,
(8) 13, 14-dihydro-6,15-diketo-$PGE_1$ methyl ester,
(9) 13, 14-dihydro-6,15-diketo-$PGE_1$ ethyl ester,
(10) 13, 14-dihydro-6,15-diketo-$PGE_1$ n-butyl ester,
(11) (±)13, 14-dihydro-6,15-diketo-$PGE_1$ ethyl ester,
(12) 13, 14-dihydro-15-keto-3R,S-methyl-$PGE_2$ methyl ester,
(13) 13, 14-dihydro-15-keto-3R,S-methyl-$PGE_2$ ethyl ester,
(14) 13, 14-dihydro-15-keto-16R,S-fluoro-11-dehydroxy-11R-methyl-$PGE_2$ ethyl ester,
(15) 13, 14-dihydro-15-keto-11-dehydroxy-11R-methyl-$PGE_2$ ethyl ester,
(16) 13, 14-dihydro-15-keto-16R,S-hydroxy-$PGE_2$ ethyl ester,
(17) 13, 14-dihydro-15-keto-16R,S-fluoro-$PGE_2$,
(18) 13, 14-dihydro-15-keto-16R,S-fluoro-$PGE_2$ methyl ester,
(19) 13, 14-dihydro-15-keto-16 R,S-fluoro-$PGE_2$ ethyl ester,
(20) 13, 14-dihydro-15-keto-16R,S-methyl-$PGE_2$ methyl ester,
(21) 13, 14-dihydro-15-keto-16R,S-methyl-$PGE_2$ ethyl ester,
(22) 13, 14-dihydro-15-keto-3R,S,16R,S-dimethyl-$PGE_2$ methyl ester,
(23) 13, 14-dihydro-15-keto-19-methyl-$PGE_2$ methyl ester,
(24) 13, 14-dihydro-15-keto-19-methyl-$PGE_2$ ethyl ester,
(25) 13, 14-dihydro-15-keto-20-isopropylidene-$PGE_2$ methyl ester,
(26) 13, 14-dihydro-15-keto-20-ethyl-$PGE_2$ methyl ester,
(27) 13, 14-dihydro-15-keto-20-ethyl-$PGE_2$ ethyl ester,

(28) 13, 14-dihydro-15-keto-20-ethyl-11-dehydroxy-11R-methyl-$PGE_2$ methyl ester,
(29) 13, 14-dihydro-15-keto-20-n-propyl-$PGE_2$ methyl ester,
(30) 13, 14-dihydro-15-keto-20-ethyl-$PGE_1$ methyl ester,
(31) 13, 14-dihydro-6,15-diketo-16R,S-fluoro-$PGE_1$ ethyl ester,
(32) 13, 14-dihydro-6,15-diketo-16R,S-fluoro-11-dehydroxy-11R-methyl-$PGE_1$ ethyl ester,
(33) 13, 14-dihydro-6,15-diketo-16R,S-methyl-$PGE_1$ methyl ester,
(34) 13, 14-dihydro-6,15-diketo-16R,S-methyl-$PGE_1$ ethyl ester,
(35) 13, 14-dihydro-6,15-diketo-19-methyl-$PGE_1$ methyl ester,
(36) 13, 14-dihydro-6, 15-diketo-19-methyl-$PGE_1$ ethyl ester,
(37) 13, 14-dihydro-6, 15-diketo-20-methyl-$PGE_1$ ethyl ester,
(38) 13, 14-dihydro-6, 15-diketo-11-dehydroxy-11R-methyl-$PGE_1$ methyl ester,
(39) 13,14-dihydro-6,15-diketo-11-dehydroxy-11R-methyl-$PGE_1$ ethyl ester.

From the foregoing results, it can be seen that while 13, 14-dihydro-15-keto-$PGE_2$, as a physiologically and pharmacologically inactive metabolite, shows no antiulcerative effect, it can have antiulcerative effect if it is made into an ester compound of 13, 14-dihydro-15-keto-PGE or a compound similar thereto.

Test Example 2

The following 4 materials were measured as to their respctive effects of ulcer prevention, intestinal constriction, tracheorelaxation, and uteroconstriction, and examined in comparison to one another. The results are shown in Table 3.

Table-3

| Material tested | Ulcer inhibiting effect $ED_{50}$ (mg/kg) | Intestinal constriction effect | Tracheal relaxation effect | Uterus constriction effect |
|---|---|---|---|---|
| A | 0.5 | + | + | + |
| B | 0.4 | + | + | ± |
| C | >20 | - | ± | ± |
| D | 1.5 | - | - | - |

Materials tested:

A : $PGE_2$ (product of Funakoshi Yakuhin K.K.)
B : $PGE_2$ ethyl ester (produced by Applicant Co.)
C : 13, 14-dihydro-15-keto-$PGE_2$ (product of Funakoshi Yakuhin K.K.)
D : 13, 14-dihydro-15-keto-$PGE_2$ ethylester

(1) Antiulcerative effect

The procedure of Test Example 1 was followed in determining values for hydrorestraint stress-ulcer preventing effect in terms of $ED_{50}$.
+ + : alvin flux developed at a concentration lower than 1 mg/kg ;
+ : alvin flux developed at concentrations of 1 ~ 10 mg/kg ;
- : no flux developed at a concentration higher than 10 mg/kg.

(2) Intestinal constriction effect

A male Wister rat (of 250 ~ 300g in weight) was struck to death, and immediately its carotid artery was

cut to dehematize. An ileum portion located about 10 cm from the cecum was extracted, and after its contents were washed away with a Tyrode liquid, a 1.5 ~ 2 cm long part of it was cut off and hung in a Magnus tube.

The constriction of the ileum was brought to rest for 15 ~ 20 minutes until the ileum was allowed to be stabilized, and subsequently the ileum was constricted with $10^{-6}$ g/mℓ of acetylcholine. After constructions of same magnitude were had two times, the material to be tested was cumulatively administered at one-minute intervals.

Constrictions with the material tested were expressed in terms of ratios, based on constriction per $10^{-6}$ g/mℓ of acetylcholine, and values for $ED_{50}$ were determined.

+ : $ED_{50} < 10^{-6}$ M
± : $10^{-4}$ M $\leqq ED_{50} \leqq 10^{-6}$ M
- : $10^{-4}$ M $< ED_{50}$

(3) Tracheal relaxation

A male gunea pig (of about 300 g in weight) was struck to death, and its artery was cut to dehematize. Its trachea, after having been extracted, was cut open lengthwise on the opposite side to the trachea smooth muscle, and seven tracheal rings were connected by string in a chain-like pattern, same being hung in a Magnus tube.

Trachea was brought to rest for 60 ~ 90 minutes and until tracheal equilibrium was reached. Thereafter, 5.4 x $10^{-4}$ M of histamine was administered in such manner that it was cumulatively administered at 6 minutes' intervals after a constriction peak was reached. Tracheal relaxation with the material tested was expressed in terms of ratio of constrictional inhibition under histamine administering, and values for $IC_{50}$ were determined.

+ : $IC_{50} < 10^{-7}$ M
± : $10^{-5}$ M $\leqq IC_{50} \leqq 10^{-7}$ M
- : $10^{-5}$ M $< IC_{50}$

(4) Uterine constriction

A female rat (of 150g in weight) was dehematized to death, and its uterus was taken out, which was cut to a length of 1.5 - 2.5 cm. The cut uterus was hung in a magnus tube. The uterus was constricted several times with 1 mU Oxytocin. After stable uterine movement was obtained, the material to be tested was independently administered. Constrictions with the material were expressed in terms of ratios based on constriction of 1 mU of oxytocin = 100, and values of $EC_{50}$ were determined on the following standards.

+ : $EC_{50} < 10^{-7}$ M
± : $10^{-5}$ M $\leqq EC_{50} \leqq 10^{-7}$ M
- : $10^{-5}$ M $< EC_{50}$

From the results of the foregoing tests it can be seen that $PGE_2$ and $PGE_2$ ethylester can, in addition to their ulcer inhibiting effects, concurrently produce intestinal constriction, tracheal relaxation, and uterus constriction. Whilst, no pharmacological or physiological effect, such as ulcer inhibiting effect, can be found with 13,14-dihydro-15-keto-$PGE_2$. However, it can be recognized that 13,14-dihydro-15-keto-$PGE_2$ ethylester, an ester compound of said 13, 14-dihydro-15-keto-$PGE_2$, can produce a high degree of ulcer inhibiting effect, though it has no such effect as intestinal, uterus constriction, tracheal relaxation and the like.

Chart I

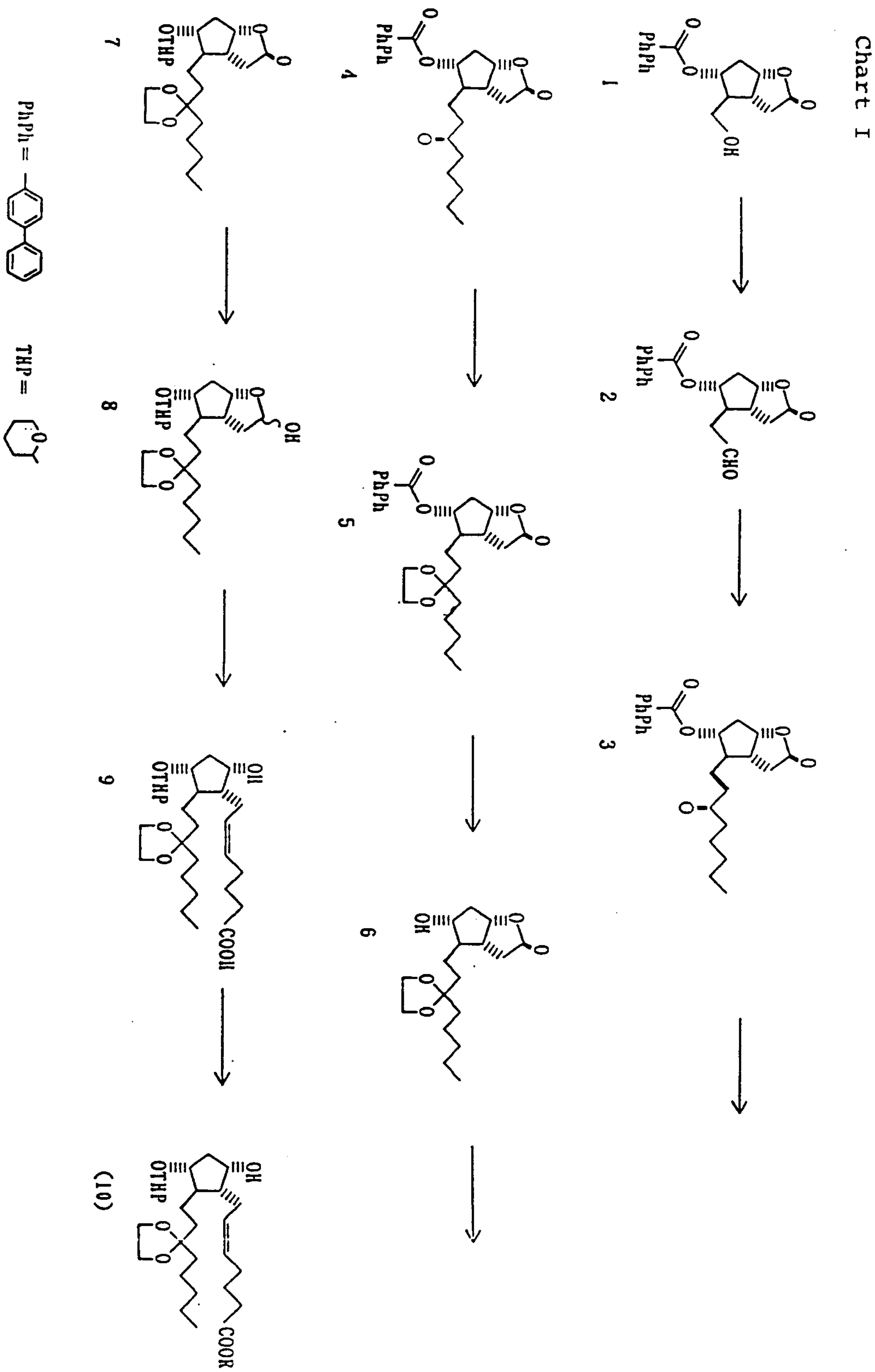

1

2

3

4

5

6

7

8

9

(10)

PhPh =

THP =

Chart I (Continued)

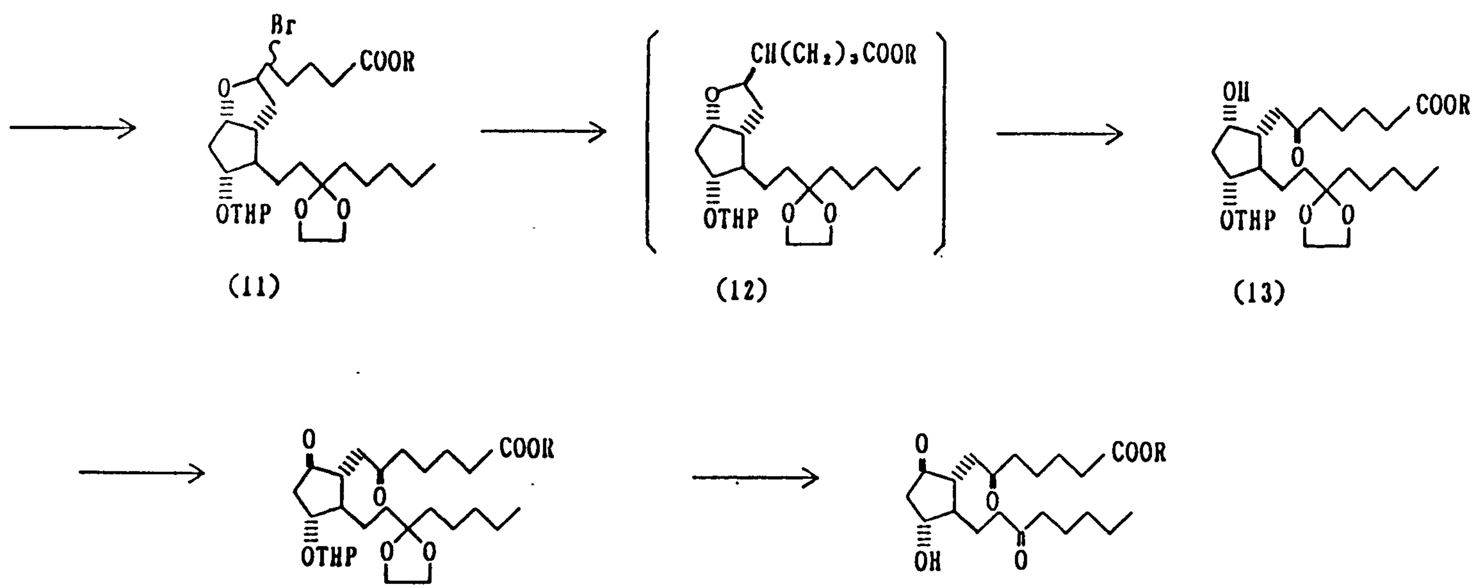

(11)

(12)

(13)

(14)

(15)

Chart II

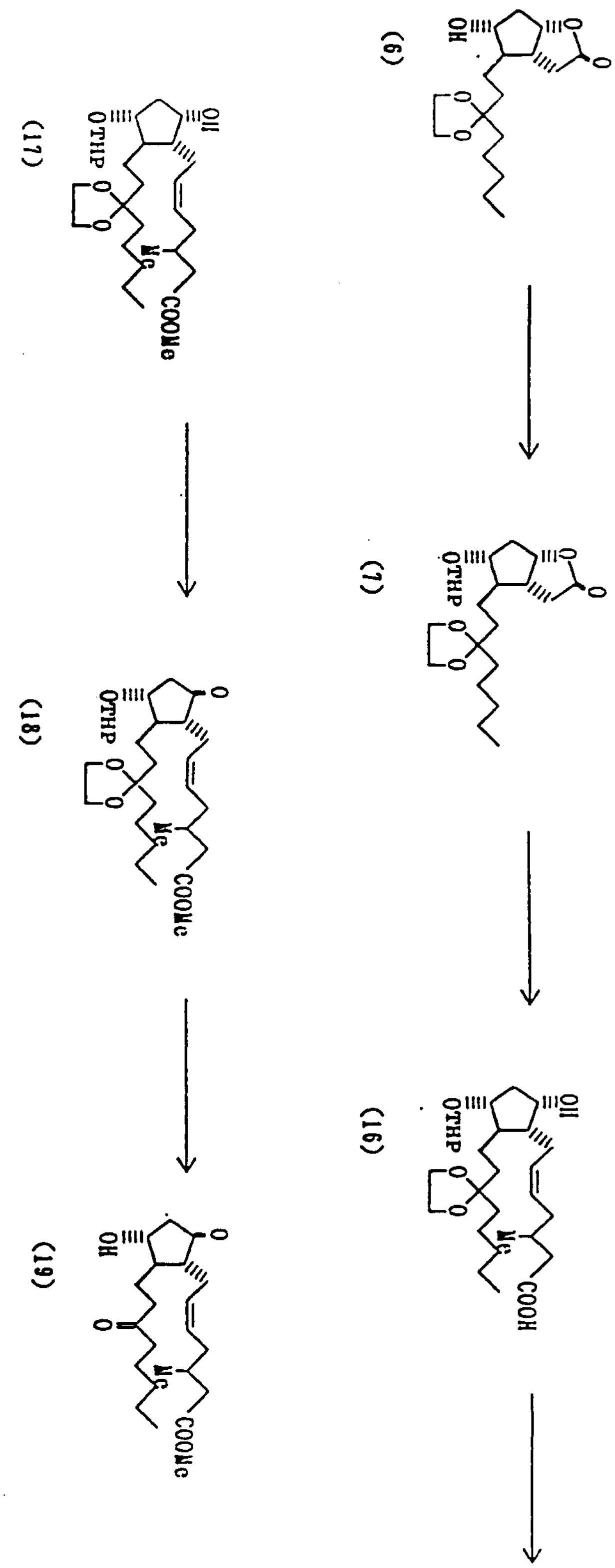

(6)

(7)

(16)

(17)

(18)

(19)

Chart III

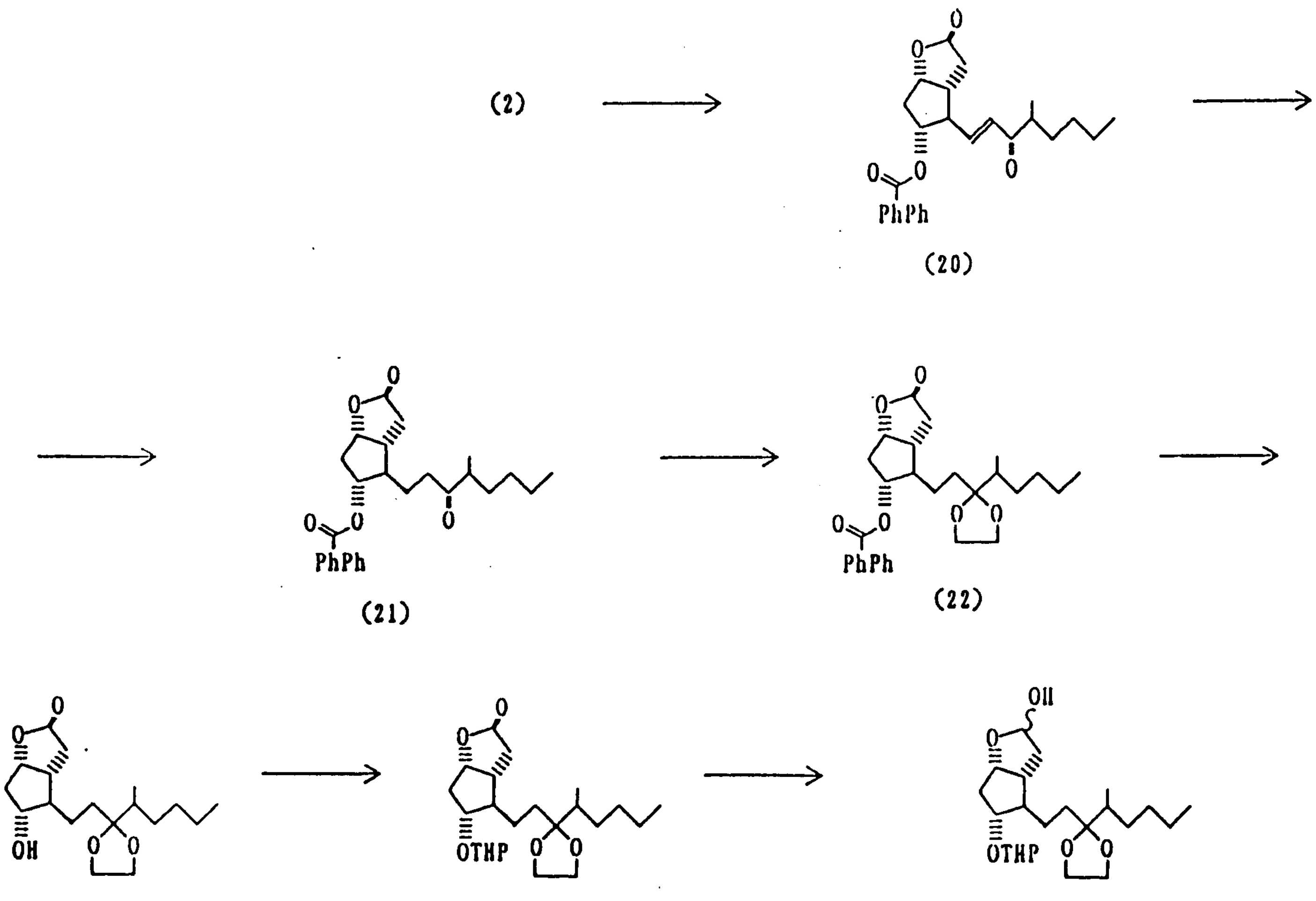

(2)

(20)

(21)

(22)

(23)

(24)

(25)

Chart III (Contiuned)

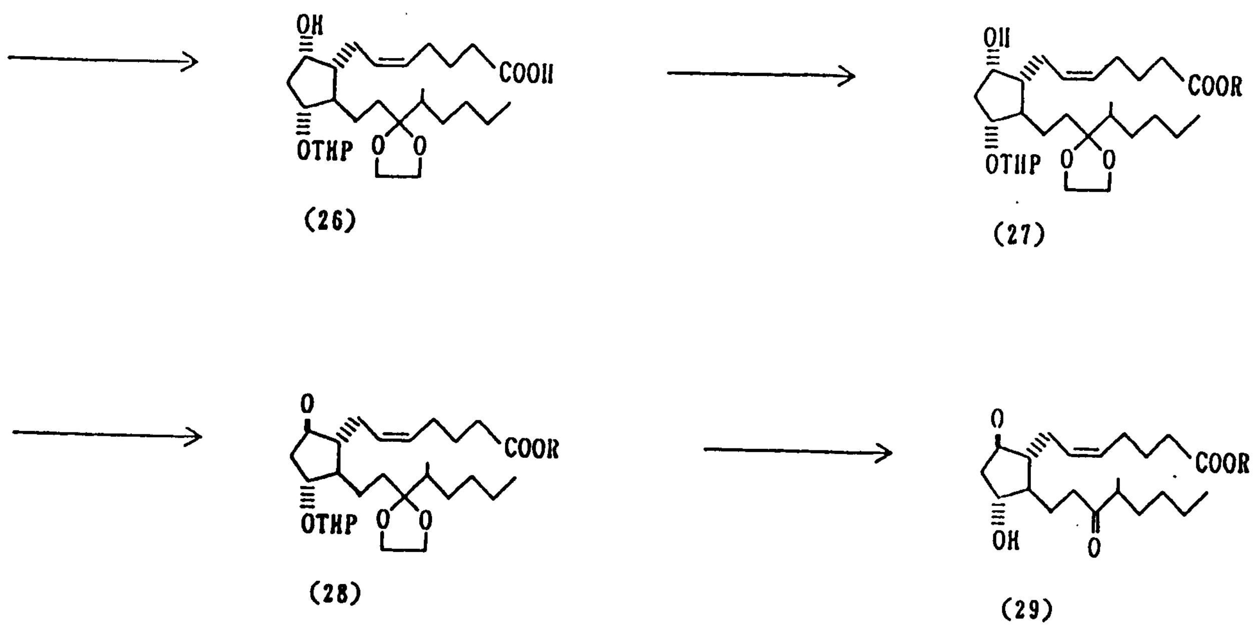

(26) (27)

(28) (29)

R : Et or Me

Chart IV

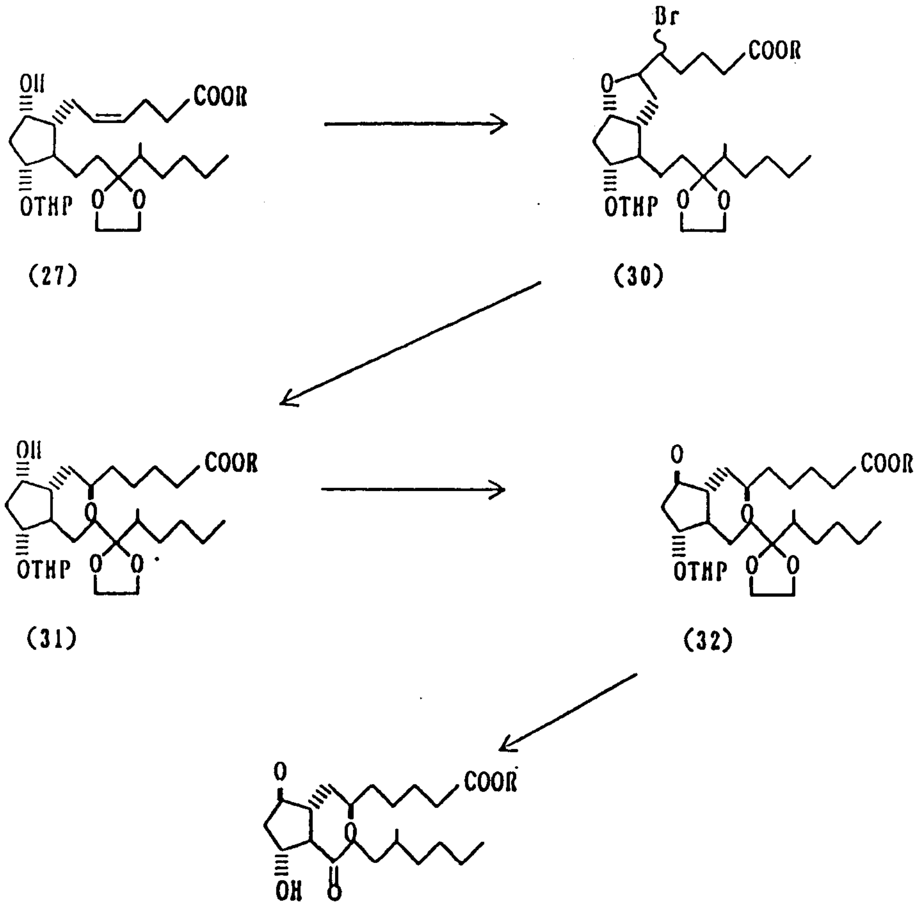

(27) (30)

(31) (32)

(33)

R : Et or Me

Chart V

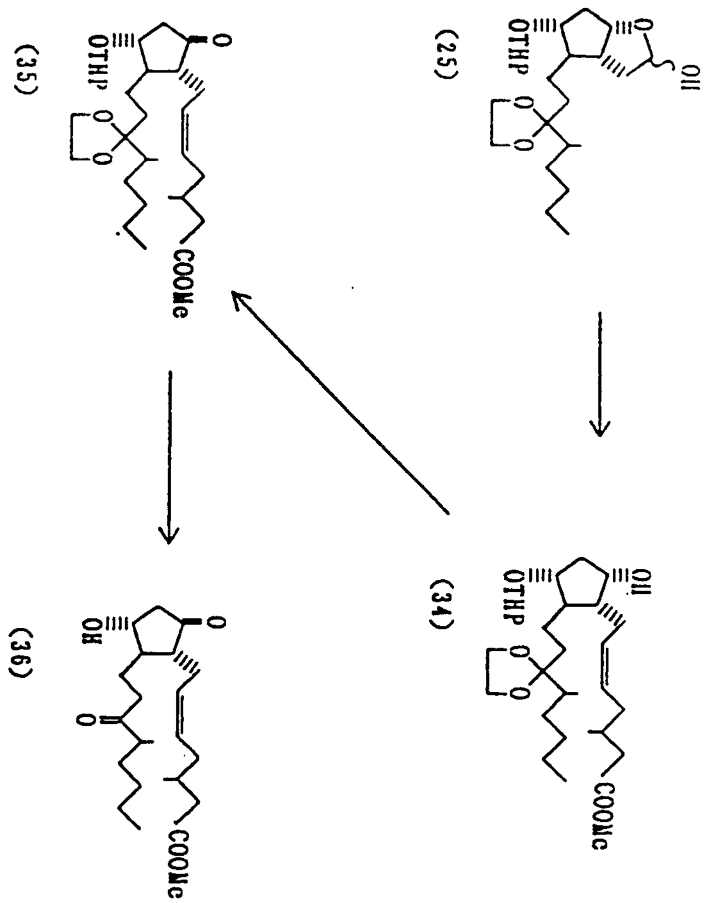

(25)

(34)

(35)

(36)

Chart VI

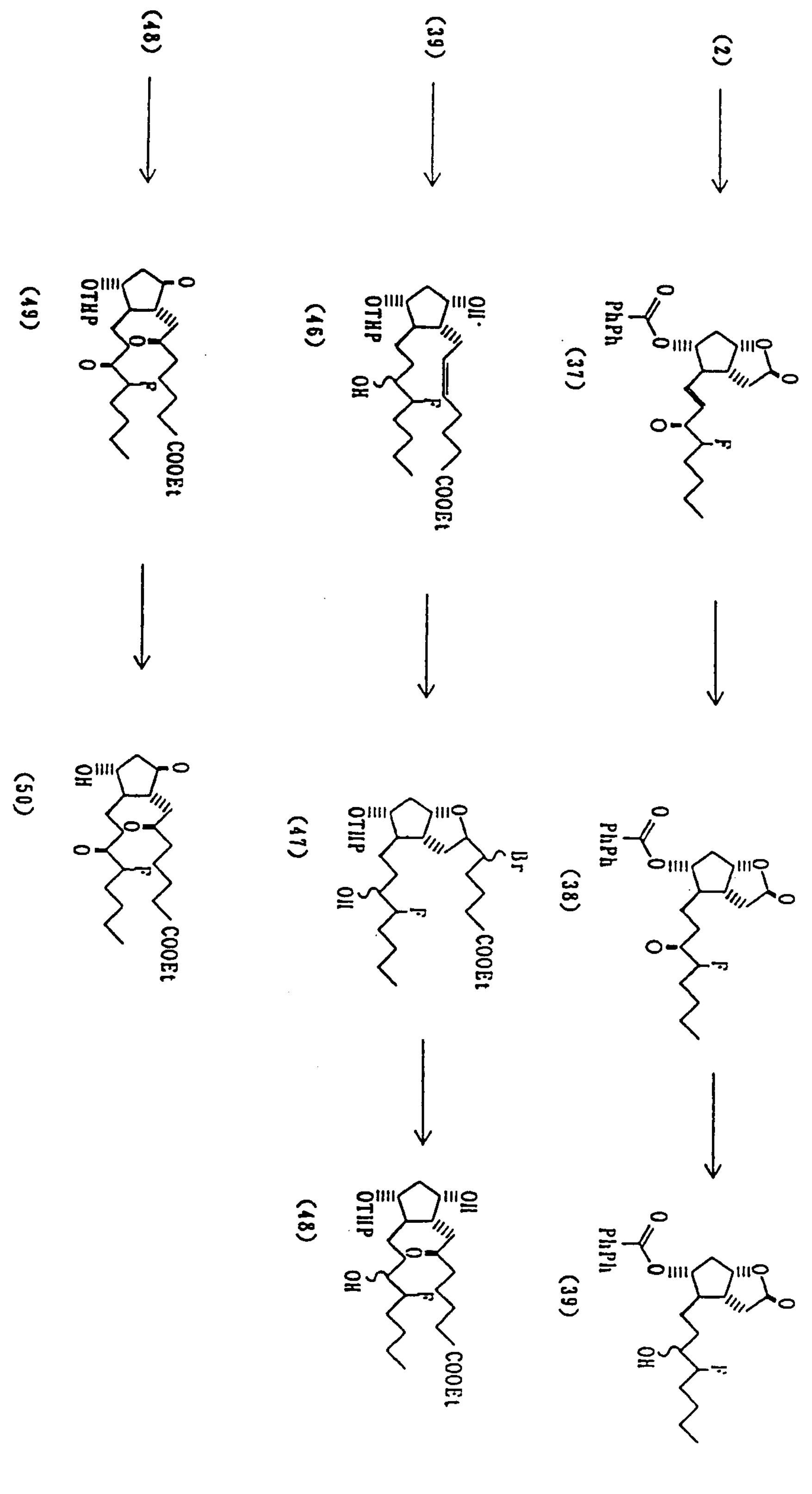

(2) → (37) → (38) → (39)

(39) → (46) → (47) → (48)

(48) → (49) → (50)

Chart VII

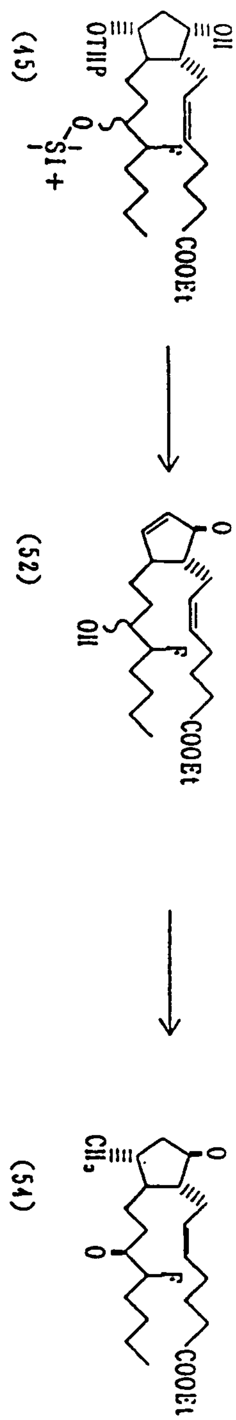

(45)

(52)

(54)

Chart VIII

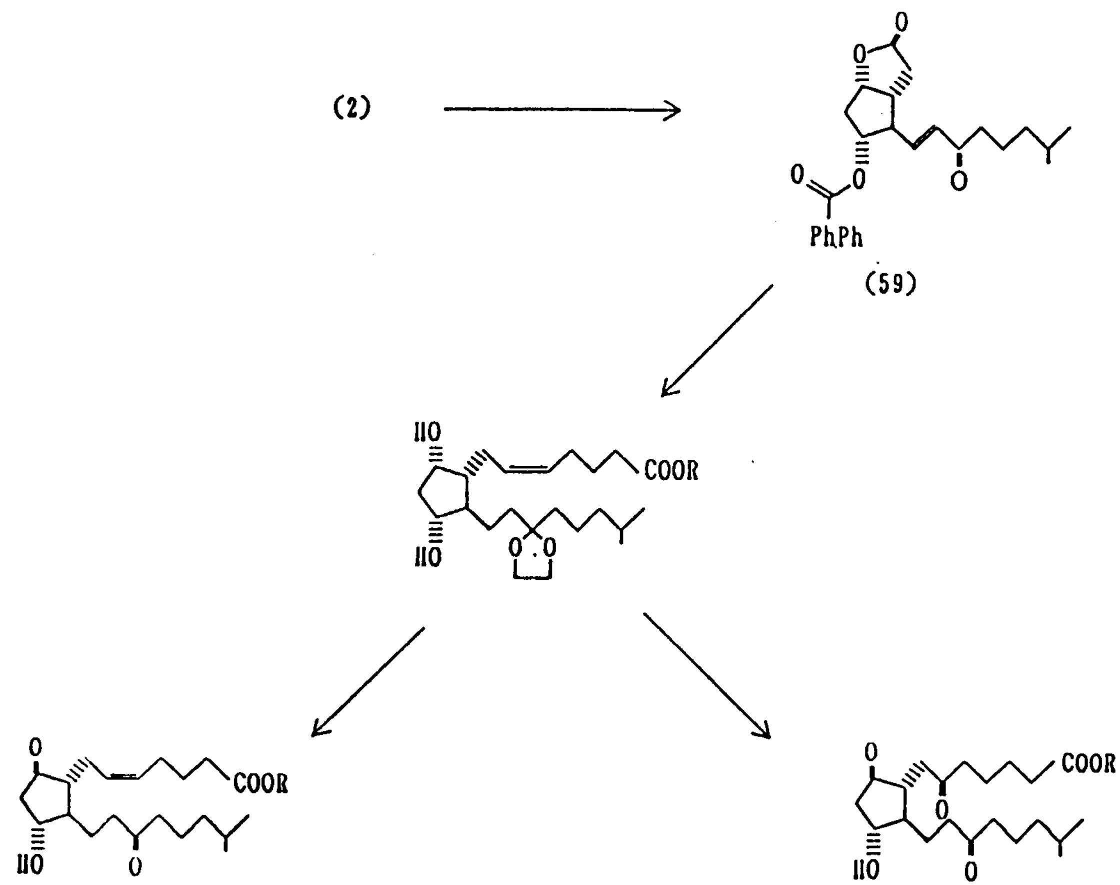

(2)

(59)

R: Et (60)
Me (61)

R : Et or Me

R: Et (62)
Me (63)

Chart IX

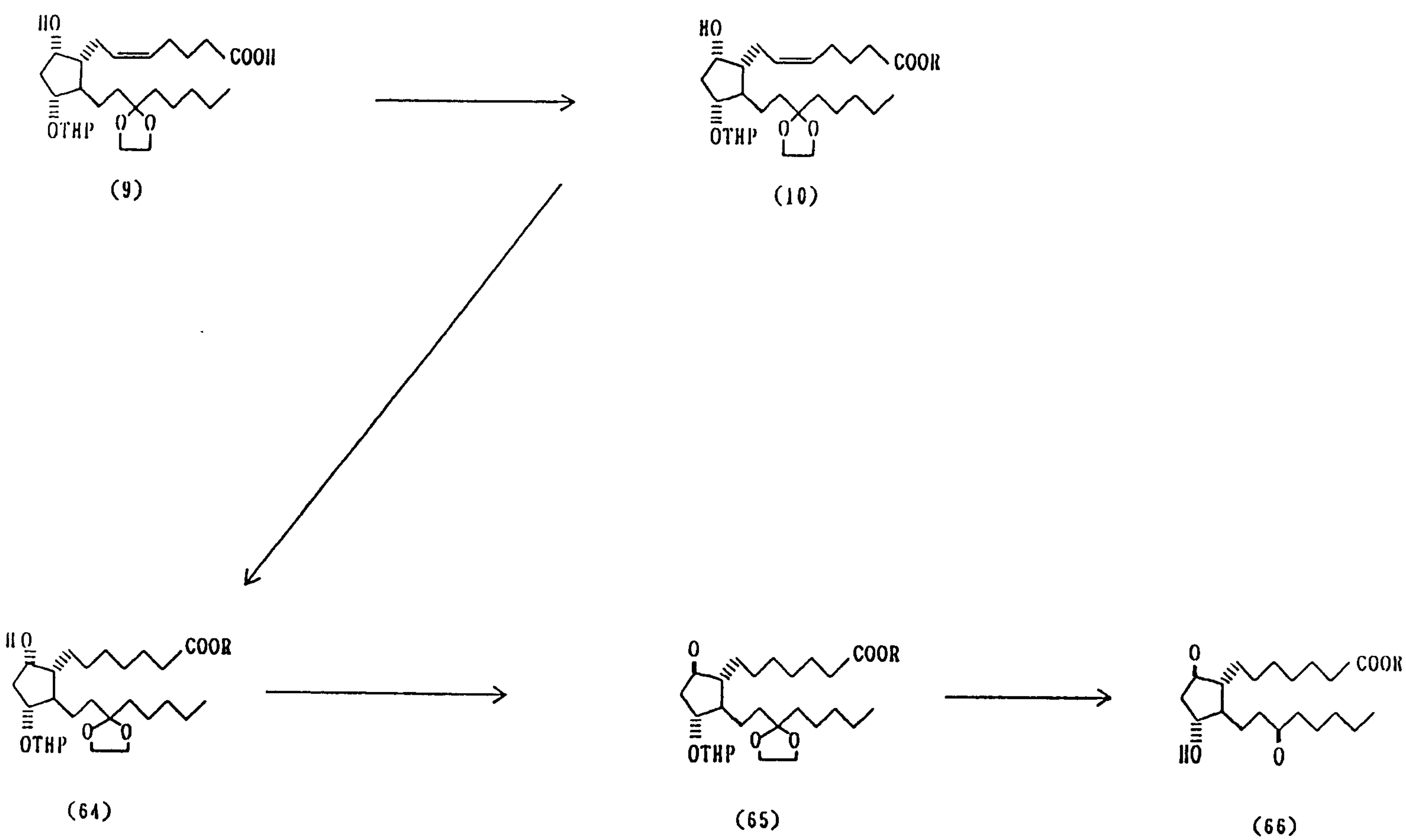

(9) (10) (64) (65) (66)

R : Et or Me

Chart X

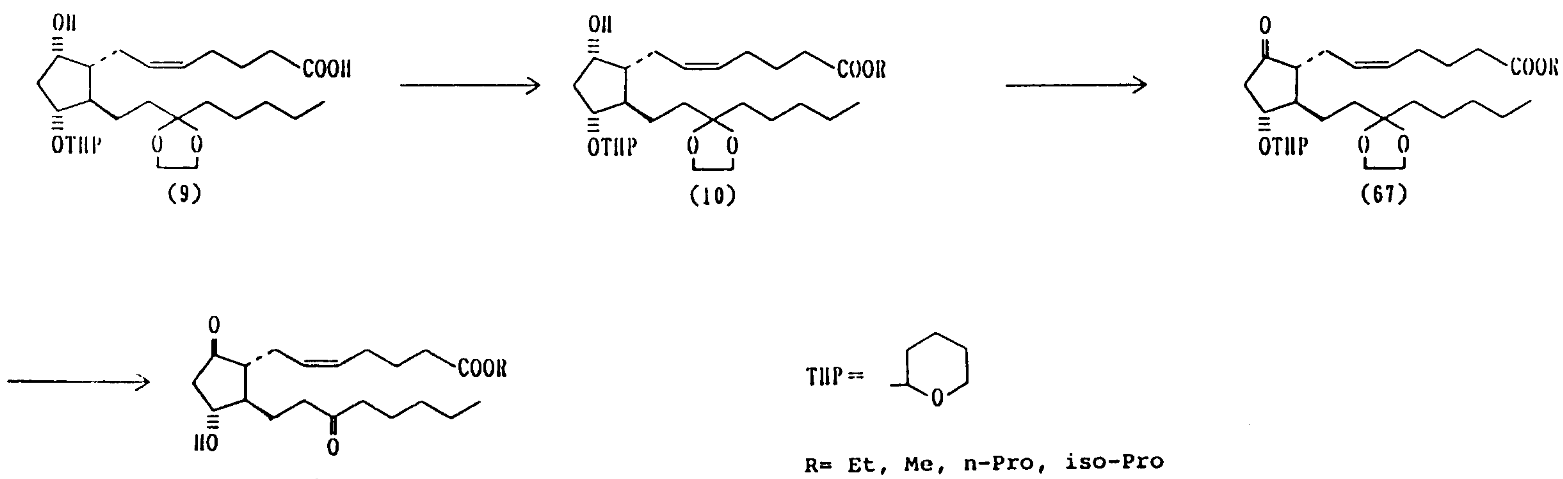

(9)

(10)

(67)

(68)

THP =

R= Et, Me, n-Pro, iso-Pro
iso-Pro, n-Bu,
Cyclopentyl, Benzyl

Chart XI

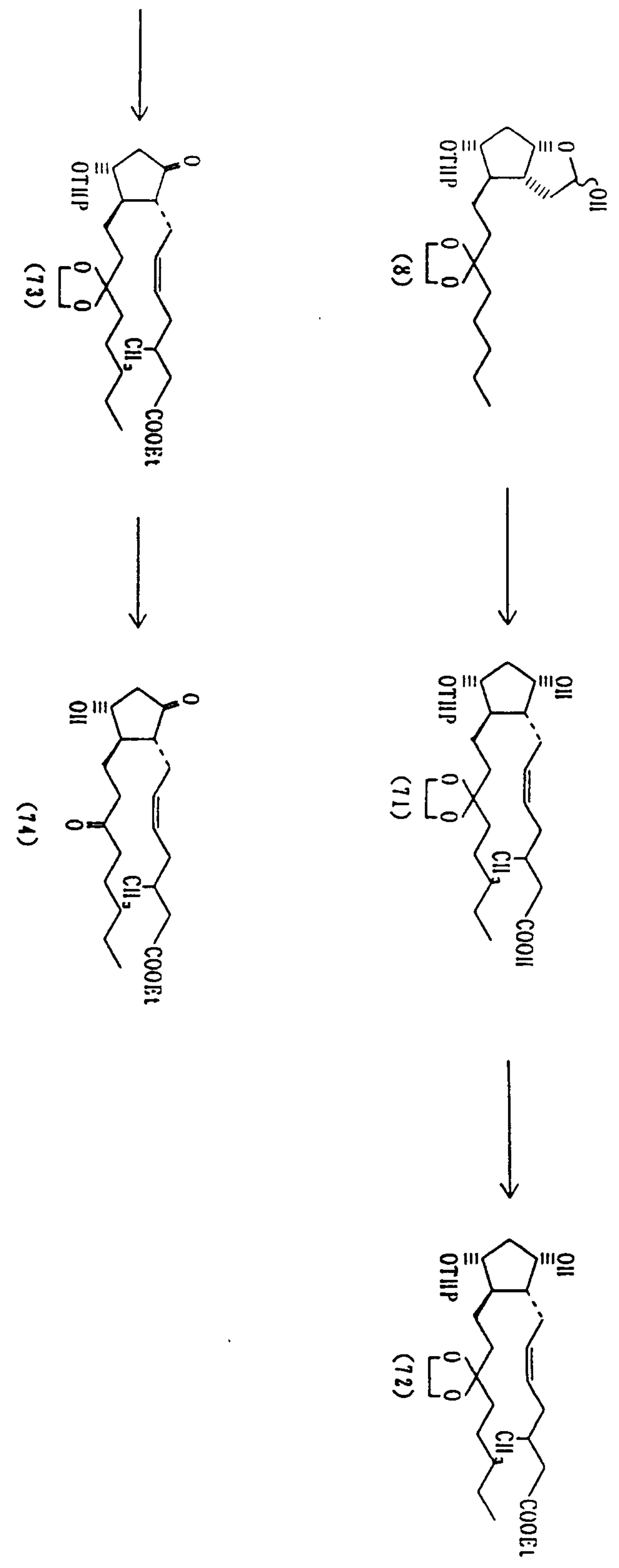

(8)

(71)

(72)

(73)

(74)

Chart XII

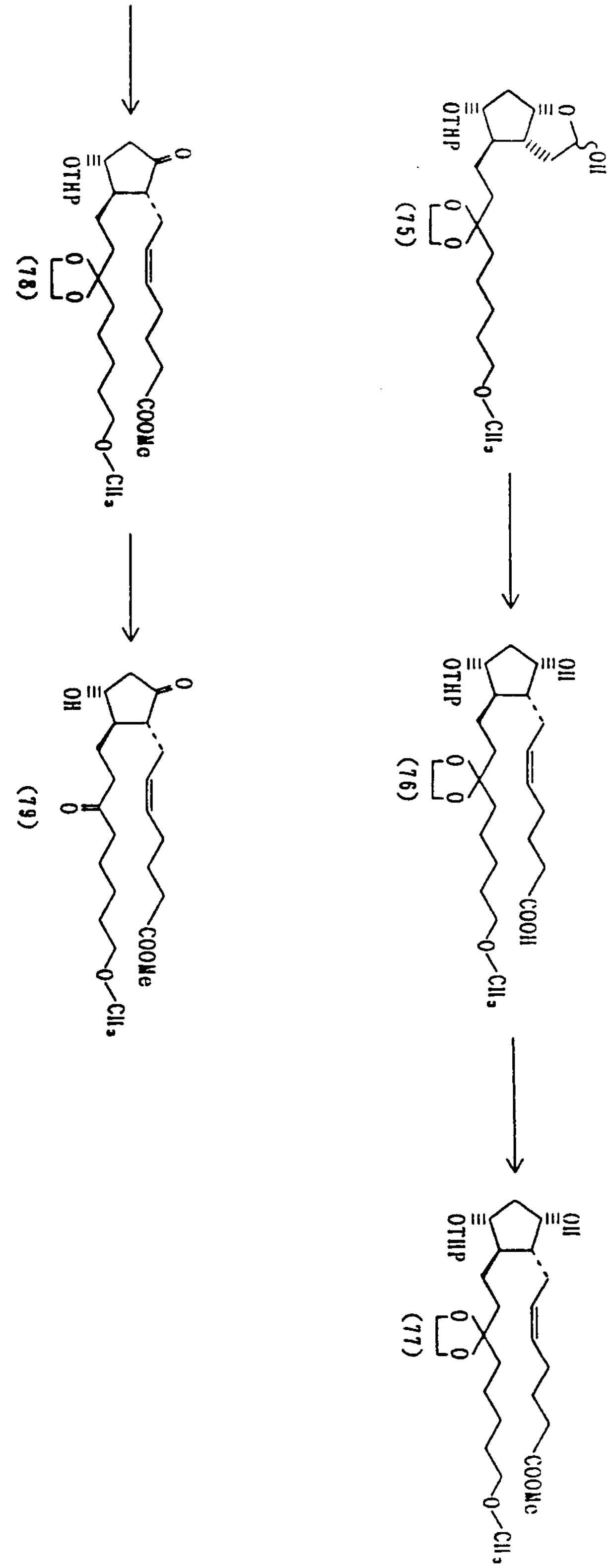

(75) (76) (77) (78) (79)

Chart XIII

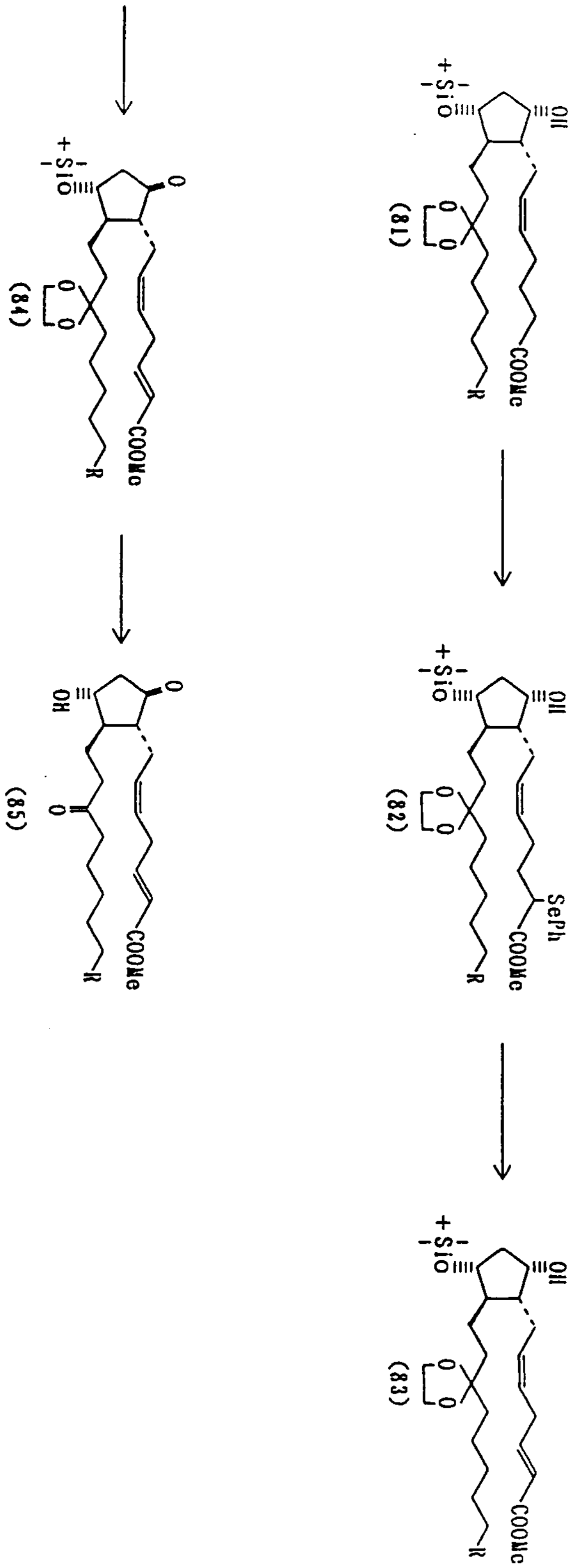

(81)

(82)

(83)

(84)

(85)

Chart XIV

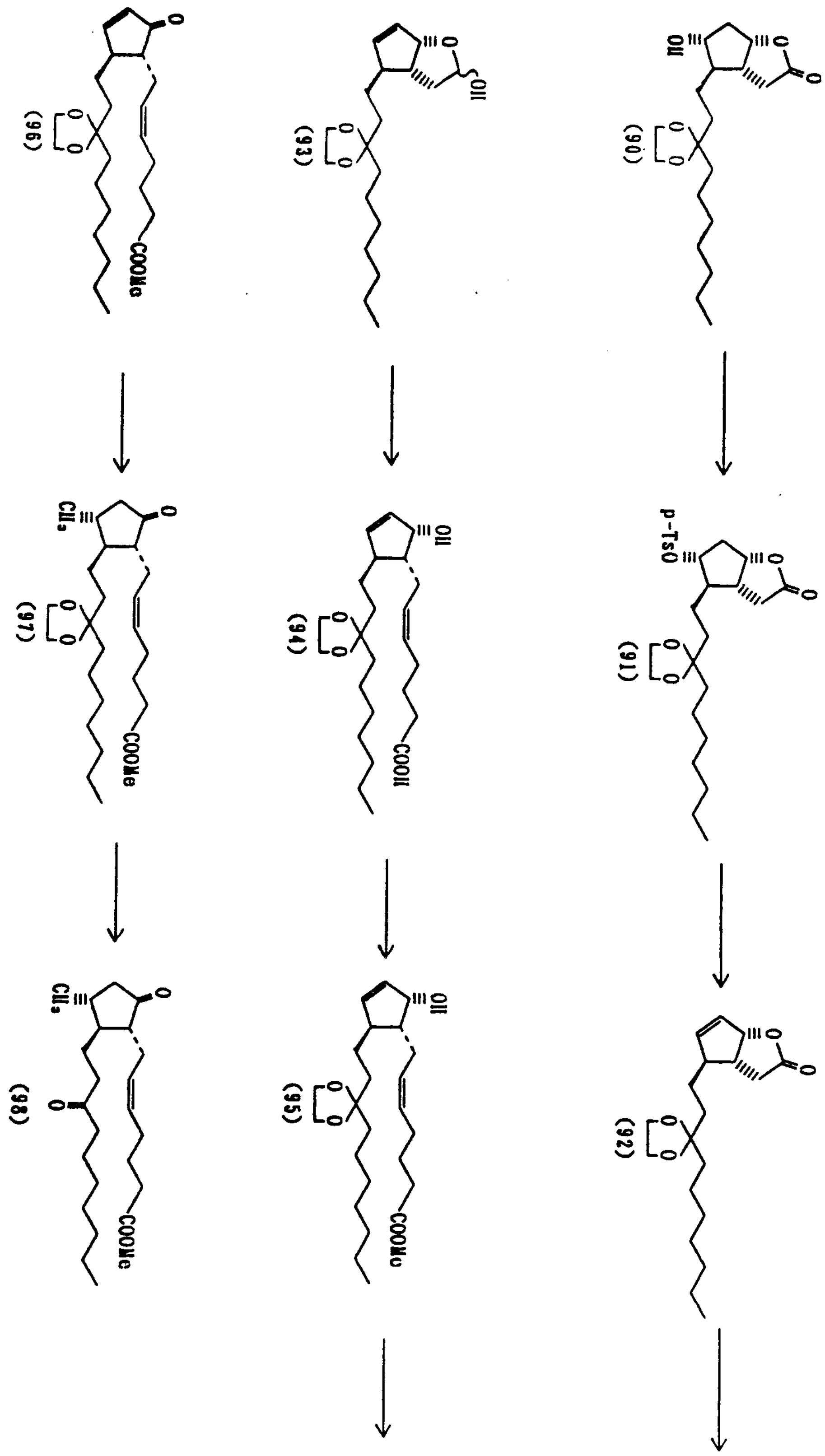

(90) (91) (92)

(93) (94) (95)

(96) (97) (98)

Chart XV

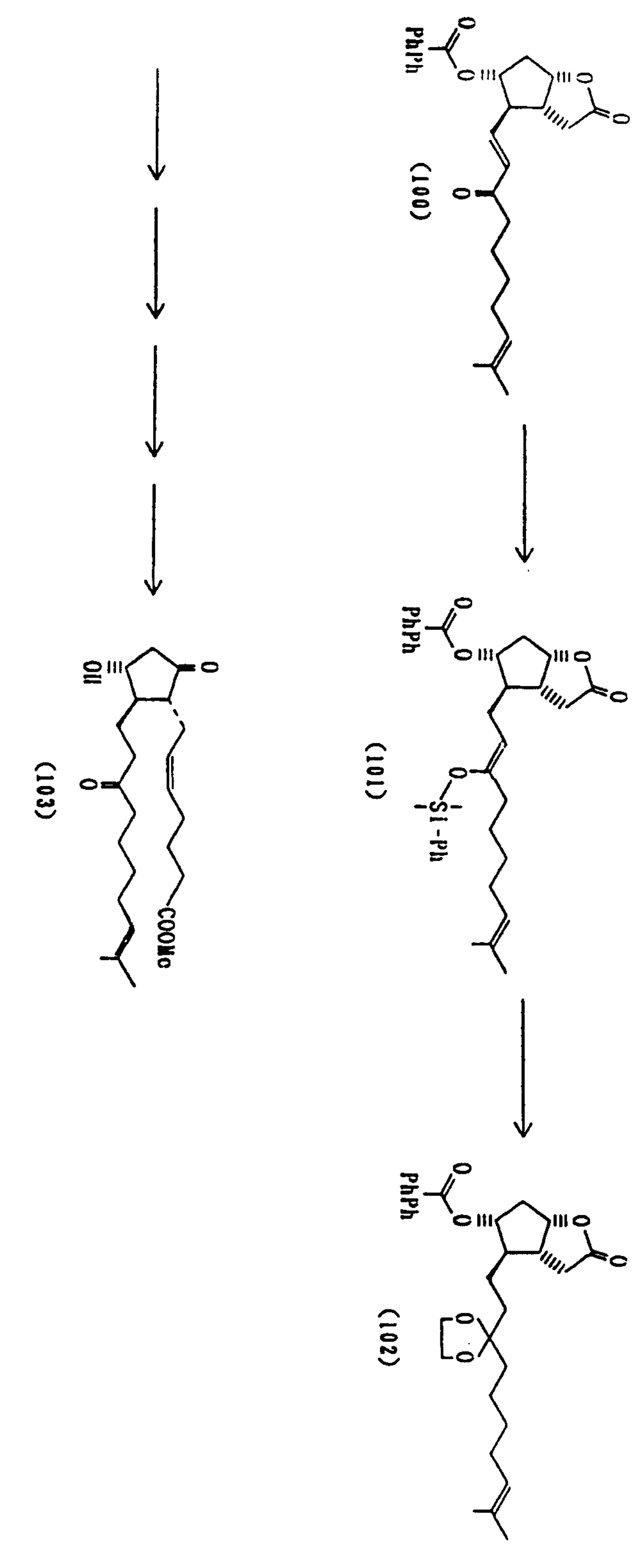

(100)

(101)

(102)

(103)

Chart XVI

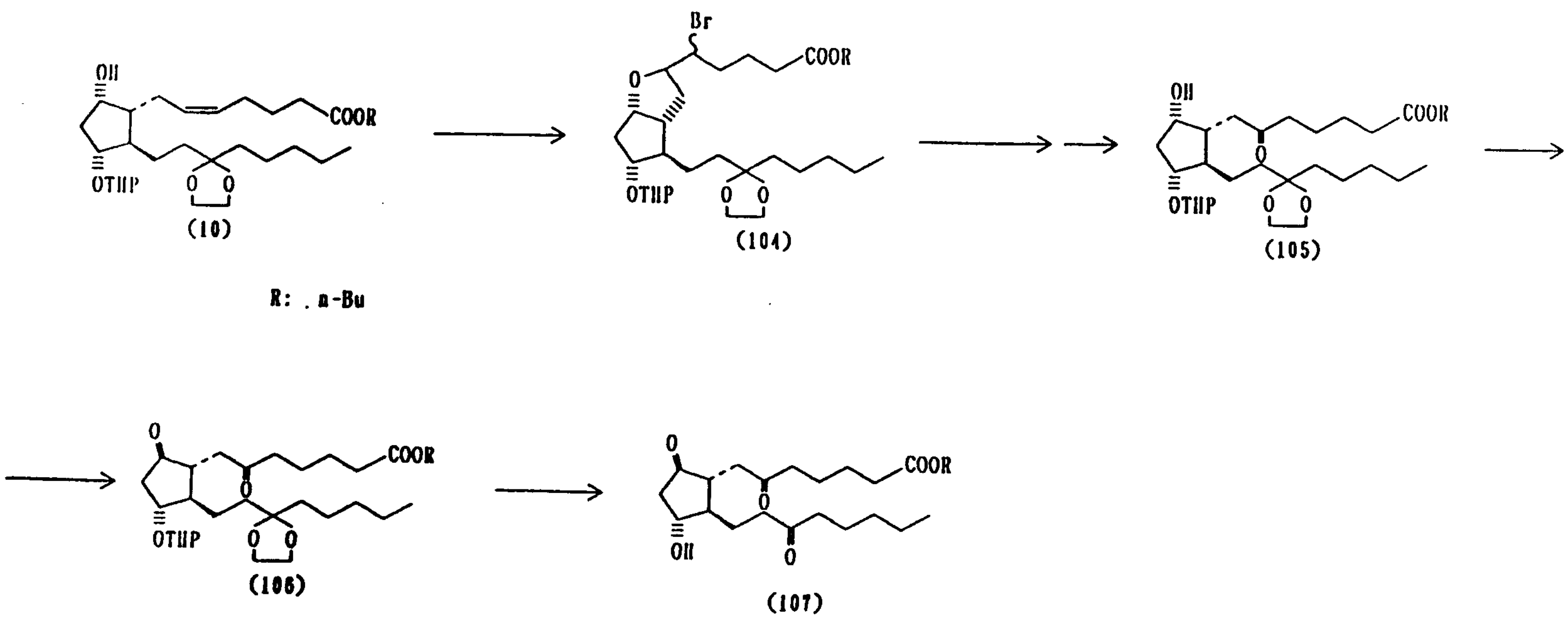

(10)

(104)

(105)

R: . n-Bu

(106)

(107)

Chart XVII

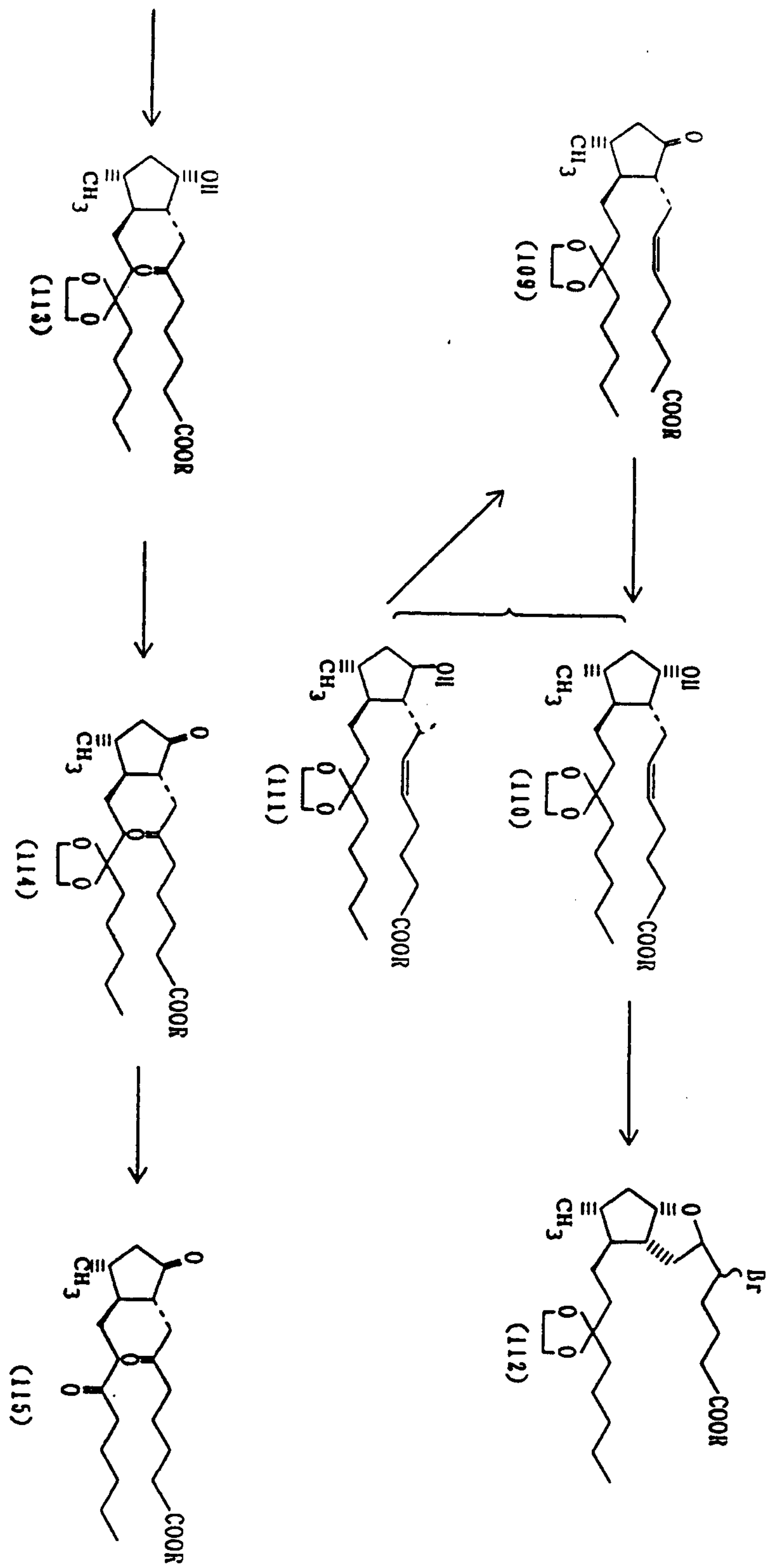

(109)

(110)

(111)

(112)

(113)

(114)

(115)

Chart X VIII

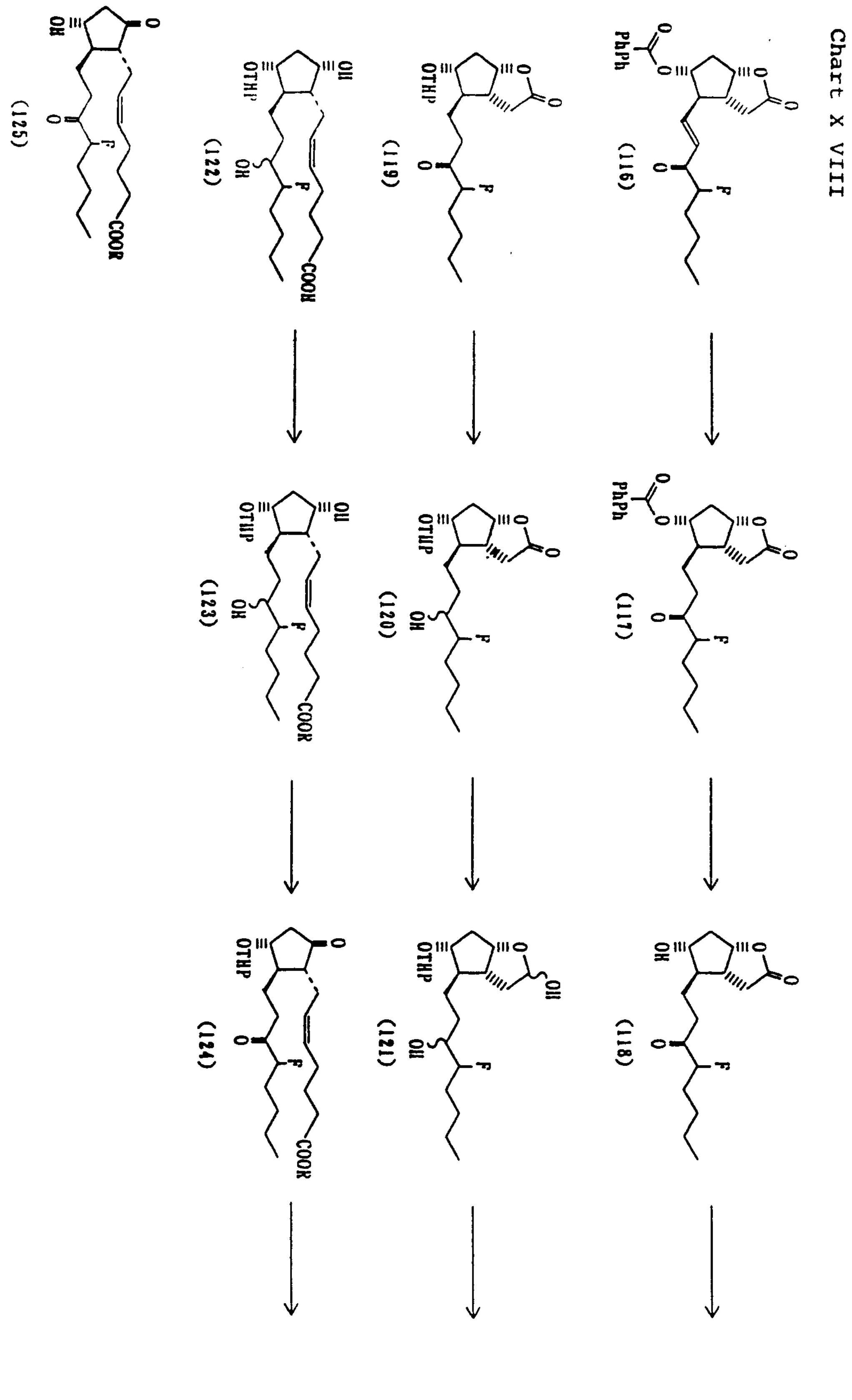

(116) (117) (118) (119) (120) (121) (122) (123) (124) (125)

Chart X IX

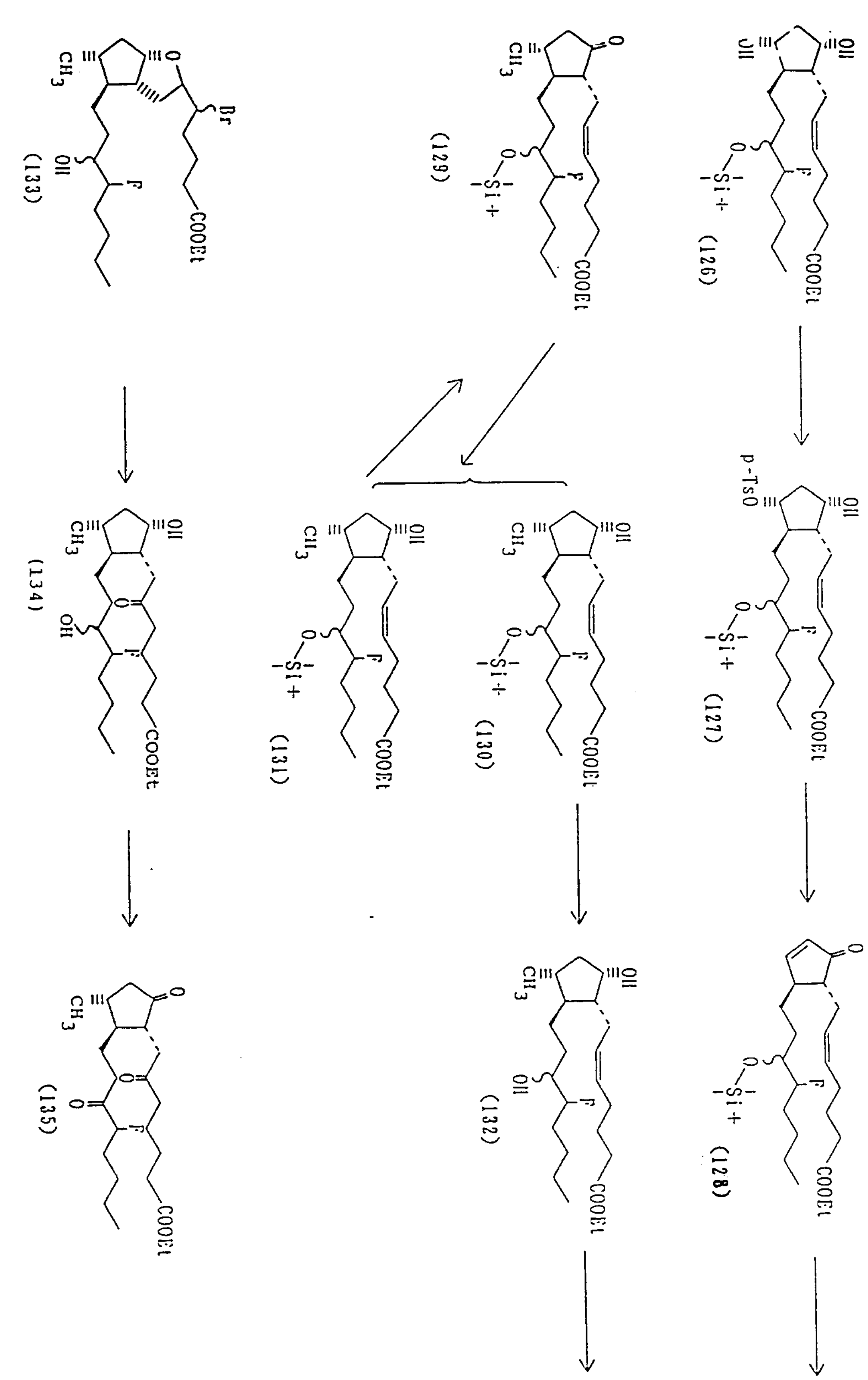

(126) (127) (128) (129) (130) (131) (132) (133) (134) (135)

Chart XX

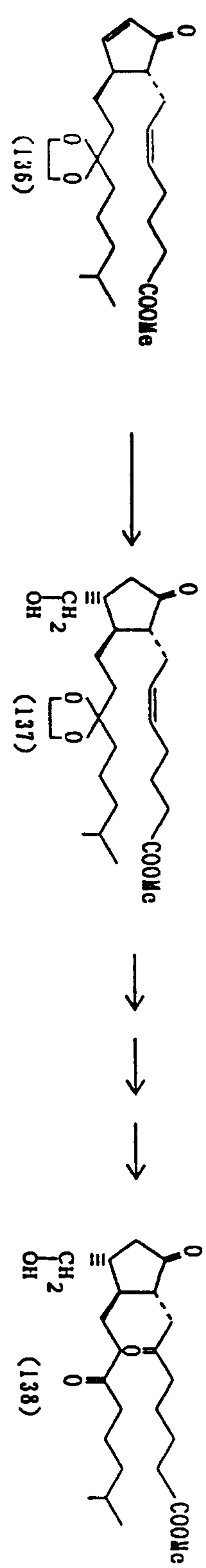

(136)

(137)

(138)

Chart XXI

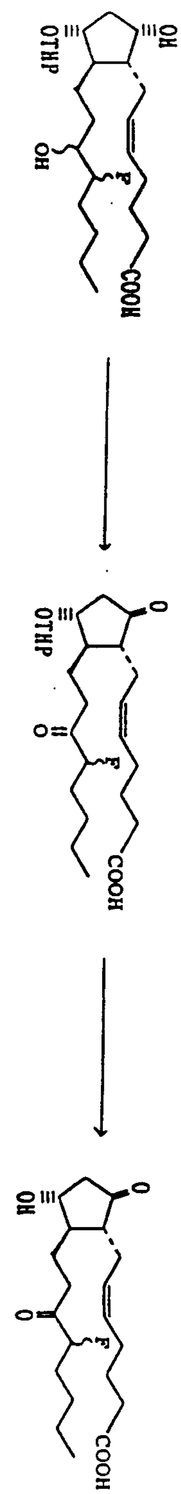

(122)

(139)

(140)

Chart X XII

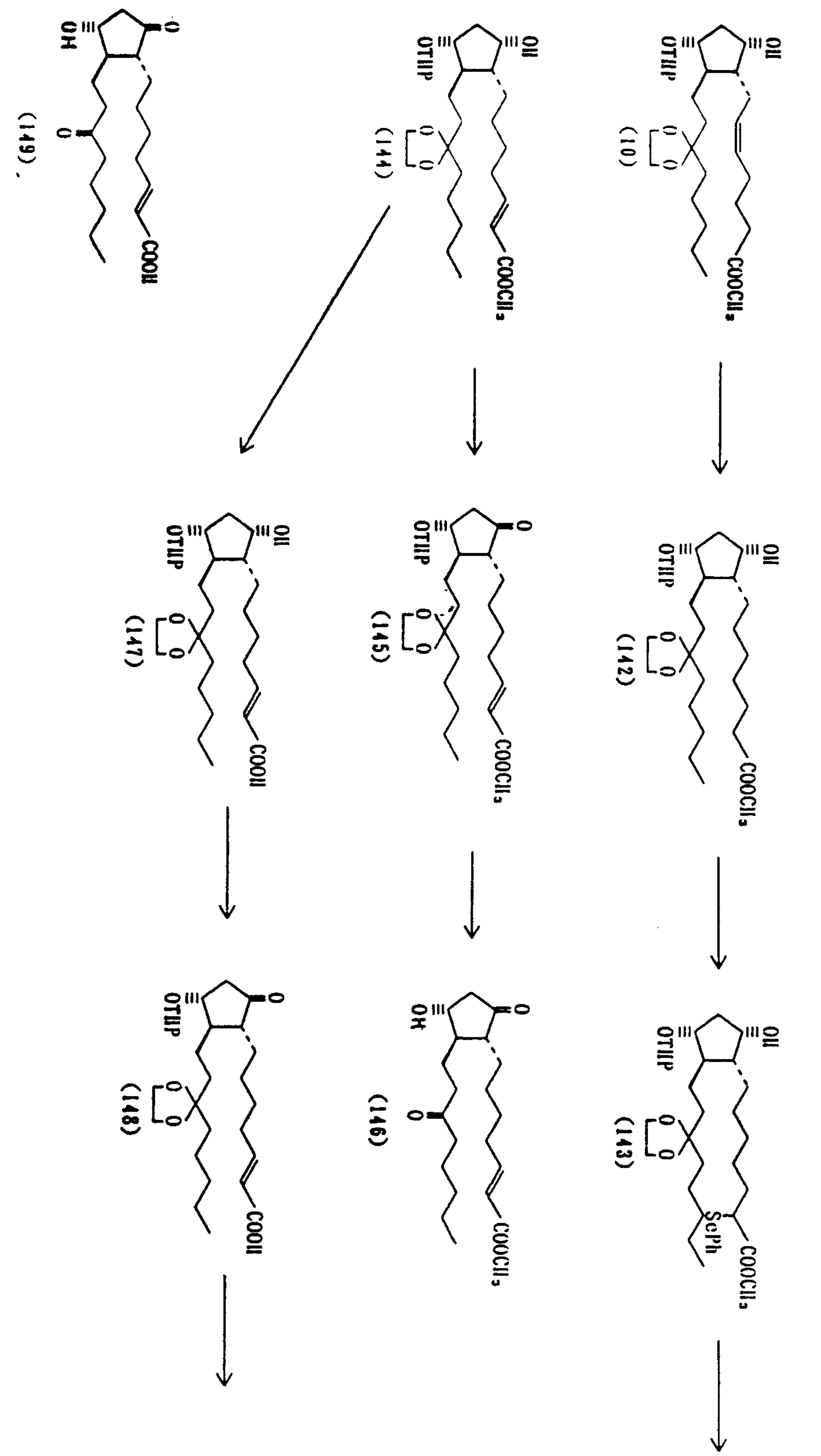

(10) (142) (143) (144) (145) (146) (147) (148) (149).

Chart X XIII

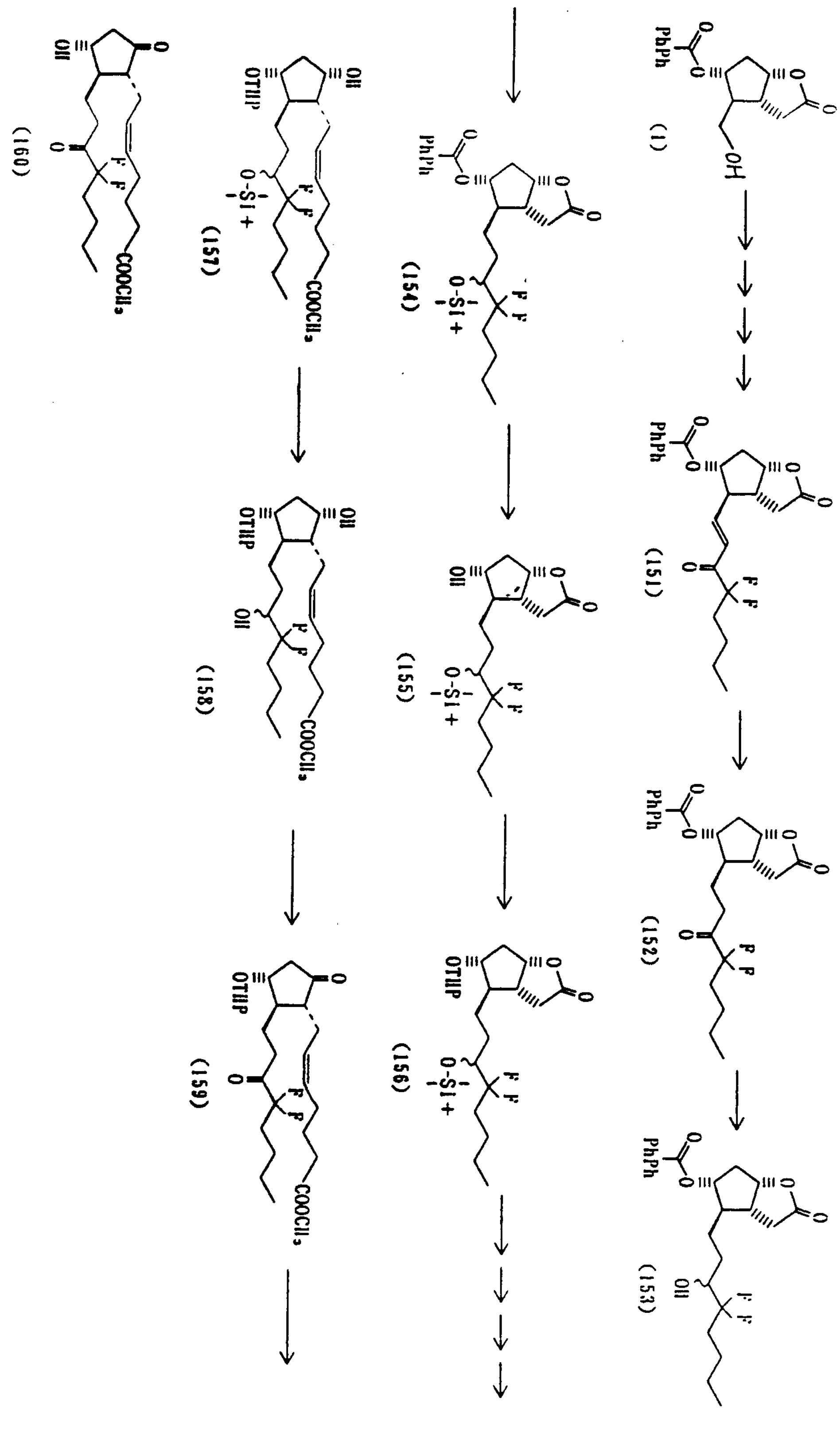

(1) (151) (152) (153) (154) (155) (156) (157) (158) (159) (160)

Chart XXIV

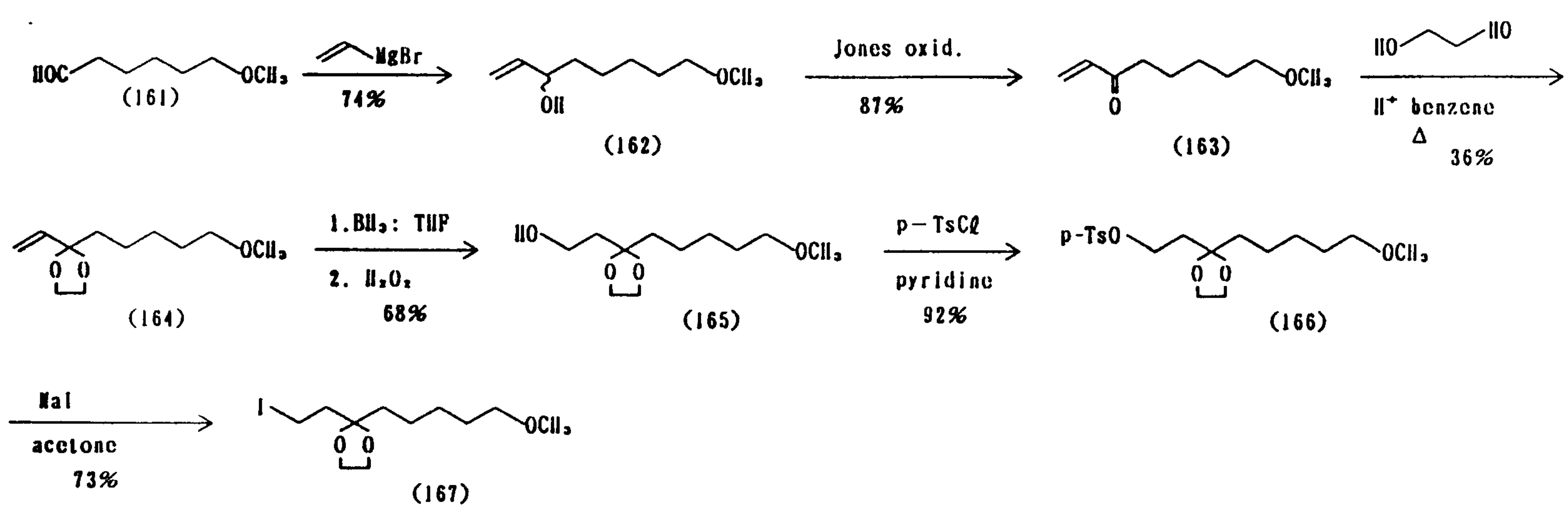


(161) (162) (163) (164) (165) (166) (167)

Chart XXV

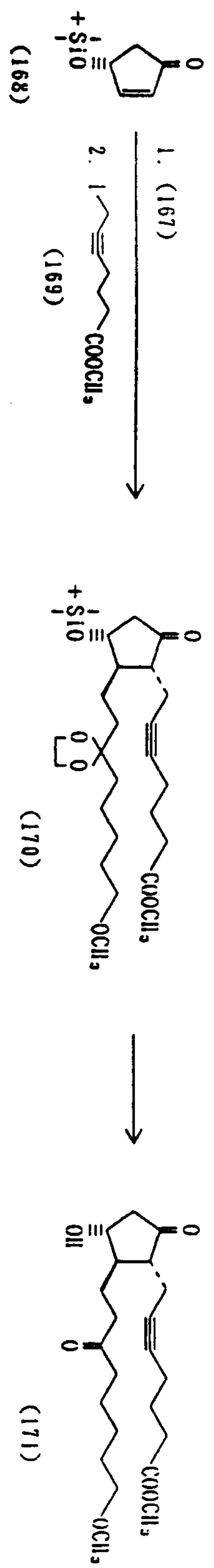


(168)

(169)

(170)

(171)

## Claims

**Claims for the following Contracting States : AT, BE, CH, DE, FR, GB, IT, LI, LU, NL, SE**

**1.** Prostaglandins E represented by a general formula:

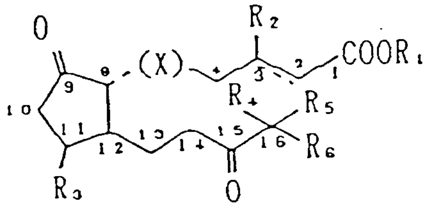

(I)

(in which X represents:

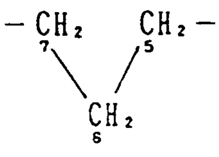 , 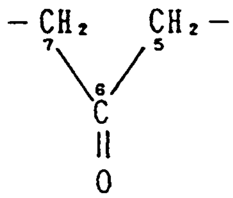 , 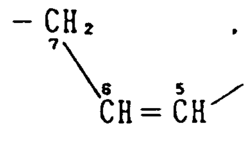 , 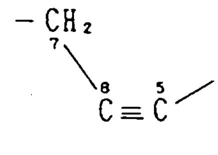

| | |
|---|---|
| $R_1$ | represents : hydrogen atom, physiologically acceptable salts, physiologically acceptable protective group, $C_1$-$C_4$ alkyl, benzyl, hydroxyalkyl; |
| $R_2$ | represents : hydrogen atom or a methyl group; |
| $R_3$ | represents : a hydroxyl, or hydroxymethyl group; |
| $R_4$ and $R_5$ | are the same or different, and signify a hydrogen atom, a methyl group, hydroxy group, or halogen atom, subject to the provisos that<br>(I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or<br>(II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond. |

**2.** Prostaglandins E as described in claim 1, wherein $R_4$ and/or $R_5$ is a halogen.

**3.** Prostaglandins E as described in claim 1, wherein $R_4$ and/or $R_5$ is a fluorine atom.

**4.** Prostaglandins E as described in claim 1, wherein $R_4$ and/or $R_5$ is a methyl group.

**5.** Prostaglandins E as described in claim 1, having a methyl group on 19 position thereof.

**6.** Prostaglandins E as described in claim 1, wherein $R_6$ is a hexyl group.

**7.** Prostaglandins E as described in claim 1, wherein $R_6$ is an isopentyl group.

**8.** Prostaglandins E as described in claim 1, wherein $R_6$ is a pentyl-2S-group.

**9.** Prostaglandins E as described in claim 1, of which carboxyl group on the terminal position of $\alpha$-chain is esterified with alkyl group.

**10.** Prostaglandins E as described in claim 1, which is 13,14-dihydro-15-keto-PGE having methyl group or fluorine atom on 16-position or alkyl ester thereof.

**11.** Prostaglandins E as described in claim 1 which is 13,14-dihydro-15-keto-16R,S-methyl-$PGE_2$ or alkyl ester thereof.

**12.** Prostaglandins E as described in claim 1 being 13,14-dihydro-6,15-diketo-16R,S-methyl-$PGE_1$ or

alkylester thereof.

**13.** Prostaglandins E as described in claim 1 being 13,14-dihydro-15-keto-16R,S-fluoro-$PGE_2$ or alkyl, ester thereof.

**14.** Prostaglandins E as described in claim 1 being 13,14-dihydro-6,15-diketo-16R,S-fluoro-$PGE_1$ or alkyl ester thereof.

**15.** Prostaglandins E as described in claim 1 being 13,14-dihydro-15-keto-19-methyl-$PGE_2$ or alkyl ester thereof.

**16.** Prostaglandins E as described in claim 1 being 13,14-dihydro-6,15-diketo-19-methyl-$PGE_1$ or alkyl ester thereof.

**17.** Prostaglandins E as described in claim 1 being 13,14-dihydro-15-keto-20-ethyl-$PGE_2$ or alkyl ester thereof.

**18.** Prostaglandins E as described in claim 1 being 13,14-dihydro-15-keto-16,16-difluoro-$PGE_2$ or alkyl ester thereof.

**19.** Prostaglandins E as described in claim 1 being 13,14-dihydro-15-keto-20-methyl-$PGE_1$ or alkyl ester thereof.

**20.** Prostaglandins E as described in claim 1 being 13,14-dihydro-15-keto-$\Delta^2$-$PGE_1$ or alkyl ester thereof.

**21.** Prostaglandins E as described in claim 1 being 13,14-dihydro-15-keto-16R,S-fluoro-20-methyl-$PGE_2$ or alkyl ester thereof.

**22.** Prostaglandins E as described in claim 1 being 13,14-dihydro-15-keto-5,6-dehydro-20-methoxy-$PGE_2$ or alkyl ester thereof.

**23.** The use of prostaglandins E expressed by a general formula:

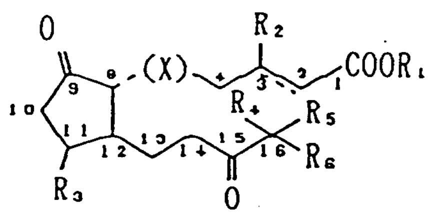

(I)

(in which x represents:

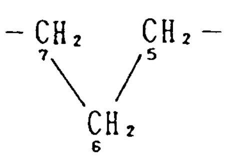 , 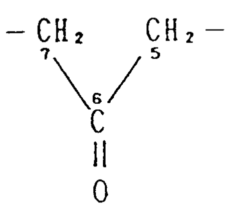 , 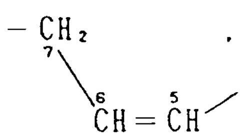 , 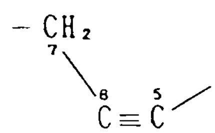

| | |
|---|---|
| $R_1$ | represents: hydrogen atom, physiologically acceptable salts, physiologically acceptable protective group, $C_1$-$C_4$ alkyl, benzyl, hydroxyalkyl; |
| $R_2$ | represents: hydrogen atom or a methyl group; |
| $R_3$ | represents: a hydroxyl, or hydroxymethyl group; |
| $R_4$ and $R_5$ | are the same or different, and signify a hydrogen atom, a methyl group, hydroxy group, or halogen atom, subject to the provisos that |

(I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or

(II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond,

for the manufacture of an anti-ulcer composition.

**24.** The use as described in claim 23 wherein $R_4$ and/or $R_5$ is a halogen.

**25.** The use as described in claim 23 wherein $R_4$ and/or $R_5$ is a fluorine atom.

**26.** The use as described in claim 23 wherein $R_4$ and/or $R_5$ is a methyl group.

**27.** The use as described in claim 23 wherein the prostaglandin E has a methyl group on 19-position.

**28.** The use as described in claim 23 wherein $R_6$ is a hexyl group.

**29.** The use as described in claim 23 wherein $R_6$ is an isopentyl group.

**30.** The use as described in claim 23 wherein $R_6$ is a pentyl-2S-group.

**31.** The use as described in claim 23 wherein the prostaglandines E of which carboxyl group on the terminal position of $\alpha$-chain is esterified with alkyl group are contained.

**32.** The use as described in claim 23 wherein the prostaglandins E are 13,14-dihydro-15-keto-PGEs having a methyl group or fluorine atom on 16-position or alkyl ester thereof.

**33.** The use as described in claim 23 wherein the prostaglandin E is 13,14-dihydro-15-keto-16R,S-methyl-$PGE_2$ alkyl ester.

**34.** The use as described in claim 23 wherein the prostaglandin E is 13,14-dihydro-6,15-diketo-16R,S-methyl-$PGE_1$-alkyl ester.

**35.** The use as described in claim 23 wherein the prostaglandin E is 13,14-dihydro-15-keto-16R,S-fluoro-$PGE_2$ or alkyl ester thereof.

**36.** The use as described in claim 23 wherein the prostaglandin E is 13,14-dihydro-6,15-diketo-16R,S-fluoro-$PGE_1$ or alkyl ester thereof.

**37.** The use as described in claim 23 wherein the prostaglandin E is 13,14-dihydro-15-keto-19-methyl-$PGE_2$ or alkyl ester thereof.

**38.** The use as described in claim 23 wherein the prostaglandin E is 13,14-dihydro-6,15-diketo-19-methyl-$PGE_2$ or alkyl ester thereof.

**39.** The use as described in claim 23 wherein the prostaglandin E is 13,14-dihydro-15-keto-20-ethyl-$PGE_2$ or alkyl ester thereof.

**40.** The use as described in claim 23 wherein the prostaglandin E is 13,14-dihydro-15-keto-16,16-difluoro-$PGE_2$ or alkyl ester thereof.

**41.** The use as described in claim 23 wherein the prostaglandin E is 13,14-dihydro-15-keto-20-methyl-$PGE_1$ or alkyl ester thereof.

**42.** The use as described in claim 23 wherein the prostaglandin E is 13,14-dihydro-15-keto-$\Delta^2$-$PGE_1$ or alkyl ester thereof.

**43.** The use as described in claim 23 wherein the prostaglandin E is 13,14-dihydro-15-keto-16R,S-fluoro-20-methyl-$PGE_2$ or alkyl ester thereof.

**44.** The use as described in claim 23 wherein the prostaglandin E is 13,14-dihydro-15-keto-5,6-dehydro-20-methoxy-$PGE_2$ or alkyl ester thereof.

**Claims for the following Contracting State : ES**

**1.** A method of preparing Prostaglandins E represented by a general formula:

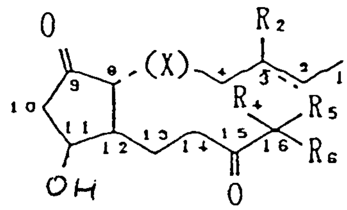

(I)

(in which X represents:

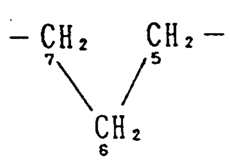
,
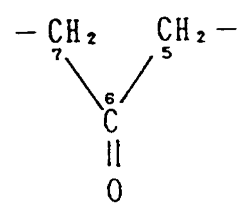
,
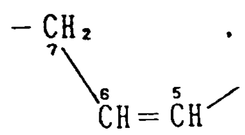
,
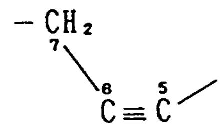

| | |
|---|---|
| $R_1$ | represents : hydrogen atom, physiologically acceptable salts, physiologically acceptable protective group, $C_1$-$C_4$ alkyl, benzyl, hydroxyalkyl; |
| $R_2$ | represents : hydrogen atom or a methyl group; |
| $R_4$ and $R_5$ | are the same or different, and signify a hydrogen atom, a methyl group, hydroxy group, or halogen atom, subject to the provisos that (I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or (II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond; |

the method comprising hydrolysing the compound represented by the formula:

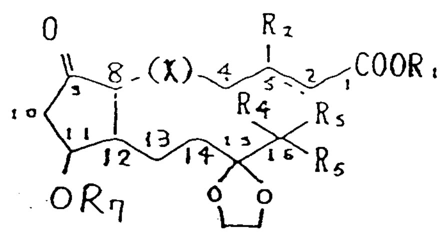

wherein the $R_7$ is tetrahydropyranyl group or t-butyldimethylsilyl group, and X, $R_1$, $R_2$, $R_4$, $R_5$ and $R_6$ are the same as the above.

2. A method of preparing prostaglandins E represented by the following formula:

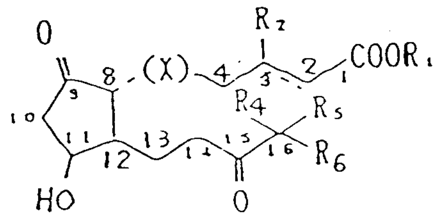

in which X, $R_1$, $R_2$, $R_4$, $R_5$ and $R_6$ are the same as defined in claim 1, which comprises hydrolyzing a compound represented by the formula:

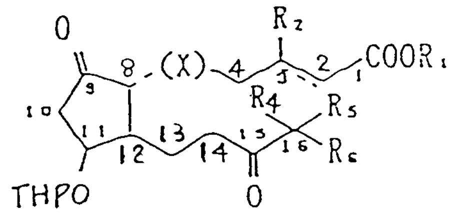

wherein the $R_1$, $R_2$, $R_4$, $R_5$ and $R_6$ are the same as the above, and THP means a tetrahydropyranyl group.

3. A method as described in claim 1, or 2, wherein $R_4$ and/or $R_5$ is a halogen.

4. A method as described in claim 1, or 2, wherein $R_4$ and/or $R_5$ is a fluorine atom.

5. A method as described in claim 1, or 2, wherein $R_4$ and/or $R_5$ is a methyl group.

6. A method as described in claim 1, or 2, having a methyl group on 19 position thereof.

7. A method as described in claim 1, or 2, wherein $R_6$ is a hexyl group.

8. A method as described in claim 1, or 2, wherein $R_6$ is an isopentyl group.

9. A method as described in claim 1, or 2, wherein $R_6$ is a pentyl-2S-group.

10. A method as described in claim 1, or 2, wherein the carboxyl group on the terminal position of α-chain is esterified with alkyl group.

11. A method as described in claim 1, or 2 for producing a 13,14-dihydro-15-keto-PGE having methyl group or fluorine atom on 16-position or alkyl ester thereof.

12. A method as described in claim 1 or 2, for producing a 13,14-dihydro-15-keto-16R,S-methyl-$PGE_2$ or alkyl ester thereof.

13. A method as described in claim 1 or 2, for producing a 13,14-dihydro-6,15-diketo-16R,S-methyl-$PGE_1$ or alkylester thereof.

14. A method as described in claim 1 or 2, for producing a 13,14-dihydro-15-keto-16R,S-fluoro-$PGE_2$ or alkyl ester thereof.

15. A method as described in claim 1 or 2, for producing a 13,14-dihydro-6,15-diketo-16R,S-fluoro-$PGE_1$ or

alkyl ester thereof.

**16.** A method as described in claim 1 or 2, for producing a 13,14-dihydro-15-keto-19-methyl-$PGE_2$ or alkyl ester thereof.

**17.** A method as described in claim 1 or 2, for producing a 13,14-dihydro-6,15-diketo-19-methyl-$PGE_1$ or alkyl ester thereof.

**18.** A method as described in claim 1 or 2, for producing a 13,14-dihydro-15-keto-20-ethyl-$PGE_2$ or alkyl ester thereof.

**19.** A method as described in claim 1 or 2, for producing a 13,14-dihydro-15-keto-16,16-difluoro-$PGE_2$ or alkyl ester thereof.

**20.** A method as described in claim 1 or 2, for producing a 13,14-dihydro-15-keto-20-methyl-$PGE_1$ or alkyl ester thereof.

**21.** A method as described in claim 1 or 2, for producing a 13,14-dihydro-15-keto-$\Delta^2$-$PGE_1$ or alkyl ester thereof.

**22.** A method as described in claim 1 or 2, for producing a 13,14-dihydro-15-keto-16R,S-fluoro-20-methyl-$PGE_2$ or alkyl ester thereof.

**23.** A method as described in claim 1 or 2, for producing a 13,14-dihydro-15-keto-5,6-dehydro-20-methoxy-$PGE_2$ or alkyl ester thereof.

**24.** A method for producing an antiulcer composition comprising admixing a prostaglandins E expressed by a general formula:

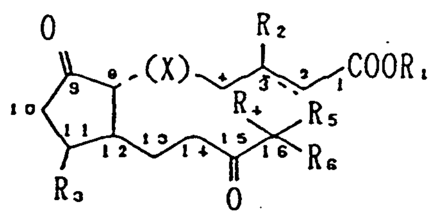

(I)

(in which X represents:

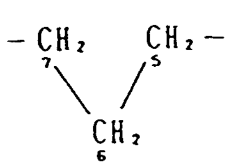
.
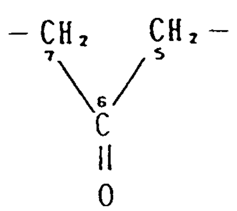
.
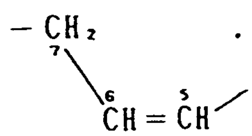
.
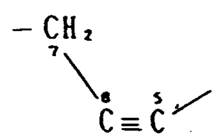

| | |
|---|---|
| $R_1$ | represents: hydrogen atom, physiologically acceptable salts, physiologically acceptable protective group, $C_1$-$C_4$ alkyl, benzyl, hydroxyalkyl; |
| $R_2$ | represents: hydrogen atom or a methyl group; |
| $R_3$ | represents: a hydroxyl, or hydroxymethyl group; |
| $R_4$ and $R_5$ | are the same or different, and signify a hydrogen atom, a methyl group, hydroxy group, or halogen atom, subject to the provisos that (I) when at least one of $R_4$ and $R_5$ is a methyl group, a hydroxy group or a halogen atom, $R_6$ is a $C_1$-$C_9$ alkyl group which may have a branch or a double bond, or $C_1$- |

$C_9$ alkyl group having an alkoxy-substituent group; or
(II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond; and
a pharmaceutically acceptable excipient.

**25.** The method as described in claim 24 wherein $R_4$ and/or $R_5$ is a halogen.

**26.** The use as described in claim 24 wherein $R_4$ and/or $R_5$ is a fluorine atom.

**27.** The use as described in claim 24 wherein $R_4$ and/or $R_5$ is a methyl group.

**28.** The method as described in claim 24 wherein the prostaglandin E has a methyl group on 19-position.

**29.** The method as described in claim 24 wherein $R_6$ is a hexyl group.

**30.** The method as described in claim 24 wherein $R_6$ is an isopentyl group.

**31.** The method as described in claim 24 wherein $R_6$ is a pentyl-2S-group.

**32.** The method as described in claim 24 wherein the prostaglandines E of which carboxyl group on the terminal position of $\alpha$-chain is esterified with alkyl group are contained.

**33.** The method as described in claim 24 wherein the prostaglandins E are 13,14-dihydro-15-keto-PGEs having a methyl group or fluorine atom on 16-position or alkyl ester thereof.

**34.** The method as described in claim 24 wherein the prostaglandin E is 13,14-dihydro-15-keto-16R,S-methyl-$PGE_2$ alkyl ester.

**35.** The method as described in claim 24 wherein the prostaglandin E is 13,14-dihydro-6,15-diketo-16R,S-methyl-$PGE_1$-alkyl ester.

**36.** The method as described in claim 24 wherein the prostaglandin E is 13,14-dihydro-15-keto-16R,S-fluoro-$PGE_2$ or alkyl ester thereof.

**37.** The method as described in claim 24 wherein the prostaglandin E is 13,14-dihydro-6,15-diketo-16R,S-fluoro-$PGE_1$ or alkyl ester thereof.

**38.** The method as described in claim 24 wherein the prostaglandin E is 13,14-dihydro-15-keto-19-methyl-$PGE_2$ or alkyl ester thereof.

**39.** The method as described in claim 24 wherein the prostaglandin E is 13,14-dihydro-6,15-diketo-19-methyl-$PGE_2$ or alkyl ester thereof.

**40.** The method as described in claim 24 wherein the prostaglandin E is 13,14-dihydro-15-keto-20-ethyl-$PGE_2$ or alkyl ester thereof.

**41.** The method as described in claim 24 wherein the prostaglandin E is 13,14-dihydro-15-keto-16,16-difluoro-$PGE_2$ or alkyl ester thereof.

**42.** The method as described in claim 24 wherein the prostaglandin E is 13,14-dihydro-15-keto-20-methyl-$PGE_1$ or alkyl ester thereof.

**43.** The method as described in claim 24 wherein the prostaglandin E is 13,14-dihydro-15-keto-$\Delta^2$-$PGE_1$ or alkyl ester thereof.

**44.** The method as described in claim 24 wherein the prostaglandin E is 13,14-dihydro-15-keto-16R,S-fluoro-20-methyl-$PGE_2$ or alkyl ester thereof.

**45.** The method as described in claim 24 wherein the prostaglandin E is 13,14-dihydro-15-keto-5,6-dehydro-20-methoxy-$PGE_2$ or alkyl ester thereof.

**Claims for the following Contracting State : GR**

**1.** Prostaglandins E represented by a general formula:

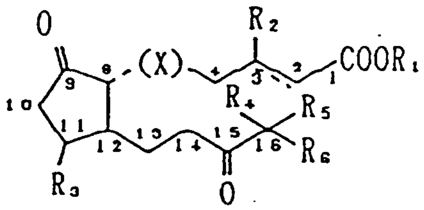

(I)

(in which X represents:

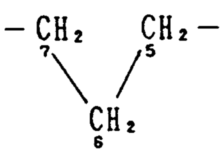 , 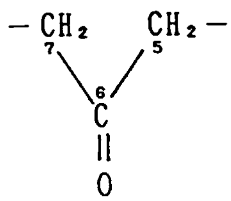 , 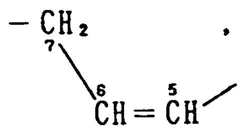 , 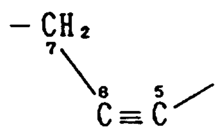

| | |
|---|---|
| $R_1$ | represents : hydrogen atom, physiologically acceptable salts, physiologically acceptable protective group, $C_1$-$C_4$ alkyl, benzyl, hydroxyalkyl; |
| $R_2$ | represents : hydrogen atom or a methyl group; |
| $R_3$ | represents : a hydroxyl, or hydroxymethyl group; |
| $R_4$ and $R_5$ | are the same or different, and signify a hydrogen atom, a methyl group, hydroxy group, or halogen atom, subject to the provisos that<br>(I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or<br>(II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond. |

**2.** Prostaglandins E as described in claim 1, wherein $R_4$ and/or $R_5$ is a halogen.

**3.** Prostaglandins E as described in claim 1, wherein $R_4$ and/or $R_5$ is a fluorine atom.

**4.** Prostaglandins E as described in claim 1, wherein $R_4$ and/or $R_5$ is a methyl group.

**5.** Prostaglandins E as described in claim 1, having a methyl group on 19 position thereof.

**6.** Prostaglandins E as described in claim 1, wherein $R_6$ is a hexyl group.

**7.** Prostaglandins E as described in claim 1, wherein $R_6$ is an isopentyl group.

**8.** Prostaglandins E as described in claim 1, wherein $R_6$ is a pentyl-2S-group.

**9.** Prostaglandins E as described in claim 1, of which carboxyl group on the terminal position of $\alpha$-chain is esterified with alkyl group.

**10.** Prostaglandins E as described in claim 1, which is 13,14-dihydro-15-keto-PGE having methyl group or fluorine atom on 16-position or alkyl ester thereof.

**11.** Prostaglandins E as described in claim 1 which is 13,14-dihydro-15-keto-16R,S-methyl-$PGE_2$ or alkyl ester thereof.

**12.** Prostaglandins E as described in claim 1 being 13,14-dihydro-6,15-diketo-16R,S-methyl-$PGE_1$ or alkylester thereof.

**13.** Prostaglandins E as described in claim 1 being 13,14-dihydro-15-keto-16R,S-fluoro-$PGE_2$ or alkyl ester thereof.

**14.** Prostaglandins E as described in claim 1 being 13,14-dihydro-6,15-diketo-16R,S-fluoro-$PGE_1$ or alkyl ester thereof.

**15.** Prostaglandins E as described in claim 1 being 13,14-dihydro-15-keto-19-methyl-$PGE_2$ or alkyl ester thereof.

**16.** Prostaglandins E as described in claim 1 being 13,14-dihydro-6,15-diketo-19-methyl-$PGE_1$ or alkyl ester thereof.

**17.** Prostaglandins E as described in claim 1 being 13,14-dihydro-15-keto-20-ethyl-$PGE_2$ or alkyl ester thereof.

**18.** Prostaglandins E as described in claim 1 being 13,14-dihydro-15-keto-16,16-difluoro-$PGE_2$ or alkyl ester thereof.

**19.** Prostaglandins E as described in claim 1 being 13,14-dihydro-15-keto-20-methyl-$PGE_1$ or alkyl ester thereof.

**20.** Prostaglandins E as described in claim 1 being 13,14-dihydro-15-keto-$\Delta^2$-$PGE_1$ or alkyl ester thereof.

**21.** Prostaglandins E as described in claim 1 being 13,14-dihydro-15-keto-16R,S-fluoro-20-methyl-$PGE_2$ or alkyl ester thereof.

**22.** Prostaglandins E as described in claim 1 being 13,14-dihydro-15-keto-5,6-dehydro-20-methoxy-$PGE_2$ or alkyl ester thereof.

**23.** A method of producing an antiulcer composition comprising admixing a prostaglandin E expressed by a general formula:

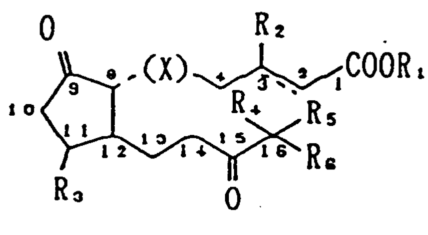

(I)

(in which x represents:

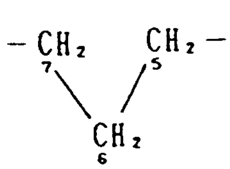 . 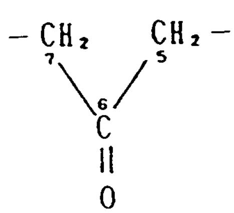 . 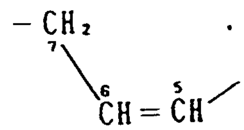 . 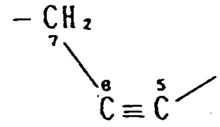

| | |
|---|---|
| $R_1$ | represents: hydrogen atom, physiologically acceptable salts, physiologically acceptable protective group, $C_1$-$C_4$ alkyl, benzyl, hydroxyalkyl; |
| $R_2$ | represents: hydrogen atom or a methyl group; |
| $R_3$ | represents: a hydroxyl, or hydroxymethyl group; |
| $R_4$ and $R_5$ | are the same or different, and signify a hydrogen atom, a methyl group, hydroxy group, or halogen atom, subject to the provisos that (I) when at least one of $R_4$ and $R_5$ is a methyl group, a hydroxy group or a halogen atom, $R_6$ is a $C_1$-$C_9$ alkyl group which may have a branch or a double bond, or $C_1$-$C_9$ alkyl group having an alkoxy-substituent group; or (II) when $R_4$ and $R_5$ are both hydrogen atoms, $R_6$ is a $C_5$-$C_9$ alkyl group which may have a branch or a double bond, or $C_1$-$C_9$ alkyl group having an alkoxy-substituent group, in which $C_2$-$C_3$ bond may be a double bond; and |

a pharmaceutically acceptable excipient.

**24.** The method as described in claim 23 wherein $R_4$ and/or $R_5$ is a halogen.

**25.** The method as described in claim 23 wherein $R_4$ and/or $R_5$ is a fluorine atom.

**26.** The method as described in claim 23 wherein $R_4$ and/or $R_5$ is a methyl group.

**27.** The method as described in claim 23 wherein the prostaglandin E has a methyl group on 19-position.

**28.** The method as described in claim 23 wherein $R_6$ is a hexyl group.

**29.** The method as described in claim 23 wherein $R_6$ is an isopentyl group.

**30.** The method as described in claim 23 wherein $R_6$ is a pentyl-2S-group.

**31.** The method as described in claim 23 wherein the prostaglandines E of which carboxyl group on the terminal position of $\alpha$-chain is esterified with alkyl group are contained.

**32.** The method as described in claim 23 wherein the prostaglandins E are 13,14-dihydro-15-keto-PGEs having a methyl group or fluorine atom on 16-position or alkyl ester thereof.

**33.** The method as described in claim 23 wherein the prostaglandin E is 13,14-dihydro-15-keto-16R,S-methyl-$PGE_2$ alkyl ester.

**34.** The method as described in claim 23 wherein the prostaglandin E is 13,14-dihydro-6,15-diketo-16R,S-methyl-$PGE_1$-alkyl ester.

**35.** The method as described in claim 23 wherein the prostaglandin E is 13,14-dihydro-15-keto-16R,S-fluoro-$PGE_2$ or alkyl ester thereof.

**36.** The method as described in claim 23 wherein the prostaglandin E is 13,14-dihydro-6,15-diketo-16R,S-fluoro-$PGE_1$ or alkyl ester thereof.

**37.** The method as described in claim 23 wherein the prostaglandin E is 13,14-dihydro-15-keto-19-methyl-$PGE_2$ or alkyl ester thereof.

**38.** The method as described in claim 23 wherein the prostaglandin E is 13,14-dihydro-6,15-diketo-19-methyl-$PGE_2$ or alkyl ester thereof.

**39.** The method as described in claim 23 wherein the prostaglandin E is 13,14-dihydro-15-keto-20-ethyl-$PGE_2$ or alkyl ester thereof.

**40.** The method as described in claim 23 wherein the prostaglandin E is 13,14-dihydro-15-keto-16,16-difluoro-$PGE_2$ or alkyl ester thereof.

**41.** The method as described in claim 23 wherein the prostaglandin E is 13,14-dihydro-15-keto-20-methyl-$PGE_1$ or alkyl ester thereof.

**42.** The method as described in claim 23 wherein the prostaglandin E is 13,14-dihydro-15-keto-$\Delta^2$-$PGE_1$ or alkyl ester thereof.

**43.** The method as described in claim 23 wherein the prostaglandin E is 13,14-dihydro-15-keto-16R,S-fluoro-20-methyl-$PGE_2$ or alkyl ester thereof.

**44.** The method as described in claim 23 wherein the prostaglandin E is 13,14-dihydro-15-keto-5,6-dehydro-20-methoxy-$PGE_2$ or alkyl ester thereof.

**Patentansprüche**
**Patentansprüche für folgende Vertragsstaaten : AT, BE, CH, DE, FR, GB, IT, LI, LU, NL, SE**

**1.** Prostaglandine E, dargestellt durch die allgemeine Formel

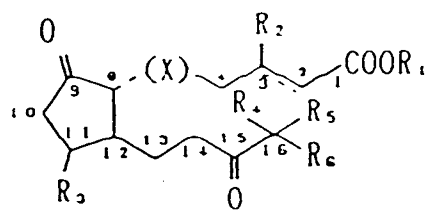

(I)

worin X

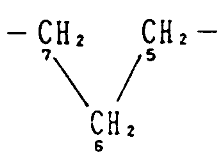 , 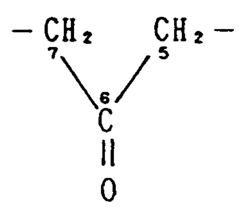 , 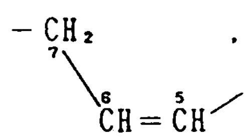 , 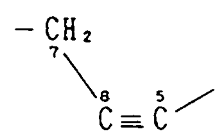

bedeutet;
$R_1$ ein Wasserstoffatom, physiologisch annehmbare Salze, physiologisch annehmbare Schutzgruppen, $C_1$-$C_4$-Alkyl, Benzyl, Hydroxyalkyl bedeutet;
$R_2$ ein Wasserstoffatom oder eine Methylgruppe bedeutet;
$R_3$ eine Hydroxyl- oder Hydroxymethylgruppe bedeutet;
$R_4$ und $R_5$ gleich oder unterschiedlich sind und ein Wasserstoffatom, eine Methylgruppe, eine Hydroxygruppe oder ein Halogenatom bedeuten, mit der Maßgabe, daß

(I) wenn in mindestens einer der Substituenten $R_4$ und $R_5$ eine Methylgruppe, eine Hydroxygruppe oder ein Halogen bedeutet, $R_6$ eine $C_1$-$C_9$-Alkylgruppe, die eine Verzweigung oder Doppelbindung aufweisen kann oder eine $C_1$-$C_9$-Alkylgruppe, die eine Alkoxysubstituentengruppe aufweist, bedeutet; oder

(II) wenn $R_4$ und $R_5$ beide Wasserstoffatome bedeuten, $R_6$ eine $C_5$-$C_9$-Alkylgruppe, die eine

verzweigung oder Doppelbindung aufweisen kann oder eine $C_1$-$C_9$-Alkylgruppe, die eine Alkoxysubstituentengruppe aufweisen kann, wobei die $C_2$-$C_3$-Bindung eine Doppelbindung sein kann, bedeutet.

**2.** Prostaglandine E nach Anspruch 1, dadurch **gekennzeichnet,** daß $R_4$ und/oder $R_5$ ein Halogen bedeuten.

**3.** Prostaglandine E nach Anspruch 1, dadurch **gekennzeichnet,** daß $R_4$ und/oder $R_5$ ein Fluoratom bedeuten.

**4.** Prostaglandine E nach Anspruch 1, dadurch **gekennzeichnet,** daß $R_4$ und/oder $R_5$ eine Methylgruppe bedeuten.

**5.** Prostaglandine E nach Anspruch 1, dadurch **gekennzeichnet,** daß sie eine Methylgruppe an ihrer 19-Stellung aufweisen.

**6.** Prostaglandine E nach Anspruch 1, dadurch **gekennzeichnet,** daß $R_6$ eine Hexylgruppe bedeutet.

**7.** Prostaglandine E nach Anspruch 1 dadurch **gekennzeichnet,** daß $R_6$ eine Isopentylgruppe bedeutet.

**8.** Prostaglandine E nach Anspruch 1, dadurch **gekennzeichnet,** daß $R_6$ eine Pentyl-2S-Gruppe bedeutet.

**9.** Prostaglandine E nach Anspruch 1, dadurch **gekennzeichnet,** daß die Carboxylgruppe an der Endstellung der $\alpha$-Kette mit einer Alkylgruppe verestert ist.

**10.** Prostaglandine E nach Anspruch 1, nämlich 13,14-Dihydro-15-keto-PGE mit einer Methylgruppe oder einem Fluoratom an der 16-Stellung oder ein Alkylester davon.

**11.** Prostaglandine E nach Anspruch 1, nämlich 13-14-Dihydro-15-keto-16R,S-methyl-$PGE_2$ oder ein Alkylester davon.

**12.** Prostaglandine E nach Anspruch 1, nämlich 13,14-Dihydro-6,15-diketo-16R,S-methyl-$PGE_1$ oder ein Alkylester davon.

**13.** Prostaglandine E nach Anspruch 1, nämlich 13,14-Dihydro-15-keto-16R,16-fluoro-PGE2 oder ein Alkylester davon.

**14.** Prostaglandine E nach Anspruch 1, nämlich 13,14-Dihydro-6,15-diketo-16R,S-fluor-$PGE_1$ oder ein Alkylester davon.

**15.** Prostaglandine E nach Anspruch 1, nämlich 13,14-Dihydro-15-keto-19-methyl-$PGE_2$ oder ein Alkylester davon.

**16.** Prostaglandine E nach Anspruch 1, nämlich 13,14-Dihydro-6,15-diketo-19-methyl-$PGE_1$ oder ein Alkylester davon.

**17.** Prostaglandine E nach Anspruch 1, nämlich 13,14-Dihydro-15-keto-20-ethyl-$PGE_2$ oder ein Alkylester davon.

**18.** Prostaglandine E nach Anspruch 1, nämlich 13,14-Dihydro-15-keto-16,16-difluor-$PGE_2$ oder ein Alkylester davon.

**19.** Prostaglandine E nach Anspruch 1, nämlich 13,14-Dihydro-15-keto-20-methyl-$PGE_1$ oder ein Alkylester davon.

**20.** Prostaglandine E nach Anspruch 1, nämlich 13,14-Dihydro-15-keto-$\Delta^2$-$PGE_1$ oder ein Alklyester davon.

**21.** Prostaglandine E nach Anspruch 1, nämlich 13,14-Dihydro-15-keto-16R,S-fluor-20-methyl-$PGE_2$ oder ein Alkylester davon.

**22.** Prostaglandine E nach Anspruch 1, nämlich 13,14-Dihydro-15-keto-5,6-dehydro-20-methoxy-$PGE_2$ oder ein Alkylester davon.

**23.** Die Verwendung von Prostaglandinen E, dargestellt durch die allgemeine Formel

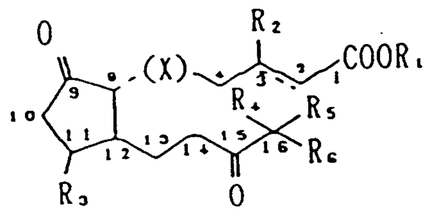

(I)

worin X

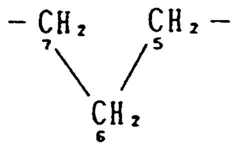 . 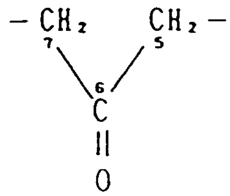 . 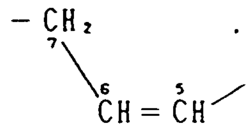 . 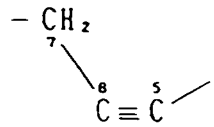

bedeutet;
$R_1$ ein Wasserstoffatom, physiologisch annehmbare Salze, physiologisch annehmbare Schutzgruppen, $C_1$-$C_4$-Alkyl, Benzyl, Hydroxyalkyl bedeutet;
$R_2$ ein Wasserstoffatom oder eine Methylgruppe bedeutet;
$R_3$ eine Hydroxyl- oder Hydroxymethylgruppe bedeutet;
$R_4$ und $R_5$ gleich oder unterschiedlich sind und ein Wasserstoffatom, eine Methylgruppe, eine Hydroxygruppe oder ein Halogenatom bedeuten, mit der Maßgabe, daß

(I) wenn in mindestens einer der Substituenten $R_4$ und $R_5$ eine Methylgruppe, eine Hydroxygruppe oder ein Halogen bedeutet, $R_6$ eine $C_1$-$C_9$-Alkylgruppe, die eine Verzweigung oder Doppelbindung aufweisen kann oder eine $C_1$-$C_9$-Alkylgruppe, die eine Alkoxysubstituentengruppe aufweist, bedeutet; oder

(II) wenn $R_4$ und $R_5$ beide Wasserstoffatome bedeuten, $R_6$ eine $C_5$-$C_9$-Alkylgruppe, die eine Verzweigung oder Doppelbindung aufweisen kann oder eine $C_1$-$C_9$-Alkylgruppe, die eine Alkoxysubstituentengruppe aufweisen kann, wobei die $C_2$-$C_3$-Bindung eine Doppelbindung sein kann, bedeutet,

für die Herstellung eines Anti-Ulcer-Mittels.

**24.** Die Verwendung nach Anspruch 23, worin $R_4$ und/oder $R_5$ ein Halogenatom bedeuten.

**25.** Die Verwendung nach Anspruch 23, worin $R_4$ und/oder $R_5$ ein Fluoratom bedeuten.

**26.** Die Verwendung nach Anspruch 23, worin $R_4$ und/oder $R_5$ eine Methylgruppe bedeuten.

**27.** Die Verwendung nach Anspruch 23, worin das Prostaglandin E eine Methylgruppe in der 19-Stellung aufweist.

**28.** Die Verwendung nach Anspruch 23, worin $R_6$ eine Hexylgruppe bedeutet.

**29.** Die Verwendung nach Anspruch 23, worin $R_6$ eine Isopentylgruppe bedeutet.

**30.** Die Verwendung nach Anspruch 23, worin $R_6$ eine Pentyl-2S-Gruppe bedeutet.

**31.** Die Verwendung nach Anspruch 23, wobei die Prostaglandine E, deren Carboxylgruppe an der Endstellung der $\alpha$-Kette mit einer Alkylgruppe verestert ist, verwendet werden.

**32.** Die Verwendung nach Anspruch 23, wobei die Prostaglandine E 13,14-Dihydro-15-keto-PGEs mit einer Methylgruppe oder einem Fluoratom an der 16-Stellung oder die Alkylester davon sind.

**33.** Die Verwendung nach Anspruch 23, wobei das Prostaglandin E ein 13,14-Dihydro-15-keto-16R,S-methyl-$PGE_2$-alkylester ist.

**34.** Die Verwendung nach Anspruch 23, wobei das Prostaglandin E ein 13,14-Dihydro-6,15-diketo-16R,S-methyl-$PGE_1$-alkylester ist.

**35.** Die Verwendung nach Anspruch 23, wobei das Prostaglandin E ein 13,14-Dihydro-15-keto-16R,S-fluoro-$PGE_2$ oder ein Alkylester davon ist.

**36.** Die Verwendung nach Anspruch 23, wobei das Prostaglandin E ein 13,14-Dihydro-6,15-diketo-16R,S-fluor-$PGE_1$ oder ein Alkylester davon ist.

**37.** Die Verwendung nach Anspruch 23, wobei das Prostaglandin E ein 13,14-Dihydro-15-keto-19-methyl-$PGE_2$ oder ein Alkylester davon ist.

**38.** Die Verwendung nach Anspruch 23, wobei das Prostaglandin E ein 13,14-Dihydro-6,15-diketo-19-methyl-$PGE_2$ oder ein Alkylester davon ist.

**39.** Die Verwendung nach Anspruch 23, wobei das Prostaglandin E ein 13,14-Dihydro-15-keto-20-ethyl-$PGE_2$ oder ein Alkylester davon ist.

**40.** Die Verwendung nach Anspruch 23, wobei das Prostaglandin E ein 13,14-Dihydro-15-keto-16,16-difluor-$PGE_2$ oder ein Alkylester davon ist.

**41.** Die Verwendung nach Anspruch 23, wobei das Prostaglandin E ein 13,14-Dihydro-15-keto-20-methyl-$PGE_1$ oder ein Alkylester davon ist.

**42.** Die Verwendung nach Anspruch 23, wobei das Prostaglandin E ein 13,14-Dihydro-15-keto-$\Delta^2$-$PGE_1$ oder ein Alkylester davon ist.

**43.** Die Verwendung nach Anspruch 23, wobei das Prostaglandin E ein 13,14-Dihydro-15-keto-16R,S-fluor-20-methyl-$PGE_2$ oder ein Alkylester davon ist.

**44.** Die Verwendung nach Anspruch 23, wobei das Prostaglandin E ein 13,14-Dihydro-15-keto-5,6-dehydro-20-methoxy-$PGE_2$ oder ein Alkylester davon ist.

**Patentansprüche für folgenden Vertragsstaat : ES**

**1.** Verfahren zur Herstellung von Prostaglandinen E, dargestellt durch die allgemeine Formel

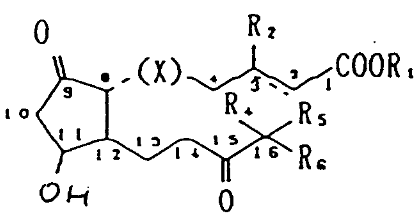

(I)

worin X

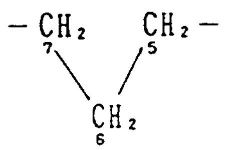 , 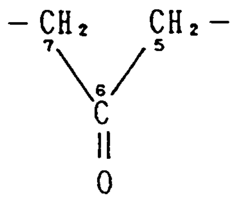 , 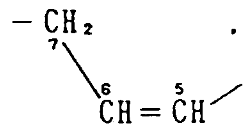 , 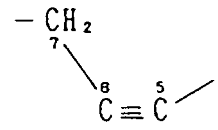

bedeutet;

$R_1$ ein Wasserstoffatom, physiologisch annehmbare Salze, physiologisch annehmbare Schutzgruppen, $C_1$-$C_4$-Alkyl, Benzyl, Hydroxyalkyl bedeutet;

$R_2$ ein Wasserstoffatom oder eine Methylgruppe bedeutet;

$R_4$ und $R_5$ gleich oder unterschiedlich sind und ein Wasserstoffatom, eine Methylgruppe, eine Hydroxygruppe oder ein Halogenatom bedeuten, mit der Maßgabe, daß

(I) wenn in mindestens einer der Substituenten $R_4$ und $R_5$ eine Methylgruppe, eine Hydroxygruppe oder ein Halogen bedeutet, $R_6$ eine $C_1$-$C_9$-Alkylgruppe, die eine Verzweigung oder Doppelbindung aufweisen kann oder eine $C_1$-$C_9$-Alkylgruppe, die eine Alkoxysubstituentengruppe aufweist, bedeutet; oder

(II) wenn $R_4$ und $R_5$ beide Wasserstoffatome bedeuten, $R_6$ eine $C_5$-$C_9$-Alkylgruppe, die eine Verzweigung oder Doppelbindung aufweisen kann oder eine $C_1$-$C_9$-Alkylgruppe, die eine Alkoxysubstituentengruppe aufweisen kann, wobei die $C_2$-$C_3$-Bindung eine Doppelbindung sein kann, bedeutet;

dadurch **gekennzeichnet,** daß eine Verbindung der Formel

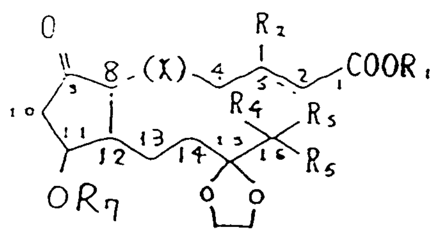

worin $R_7$ eine Tetrahydropyranylgruppe oder eine t-Butyldimethylsilylgruppe bedeutet, und X, $R_1$, $R_2$, $R_4$, $R_5$ und $R_6$ die gleiche Bedeutung wie oben angegeben besitzen, hydrolisiert wird.

**2.** Verfahren zur Herstellung von Prostaglandinen E, dargestellt durch die folgende Formel:

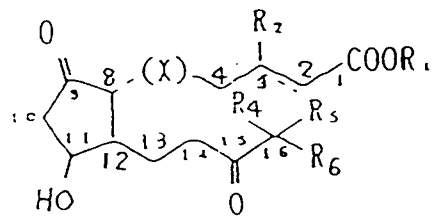

worin X, $R_1$, $R_2$, $R_4$, $R_5$ und $R_6$ die in Anspruch 1 gegebenen Definitionen besitzen, dadurch **gekennzeichnet,** daß eine Verbindung der Formel

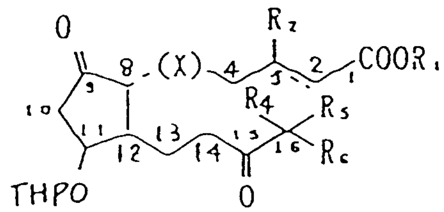

worin $R_1$, $R_2$, $R_4$, $R_5$ und $R_6$ die gleichen Bedeutungen wie oben besitzen und THP eine Tetrahydropyranylgruppe bedeutet, hydrolisiert wird.

3. Verfahren nach einem der Ansprüche 1 oder 2, dadurch **gekennzeichnet,** daß $R_4$ und/oder $R_5$ Halogen bedeuten.

4. Verfahren nach einem der Ansprüche 1 oder 2, dadurch **gekennzeichnet,** daß $R_4$ und/oder $R_5$ ein Fluoratom bedeuten.

5. Verfahren nach einem der Ansprüche 1 oder 2, dadurch **gekennzeichnet,** daß $R_4$ und/oder $R_5$ eine Methylgruppe bedeuten.

6. Verfahren nach einem der Ansprüche 1 oder 2, dadurch **gekennzeichnet,** daß in der 19-stellung eine Methylgruppe vorhanden ist.

7. Verfahren nach einem der Ansprüche 1 oder 2, dadurch **gekennzeichnet,** daß $R_6$ eine Hexylgruppe bedeutet.

8. Verfahren nach einem der Ansprüche 1 oder 2, dadurch **gekennzeichnet,** daß $R_6$ eine Isopentylgruppe bedeutet.

9. Verfahren nach einem der Ansprüche 1 oder 2, dadurch **gekennzeichnet,** daß $R_6$ eine Pentyl-2S-Gruppe bedeutet.

10. Verfahren nach einem der Ansprüche 1 oder 2, dadurch **gekennzeichnet,** daß die Carboxylgruppe an der Endstelle der α-Kette mit einer Alkylgruppe verestert ist.

11. Verfahren nach einem der Ansprüche 1 oder 2, dadurch **gekennzeichnet,** daß 13,14-Dihydro-15-keto-PGE, das eine Methylgruppe oder eine Fluroatom an der 16-Stellung aufweist, oder ein Alkylester davon hergestellt wird.

12. Verfahren nach einem der Ansprüche 1 oder 2, dadurch **gekennzeichnet,** daß 13,14-Dihydro-15-keto-16R,S-methyl-$PGE_2$ oder ein Alkylester davon hergestellt wird.

13. Verfahren nach einem der Ansprüche 1 oder 2, dadurch **gekennzeichnet,** daß 13,14-Dihydro-6,15-diketo-16R,S-methyl-$PGE_1$ oder ein Alkylester davon hergestellt wird.

14. Verfahren nach einem der Ansprüche 1 oder 2, dadurch **gekennzeichnet,** daß 13,14-Dihydro-15-keto-16R,S-fluor-$PGE_2$ oder ein Alkylester davon hergestellt wird.

15. Verfahren nach einem der Ansprüche 1 oder 2, dadurch **gekennzeichnet,** daß 13,14-Dihydro-6,15-diketo-16R,S-fluor-$PGE_1$ oder ein Alkylester davon hergestellt wird.

16. Verfahren nach einem der Ansprüche 1 oder 2, dadurch **gekennzeichnet,** daß 13,14-Dihydro-15-keto-19-methyl-$PGE_2$ oder ein Alkylester davon hergestellt wird.

17. Verfahren nach einem der Ansprüche 1 oder 2, dadurch **gekennzeichnet,** daß 13,14-Dihydro-6,15-diketo-19-methyl-$PGE_1$ oder ein Alkylester davon hergestellt wird.

**18.** Verfahren nach einem der Ansprüche 1 oder 2, dadurch **gekennzeichnet,** daß 13,14-Dihydro-15-keto-20-ethyl-$PGE_2$ oder ein Alkylester davon hergestellt wird.

**19.** Verfahren nach einem der Ansprüche 1 oder 2, dadurch **gekennzeichnet,** daß 13,14-Dihydro-15-keto-16,16-difluor-$PGE_2$ oder ein Alkylester davon hergestellt wird.

**20.** Verfahren nach einem der Ansprüche 1 oder 2, dadurch **gekennzeichnet,** daß 13,14-Dihydro-15-keto-20-methyl-$PGE_1$ oder ein Alkylester davon hergestellt wird.

**21.** Verfahren nach einem der Ansprüche 1 oder 2, dadurch **gekennzeichnet,** daß 13,14-Dihydro-15-keto-$\Delta^2$-$PGE_1$ oder ein Alkylester davon hergestellt wird.

**22.** Verfahren nach einem der Ansprüche 1 oder 2, dadurch **gekennzeichnet,** daß 13,14-Dihydro-15-keto-16R,S-fluor-20-methl-$PGE_2$ oder ein Alkylester davon hergestellt wird.

**23.** Verfahren nach einem der Ansprüche 1 oder 2, dadurch **gekennzeichnet,** daß 13,14-Dihydro-15-keto-5,6-dehydro-20-methoxy-$PGE_2$ oder ein Alkylester davon hergestellt wird.

**24.** Verfahren zur Herstellung eines Anti-Ulcer-Mittels, dadurch **gekennzeichnet,** daß Prostaglandine E, dargestellt durch die allgemeine Formel

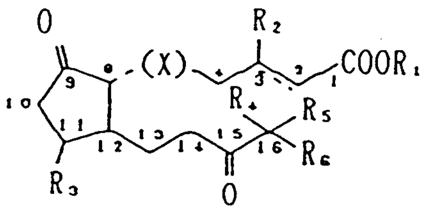

(I)

worin X

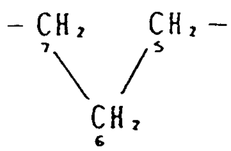 . 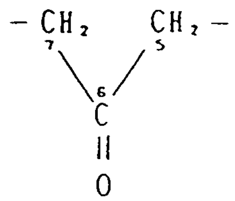 . 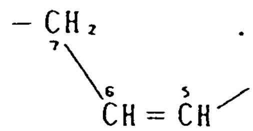 . 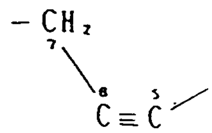

bedeutet;

$R_1$ ein Wasserstoffatom, physiologisch annehmbare Salze, physiologisch annehmbare Schutzgruppen, $C_1$-$C_4$-Alkyl, Benzyl, Hydroxyalkyl bedeutet;

$R_2$ ein Wasserstoffatom oder eine Methylgruppe bedeutet;

$R_3$ eine Hydroxyl- oder Hydroxymethylgruppe bedeutet;

$R_4$ und $R_5$ gleich oder unterschiedlich sind und ein Wasserstoffatom, eine Methylgruppe, eine Hydroxygruppe oder ein Halogenatom bedeuten, mit der Maßgabe, daß

(I) wenn in mindestens einer der Substituenten $R_4$ und $R_5$ eine Methylgruppe, eine Hydroxygruppe oder ein Halogen bedeutet, $R_6$ eine $C_1$-$C_9$-Alkylgruppe, die eine Verzweigung oder Doppelbindung aufweisen kann oder eine $C_1$-$C_9$-Alkylgruppe, die eine Alkoxysubstituentengruppe aufweist, bedeutet; oder

(II) wenn $R_4$ und $R_5$ beide Wasserstoffatome bedeuten, $R_6$ eine $C_5$-$C_9$-Alkylgruppe, die eine Verzweigung oder Doppelbindung aufweisen kann oder eine $C_1$-$C_9$-Alkylgruppe, die eine Alkoxysubstituentengruppe aufweisen kann, wobei die $C_2$-$C_3$-Bindung eine Doppelbindung sein kann, bedeutet und

ein pharmazeutisch annehmbarer Träger vermischt werden.

**25.** Verfahren nach Anspruch 24, dadurch **gekennzeichnet,** daß $R_4$ und/oder $R_5$ ein Halogen bedeuten.

**26.** Verfahren nach Anspruch 24, dadurch **gekennzeichnet,** daß $R_4$ und/oder $R_5$ ein Fluoratom bedeuten.

**27.** Verfahren nach Anspruch 24, dadurch **gekennzeichnet,** daß $R_4$ und/oder $R_5$ eine Methylgruppe bedeuten.

**28.** Verfahren nach Anspruch 24, dadurch **gekennzeichnet,** daß das Prostaglandin E eine Methylgruppe an der 19-Stellung aufweist.

**29.** Verfahren nach Anspruch 24, dadurch **gekennzeichnet,** daß $R_6$ eine Hexylgruppe bedeutet.

**30.** Verfahren nach Anspruch 24, dadurch **gekennzeichnet,** daß $R_6$ eine Isopentylgruppe bedeutet.

**31.** Verfahren nach Anspruch 24, dadurch **gekennzeichnet,** daß $R_6$ eine Pentyl-2S-Gruppe bedeutet.

**32.** Verfahren nach Anspruch 24, dadurch **gekennzeichnet,** daß bei den Prostaglandinen E die Carboxylgruppe an der Endstellung der $\alpha$-Kette mit einer Alkylgruppe verestert ist.

**33.** Verfahren nach Anspruch 24, dadurch **gekennzeichnet,** daß als Prostaglandine E 13,14-Dihydro-15-keto-PGEs mit einer Methylgruppe oder einem Fluoratom an der 16-Stellung oder ein Alkylester davon verwendet werden.

**34.** Verfahren nach Anspruch 24, dadurch **gekennzeichnet,** daß als Prostaglandin E 13,14-Dihydro-15-keto-16R,S-methyl-$PGE_2$-alkylester verwendet wird.

**35.** Verfahren nach Anspruch 24, dadurch **gekennzeichnet,** daß als Prostaglandin E 13,14-Dihydro-6,15-diketo-16R,S-methyl-$PGE_1$-alkylester verwendet wird.

**36.** Verfahren nach Anspruch 24, dadurch **gekennzeichnet,** daß als Prostaglandin E 13,14-Dihydro-15-keto-16R,S-fluor-$PGE_2$ oder ein Alkylester davon verwendet wird.

**37.** Verfahren nach Anspruch 24, dadurch **gekennzeichnet,** daß als Prostaglandin E 13,14-Dihydro-6,15-diketo-16R,S-fluor-$PGE_1$ oder ein Alkylester davon verwendet wird.

**38.** Verfahren nach Anspruch 24, dadurch **gekennzeichnet,** daß als Prostaglandin E 13,14-Dihydro-15-keto-19-methyl-$PGE_2$ oder ein Alkylester davon verwendet wird.

**39.** Verfahren nach Anspruch 24, dadurch **gekennzeichnet,** daß als Prostaglandin E 13,14-Dihydro-6,15-diketo-19-methyl-$PGE_2$ oder ein Alkylester davon verwendet wird.

**40.** Verfahren nach Anspruch 24, dadurch **gekennzeichnet,** daß als Prostaglandin E 13,14-Dihydro-15-keto-20-ethyl-$PGE_2$ oder ein Alkylester davon verwendet wird.

**41.** Verfahren nach Anspruch 24, dadurch **gekennzeichnet,** daß als Prostaglandin E 13,14-Dihydro-15-keto-16,16-difluor-$PGE_2$ oder ein Alkylester davon hergestellt wird.

**42.** Verfahren nach Anspruch 24, dadurch **gekennzeichnet,** daß als Prostaglandin E 13,14-Dihydro-15-keto-20-methyl-$PGE_1$ oder ein Alkylester davon hergestellt wird.

**43.** Verfahren nach Anspruch 24, dadurch **gekennzeichnet,** daß als Prostaglandin E 13,14-Dihydro-15-keto-$\Delta^2$-PGE1 oder ein Alkylester davon hergestellt wird.

**44.** Verfahren nach Anspruch 24, dadurch **gekennzeichnet,** daß als Prostaglandin E 13,14-Dihydro-15-keto-16R,S-fluor-20-methyl-$PGE_2$ oder ein Alkylester davon hergestellt wird.

**45.** Verfahren nach Anspruch 24, dadurch **gekennzeichnet,** daß als Prostaglandin E 13,14-Dihydro-15-keto-5,6-dehydro-20-methoxy-$PGE_2$ oder ein Alkylester davon hergestellt wird.

**Patentansprüche für folgenden Vertragsstaat : GR**

**1.** Prostaglandine E, dargestellt durch die allgemeine Formel

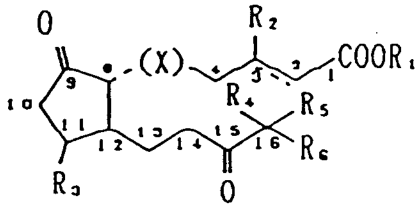

(I)

worin X

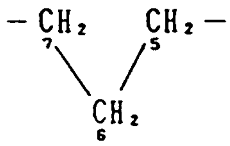 , 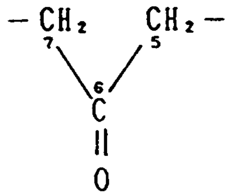 , 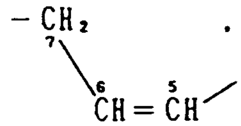 , 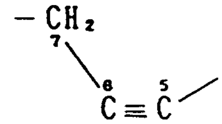

bedeutet;
$R_1$ ein Wasserstoffatom, physiologisch annehmbare Salze, physiologisch annehmbare Schutzgruppen, $C_1$-$C_4$-Alkyl, Benzyl, Hydroxyalkyl bedeutet;
$R_2$ ein Wasserstoffatom oder eine Methylgruppe bedeutet;
$R_3$ eine Hydroxyl- oder Hydroxymethylgruppe bedeutet;
$R_4$ und $R_5$ gleich oder unterschiedlich sind und ein Wasserstoffatom, eine Methylgruppe, eine Hydroxygruppe oder ein Halogenatom bedeuten, mit der Maßgabe, daß
(I) wenn in mindestens einer der Substituenten $R_4$ und $R_5$ eine Methylgruppe, eine Hydroxygruppe oder ein Halogen bedeutet, $R_6$ eine $C_1$-$C_9$-Alkylgruppe, die eine Verzweigung oder Doppelbindung aufweisen kann oder eine $C_1$-$C_9$-Alkylgruppe, die eine Alkoxysubstituentengruppe aufweist, bedeutet; oder
(II) wenn $R_4$ und $R_5$ beide Wasserstoffatome bedeuten, $R_6$ eine $C_5$-$C_9$-Alkylgruppe, die eine Verzweigung oder Doppelbindung aufweisen kann oder eine $C_1$-$C_9$-Alkylgruppe, die eine Alkoxysubstituentengruppe aufweisen kann, wobei die $C_2$-$C_3$-Bindung eine Doppelbindung sein kann, bedeutet.

**2.** Prostaglandine E nach Anspruch 1, dadurch **gekennzeichnet,** daß $R_4$ und/oder $R_5$ ein Halogen bedeuten.

**3.** Prostaglandine E nach Anspruch 1, dadurch **gekennzeichnet,** daß $R_4$ und/oder $R_5$ ein Fluoratom bedeuten.

**4.** Prostaglandine E nach Anspruch 1, dadurch **gekennzeichnet,** daß $R_4$ und/oder $R_5$ eine Methylgruppe bedeuten.

**5.** Prostaglandine E nach Anspruch 1, dadurch **gekennzeichnet,** daß sie eine Methylgruppe an ihrer 19-Stellung aufweisen.

**6.** Prostaglandine E nach Anspruch 1, dadurch **gekennzeichnet,** daß $R_6$ eine Hexylgruppe bedeutet.

**7.** Prostaglandine E nach Anspruch 1 dadurch **gekennzeichnet,** daß $R_6$ eine Isopentylgruppe bedeutet.

**8.** Prostaglandine E nach Anspruch 1, dadurch **gekennzeichnet,** daß $R_6$ eine Pentyl-2S-Gruppe bedeutet.

**9.** Prostaglandine E nach Anspruch 1, dadurch **gekennzeichnet,** daß die Carboxylgruppe an der

Endstellung der α-Kette mit einer Alkylgruppe verestert ist.

**10.** Prostaglandine E nach Anspruch 1, nämlich 13,14-Dihydro-15-keto-PGE mit einer Methylgruppe oder einem Fluoratom an der 16-Stellung oder ein Alkylester davon.

**11.** Prostaglandine E nach Anspruch 1, nämlich 13,14-Dihydro-15-keto-16R,S-methyl-$PGE_2$ oder ein Alkylester davon.

**12.** Prostaglandine E nach Anspruch 1, nämlich 13,14-Dihydro-6,15-diketo-16R,S-methyl-$PGE_1$ oder ein Alkylester davon.

**13.** Prostaglandine E nach Anspruch 1, nämlich 13,14-Dihydro-15-keto-16R,S-fluor-$PGE_2$ oder ein Alkylester davon.

**14.** Prostaglandine E nach Anspruch 1, nämlich 13,14-Dihydro-6,15-diketo-16R,S-fluor-$PGE_1$ oder ein Alkylester davon.

**15.** Prostaglandine E nach Anspruch 1, nämlich 13,14-Dihydro-15-keto-19-methyl-$PGE_2$ oder ein Alkylester davon.

**16.** Prostaglandine E nach Anspruch 1, nämlich 13,14-Dihydro-6,15-diketo-19-methyl-$PGE_1$ oder ein Alkylester davon.

**17.** Prostaglandine E nach Anspruch 1, nämlich 13,14-Dihydro-15-keto-20-ethyl-$PGE_2$ oder ein Alkylester davon.

**18.** Prostaglandine E nach Anspruch 1, nämlich 13,14-Dihydro-15-keto-16,16-difluor-$PGE_2$ oder ein Alkylester davon.

**19.** Prostaglandine E nach Anspruch 1, nämlich 13,14-Dihydro-15-keto-20-methyl-$PGE_1$ oder ein Alkylester davon.

**20.** Prostaglandine E nach Anspruch 1, nämlich 13,14-Dihydro-15-keto-$\Delta^2$-$PGE_1$ oder ein Alklyester davon.

**21.** Prostaglandine E nach Anspruch 1, nämlich 13,14-Dihydro-15-keto-16R,S-fluor-20-methyl-$PGE_2$ oder ein Alkylester davon.

**22.** Prostaglandine E nach Anspruch 1, nämlich 13,14-Dihydro-15-keto-5,6-dehydro-20-methoxy-$PGE_2$ oder ein Alkylester davon.

**23.** Verfahren zur Herstellung eines Anti-Ulcer-Mittels, dadurch **gekennzeichnet,** daß ein Prostaglandin E, das durch die allgemeine Formel

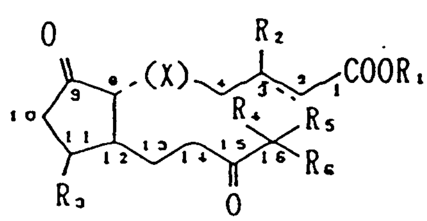

(I)

dargestellt wird, worin X

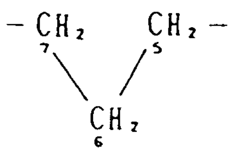 . 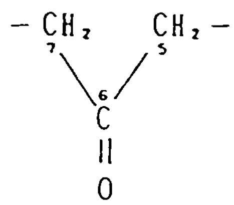 . 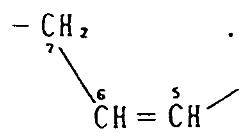 . 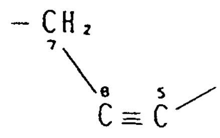

bedeutet;

$R_1$ ein Wasserstoffatom, physiologisch annehmbare Salze, physiologisch annehmbare Schutzgruppen, $C_1$-$C_4$-Alkyl, Benzyl, Hydroxyalkyl bedeutet;

$R_2$ ein Wasserstoffatom oder eine Methylgruppe bedeutet;

$R_3$ eine Hydroxyl- oder Hydroxymethylgruppe bedeutet;

$R_4$ und $R_5$ gleich oder unterschiedlich sind und ein Wasserstoffatom, eine Methylgruppe, eine Hydroxygruppe oder ein Halogenatom bedeuten, mit der Maßgabe, daß

(I) wenn in mindestens einer der Substituenten $R_4$ und $R_5$ eine Methylgruppe, eine Hydroxygruppe oder ein Halogen bedeutet, $R_6$ eine $C_1$-$C_9$-Alkylgruppe, die eine Verzweigung oder Doppelbindung aufweisen kann oder eine $C_1$-$C_9$-Alkylgruppe, die eine Alkoxysubstituentengruppe aufweist, bedeutet; oder

(II) wenn $R_4$ und $R_5$ beide Wasserstoffatome bedeuten, $R_6$ eine $C_5$-$C_9$-Alkylgruppe, die eine Verzweigung oder Doppelbindung aufweisen kann oder eine $C_1$-$C_9$-Alkylgruppe, die eine Alkoxysubstituentengruppe aufweisen kann, wobei die $C_2$-$C_3$-Bindung eine Doppelbindung sein kann, bedeutet, und

ein pharmazeutisch annehmbarer Trägerstoff vermischt werden.

**24.** Verfahren nach Anspruch 23, dadurch **gekennzeichnet,** daß $R_4$ und/oder $R_5$ ein Halogen bedeuten.

**25.** Verfahren nach Anspruch 23, dadurch **gekennzeichnet,** daß $R_4$ und/oder $R_5$ ein Fluoratom bedeuten.

**26.** Verfahren nach Anspruch 23, dadurch **gekennzeichnet,** daß $R_4$ und/oder $R_5$ eine Methylgruppe bedeuten.

**27.** Verfahren nach Anspruch 23, dadurch **gekennzeichnet,** daß das Prostaglandin E eine Methylgruppe in der 19-Stellung aufweist.

**28.** Verfahren nach Anspruch 23, dadurch **gekennzeichnet,** daß $R_6$ eine Hexylgruppe bedeutet.

**29.** Verfahren nach Anspruch 23, dadurch **gekennzeichnet,** daß $R_6$ eine Isopentylgruppe bedeutet.

**30.** Verfahren nach Anspruch 23, dadurch **gekennzeichnet,** daß $R_6$ eine Pentyl-2S-Gruppe bedeutet.

**31.** Verfahren nach Anspruch 23, dadurch **gekennzeichnet,** daß die Prostaglandine E, deren Carboxylgruppe an der Endstellung der $\alpha$-Kette mit einer Alkylgruppe verestert ist, hergestellt werden.

**32.** Verfahren nach Anspruch 23, dadurch **gekennzeichnet,** daß als Prostaglandine E 13,14-Dihydro-15-keto-PGEs mit einer Methylgruppe oder einem Fluoratom an der 16-Stellung oder die Alkylester davon hergestellt werden.

**33.** Verfahren nach Anspruch 23, dadurch **gekennzeichnet,** daß das Prostaglandin E 13,14-Dihydro-15-keto-16R,S-methyl-$PGE_2$-alkylester ist.

**34.** Verfahren nach Anspruch 23, dadurch **gekennzeichnet,** daß das Prostaglandin E 13,14-Dihydro-6,15-diketo-16R,S-methyl-$PGE_1$-alkylester ist.

**35.** Verfahren nach Anspruch 23, dadurch **gekennzeichnet,** daß das Prostaglandin E 13,14-Dihydro-15-keto-16R,S-fluor-$PGE_2$ oder ein Alkylester davon ist.

**36.** Verfahren nach Anspruch 23, dadurch **gekennzeichnet,** daß das Prostaglandin E 13,14-Dihydro-6,15-diketo-16R,S-fluor-$PGE_1$ oder ein Alkylester davon ist.

**37.** Verfahren nach Anspruch 23, dadurch **gekennzeichnet,** daß das Prostaglandin E 13,14-Dihydro-15-keto-19-methyl-$PGE_2$ oder ein Alkylester davon ist.

**38.** Verfahren nach Anspruch 23, dadurch **gekennzeichnet,** daß das Prostaglandin E 13,14-Dihydro-6,15-diketo-19-methyl-$PGE_2$ oder ein Alkylester davon ist.

**39.** Verfahren nach Anspruch 23, dadurch **gekennzeichnet,** daß das Prostaglandin E 13,14-Dihydroy-15-keto-20-ethyl-$PGE_2$ oder ein Alkylester davon ist.

**40.** Verfahren nach Anspruch 23, dadurch **gekennzeichnet,** daß das Prostaglandin E 13,14-Dihydro-15-keto-16,16-difluor-$PGE_2$ oder ein Alkylester davon ist.

**41.** Verfahren nach Anspruch 23, dadurch **gekennzeichnet,** daß das Prostaglandin E 13,14-Dihydro-15-keto-20-methyl-$PGE_1$ oder ein Alkylester davon ist.

**42.** Verfahren nach Anspruch 23, dadurch **gekennzeichnet,** daß das Prostaglandin E 13,14-Dihydro-15-keto-$\Delta^2$-$PGE_1$ oder ein Alkylester davon ist.

**43.** Verfahren nach Anspruch 23, dadurch **gekennzeichnet,** daß das Prostaglandin E 13,14-Dihydro-15-keto-16R,S-fluor-20-methyl-$PGE_2$ oder ein Alkylester davon ist.

**44.** Verfahren nach Anspruch 23, dadurch **gekennzeichnet,** daß das Prostaglandin E 13,14-Dihydro-15-keto-5,6-dehydro-20-methoxy-$PGE_2$ oder ein Alkylester davon ist.

**Revendications**

**Revendications pour les Etats contractants suivants : AT, BE, CH, DE, FR, GB, IT, LI, LU, NL, SE**

**1.** Prostaglandines E répondant à la formule générale :

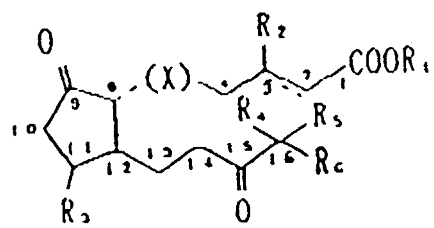

(I)

(dans laquelle X représente :

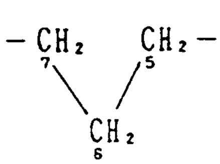 , 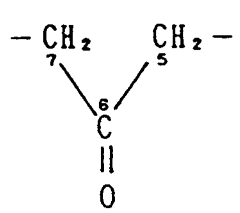 , 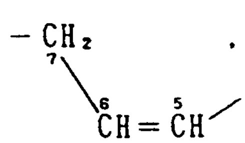 , 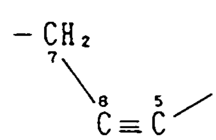

| | |
|---|---|
| $R_1$ | représente un atome d'hydrogène, des sels physiologiquement acceptables, un groupe protecteur physiologiquement acceptable, un groupe alkyle en $C_1$ à $C_4$, un groupe benzyle, un groupe hydroxyalkyle; |
| $R_2$ | représente un atome d'hydrogène ou un groupe méthyle; |
| $R_3$ | représente un groupe hydroxyle ou un groupe hydroxyméthyle; |

$R_4$ et $R_5$ sont identiques ou différents, et désignent un atome d'hydrogène, un groupe méthyle, un groupe hydroxy ou un atome d'halogène, sous réserve que:

(I) lorsqu'au moins un des symboles $R_4$ et $R_5$ est un groupe méthyle, un groupe hydroxy ou un atome d'halogène, $R_6$ est un groupe alkyle en $C_1$ à $C_9$ qui peut avoir une ramification ou une double liaison, ou un groupe alkyle en $C_1$ à $C_9$ ayant un groupe substituant alcoxy; ou

(II) lorsque $R_4$ et $R_5$ sont tous deux des atomes d'hydrogène, $R_6$ est un groupe alkyle en $C_5$ à $C_9$ qui peut avoir une ramification ou une double liaison, ou bien un groupe alkyle en $C_1$ à $C_9$ ayant un groupe alcoxy comme substituant, dans lequel la liaison en $C_2$ à $C_3$ peut être une double liaison.

**2.** Prostaglandines E selon la revendication 1, dans lesquelles $R_4$ et/ou $R_5$ sont des atomes d'halogène.

**3.** Prostaglandines E selon la revendication 1, dans lesquelles $R_4$ et/ou $R_5$ sont des atomes de fluor.

**4.** Prostaglandines E selon la revendication 1, dans lesquelles $R_4$ et/ou $R_5$ sont des groupes méthyle.

**5.** Prostaglandines E selon la revendication 1, ayant un groupe méthyle à leur position 19.

**6.** Prostaglandines E selon la revendication 1, dans lesquelles $R_6$ est un groupe hexyle.

**7.** Prostaglandines E selon la revendication 1, dans lesquelles $R_6$ est un groupe isopentyle.

**8.** Prostaglandines E selon la revendication 1, dans lesquelles $R_6$ est un groupe pentyl-2S.

**9.** Prostaglandines E selon la revendication 1, dont le groupe carboxyle à la position terminale de la chaîne $\alpha$ est estérifié par un groupe alkyle.

**10.** Prostaglandine E selon la revendication 1, qui est la 13,14-dihydro-15-céto-PGE ayant un groupe méthyle ou un atome de fluor à la position 16, ou un de ses esters alkyliques.

**11.** Prostaglandine E selon la revendication 1, qui est la 13,14-dihydro-15-céto-16R,S-méthyl-$PGE_2$ ou un de ses esters alkyliques.

**12.** Prostaglandine E selon la revendication 1, qui est la 13,14-dihydro-6,15-dicéto-16R,S-méthyl-$PGE_1$ ou un de ses esters alkyliques.

**13.** Prostaglandine selon la revendication 1, qui est une 13,14-dihydro-15-céto-16R,S-fluoro-$PGE_2$ ou son ester alkylique.

**14.** Prostaglandine E selon la revendication 1, qui est la 13,14-dihydro-6,15-dicéto-16R,S-fluoro-$PGE_1$ ou un de ses esters alkyliques.

**15.** Prostaglandine E selon la revendication 1, qui est la 13,14-dihydro-15-céto-19-méthyl-$PGE_2$ ou un de ses esters alkyliques.

**16.** Prostaglandine E selon la revendication 1, qui est la 13,14-dihydro-6,15-dicéto-19-méthyl-$PGE_1$ ou un de ses esters alkyliques.

**17.** Prostaglandine E selon la revendication 1, qui est la 13,14-dihydro-15-céto-20-éthyl-$PGE_2$ ou un de ses esters alkyliques.

**18.** Prostaglandine E selon la revendication 1, qui est la 13,14-dihydro-15-céto-16,16-difluoro-$PGE_2$ ou un de ses esters alkyliques.

**19.** Prostaglandine E selon la revendication 1, qui est la 13,14-dihydro-15-céto-20-méthyl-$PGE_1$ ou un de ses esters alkyliques.

**20.** Prostaglandine E selon la revendication 1, qui est la 13,14-dihydro-15-céto-$\Delta^2$-$PGE_1$ ou un de ses esters alkyliques.

**21.** Prostaglandine E selon la revendication 1, qui est la 13,14-dihydro-15-céto-16R,S-fluoro-20-méthyl-$PGE_2$ ou un de ses esters alkyliques.

**22.** Prostaglandine E selon la revendication 1, qui est la 13,14-dihydro-15-céto-5,6-déshydro-20-méthoxy-$PGE_2$ ou u de ses esters alkyliques.

**23.** Utilisation de prostaglandines E répondant à la formule générale :

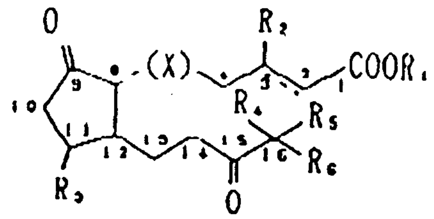

(I)

(dans laquelle x représente :

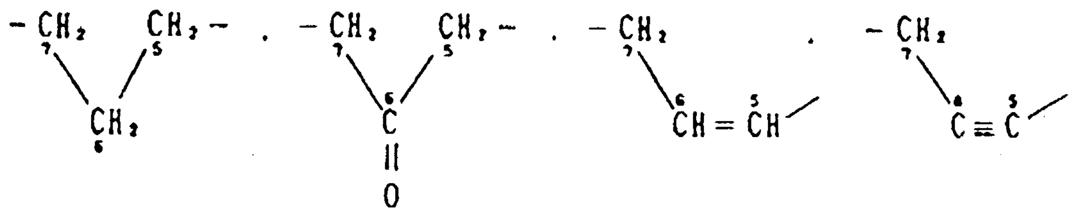

$R_1$ représente un atome d'hydrogène, des sels physiologiquement acceptables, un groupe protecteur physiologiquement acceptable, un groupe alkyle en $C_1$ à $C_4$, un groupe benzyle, un groupe hydroxyalkyle;

$R_2$ représente un atome d'hydrogène ou un groupe méthyle;

$R_3$ représente un groupe hydroxyle ou un groupe hydroxyméthyle;

$R_4$ et $R_5$ sont identiques ou différents, et représentent un atome d'hydrogène, un groupe méthyle, un groupe hydroxy ou un atome d'halogène, sous réserve que :

(I) lorsqu'au moins un des symboles $R_4$ et $R_5$ est un groupe méthyle, un groupe hydroxy ou un atome d'halogène, $R_6$ est un groupe alkyle en $C_1$ à $C_9$ qui peut avoir une ramification ou une double liaison, ou un groupe alkyle en $C_1$ à $C_9$ ayant un groupe alcoxy comme substituant; ou

(II) lorsque $R_4$ et $R_5$ sont tous deux des atomes d'hydrogène, $R_6$ est un groupe alkyle en $C_5$ à $C_9$ qui peut avoir une ramification ou une double liaison, ou un groupe alkyle en $C_1$ à $C_9$ ayant un groupe alcoxy comme substituant, dans lequel la liaison en $C_2$ à $C_3$ peut être une double liaison,

pour la fabrication d'une composition anti-ulcère.

**24.** Utilisation selon la revendication 23, dans laquelle $R_4$ et/ou $R_5$ sont des atomes d'halogène.

**25.** Utilisation telle que décrite dans la revendication 23, dons laquelle $R_4$ et/ou $R_5$ sont des atomes de fluor.

**26.** Utilisation selon la revendication 23, dans laquelle $R_4$ et/ou $R_5$ sont des groupes méthyle.

**27.** Utilisation selon la revendication 23, dans laquelle la prostaglandine E a un groupe méthyle à la position 19.

**28.** Utilisation selon la revendication 23, dans laquelle $R_6$ est un groupe hexyle.

**29.** Utilisation selon la revendication 23, dans laquelle $R_6$ est un groupe isopentyle.

**30.** Utilisation selon la revendication 23, dans laquelle $R_6$ est un groupe pentyl-2S.

**31.** Utilisation selon la revendication 23, dans laquelle les prostaglandines E ont leur groupe carboxyle à la position terminale de la chaîne $\alpha$ estérifié par un groupe alkyle.

**32.** Utilisation selon la revendication 23, dans laquelle les prostaglandines E sont des 13,14-dihydro-15-céto-PGE ayant un groupe méthyle ou un atome de fluor à la position 16, ou leurs esters alkyliques.

**33.** Utilisation selon la revendication 23, dans laquelle la prostaglandine E est un ester alkylique de la 13,14-dihydro-15-céto-16R,S-méthyl-$PGE_2$.

**34.** Utilisation selon la revendication 23, dans laquelle la prostaglandine E est un ester alkylique de la 13,14-dihydro-6,15-dicéto-16R,S-méthyl-$PGE_1$.

**35.** Utilisation selon la revendication 23, dans laquelle la prostaglandine E est la 13,14-dihydro-15-céto-16R,S-fluoro-$PGE_2$ ou un de ses esters alkyliques.

**36.** Utilisation selon la revendication 23, dans laquelle la prostaglandine E est la 13,14-dihydro-6,15-dicéto-16R,S-fluoro-$PGE_1$ ou un de ses esters alkyliques.

**37.** Utilisation selon la revendication 23, dans laquelle la prostaglandine E est la 13,14-dihydro-15-céto-19-méthyl-$PGE_2$ ou un ses esters alkyliques.

**38.** Utilisation selon la revendication 23, dans laquelle la prostaglandine E est la 13,14-dihydro-6,15-dicéto-19-méthyl-$PGE_2$ ou un de ses esters alkyliques.

**39.** Utilisation selon la revendication 23, dans laquelle la prostaglandine E est le 13,14-dihydro-15-céto-20-éthyl-$PGE_2$ ou un de ses esters alkyliques.

**40.** Utilisation selon la revendication 23, dans laquelle la prostaglandine E est la 13,14-dihydro-15-céto-16,16-difluoro-$PGE_2$ ou un de ses esters alkyliques.

**41.** Utilisation selon la revendication 23, dans laquelle la prostaglandine E est la 13,14-dihydro-15-céto-20-méthyl-$PGE_1$ ou un de ses esters alkyliques.

**42.** Utilisation selon la revendication 23, dans laquelle la prostaglandine E est la 13,14-dihydro-15-céto-$\Delta^2$-$PGE_1$ ou un de ses esters alkyliques.

**43.** Utilisation selon la revendication 23, dans laquelle la prostaglandine E est la 13,14-dihydro-15-céto-16R,S-fluoro-20-méthyl-$PGE_2$ ou un de ses esters alkyliques.

**44.** Utilisation selon la revendication 23, dans laquelle la prostaglandine E est la 13,14-dihydro-15-céto-5,6-déshydro-20-méthoxy-$PGE_2$ ou un de ses esters alkyliques.

**Revendications pour l'Etat contractant suivant : ES**

**1.** Procédé de préparation de prostaglandines E répondant à la formule générale :

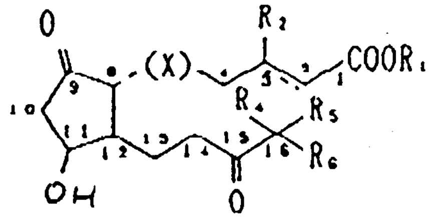

(I)

(dans laquelle X représente :

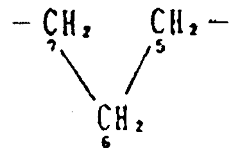 . 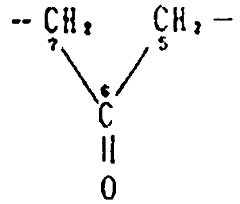 . 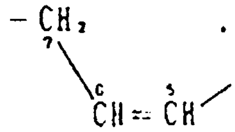 . 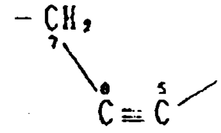

| | |
|---|---|
| $R_1$ | représente un atome d'hydrogène, des sels physiologiquement acceptables, un groupe protecteur physiologiquement acceptable, un groupe alkyle en $C_1$ à $C_4$, un groupe benzyle, un groupe hydroxyalkyle; |
| $R_2$ | représente un atome d'hydrogène ou un groupe méthyle; |
| $R_4$ et $R_5$ | sont identiques ou différents, et représentent un atome d'hydrogène, un groupe méthyle, un groupe hydroxy ou un atome d'halogène sous réserve que:<br>(I) lorsqu'au moins un des symboles $R_4$ et $R_5$ est un groupe méthyle, un groupe hydroxy ou un atome d'halogène, $R_6$ est un groupe alkyle en $C_1$ à $C_9$ qui peut avoir une ramification ou une double liaison, ou bien un groupe alkyle en $C_1$ à $C_9$ ayant comme substituant un groupe alcoxy; ou<br>(II) lorsque $R_4$ et $R_5$ sont tous deux des atomes d'hydrogène, $R_6$ est un groupe alkyle en $C_5$ à $C_9$ qui peut avoir une ramification ou une double liaison, ou bien un groupe alkyle en $C_1$ à $C_9$ ayant comme substituant un groupe alcoxy, dans lequel la liaison en $C_2$ à $C_3$ peut être une double liaison; |

le procédé comprenant l'hydrolyse du composé représenté par la formule :

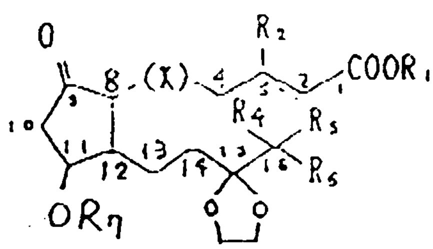

dans laquelle le symbole $R_7$ est un groupe tétrahydropyranyle ou un groupe t-butyldiméthylsilyle, et X, $R_1$, $R_2$, $R_4$, $R_5$ et $R_6$ sont les mêmes que ci-dessus.

**2.** Procédé de préparation de prostaglandines E répondant à la formule suivante :

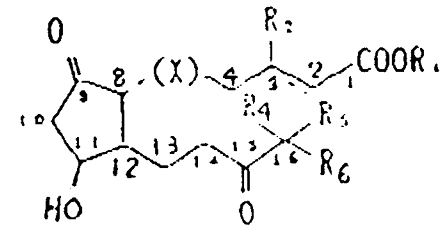

dans laquelle X, $R_1$, $R_2$, $R_4$, $R_5$ et $R_6$ sont tels que définis dans la revendication 1, qui comprend l'hydrolyse d'un composé représenté par la formule :

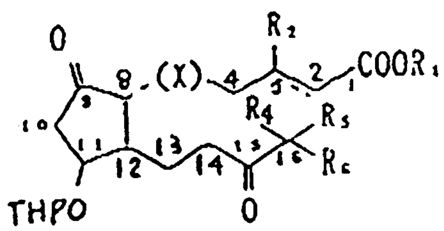

dans laquelle $R_1$, $R_2$, $R_4$, $R_5$ et $R_6$ sont les mêmes que ci-dessus et THP désigne un groupe tétrahydropyranyle.

**3.** Procédé selon les revendications 1 ou 2, dans lequel $R_4$ et/ou $R_5$ sont des atomes d'halogène.

**4.** Procédé selon les revendications 1 ou 2, dans lequel $R_4$ et/ou $R_5$ sont des atomes de fluor.

**5.** Procédé selon les revendications 1 ou 2, dans lequel $R_4$ et/ou $R_5$ sont des groupes méthyle.

**6.** Procédé selon les revendications 1 ou 2, dans lequel les prostaglandines E ont un groupe méthyle à leur position 19.

**7.** Procédé selon les revendications 1 ou 2, dans lequel $R_6$ est un groupe hexyle.

**8.** Procédé selon les revendications 1 ou 2, dans lequel $R_6$ est un groupe isopentyle.

**9.** Procédé selon les revendications 1 ou 2, dans lequel $R_6$ est un groupe pentyl-2S.

**10.** Procédé selon les revendications 1 ou 2, dans lequel le groupe carboxyle à la position terminale de la chaîne $\alpha$ est estérifié par un groupe alkyle.

**11.** Procédé selon les revendications 1 ou 2, pour préparer une 13,14-dihydro-15-céto-PGE ayant un groupe méthyle ou un atome de fluor à la position 16, ou un de ses esters alkyliques.

**12.** Procédé selon les revendications 1 ou 2, pour préparer la 13,14-dihydro-15-céto-16R,S-méthyl-$PGE_2$ ou un de ses esters alkyliques.

**13.** Procédé selon les revendications 1 ou 2, pour préparer la 13,14-dihydro-6,15-dicéto-16R,S-méthyl-$PGE_1$ ou un de ses esters alkyliques.

**14.** Procédé selon les revendications 1 ou 2, pour préparer la 13,14-dihydro-15-céto-16R,S-fluoro-$PGE_2$ ou un de ses esters alkyliques.

**15.** Procédé selon les revendications 1 ou 2, pour préparer la 13,14-dihydro-6,15-dicéto-16R,S-fluoro-$PGE_1$ ou un de ses esters alkyliques.

**16.** Procédé selon les revendications 1 ou 2, pour préparer la 13,14-dihydro-15-céto-19-méthyl-$PGE_2$ ou un de ses esters alkyliques.

**17.** Procédé selon les revendications 1 ou 2, pour préparer la 13,14-dihydro-6,15-dicéto-19-méthyl-$PGE_1$ ou un de ses esters alkyliques.

**18.** Procédé selon les revendications 1 ou 2, pour préparer la 13,14-dihydro-15-céto-20-éthyl-$PGE_2$ ou un de ses esters alkyliques.

**19.** Procédé selon les revendications 1 ou 2, pour préparer la 13,14-dihydro-15-céto-16,16-difluoro-$PGE_2$ ou un de ses esters alkyliques.

**20.** Procédé selon les revendications 1 ou 2, pour préparer la 13,14-dihydro-15-céto-20-méthyl-$PGE_1$ ou un de ses esters alkyliques.

**21.** Procédé selon les revendications 1 ou 2, pour préparer la 13,14-dihydro-15-céto-$\Delta^2$-$PGE_1$ ou un de ses esters alkyliques.

**22.** Procédé selon les revendications 1 ou 2, pour préparer la 13,14-dihydro-15-céto-16R,S-fluoro-20-méthyl-$PGE_2$ ou un de ses esters alkyliques.

**23.** Procédé selon les revendications 1 ou 2, pour préparer la 13,14-dihydro-15-céto-5,6-déshydro-20-méthoxy-$PGE_2$ ou un de ses esters alkyliques.

**24.** Procédé pour préparer une composition anti-ulcère, comprenant le fait de mélanger une prostaglandine E répondant à la formule générale :

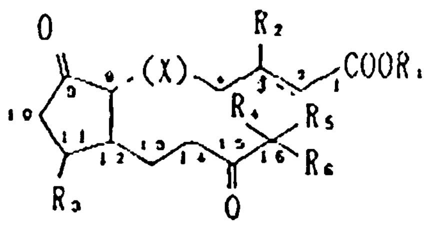

(I)

(dans laquelle X représente :

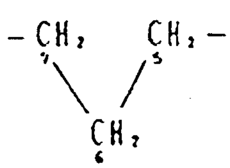 . 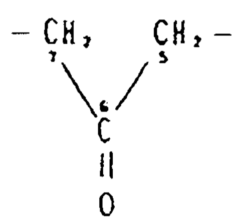 . 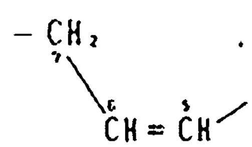 . 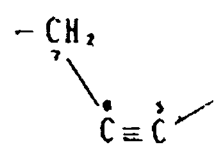

| | |
|---|---|
| $R_1$ | représente un atome d'hydrogène, des sels physiologiquement acceptables, un groupe protecteur physiologiquement acceptable, un groupe alkyle en $C_1$ à $C_4$, un groupe benzyle, un groupe hydroxyalkyle; |
| $R_2$ | représente un atome d'hydrogène ou un groupe méthyle; |
| $R_3$ | représente un groupe hydroxyle ou un groupe hydroxyméthyle; |
| $R_4$ et $R_5$ | sont identiques ou différents, et représentent un atome d'hydrogène, un groupe méthyle, un groupe, hydroxy ou un atome d'halogène, sous réserve que : (I) lorsqu'au moins un des symboles $R_4$ et $R_5$ est un groupe méthyle, un groupe hydroxy ou un atome d'halogène, $R_6$ est un groupe alkyle en $C_1$ à $C_9$ qui peut avoir |

une ramification ou une double liaison, ou un groupe alkyle en $C_1$ à $C_9$ ayant comme substituant un groupe alcoxy; ou

(II) lorsque $R_4$ et $R_5$ sont tous deux des atomes d'hydrogène, $R_6$ est un groupe alkyle en $C_5$ à $C_9$ qui peut avoir une ramification ou une double liaison, ou un groupe alkyle en $C_1$ à $C_9$ ayant comme substituant un groupe alcoxy, dans lequel la liaison en $C_2$ à $C_3$ peut être une double liaison,

et un excipient pharmaceutiquement acceptable.

**25.** Procédé selon la revendication 24, dans lequel $R_4$ et/ou $R_5$ sont des atomes d'halogène.

**26.** Utilisation selon la revendication 24, dans laquelle $R_4$ et/ou $R_5$ sont des atomes de fluor.

**27.** Utilisation selon la revendication 24, dans laquelle $R_4$ et/ou $R_5$ sont des groupes méthyle.

**28.** Procédé selon la revendication 24, dans lequel la prostaglandine E a un groupe méthyle à la position 19.

**29.** Procédé selon la revendication 24, dans lequel $R_6$ est un groupe hexyle.

**30.** Procédé selon la revendication 24, dans lequel $R_6$ est un groupe isopentyle.

**31.** Procédé selon la revendication 24, dans lequel $R_6$ est un groupe pentyl-2S.

**32.** Procédé selon la revendication 24, dans lequel les prostaglandines E ont leur groupe carboxyle à la position terminale de la chaîne $\alpha$ estérifié par un groupe alkyle.

**33.** Procédé selon la revendication 24, dans lequel les prostaglandines E sont des 13,14-dihydro-15-céto-PGE ayant un groupe méthyle ou un atome de fluor à la position 16, ou leurs esters alkyliques.

**34.** Procédé selon la revendication 24, dans lequel la prostaglandine E est un ester alkylique de la 13,14-dihydro-15-céto-16R,S-méthyl-$PGE_2$.

**35.** Procédé selon la revendication 24, dans lequel la prostaglandine E est un ester alkylique de la 13,14-dihydro-6,15-dicéto-16R,S-méthyl-$PGE_1$.

**36.** Procédé selon la revendication 24, dans lequel la prostaglandine E est la 13,14-dihydro-15-céto-16R,S-fluoro-$PGE_2$ ou un de ses esters alkyliques.

**37.** Procédé selon la revendication 24, dans lequel la prostaglandine E est la 13,14-dihydro-6,15-dicéto-16R,S-fluoro-$PGE_1$ ou un de ses esters alkyliques.

**38.** Procédé selon la revendication 24, dans lequel la prostaglandine E est la 13,14-dihydro-15-céto-19-méthyl-$PGE_2$ ou un ses esters alkyliques.

**39.** Procédé selon la revendication 24, dans lequel la prostaglandine E est la 13,14-dihydro-6,15-dicéto-19-méthyl-$PGE_2$ ou un de ses esters alkyliques.

**40.** Procédé selon la revendication 24, dans lequel la prostaglandine E est la 13,14-dihydro-15-céto-20-éthyl-$PGE_2$ ou un de ses esters alkyliques.

**41.** Procédé selon la revendication 24, dans lequel la prostaglandine E est la 13,14-dihydro-15-céto-16,16-difluoro-$PGE_2$ ou un de ses esters alkyliques.

**42.** Procédé selon la revendication 24, dans lequel la prostaglandine E est la 13,14-dihydro-15-céto-20-méthyl-$PGE_1$ ou un de ses esters alkyliques.

**43.** Procédé selon la revendication 24, dans lequel la prostaglandine E est la 13,14-dihydro-15-céto-$\Delta^2$-$PGE_1$ ou un de ses esters alkyliques.

**44.** Procédé selon la revendication 24, dans lequel la prostaglandine E est la 13,14-dihydro-15-céto-16R,S-fluoro-20-méthyl-$PGE_2$ ou un de ses esters alkyliques.

**45.** Procédé selon la revendication 24, dans lequel la prostaglandine E est la 13,14-dihydro-15-céto-5,6-déshydro-20-méthoxy-$PGE_2$ ou un de ses esters alkyliques.

**Revendications pour l'Etat contractant suivant : GR**

**1.** Prostaglandines E représentées par la formule générale :

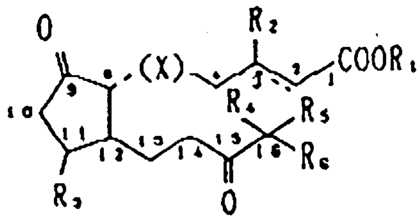

(I)

(dans laquelle X représente :

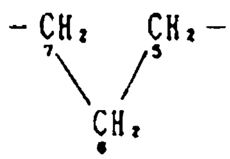
,
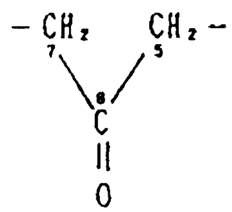
,
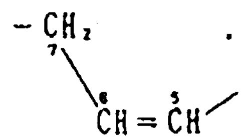
,
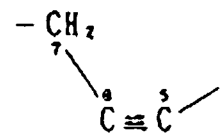

| | |
|---|---|
| $R_1$ | représente un atome d'hydrogène, des sels physiologiquement acceptables, un groupe protecteur physiologiquement acceptable, un groupe alkyle en $C_1$ à $C_4$, un groupe benzyle, un groupe hydroxyalkyle; |
| $R_2$ | représente un atome d'hydrogène ou un groupe méthyle; |
| $R_3$ | représente un groupe hydroxyle ou un groupe hydroxyméthyle; |
| $R_4$ et $R_5$ | sont identiques ou différents, et désignent un atome d'hydrogène, un groupe méthyle, un groupe hydroxy ou un atome d'halogène, sous réserve que: |

(I) lorsqu'au moins un des symboles $R_4$ et $R_5$ est un groupe méthyle, un groupe hydroxy ou un atome d'halogène, $R_6$ est un groupe alkyle en $C_1$ à $C_9$ qui peut avoir une ramification ou une double liaison, ou un groupe alkyle en $C_1$ à $C_9$ ayant un groupe alcoxy comme substituant; ou

(II) lorsque $R_4$ et $R_5$ sont tous deux des atomes d'hydrogène, $R_6$ est un groupe alkyle en $C_5$ à $C_9$ qui peut avoir une ramification ou une double liaison, ou bien un groupe alkyle en $C_1$ à $C_9$ ayant un groupe alcoxy comme substituant, dans lequel la liaison en $C_2$ à $C_3$ peut être une double liaison.

**2.** Prostaglandines E selon la revendication 1, dans lesquelles $R_4$ et/ou $R_5$ sont des atomes d'halogène.

**3.** Prostaglandines E selon la revendication 1, dans lesquelles $R_4$ et/ou $R_5$ sont des atomes de fluor.

**4.** Prostaglandines E selon la revendication 1, dans lesquelles $R_4$ et/ou $R_5$ sont des groupes méthyle.

**5.** Prostaglandines E selon la revendication 1, ayant un groupe méthyle à leur position 19.

**6.** Prostaglandines E selon la revendication 1, dans lesquelles $R_6$ est un groupe hexyle.

7. Prostaglandines E selon la revendication 1, dans lesquelles $R_6$ est un groupe isopentyle.

8. Prostaglandines E selon la revendication 1, dans lesquelles $R_6$ est un groupe pentyl-2S.

9. Prostaglandines E selon la revendication 1, dans lesquelles le groupe carboxyle à la position terminale de la chaîne $\alpha$ est estérifié par un groupe alkyle.

10. Prostaglandine E selon la revendication 1, qui est une 13,14-dihydro-15-céto-PGE ayant un groupe méthyle on un atome de fluor à la position 16, ou un de ses esters alkyliques.

11. Prostaglandine E selon la revendication 1, qui est une 13,14-dihydro-15-céto-16R,S-méthyl-$PGE_2$ ou un de ses esters alkyliques.

12. Prostaglandine E selon la revendication 1, qui est une 13,14-dihydro-6,15-dicéto-16R,S-méthyl-$PGE_1$ ou un de ses esters alkyliques.

13. Prostaglandine E selon la revendication 1, qui est une 13,14-dihydro-15-céto-16R,S-fluoro-$PGE_2$ ou un de ses esters alkyliques.

14. Prostaglandine E selon la revendication 1, qui est une 13,14-dihydro-6,15-dicéto-16R,S-fluoro-$PGE_1$ ou un de ses esters alkyliques.

15. Prostaglandine E selon la revendication 1, qui est une 13,14-dihydro-15-céto-19-méthyl-$PGE_2$ ou un de ses esters alkyliques.

16. Prostaglandine E selon la revendication 1, qui est une 13,14-dihydro-6,15-dicéto-19-méthyl-$PGE_1$ ou un de ses esters alkyliques.

17. Prostaglandine E selon la revendication 1, qui est une 13,14-dihydro-15-céto-20-éthyl-$PGE_2$ ou un de ses esters alkyliques.

18. Prostaglandine E selon la revendication 1, qui est une 13,14-dihydro-15-céto-16,16-difluoro-$PGE_2$ ou un de ses esters alkyliques.

19. Prostaglandine E selon la revendication 1, qui est une 13,14-dihydro-15-céto-20-méthyl-$PGE_1$ ou un de ses esters alkyliques.

20. Prostaglandine E selon la revendication 1, qui est une 13,14-dihydro-15-céto-$\Delta^2$-$PGE_1$ ou un de ses esters alkyliques.

21. Prostaglandine E selon la revendication 1, qui est une 13,14-dihydro-15-céto-16R,S-fluoro-20-méthyl-$PGE_2$ ou un de ses esters alkyliques.

22. Prostaglandine E selon la revendication 1, qui est une 13,14-dihydro-15-céto-5,6-déshydro-20-méthoxy-$PGE_2$ ou un de ses esters alkyliques.

23. Procédé pour préparer une composition anti-ulcère comprenant le mélange d'une prostaglandine E répondant à la formule générale :

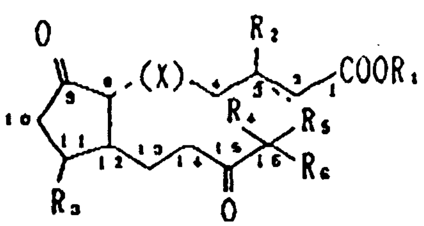

(I)

(dans laquelle x représente :

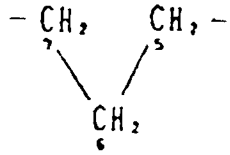 . 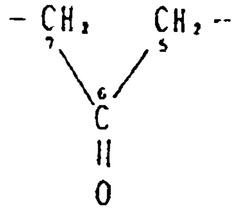 . 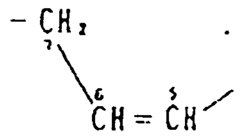 . 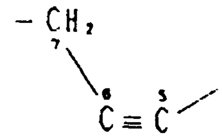

$R_1$ représente un atome d'hydrogène, des sels physiologiquement acceptables, un groupe protecteur physiologiquement acceptable, un groupe alkyle en $C_1$ à $C_4$, un groupe benzyle, un groupe hydroxyalkyle;

$R_2$ représente un atome d'hydrogène ou un groupe méthyle;

$R_3$ représente un groupe hydroxyle ou un groupe hydroxyméthyle;

$R_4$ et $R_5$ sont identiques ou différents, et représentent un atome d'hydrogène, un groupe méthyle, un groupe hydroxy ou un atome d'halogène sous réserve que :

(I) lorsqu'au moins un des symboles $R_4$ et $R_5$ est un groupe méthyle, un groupe hydroxy ou un atome d'halogène, $R_6$ est un groupe alkyle en $C_1$ à $C_9$ qui peut avoir une ramification ou une double liaison, ou un groupe alkyle en $C_1$ à $C_9$ ayant un groupe alcoxy comme substituant; ou

(II) lorsque $R_4$ et $R_5$ sont tous deux des atomes d'hydrogène, $R_6$ est un groupe alkyle en $C_5$ à $C_9$ qui peut avoir une ramification ou une double liaison, ou un groupe alkyle en $C_1$ à $C_9$ ayant un groupe alcoxy comme substituant, dans lequel la liaison en $C_2$ à $C_3$ peut être une double liaison,

et d'un excipient pharmaceutiquement acceptable.

**24.** Procédé selon la revendication 23, dans lequel $R_4$ et/ou $R_5$ sont des atomes d'halogène.

**25.** Procédé selon la revendication 23, dans lequel $R_4$ et/ou $R_5$ sont des atomes de fluor.

**26.** Procédé selon la revendication 23, dans lequel $R_4$ et/ou $R_5$ sont des groupes méthyle.

**27.** Procédé selon la revendication 23, dans lequel la prostaglandine E a un groupe méthyle à la position 19.

**28.** Procédé selon la revendication 23, dans lequel $R_6$ est un groupe hexyle.

**29.** Procédé selon la revendication 23, dans lequel $R_6$ est un groupe isopentyle.

**30.** Procédé selon la revendication 23, dans lequel $R_6$ est un groupe pentyl-2S.

**31.** Procédé selon la revendication 23, dans lequel le groupe carboxyle à la position terminale de la chaîne $\alpha$ des prostaglandines est estérifié par un groupe alkyle.

**32.** Procédé selon la revendication 23, dans lequel les prostaglandines E sont des 13,14-dihydro-15-céto-PGE ayant un groupe méthyle ou un atome de fluor à la position 16, ou leurs esters alkyliques.

**33.** Procédé selon la revendication 23, dans lequel la prostaglandine E est un ester alkylique de la 13,14-dihydro-15-céto-16R,S-méthyl-$PGE_2$.

**34.** Procédé selon la revendication 23, dans lequel la prostaglandine E est un ester alkylique de la 13,14-dihydro-6,15-dicéto-16R,S-méthyl-$PGE_1$.

**35.** Procédé selon la revendication 23, dans lequel la prostaglandine E est la 13,14-dihydro-15-céto-16R,S-fluoro-$PGE_2$ ou un de ses esters alkyliques.

**36.** Procédé la revendication 23, dans lequel la prostaglandine E est la 13,14-dihydro-6,15-dicéto-16R,S-fluoro-$PGE_1$ ou un de ses esters alkyliques.

**37.** Procédé selon la revendication 23, dans lequel la prostaglandine E est la 13,14-dihydro-15-céto-19-méthyl-$PGE_2$ ou un ses esters alkyliques.

**38.** Procédé selon la revendication 23, dans lequel la prostaglandine E est une 13,14-dihydro-6,15-dicéto-19-méthyl-$PGE_2$ ou un de ses esters alkyliques.

**39.** Procédé selon la revendication 23, dans lequel la prostaglandine E est la 13,14-dihydro-15-céto-20-éthyl-$PGE_2$ ou un de ses esters alkyliques.

**40.** Procédé selon la revendication 23, dans lequel la prostaglandine E est la 13,14-dihydro-15-céto-16,16-difluoro-$PGE_2$ ou un de ses esters alkyliques.

**41.** Procédé selon la revendication 23, dans lequel la prostaglandine E est la 13,14-dihydro-15-céto-20-méthyl-$PGE_1$ ou un de ses esters alkyliques.

**42.** Procédé selon la revendication 23, dans lequel la prostaglandine E est la 13,14-dihydro-15-céto-$\Delta^2$-$PGE_1$ ou un de ses esters alkyliques.

**43.** Procédé selon la revendication 23, dans lequel la prostaglandine E est la 13,14-dihydro-15-céto-16R,S-fluoro-20-méthyl-$PGE_2$ ou un de ses esters alkyliques.

**44.** Procédé selon la revendication 23, dans lequel la prostaglandine E est la 13,14-dihydro-15-céto-5,6-déshydro-20-méthoxy-$PGE_2$ ou un de ses esters alkyliques.

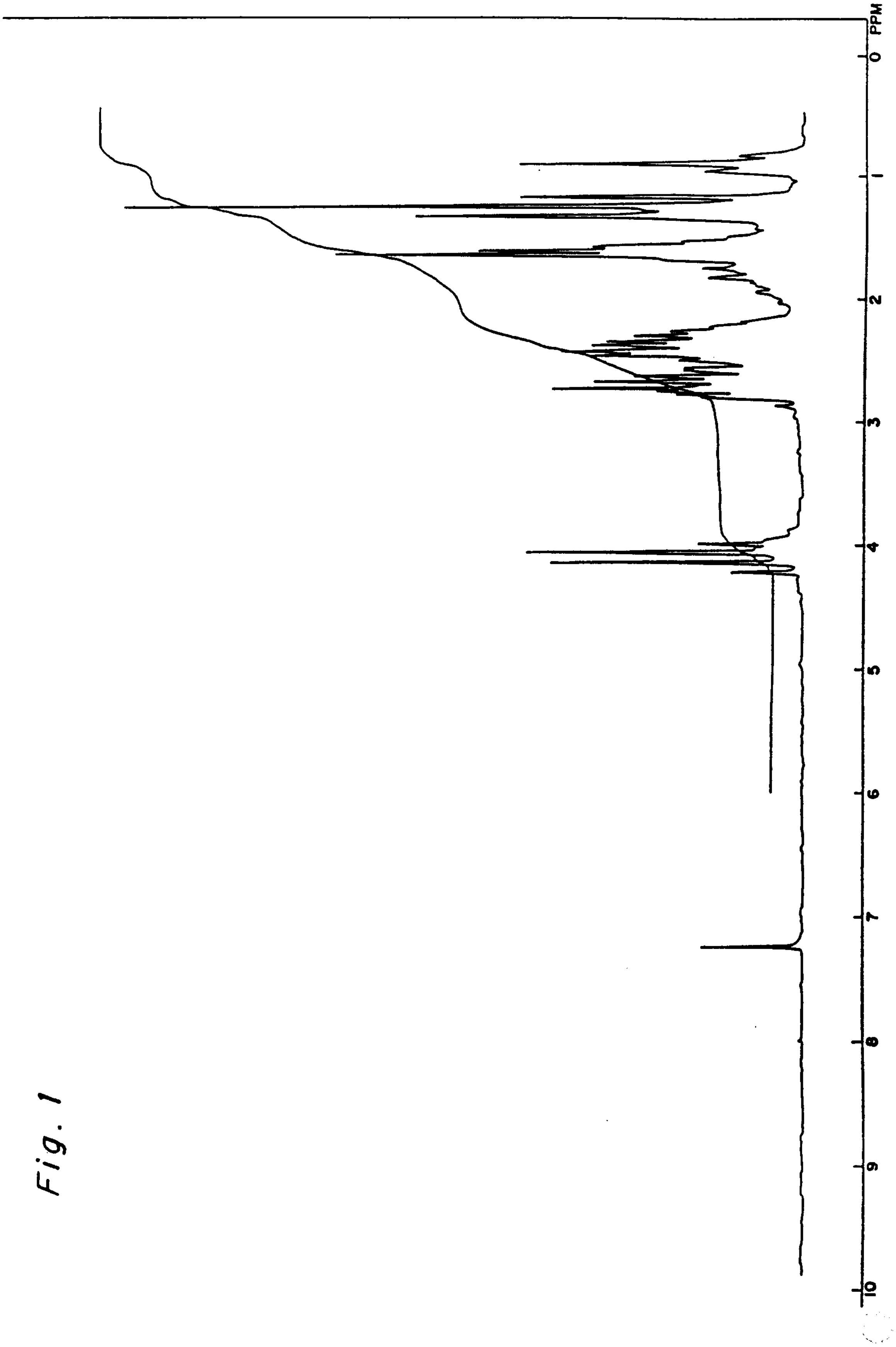


Fig. 1

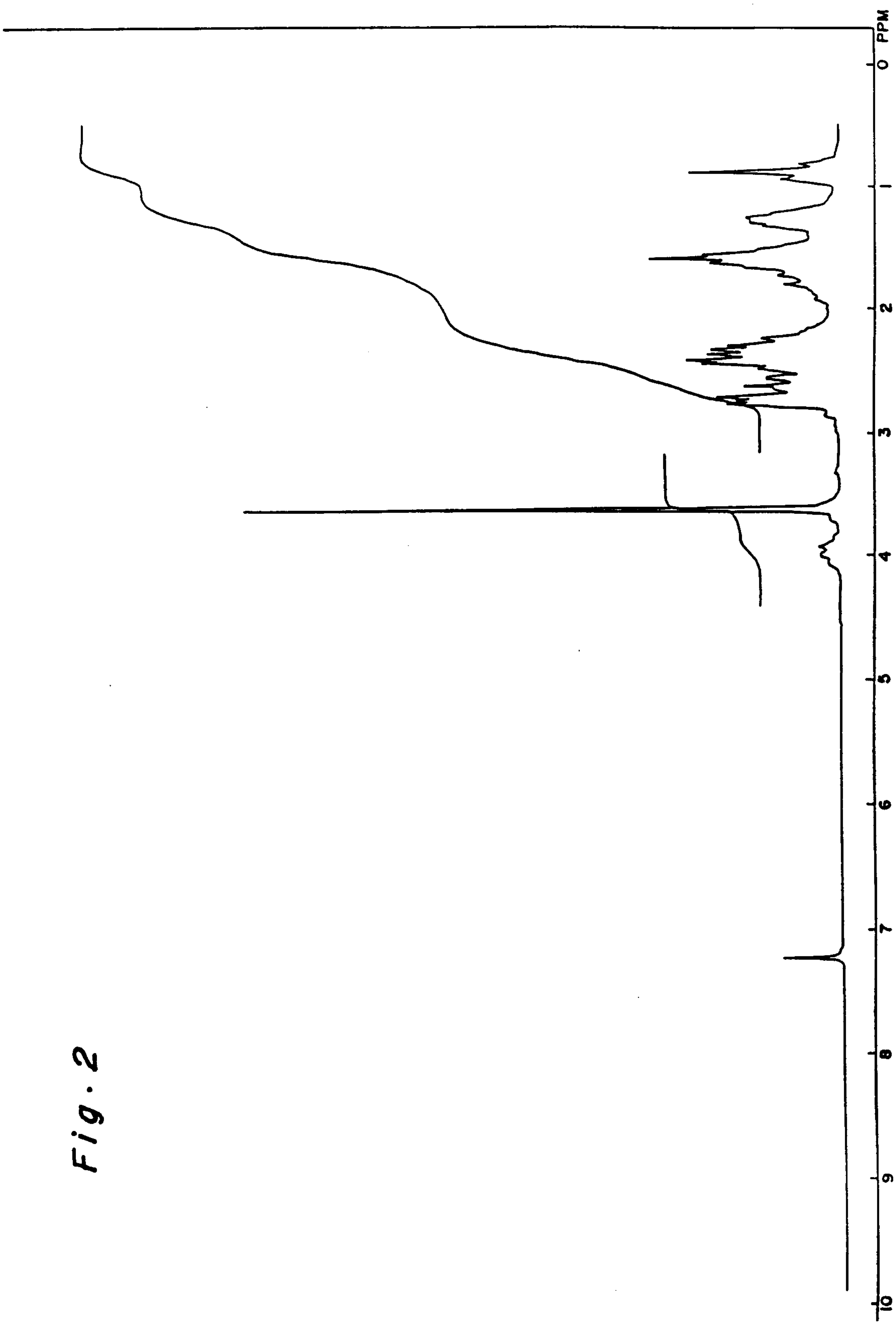


Fig. 2

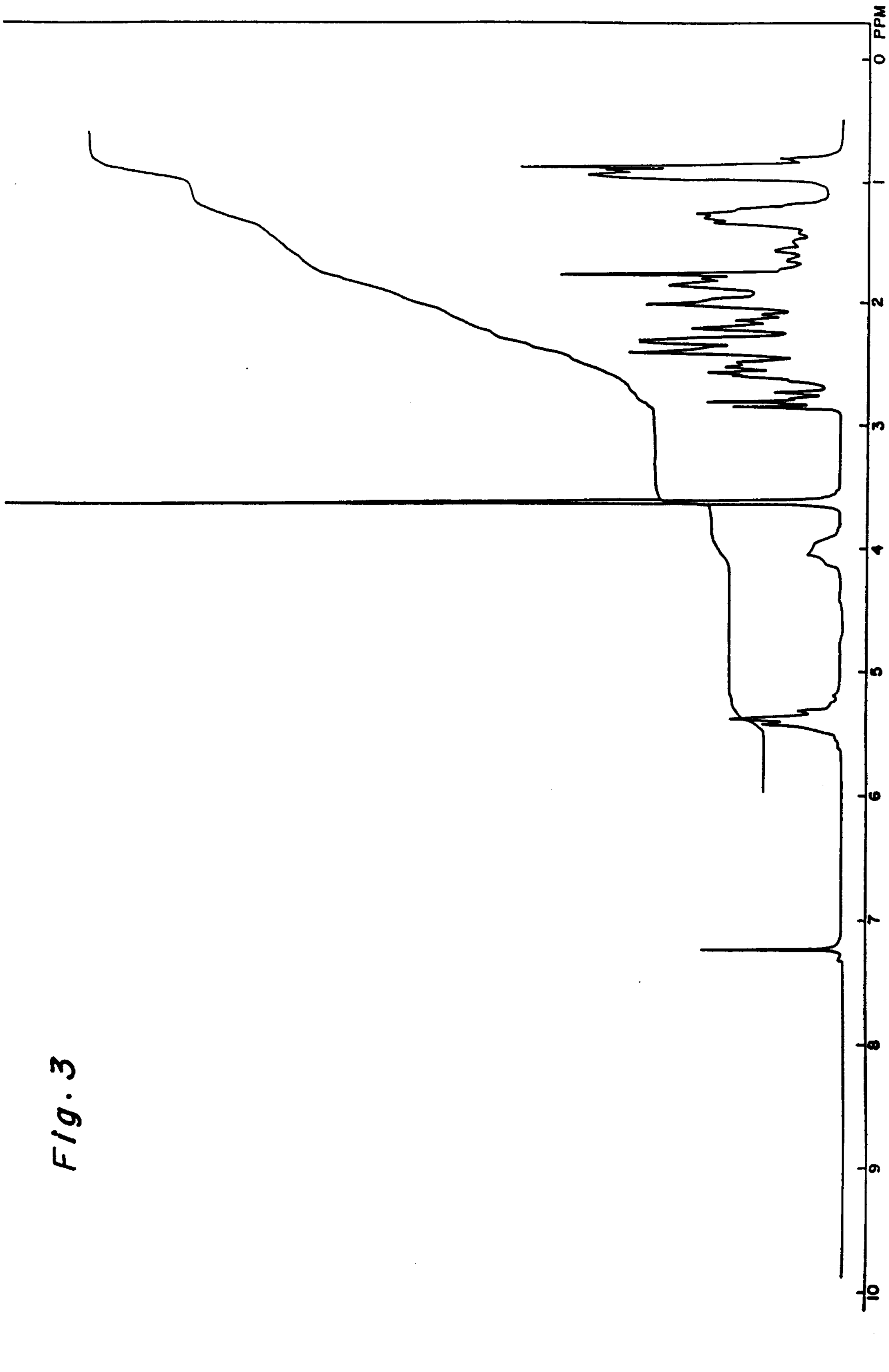


Fig. 3

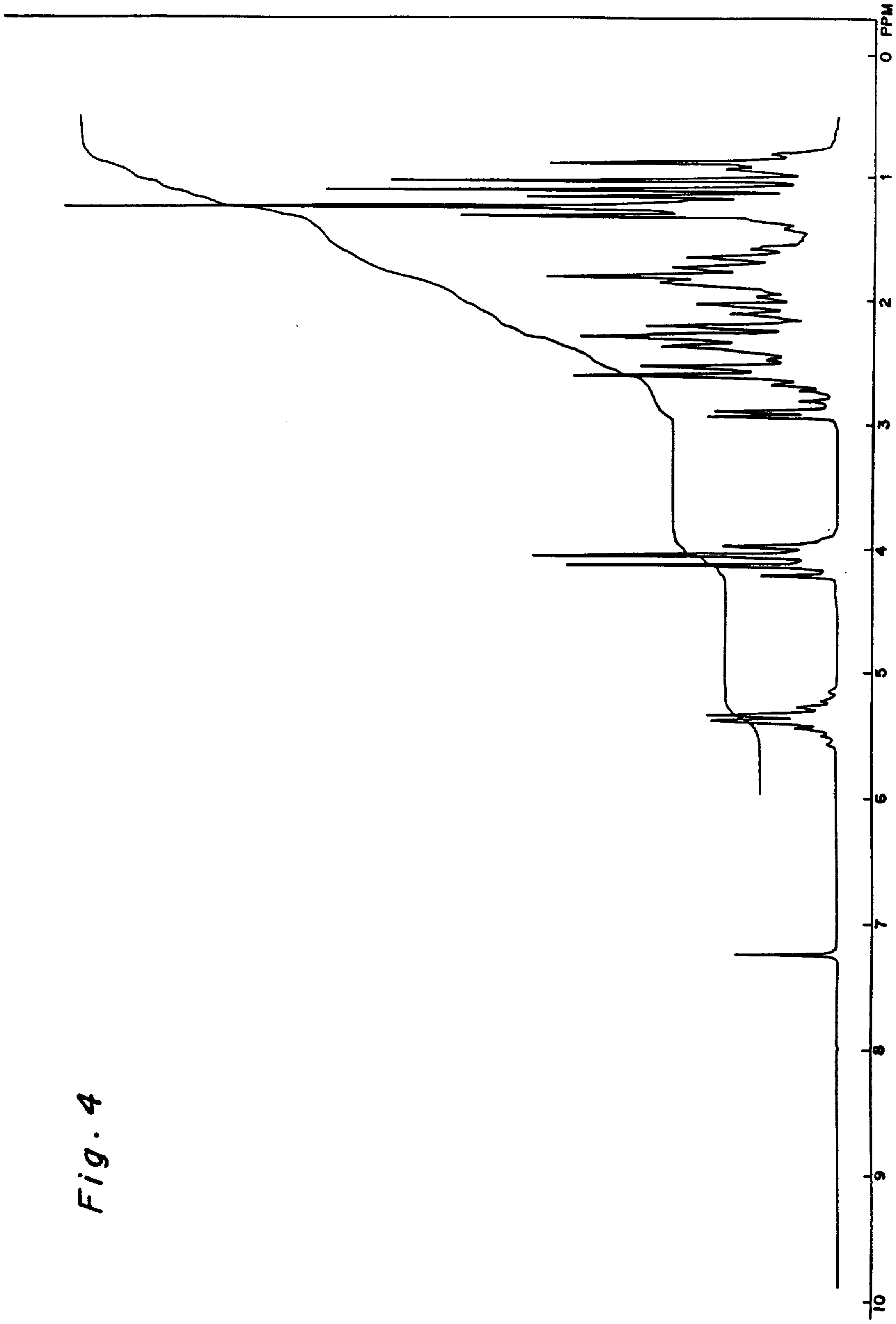


Fig. 4

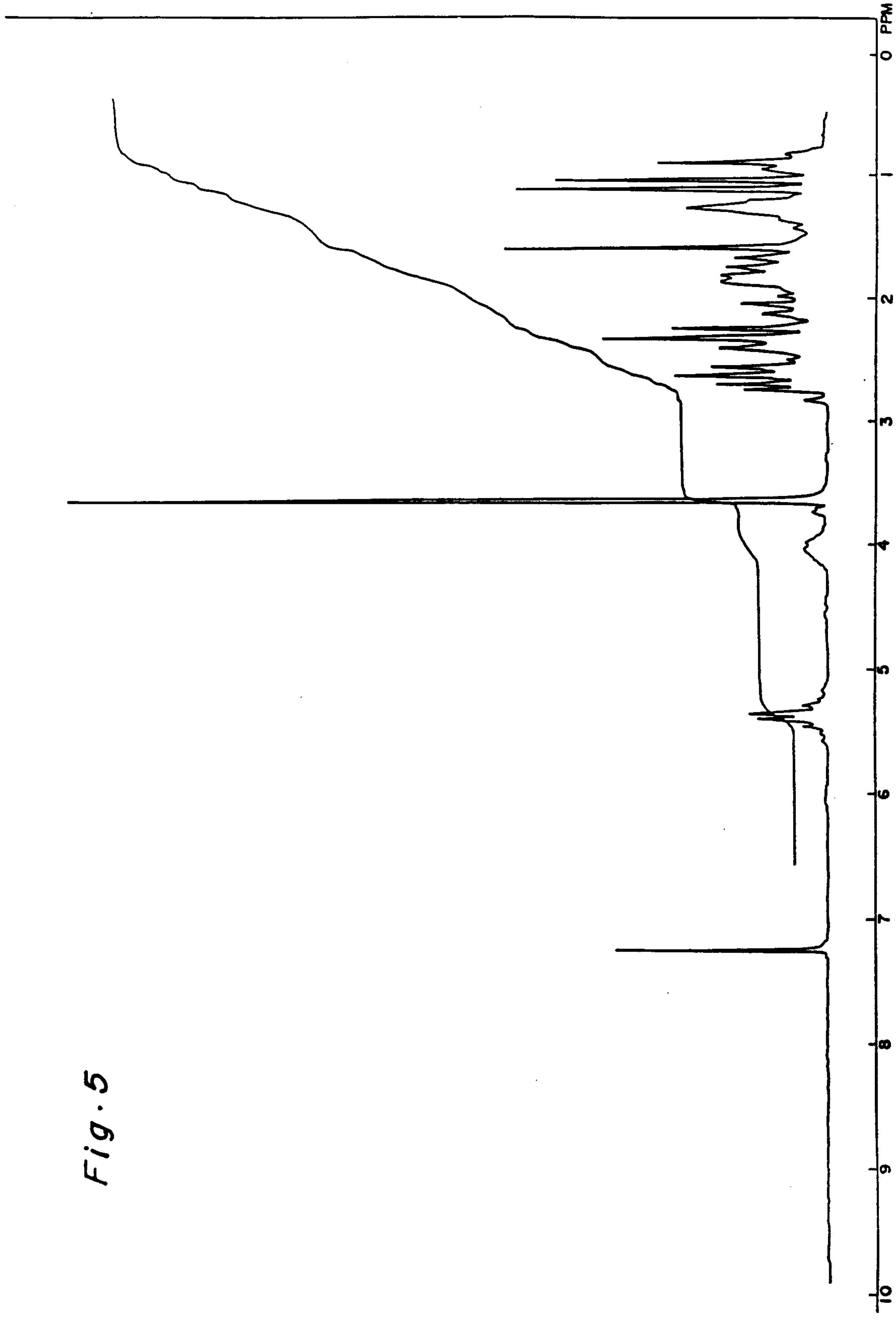


Fig. 5

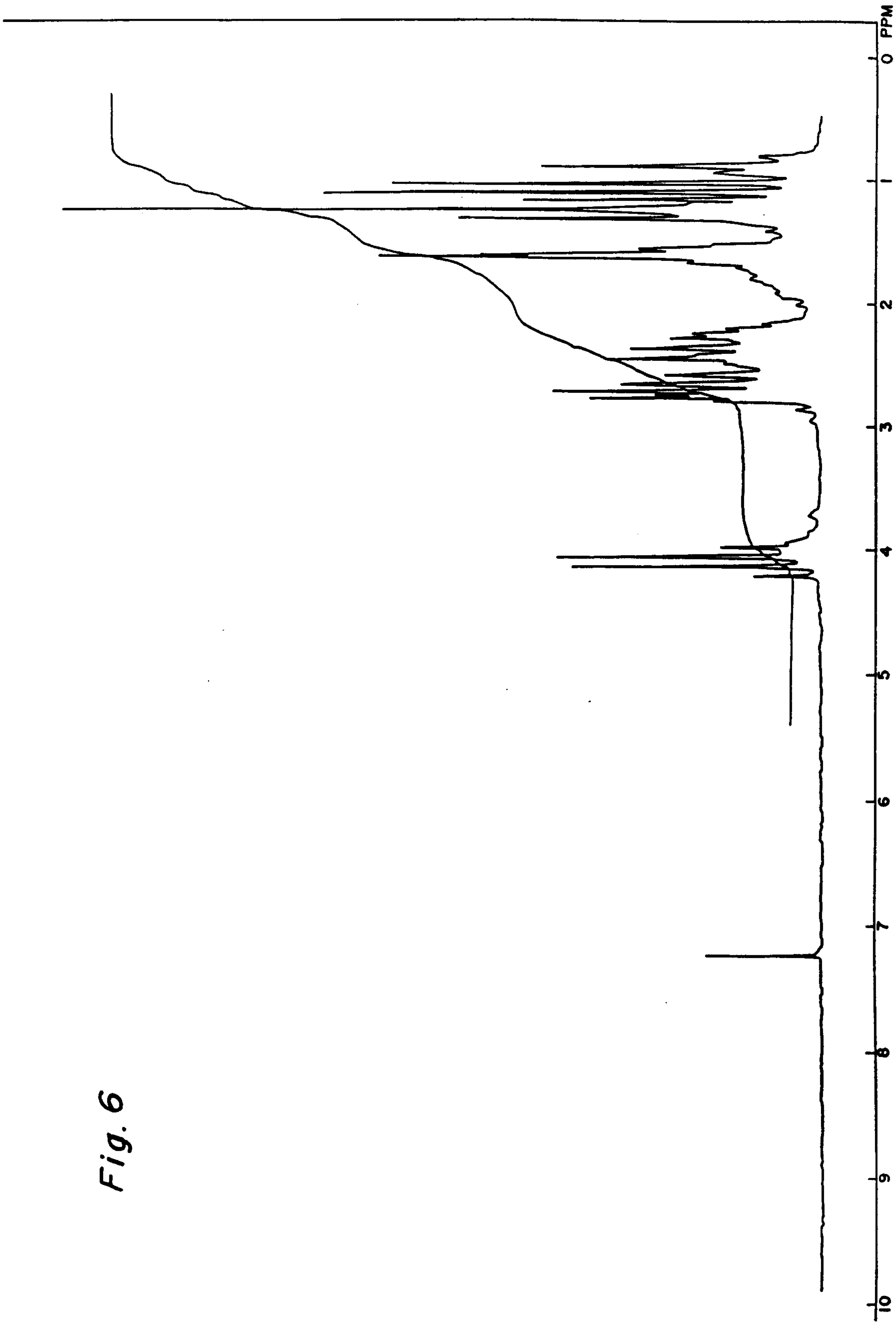


Fig. 6

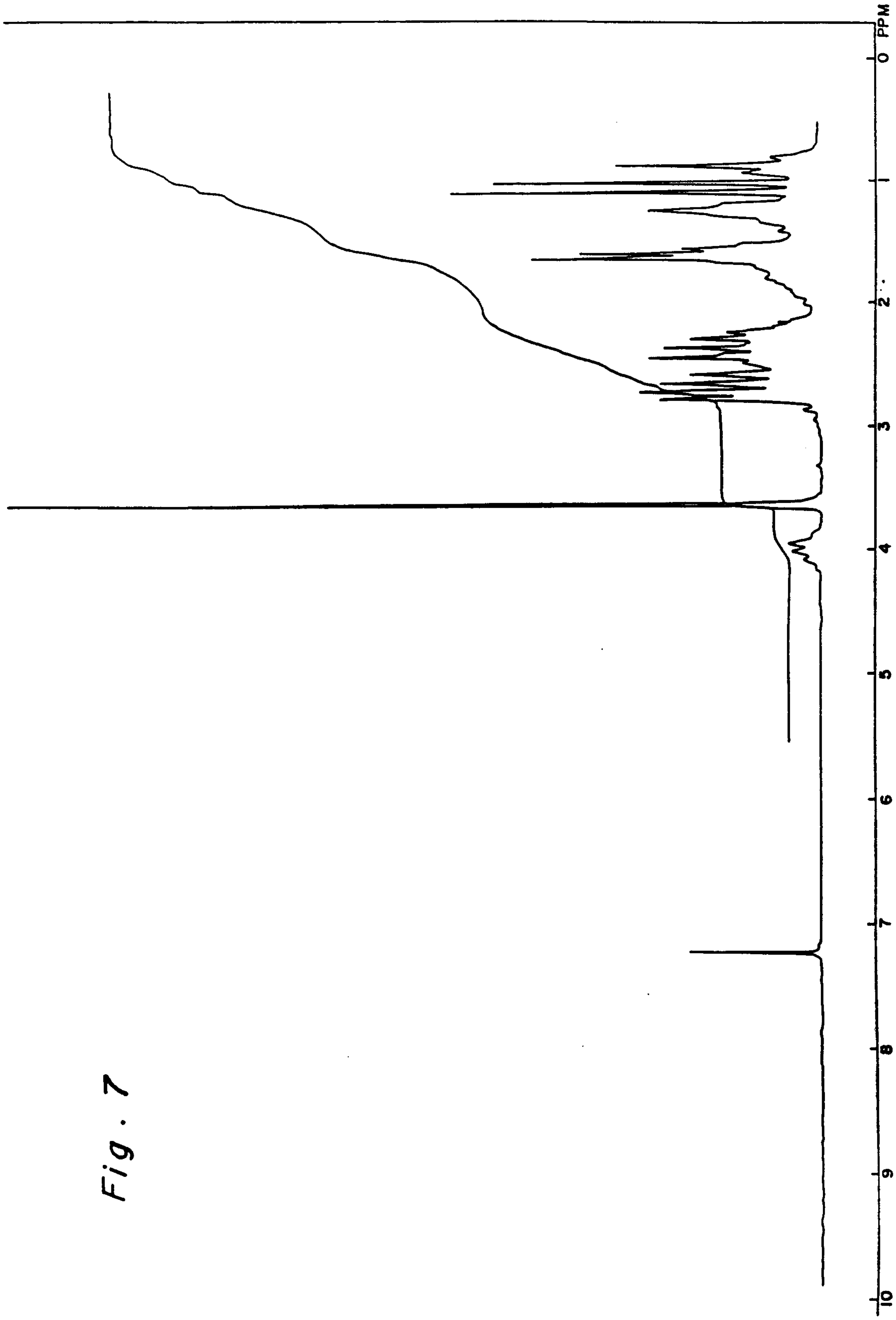


Fig. 7

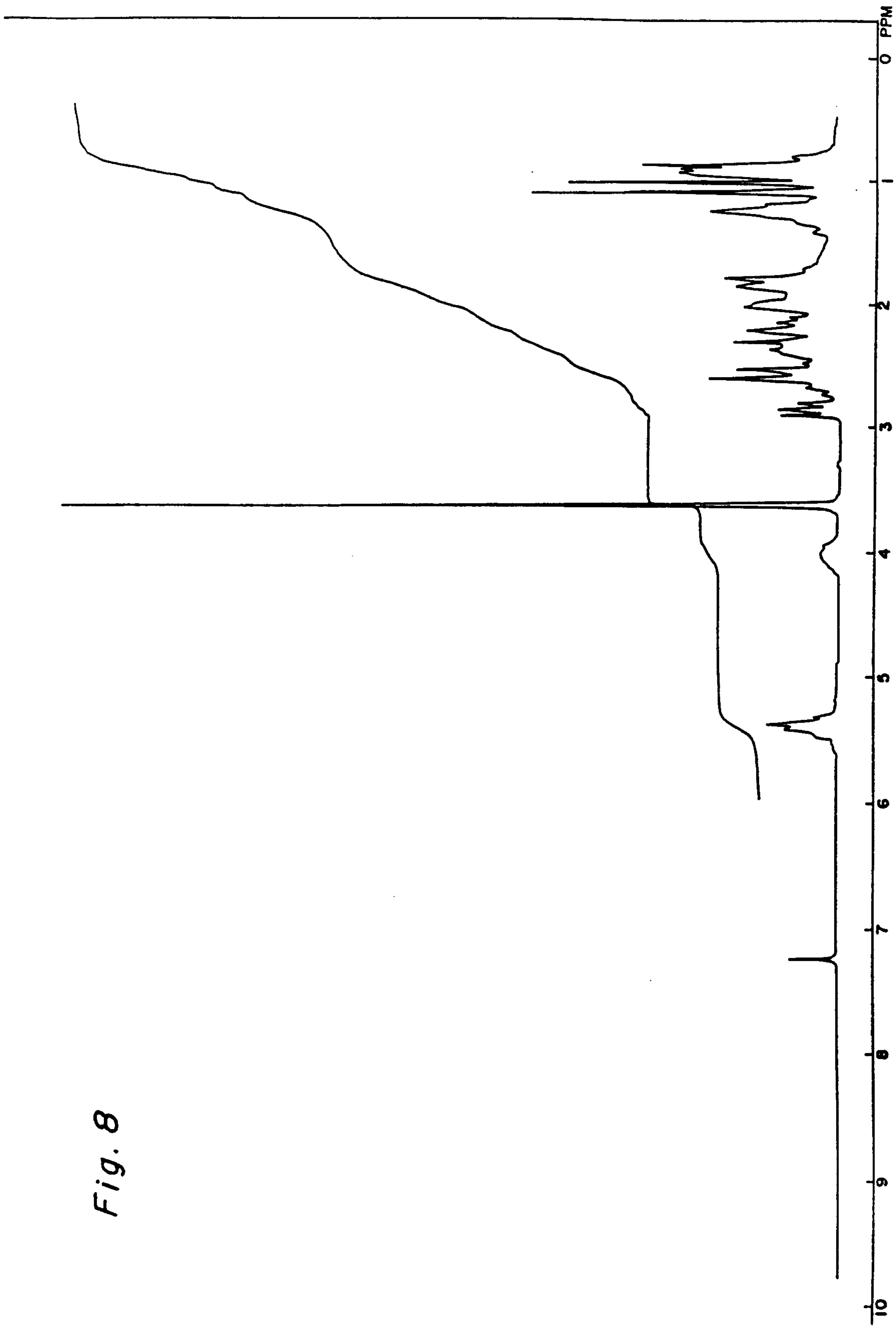


Fig. 8

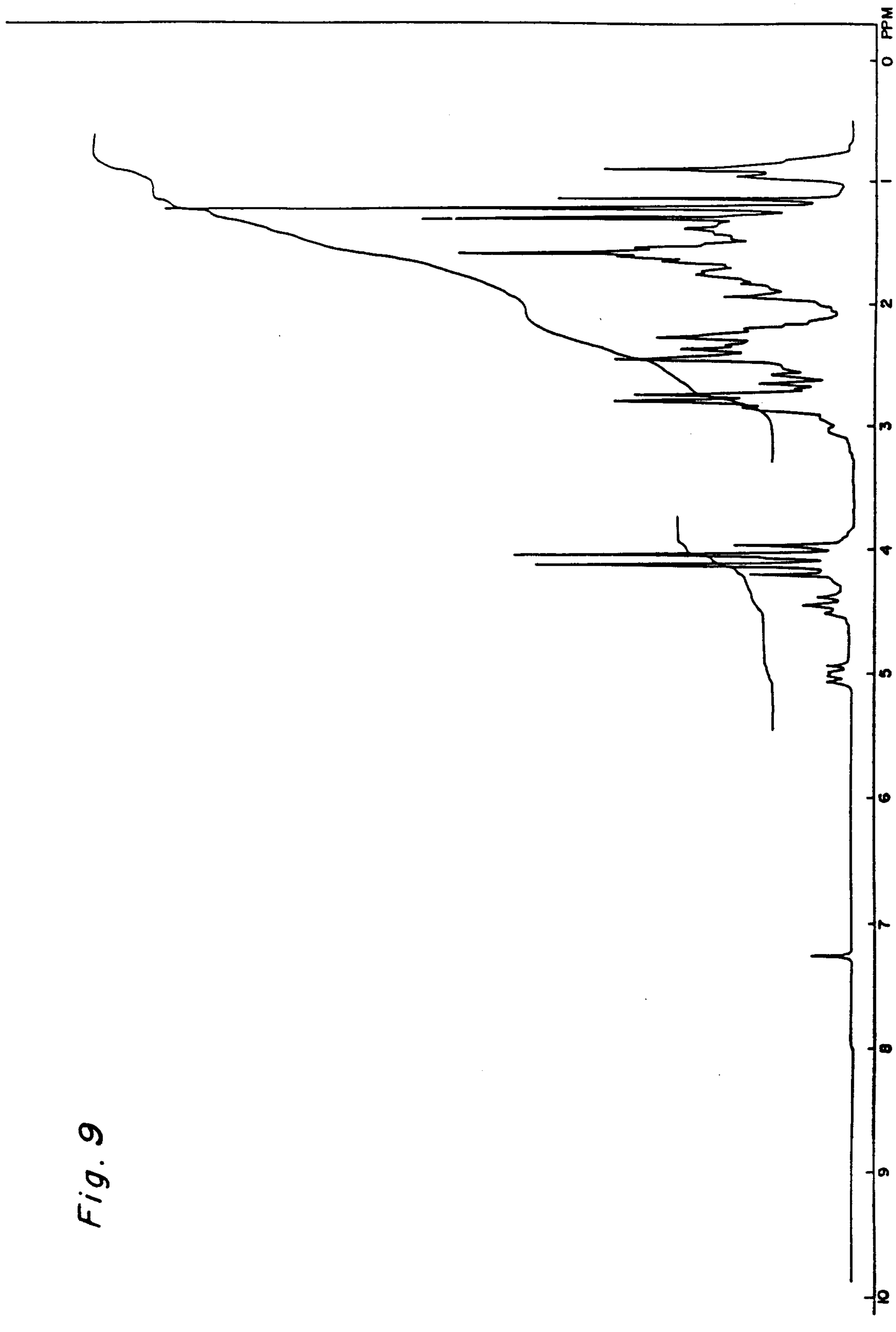


Fig. 9

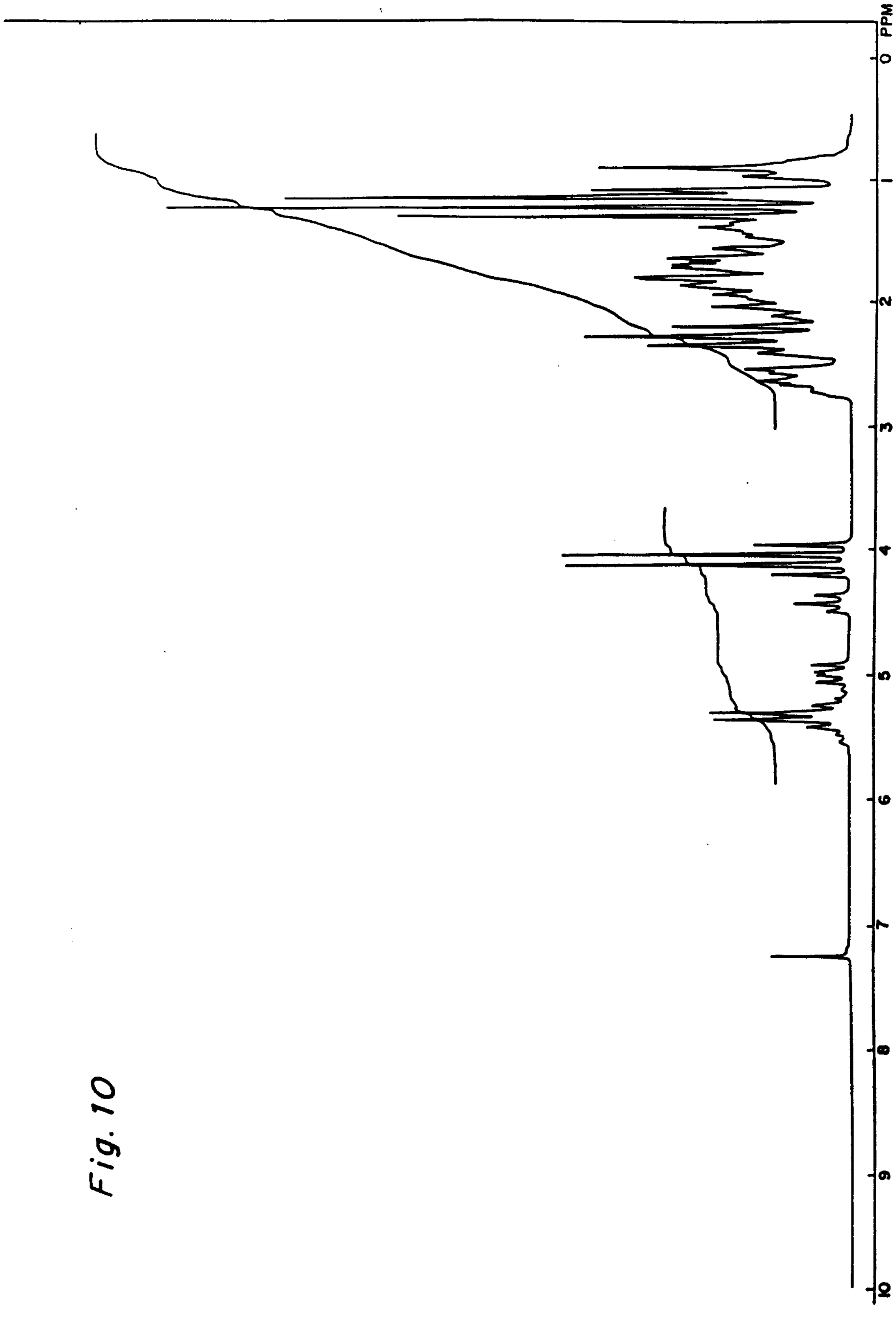


Fig. 10

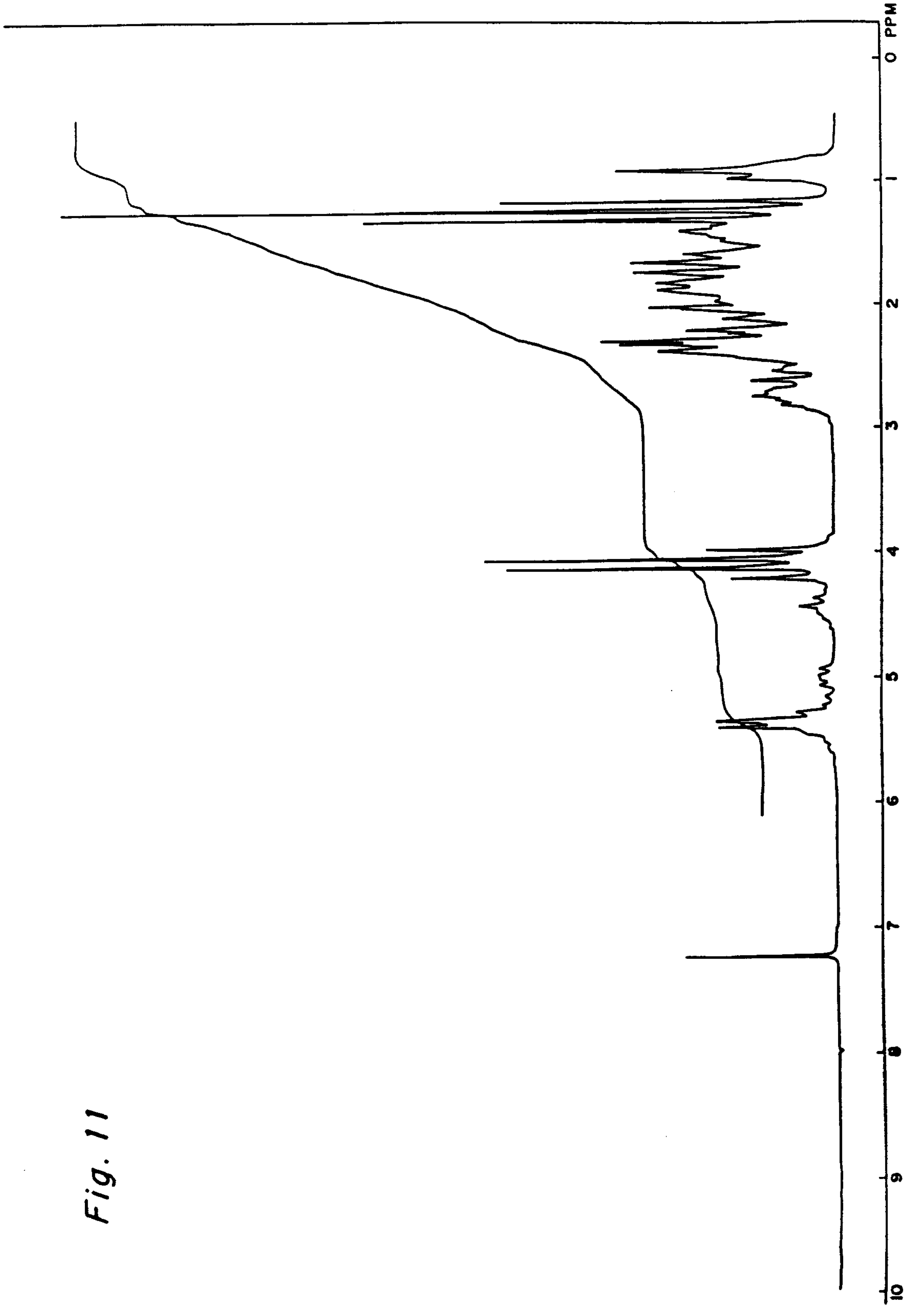


Fig. 11

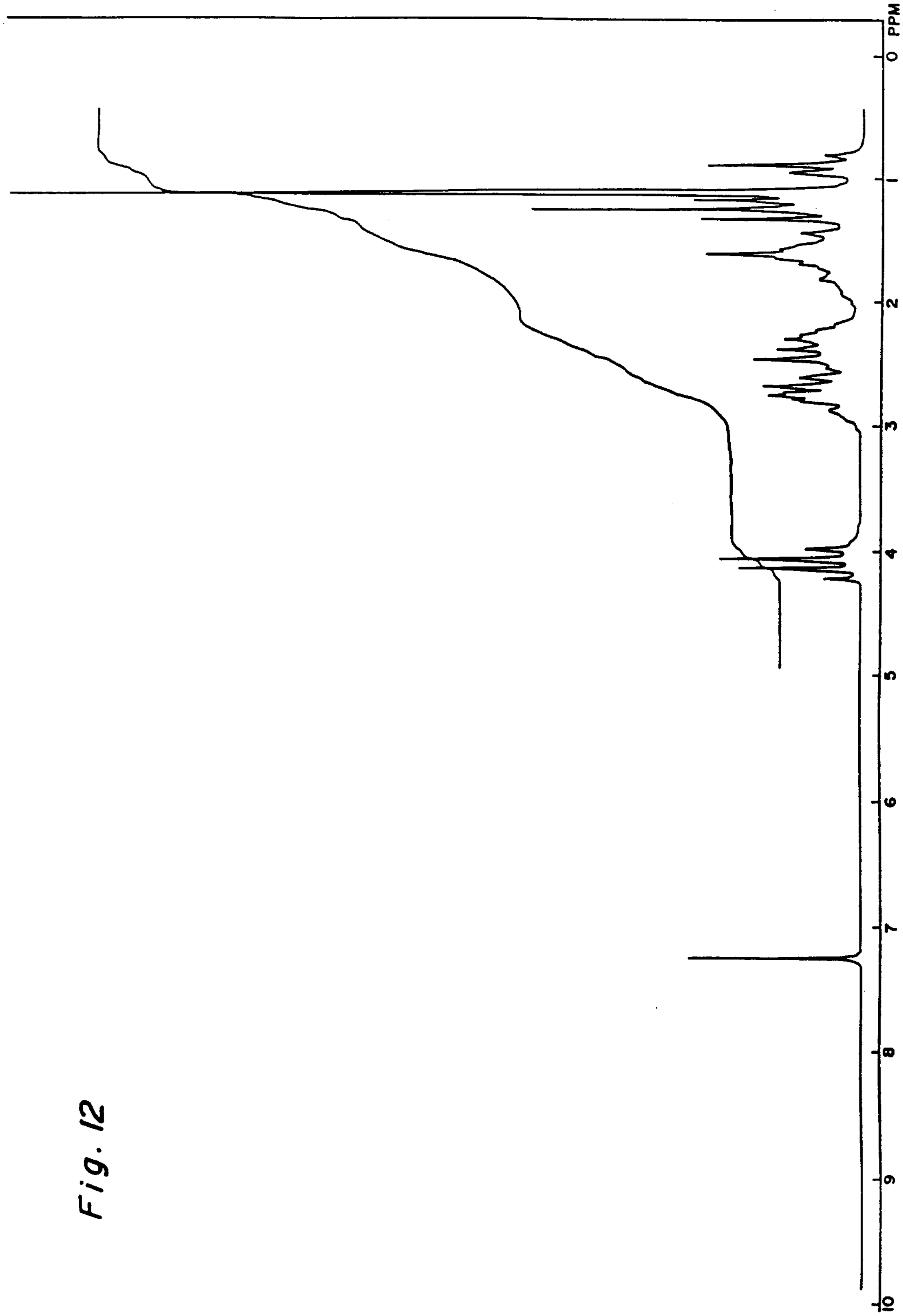


Fig. 12

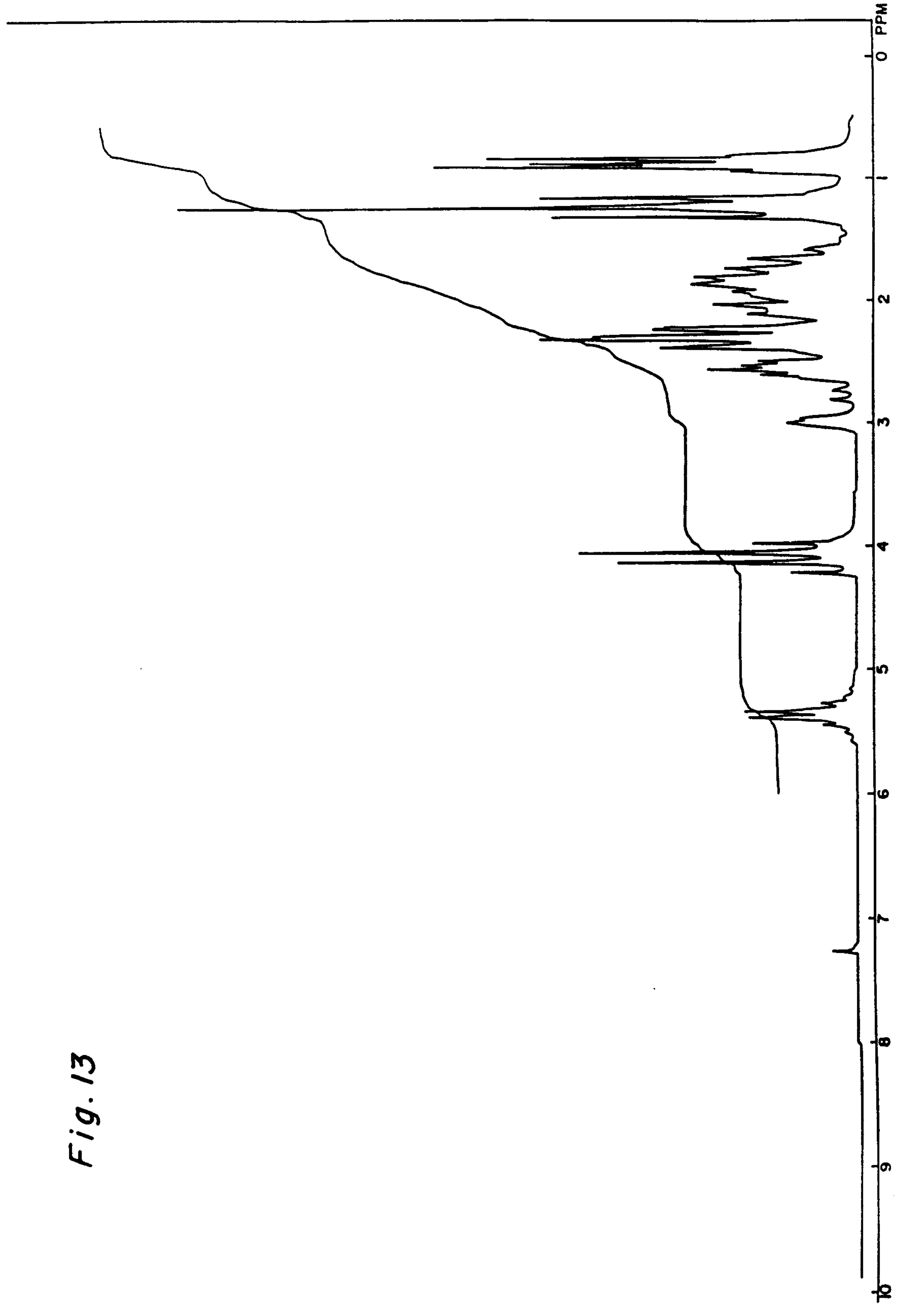


Fig. 13

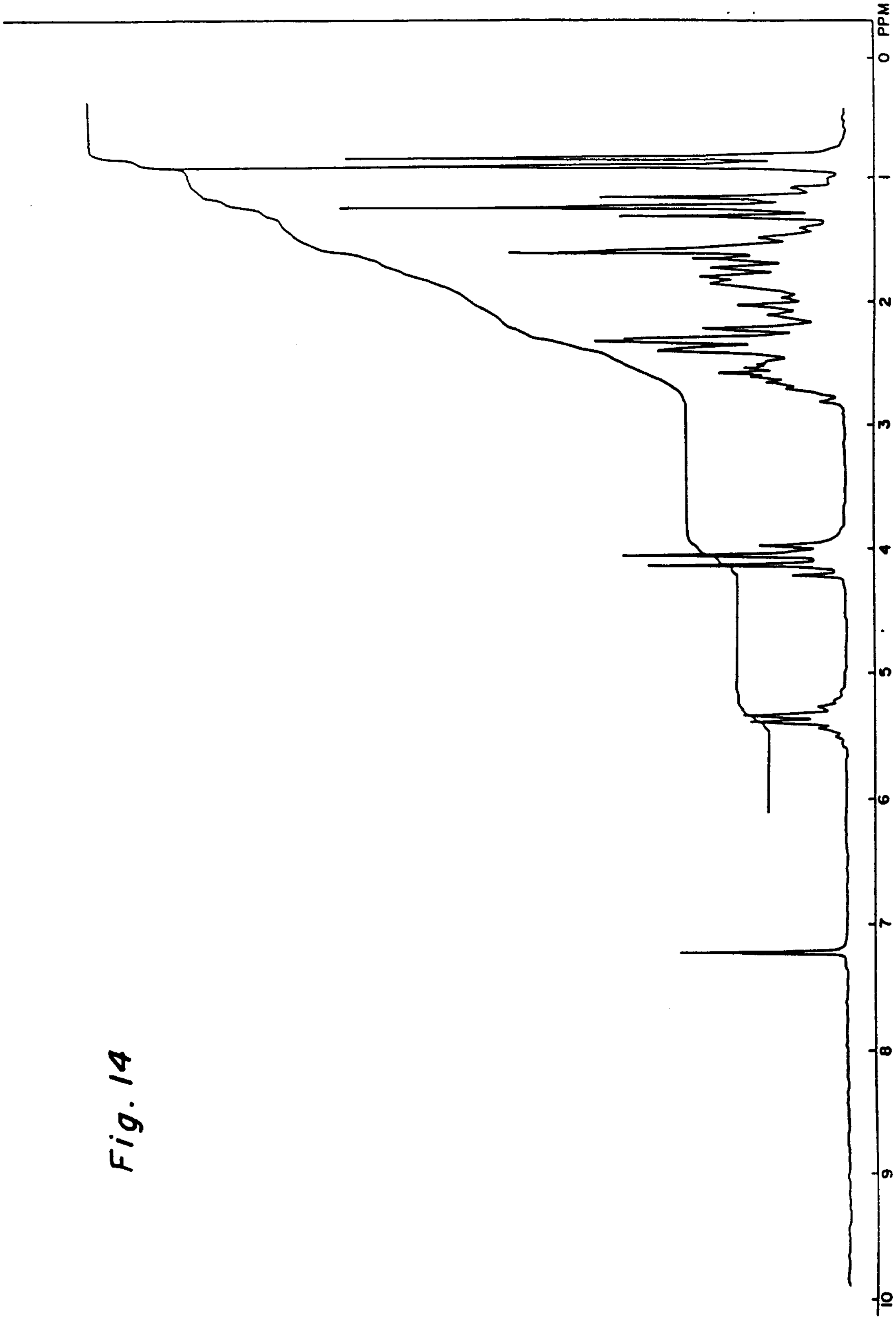


Fig. 14

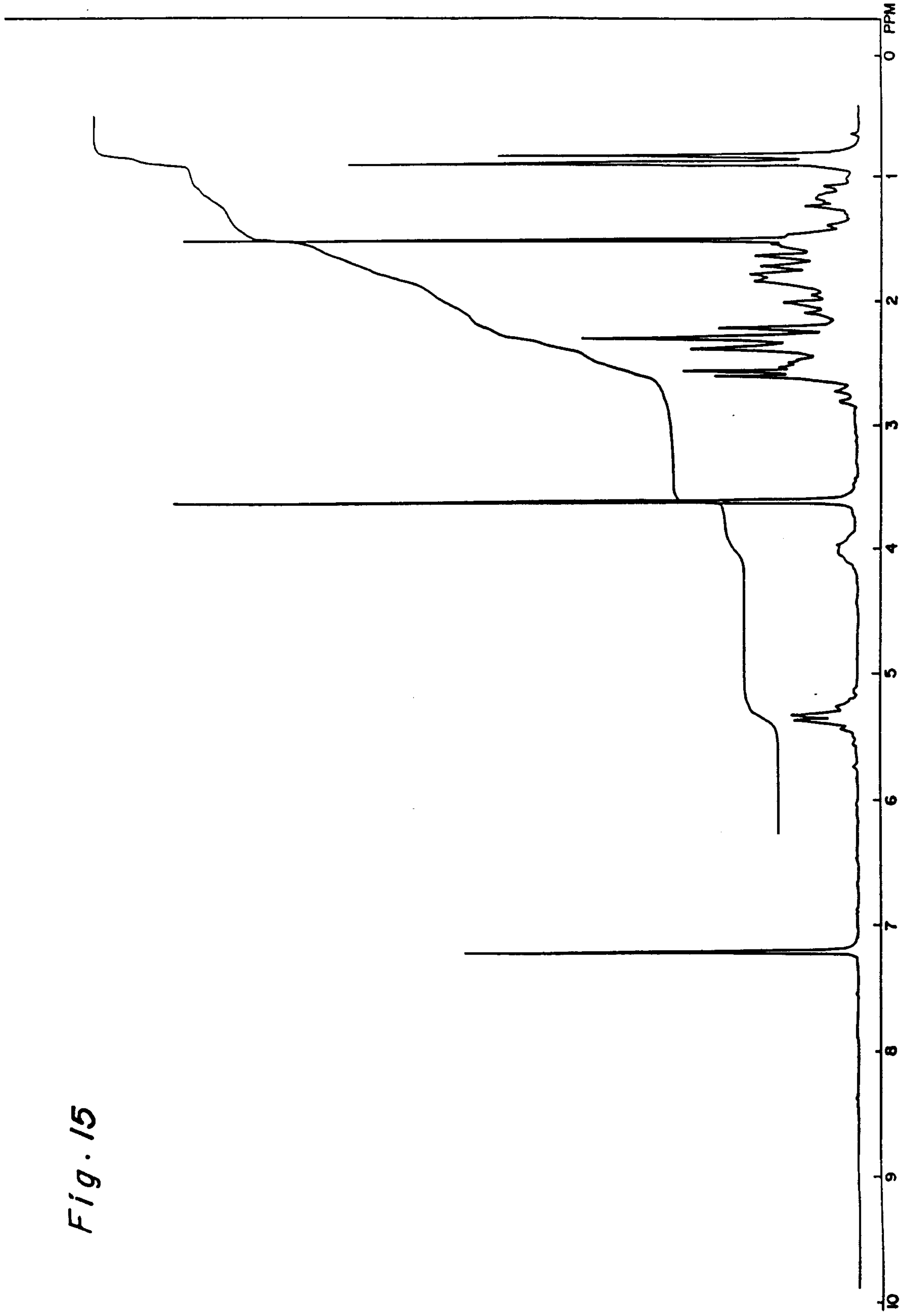


Fig. 15

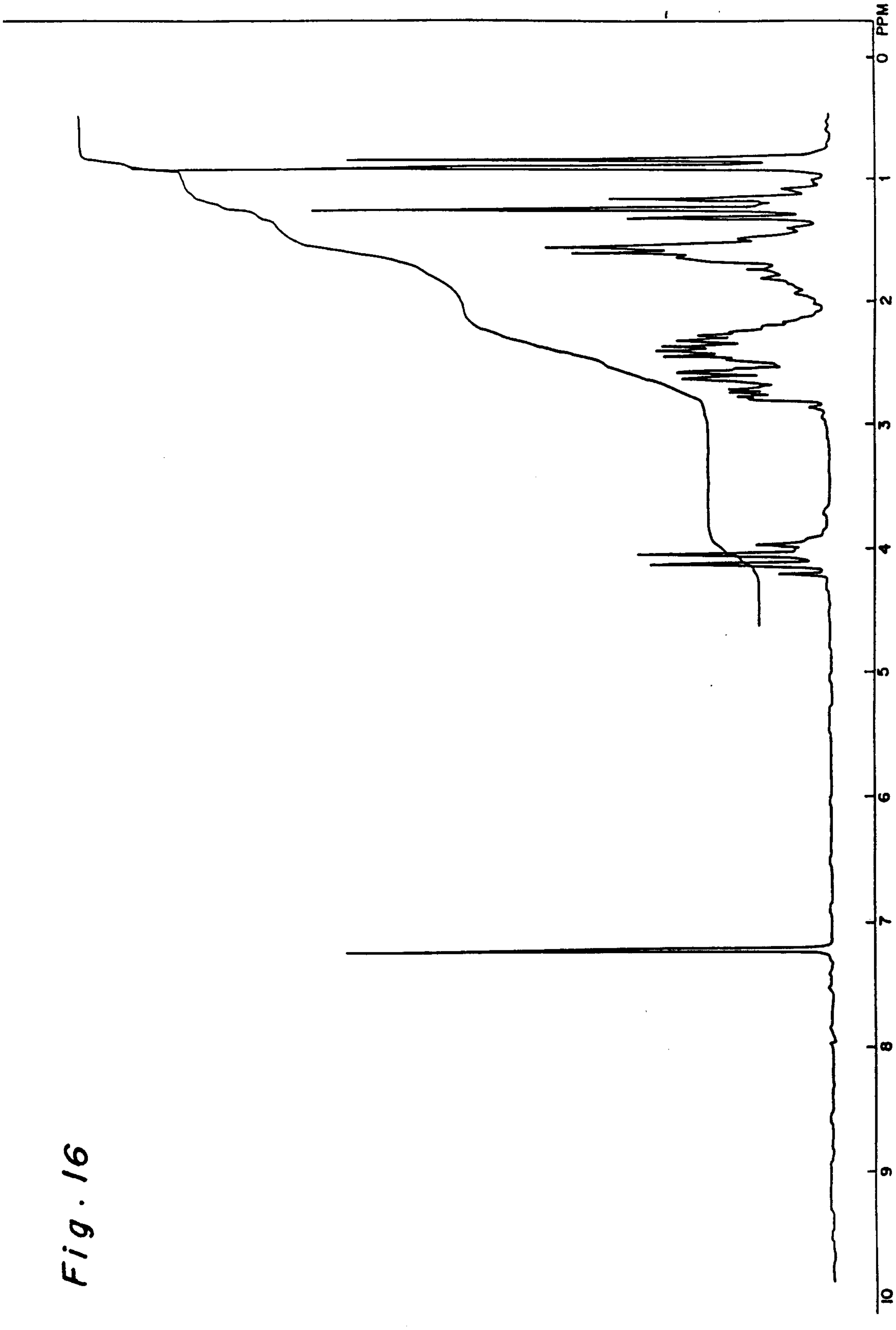


Fig. 16

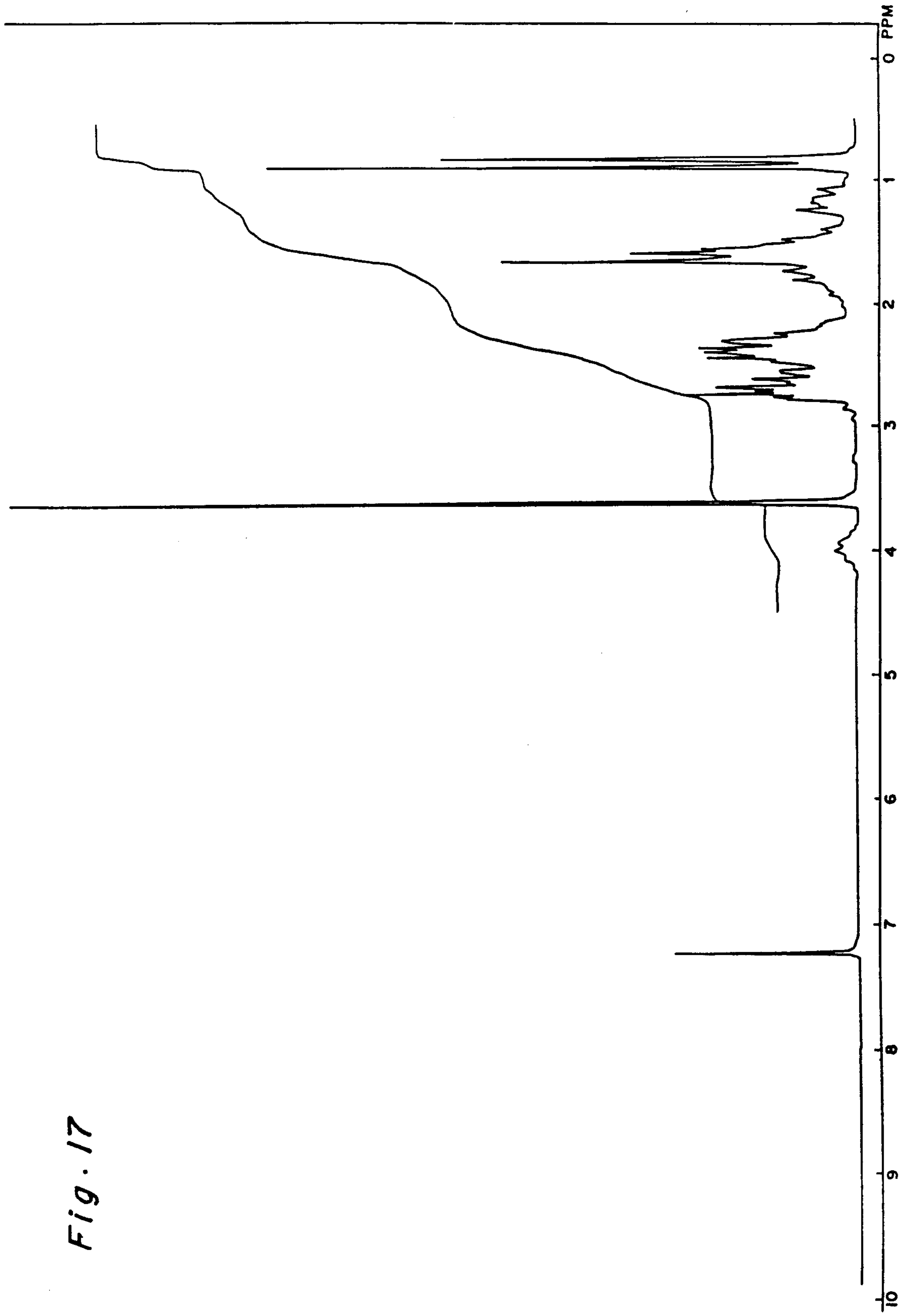


Fig. 17

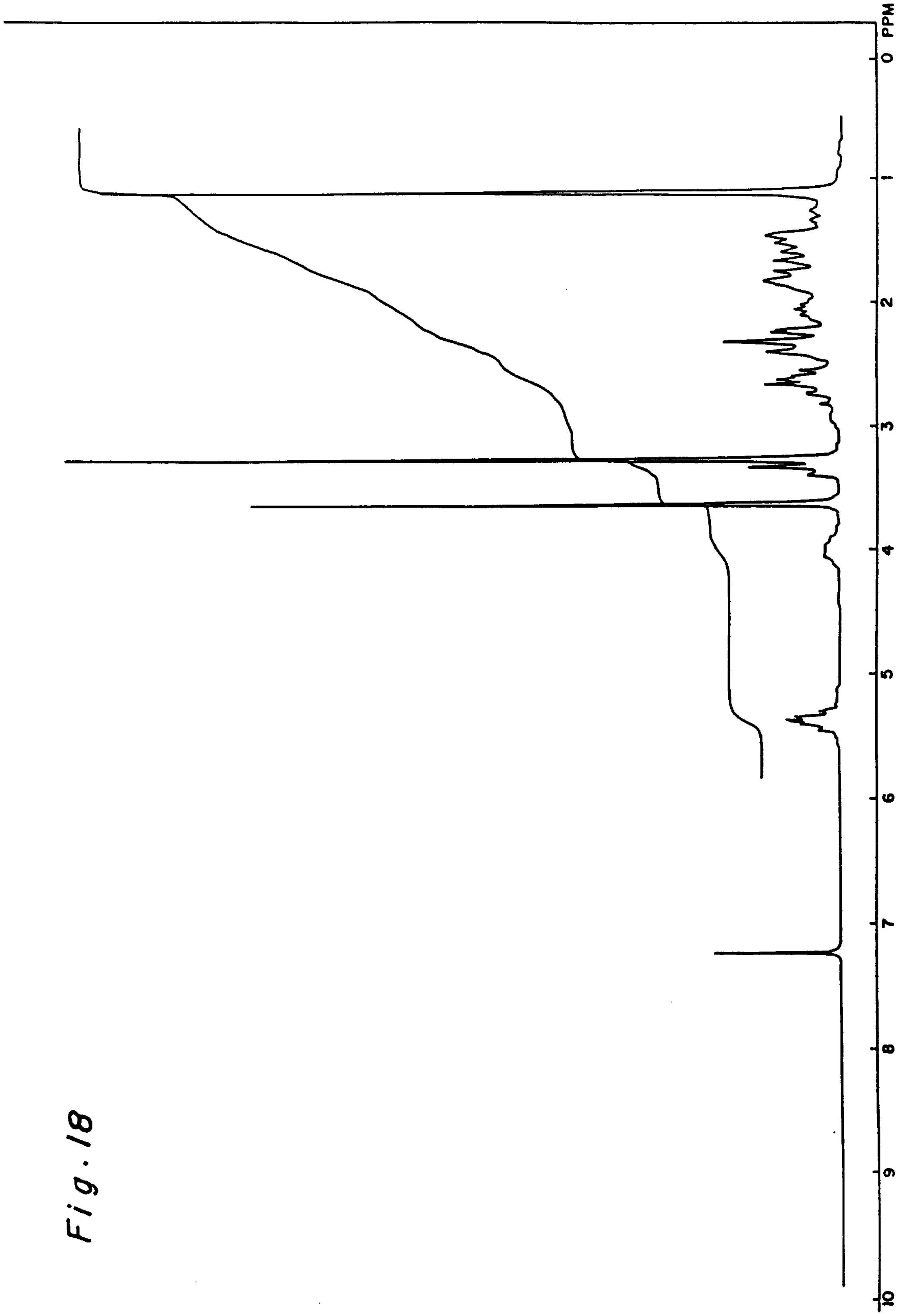


Fig. 18

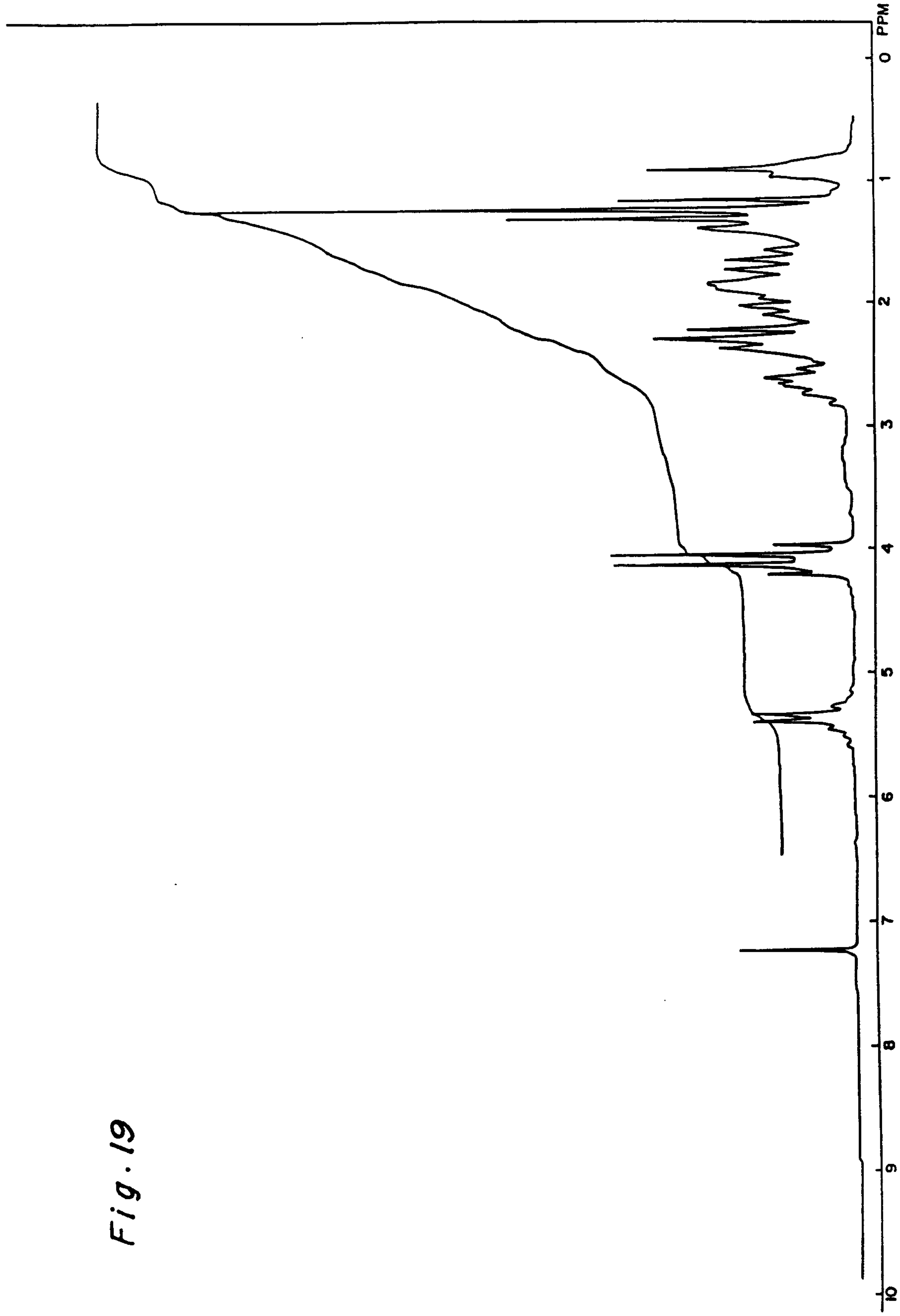


Fig. 19

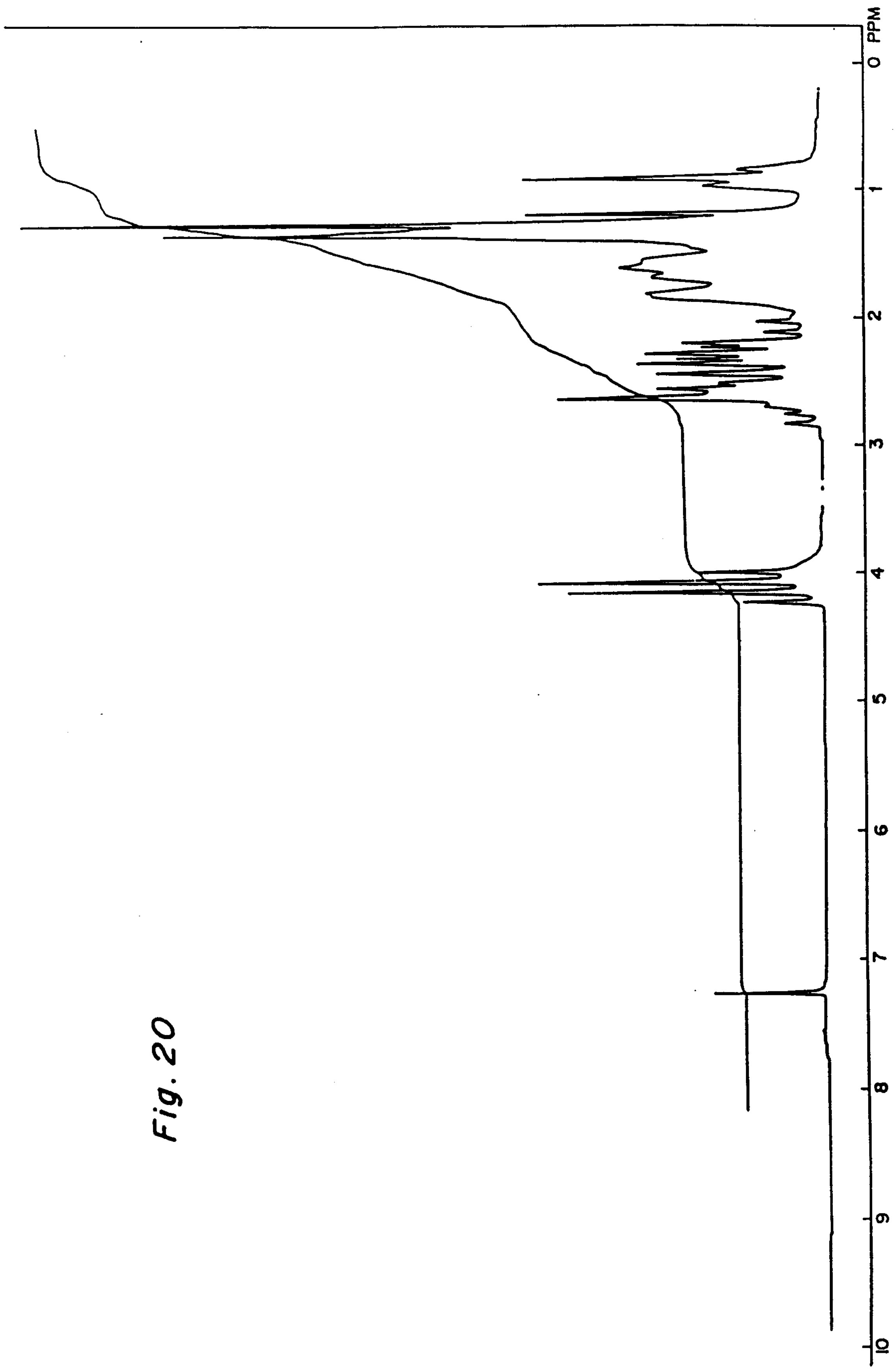


Fig. 20

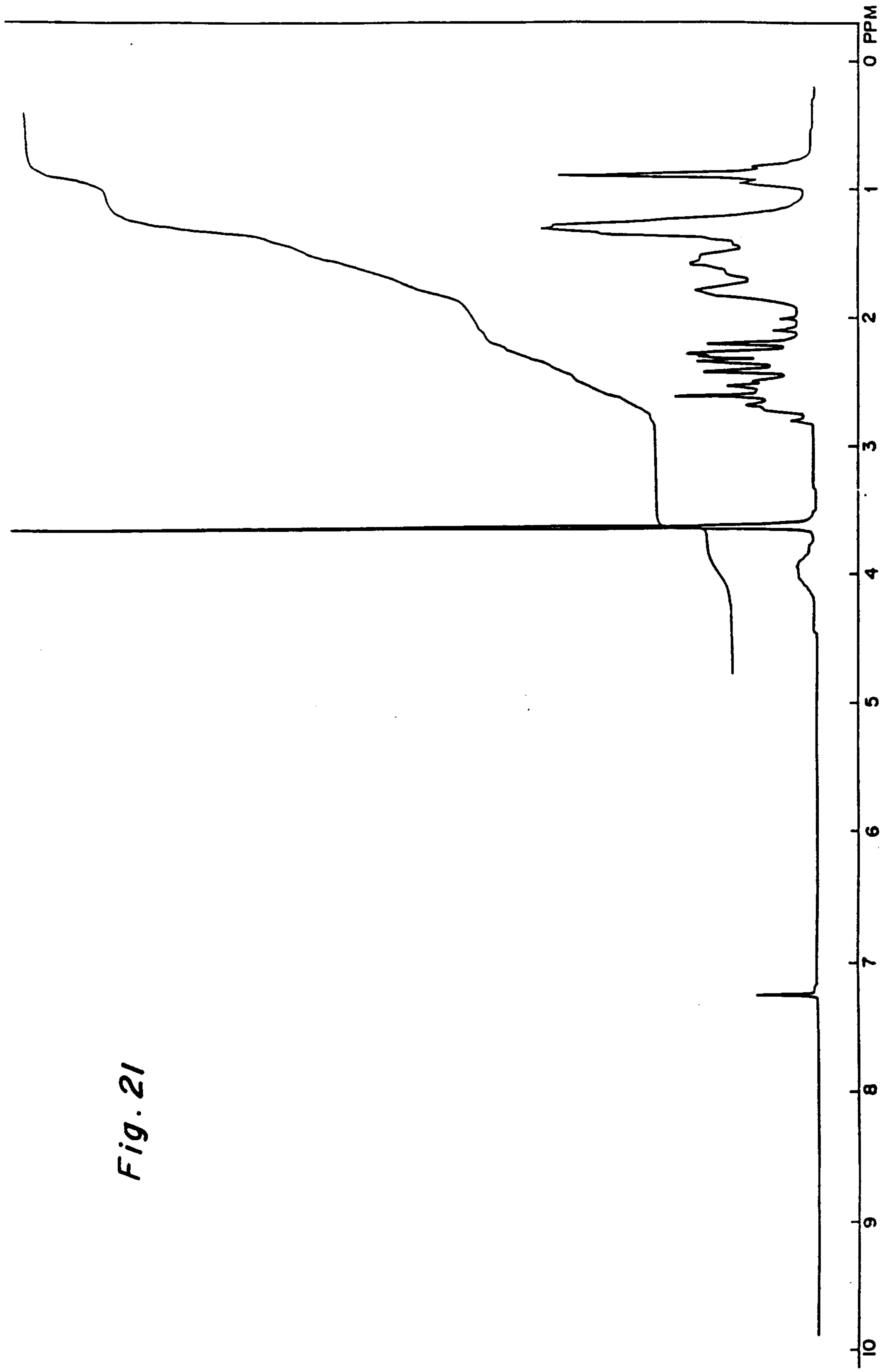


Fig. 21

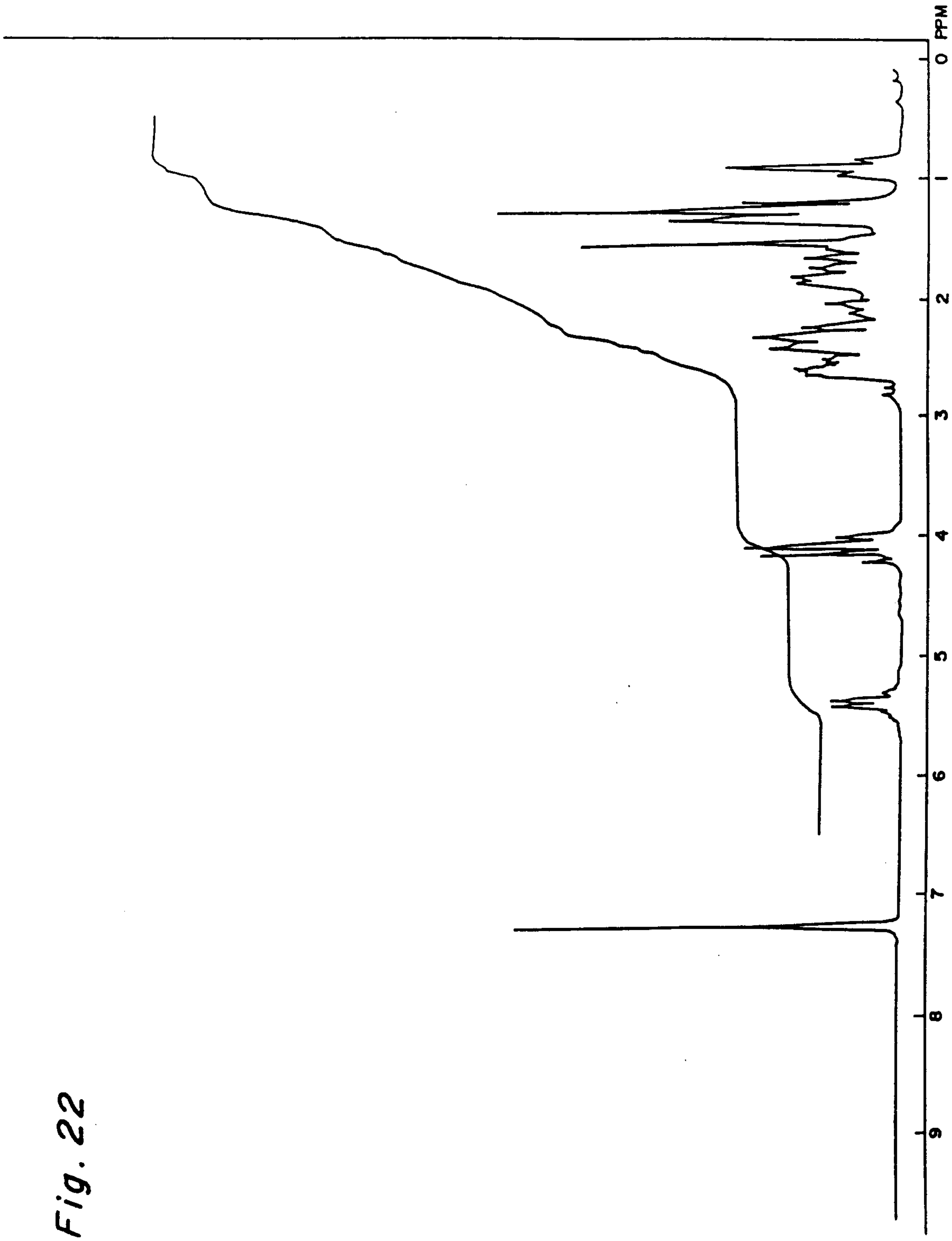


Fig. 22

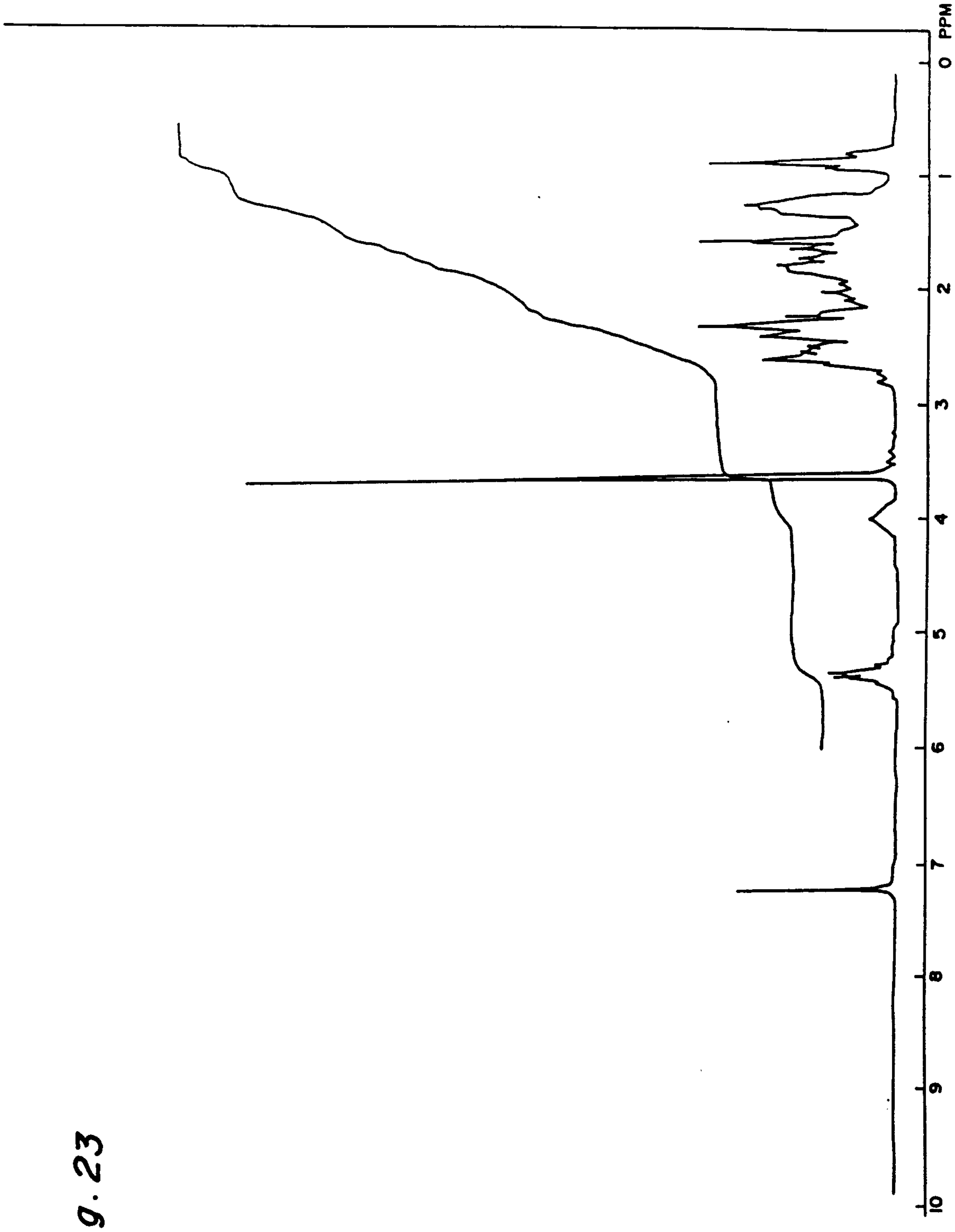


Fig. 23

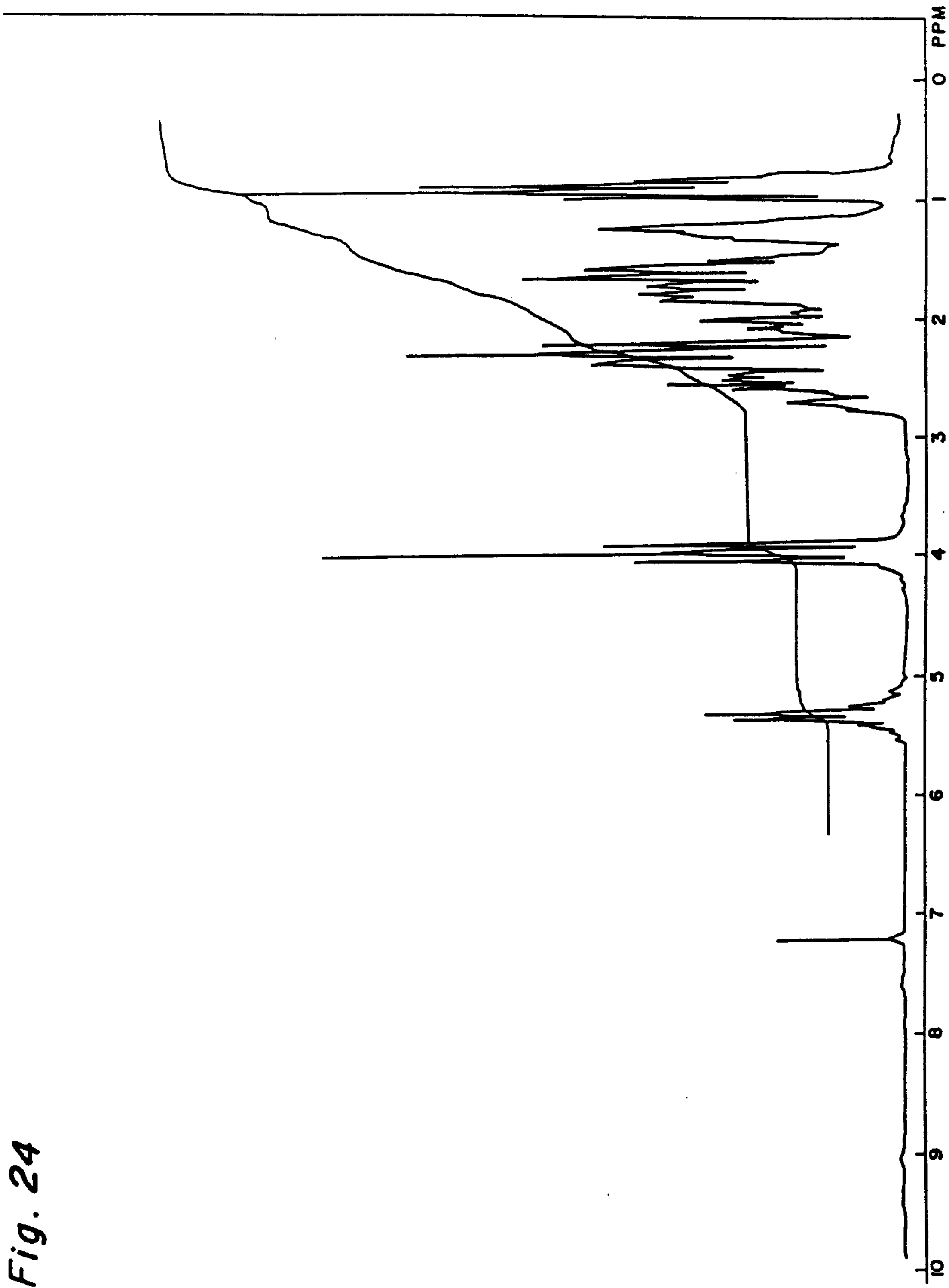


Fig. 24

Fig. 25

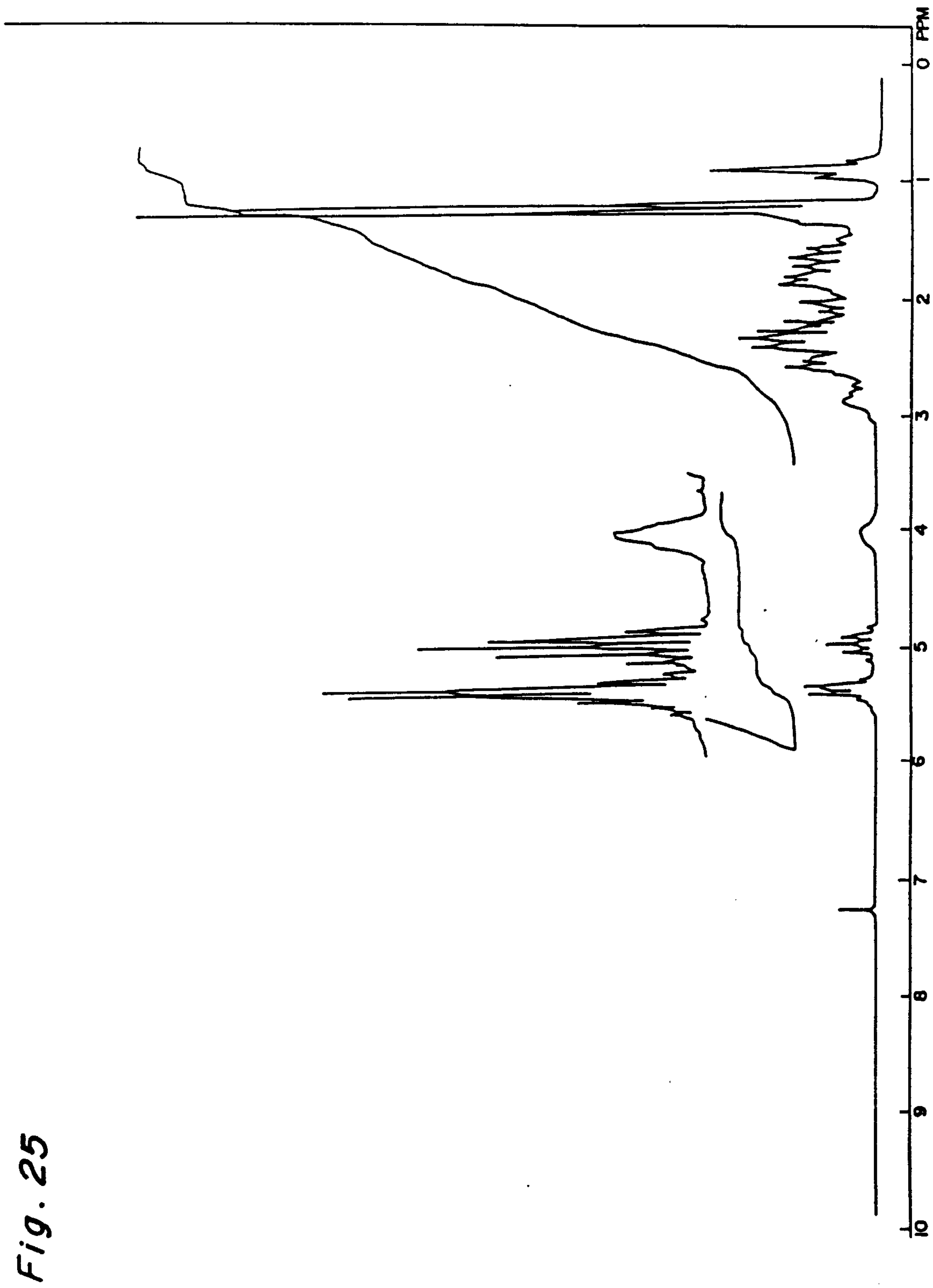

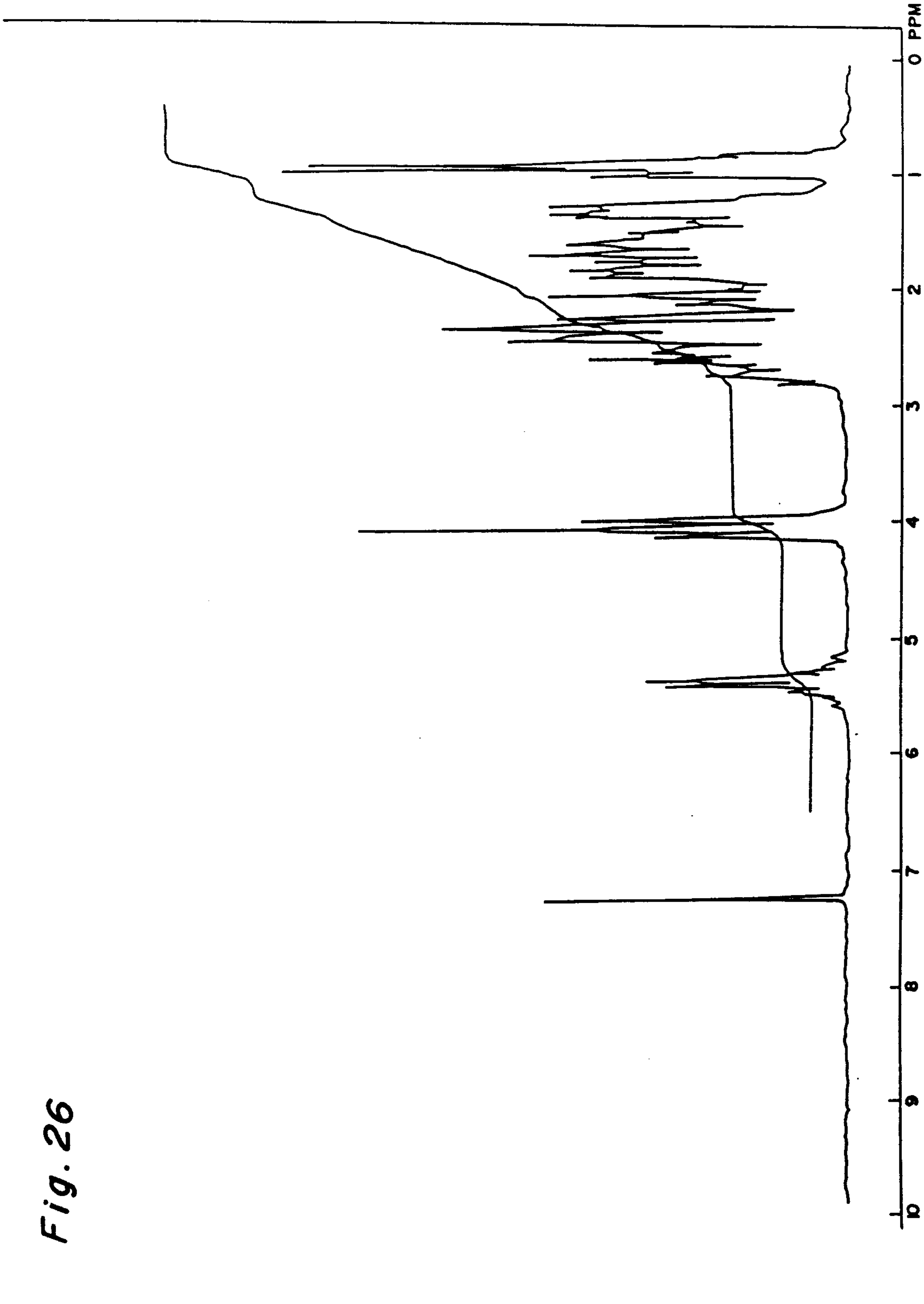


Fig. 26

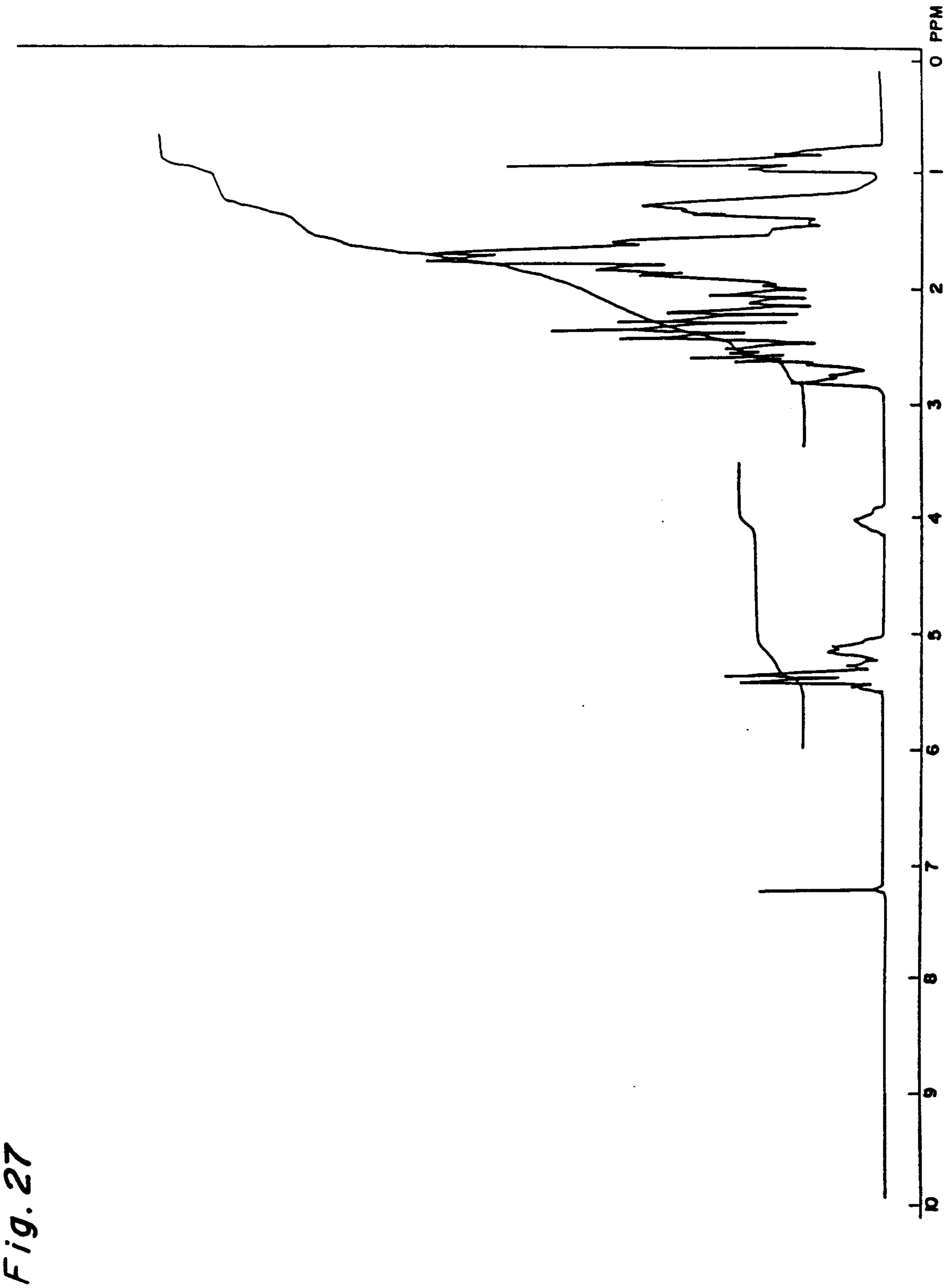


Fig. 27

Fig. 28

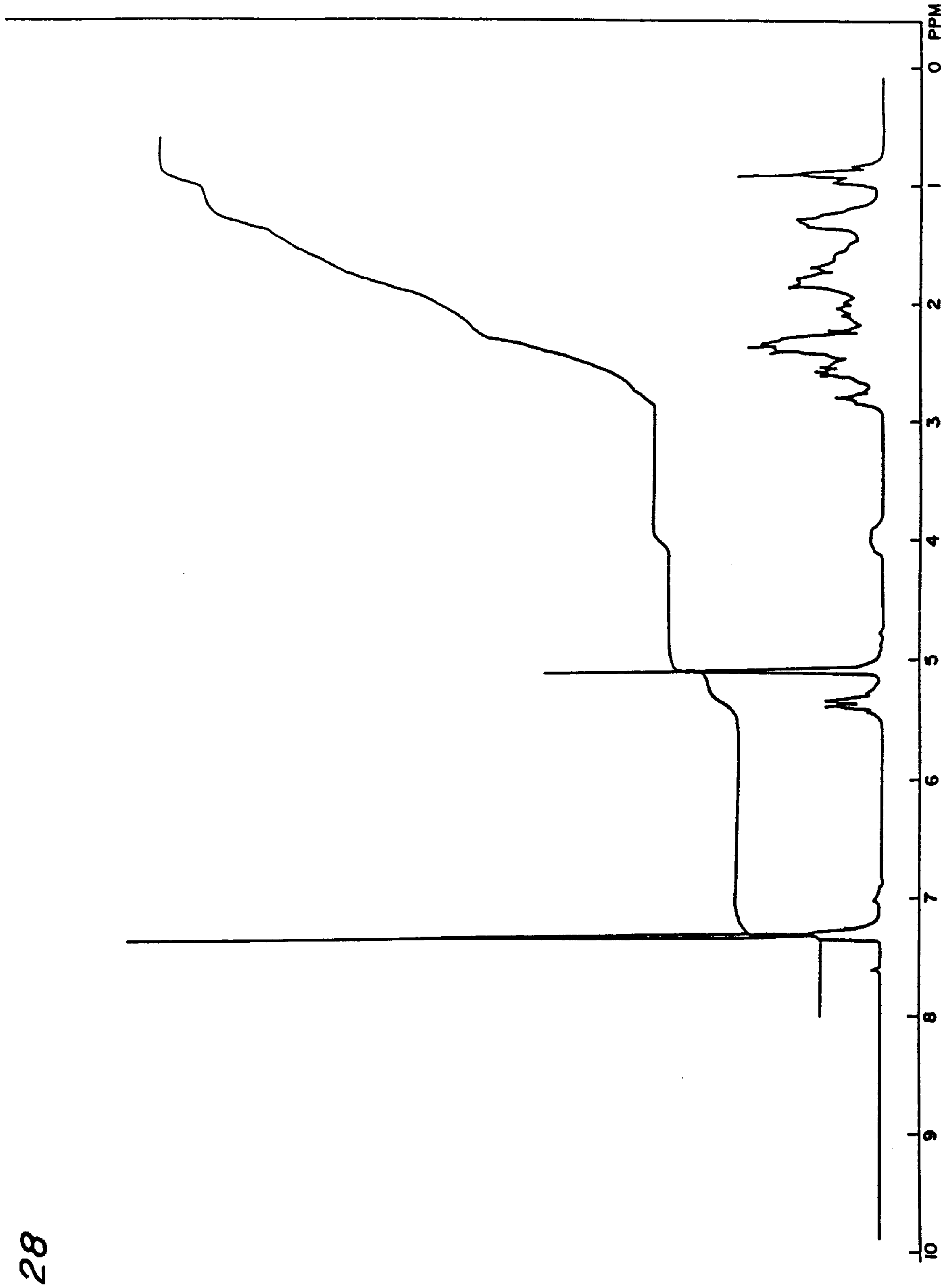

Fig. 29

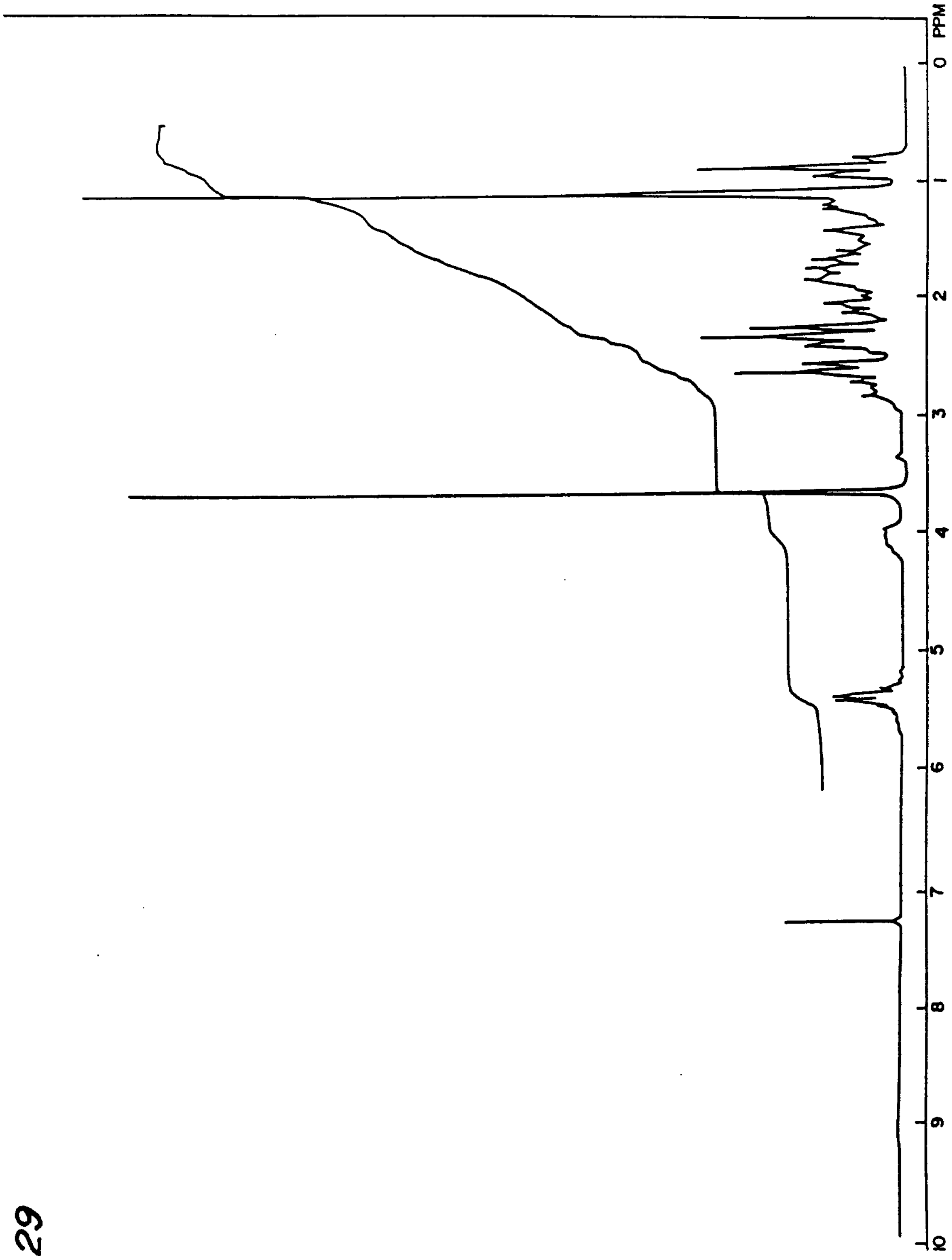

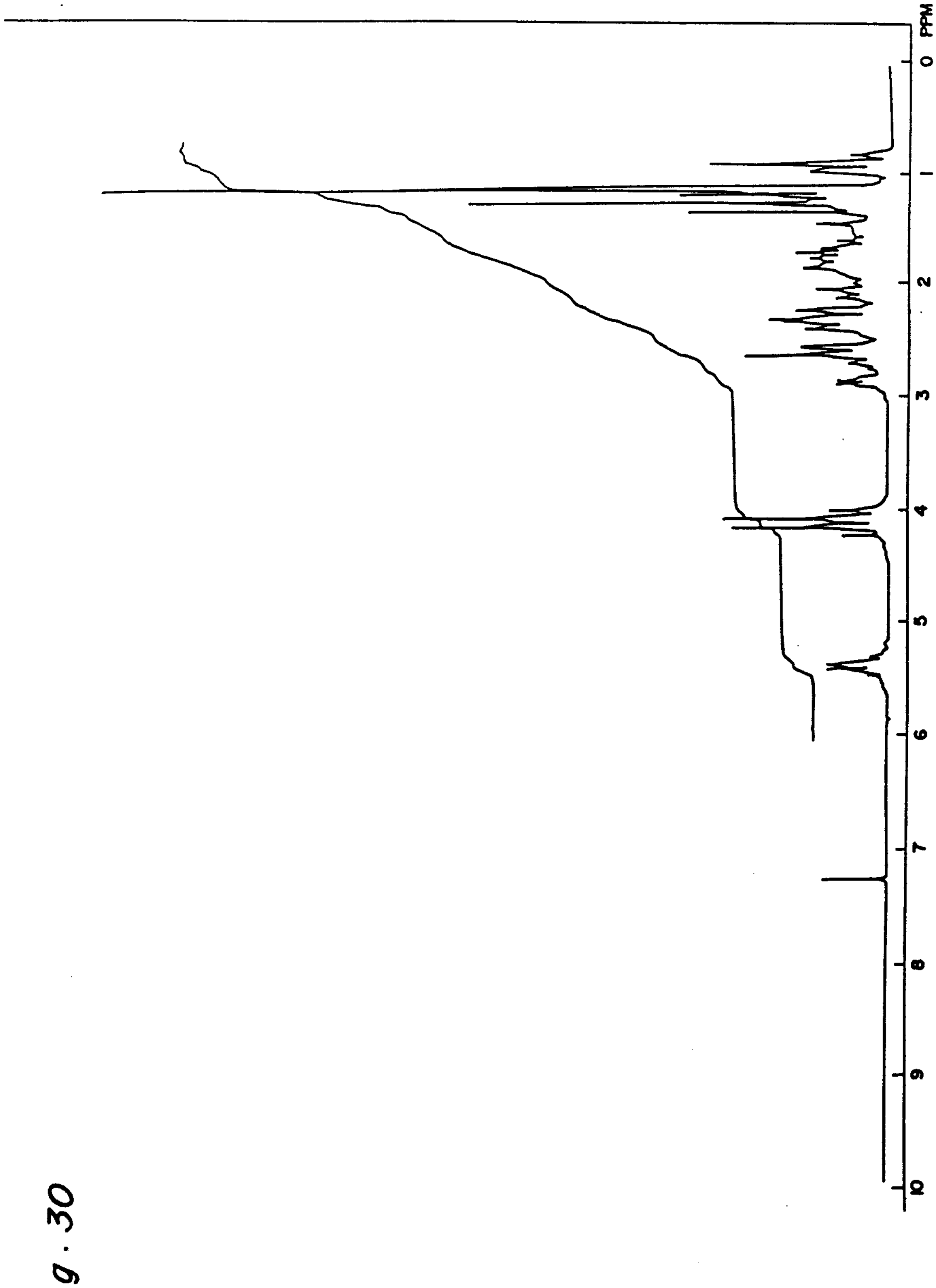


Fig. 30

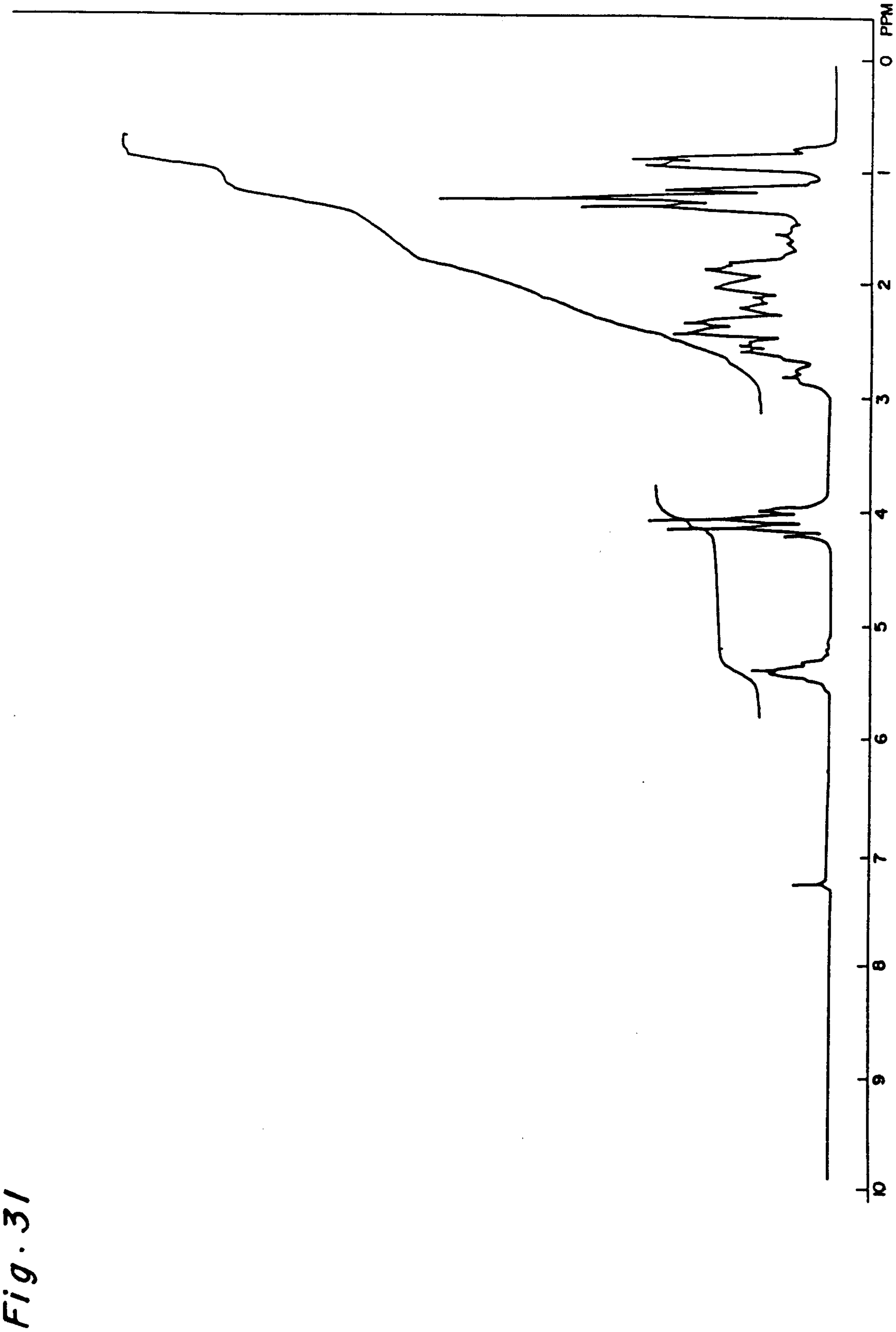


Fig. 31

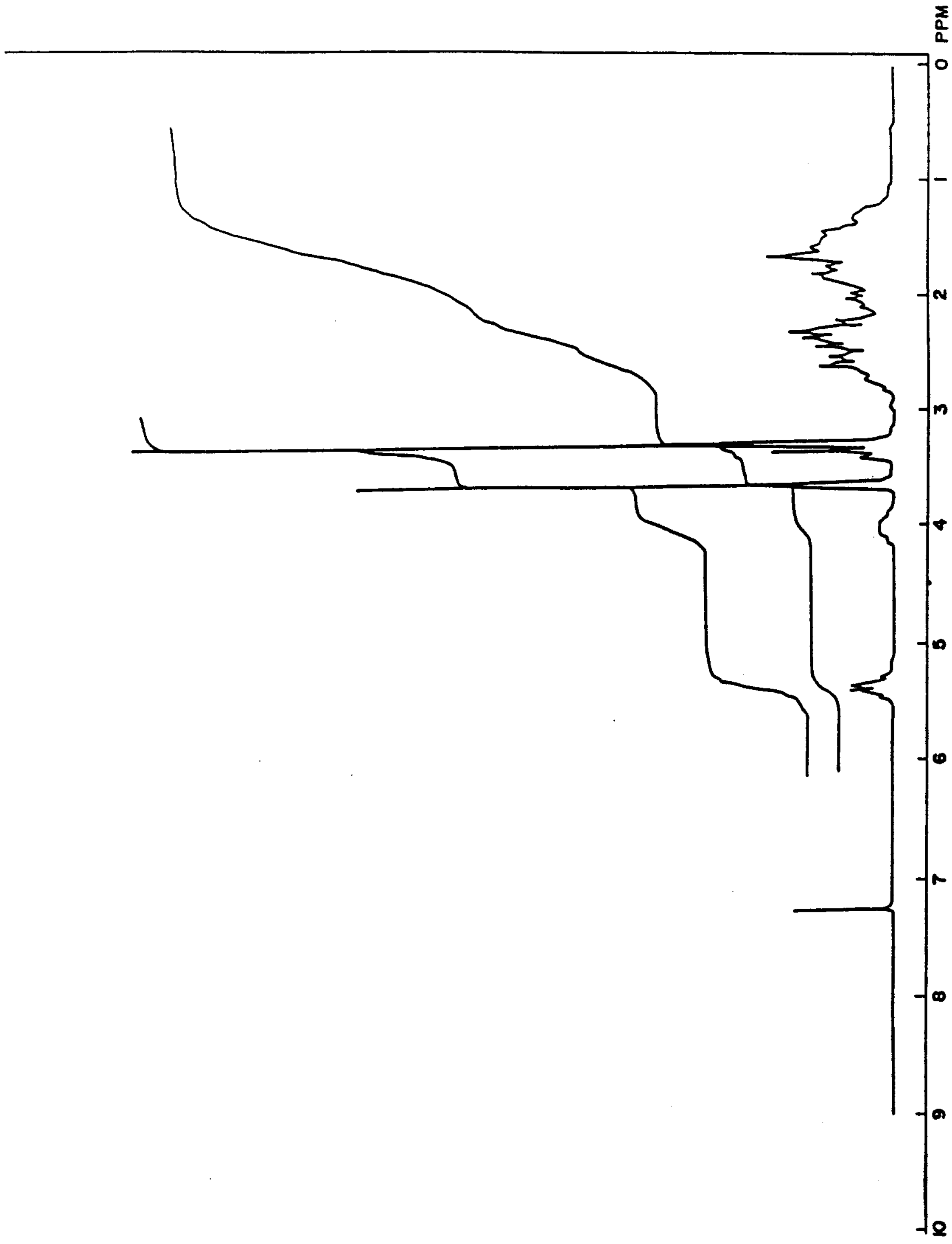


Fig. 32

Fig. 33

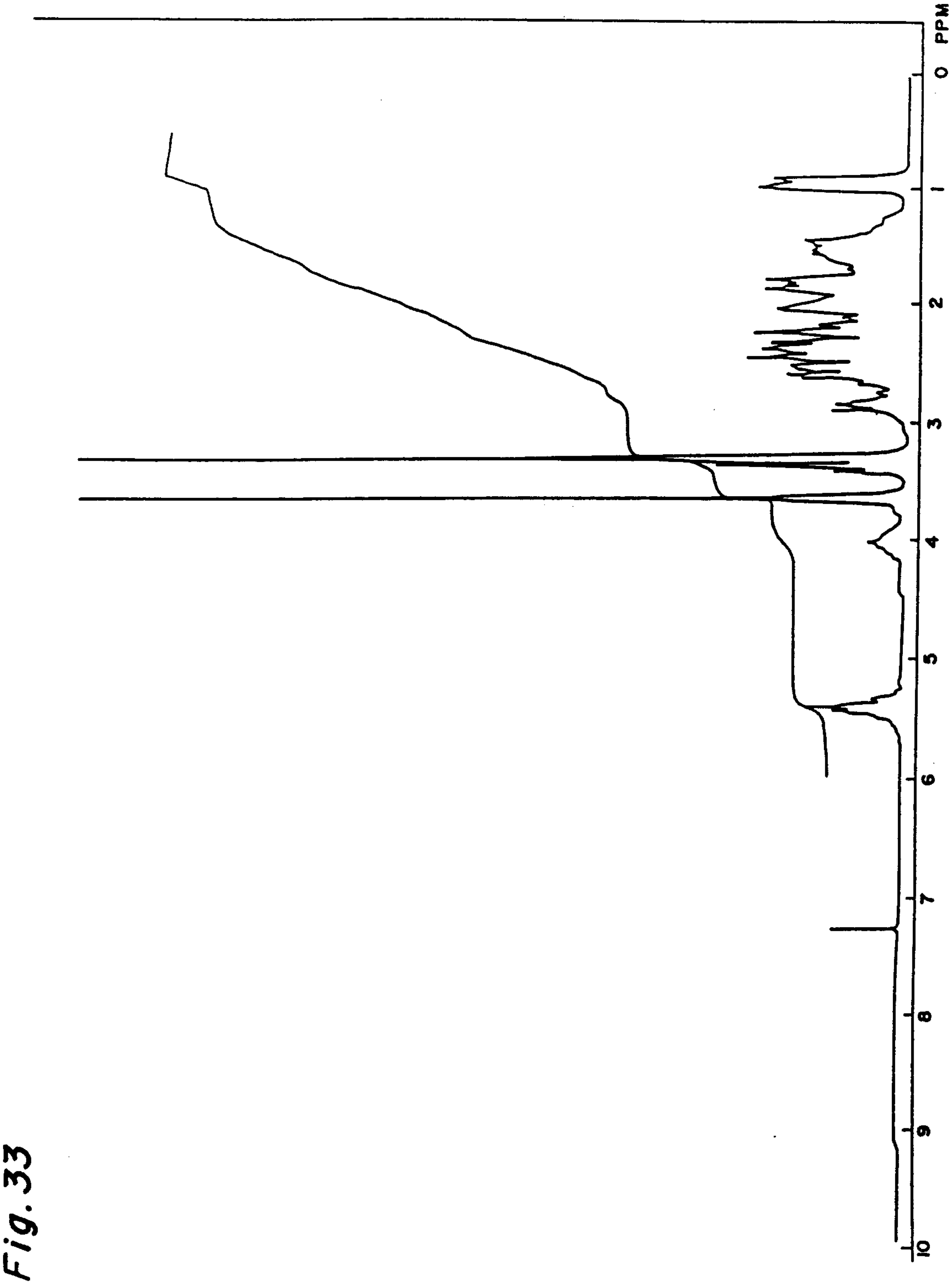

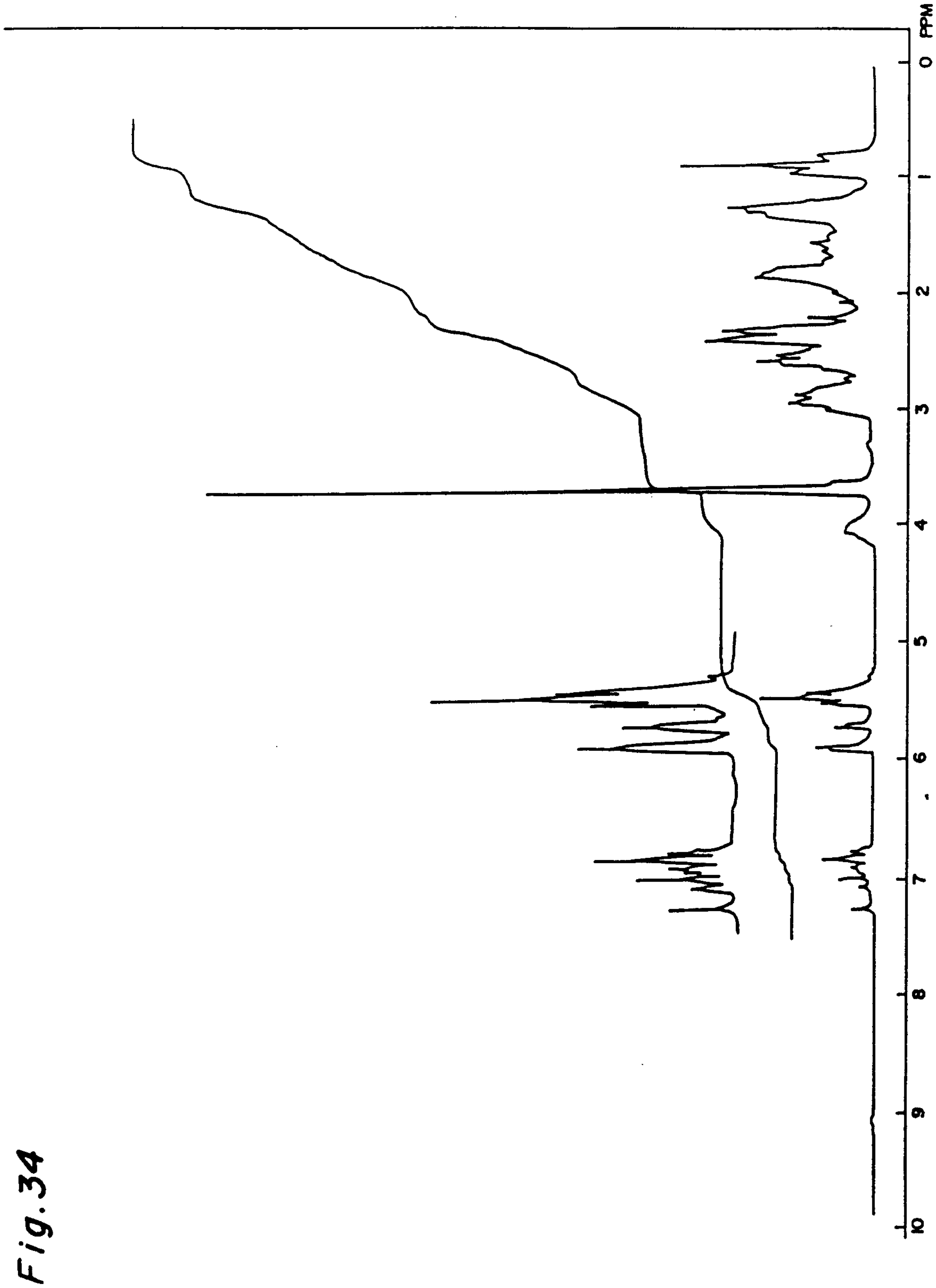


Fig.34

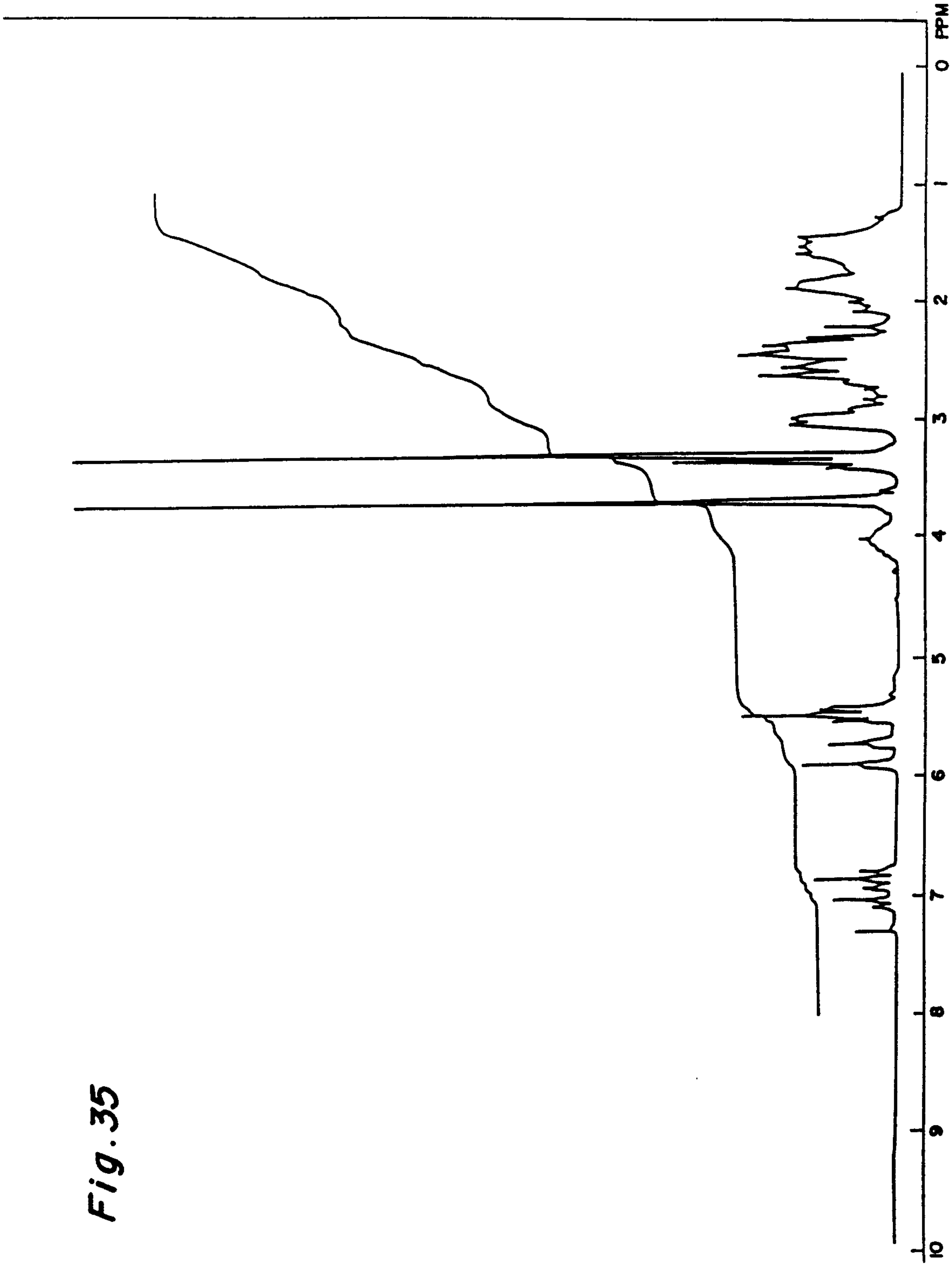


Fig.35

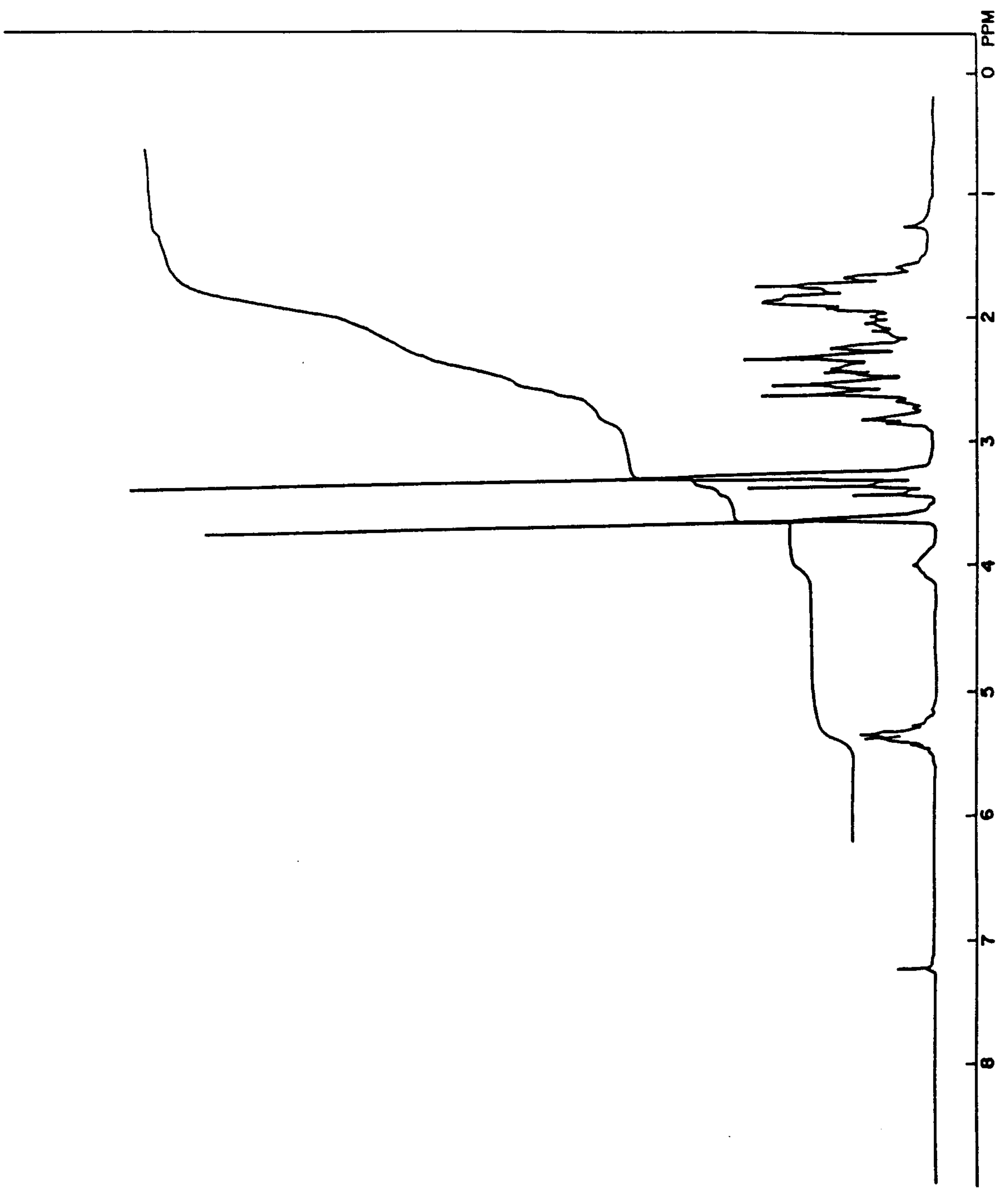


Fig.36

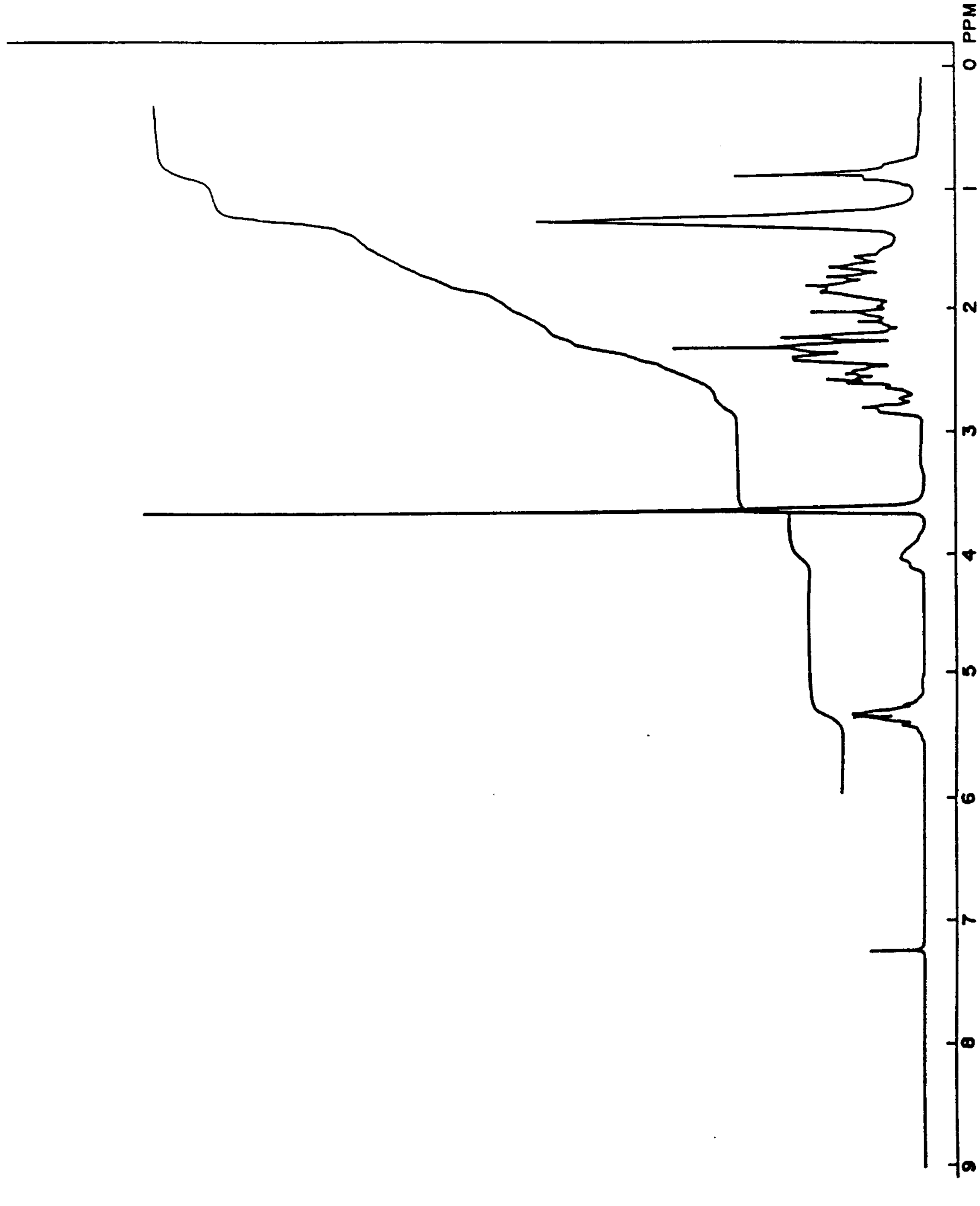


Fig.37

Fig.38

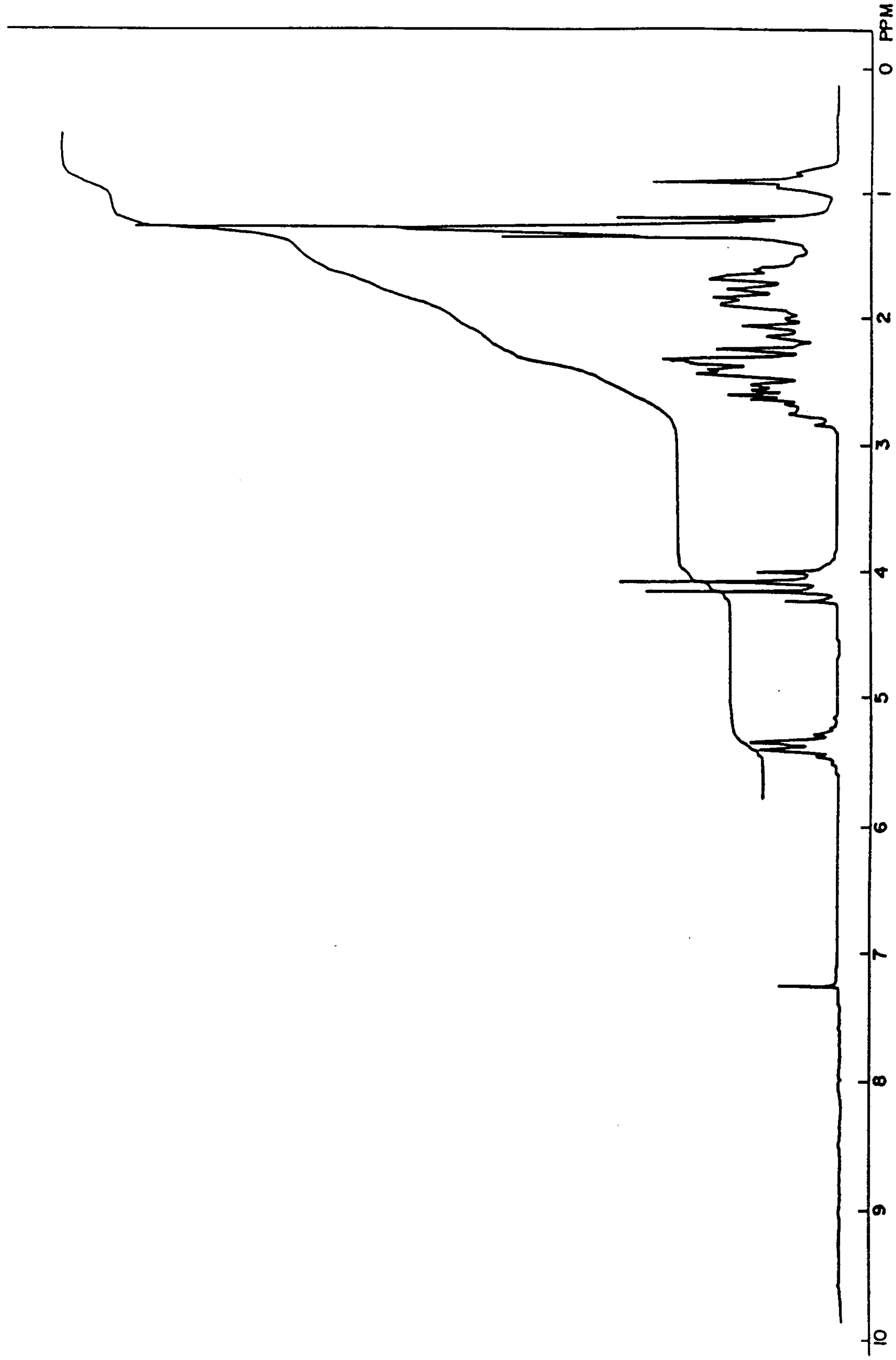

Fig.39

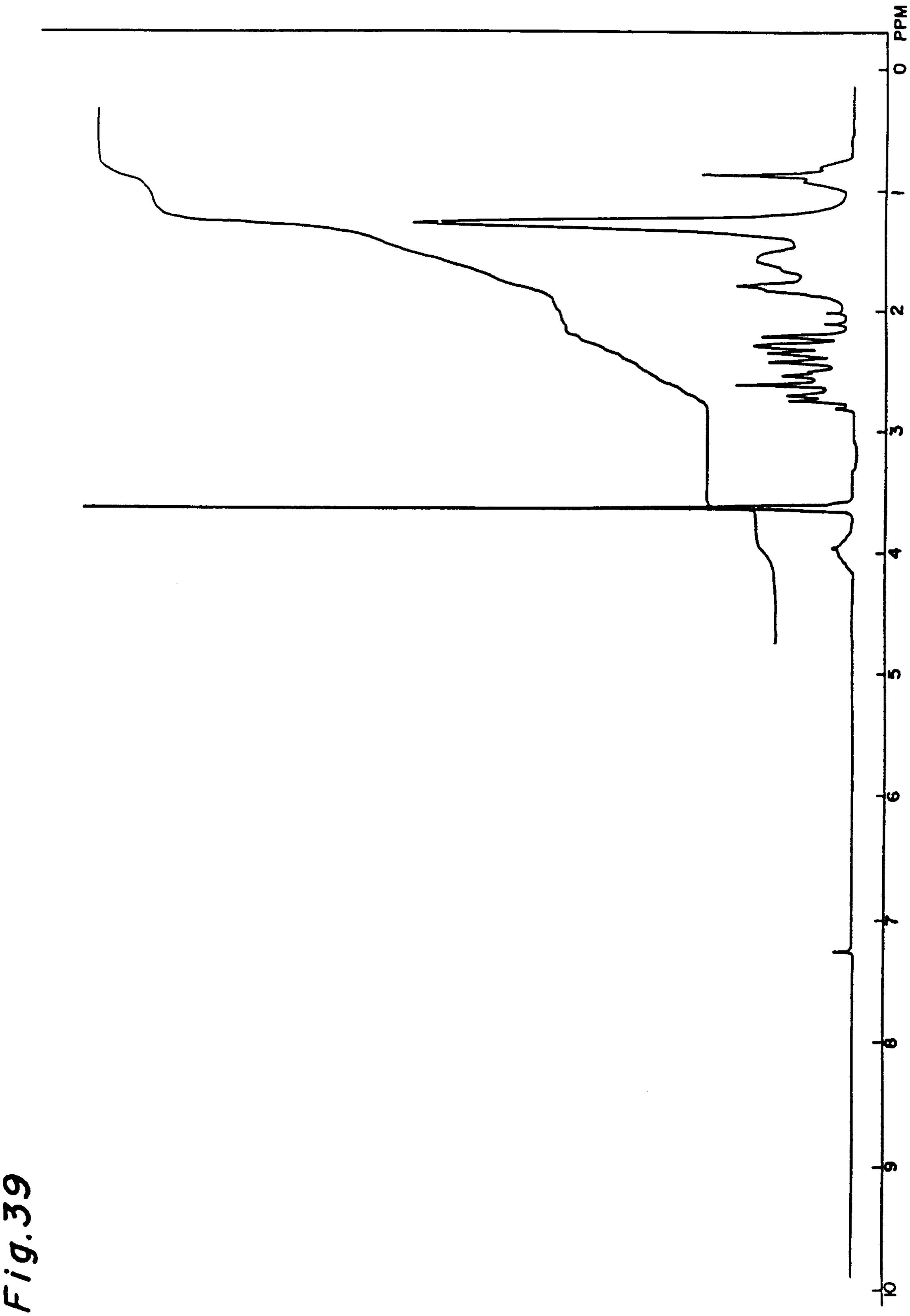

Fig. 40

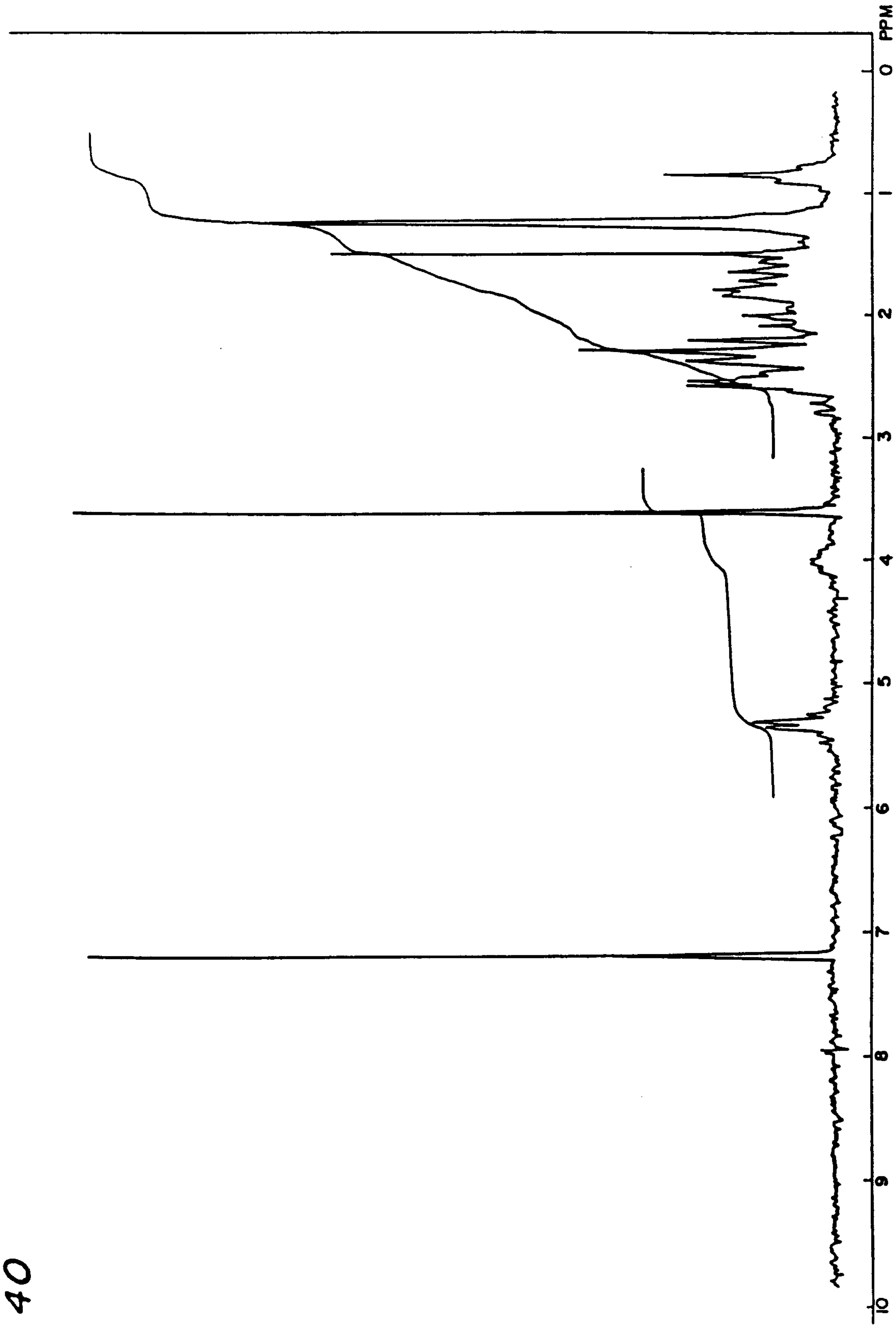

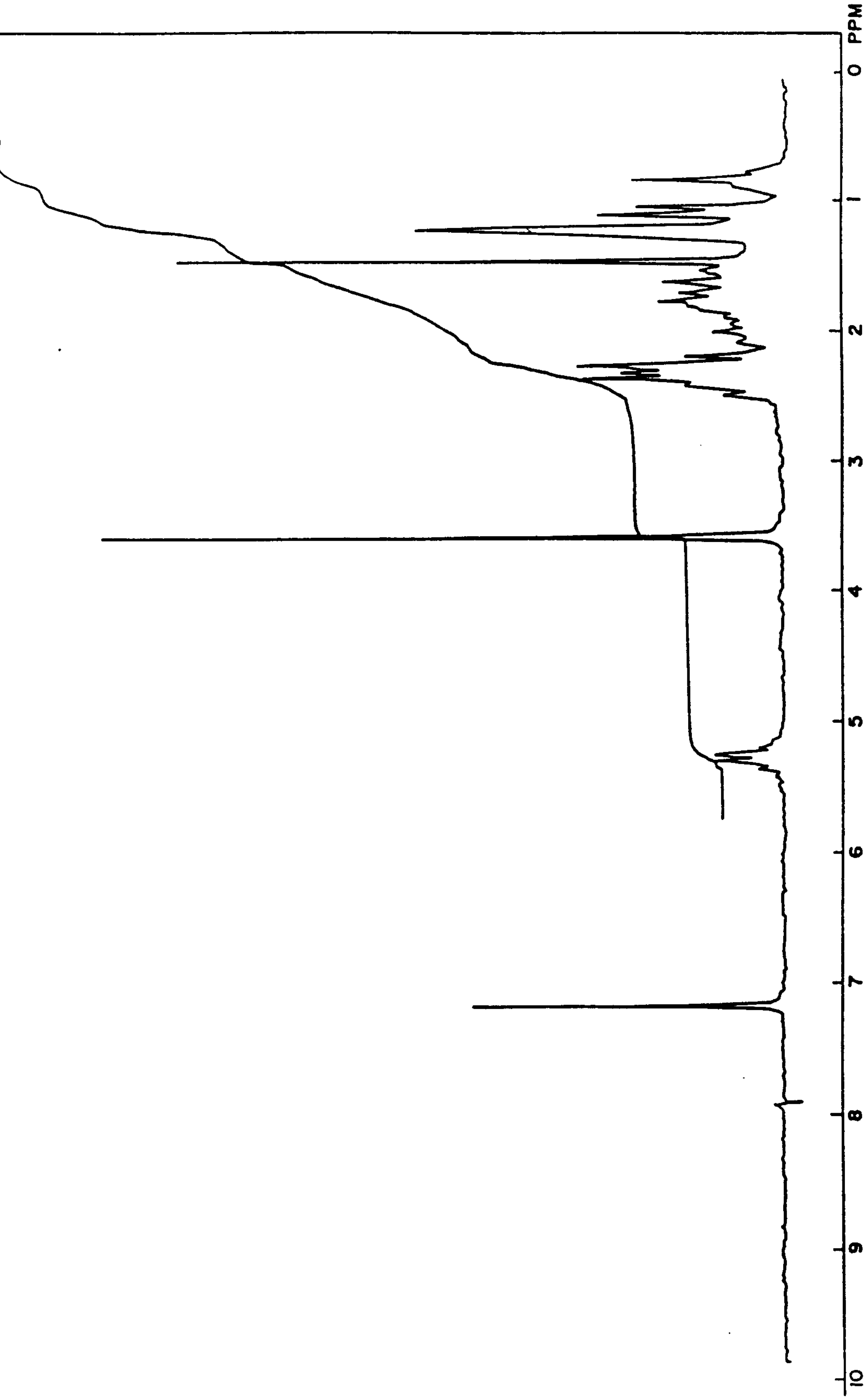


Fig. 41

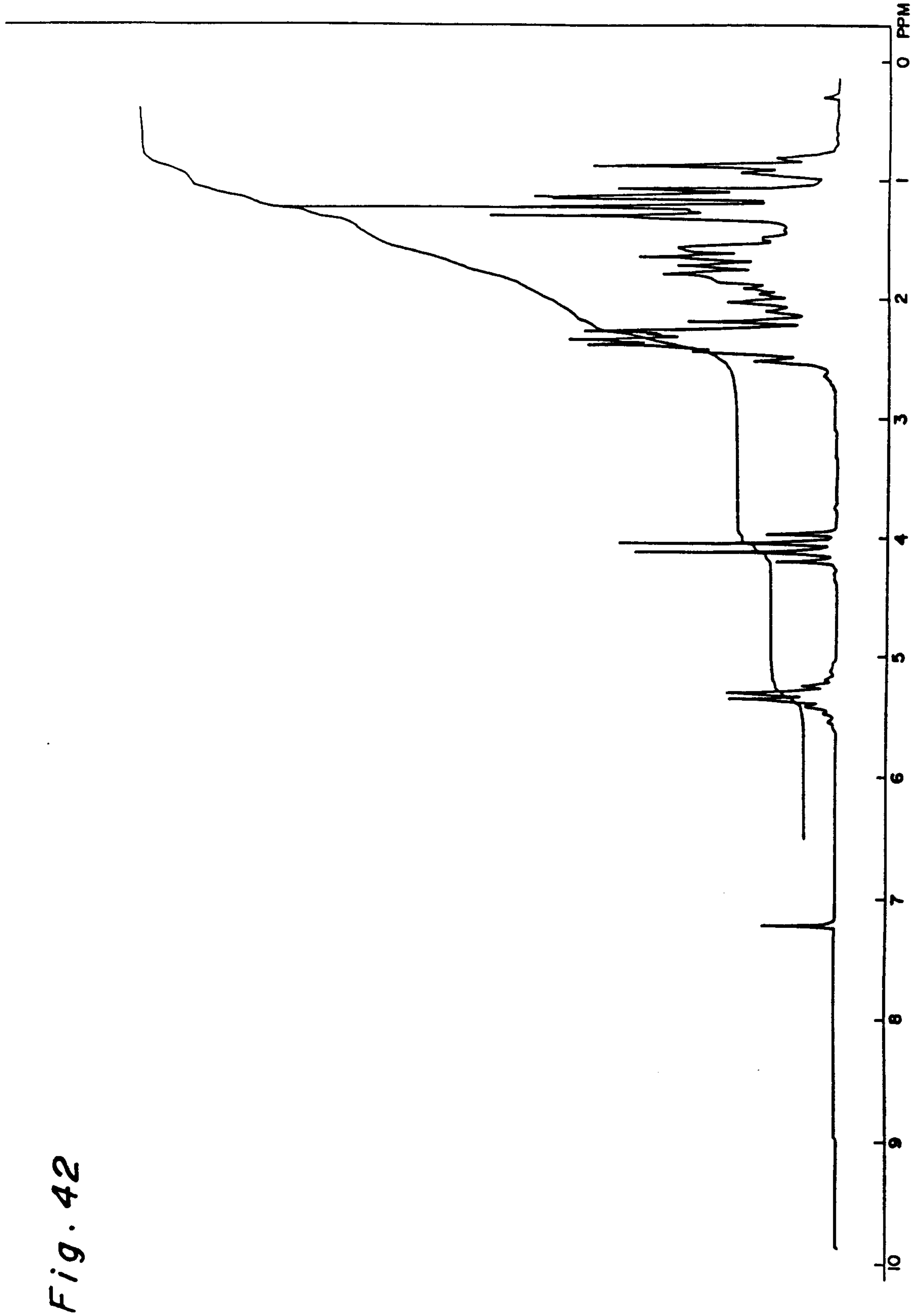


Fig. 42

Fig. 43

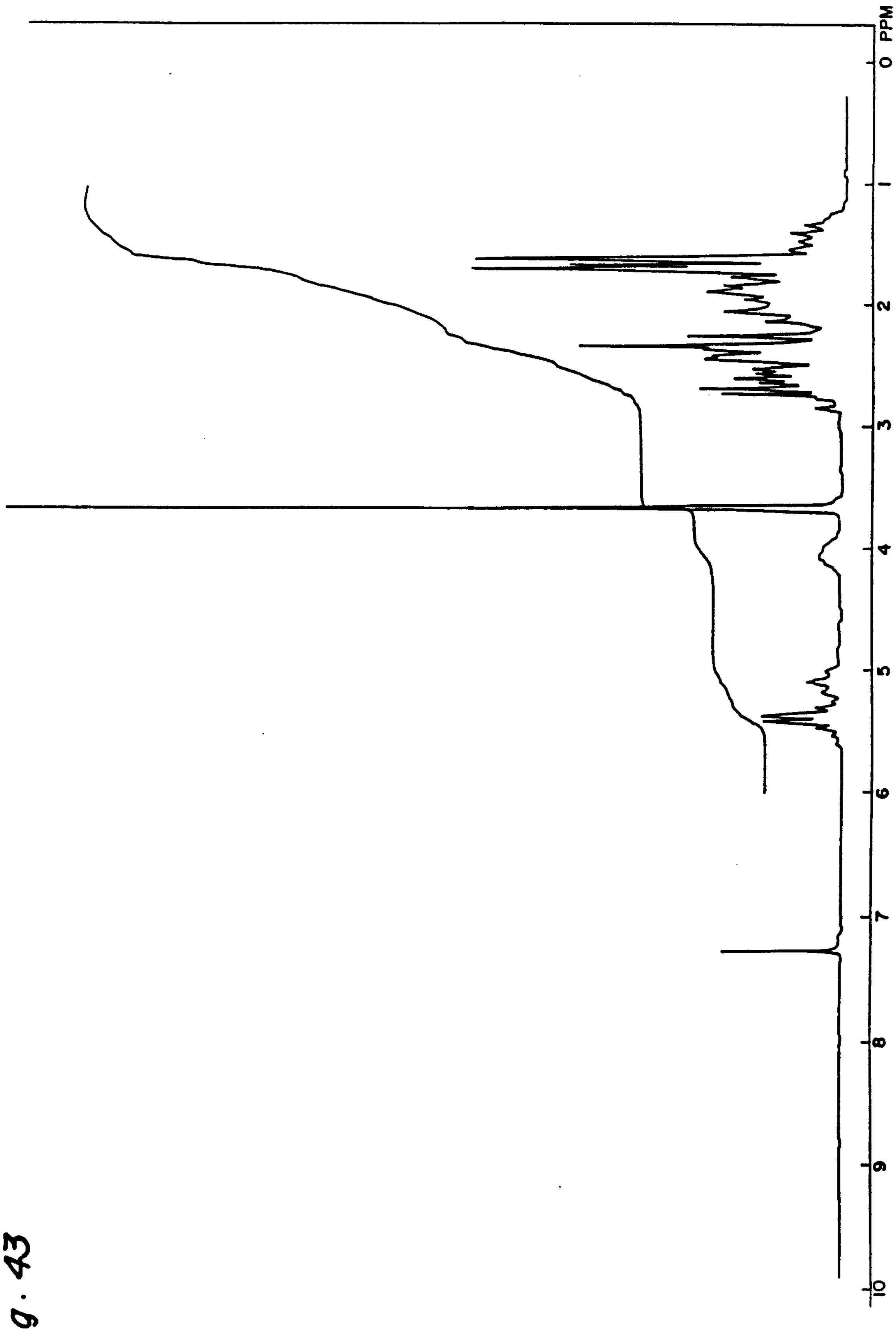

Fig. 44

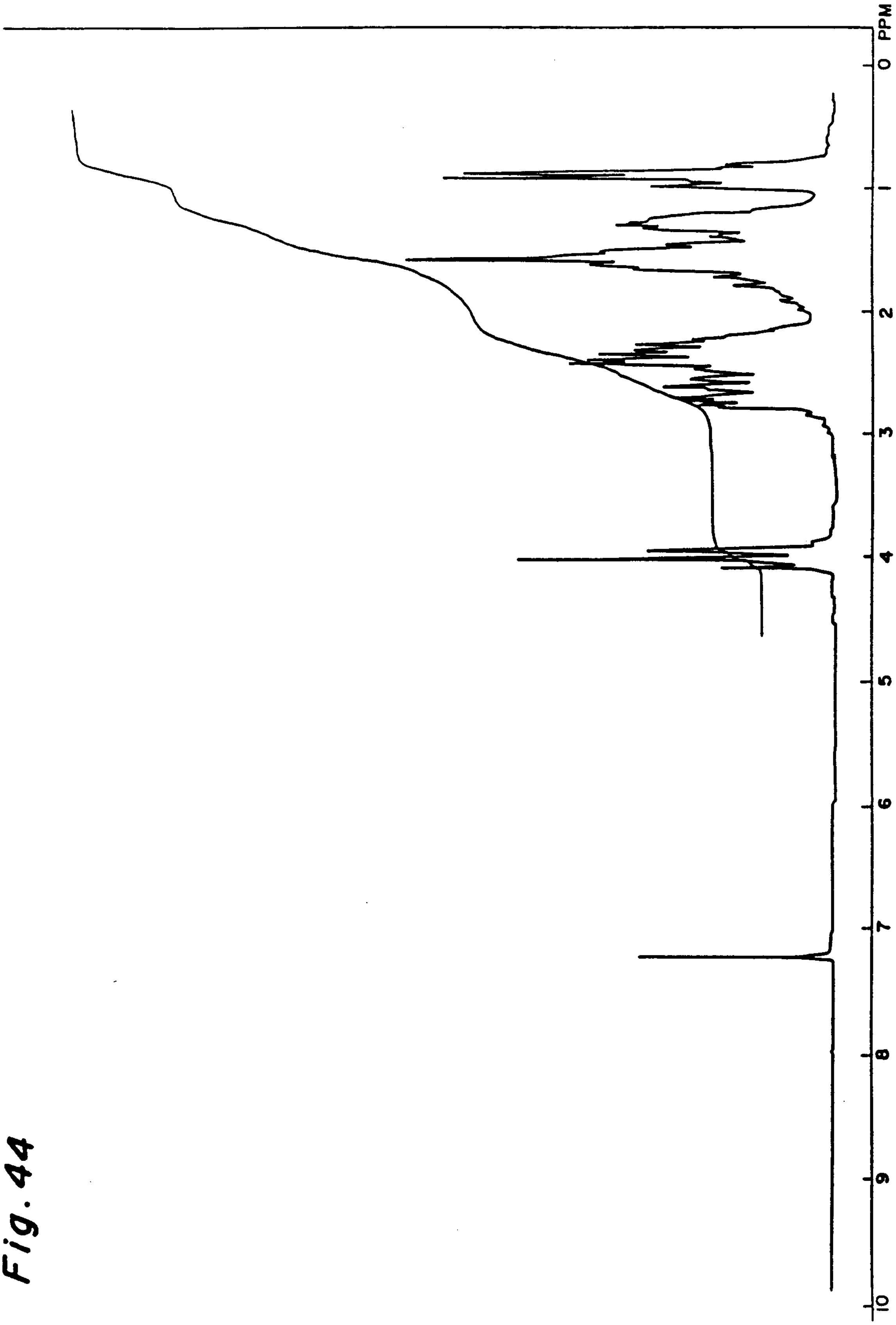

Fig.45

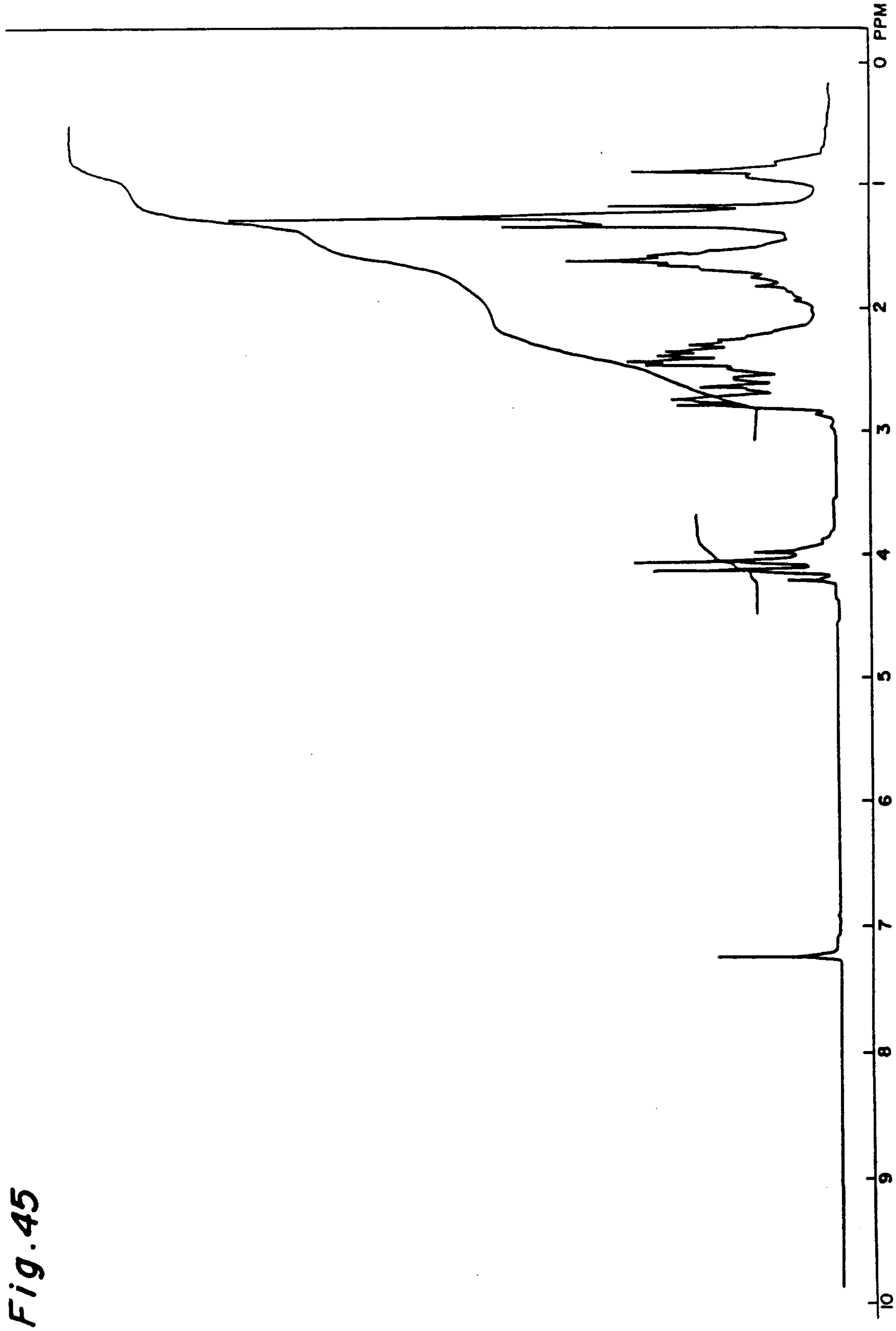

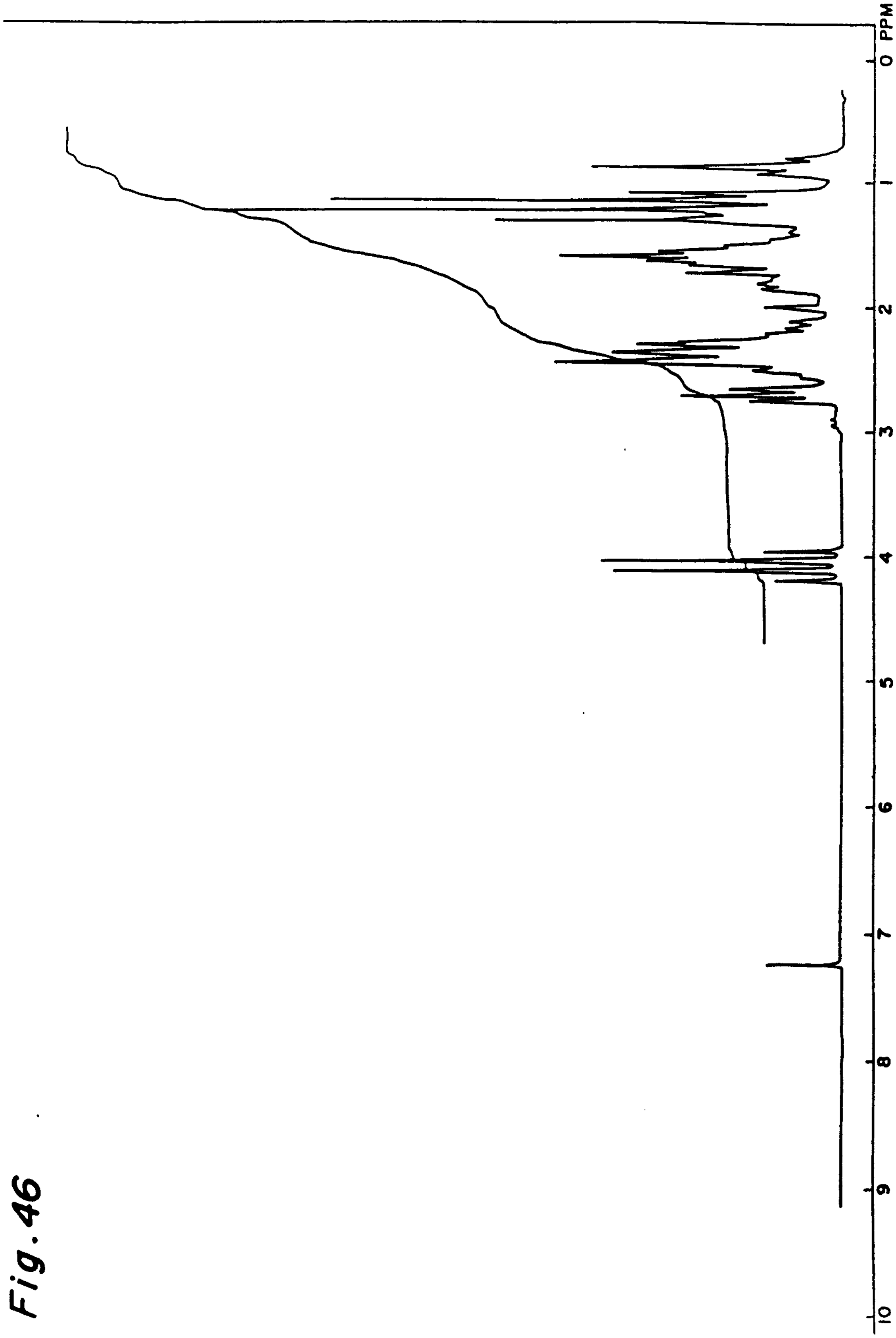


Fig. 46

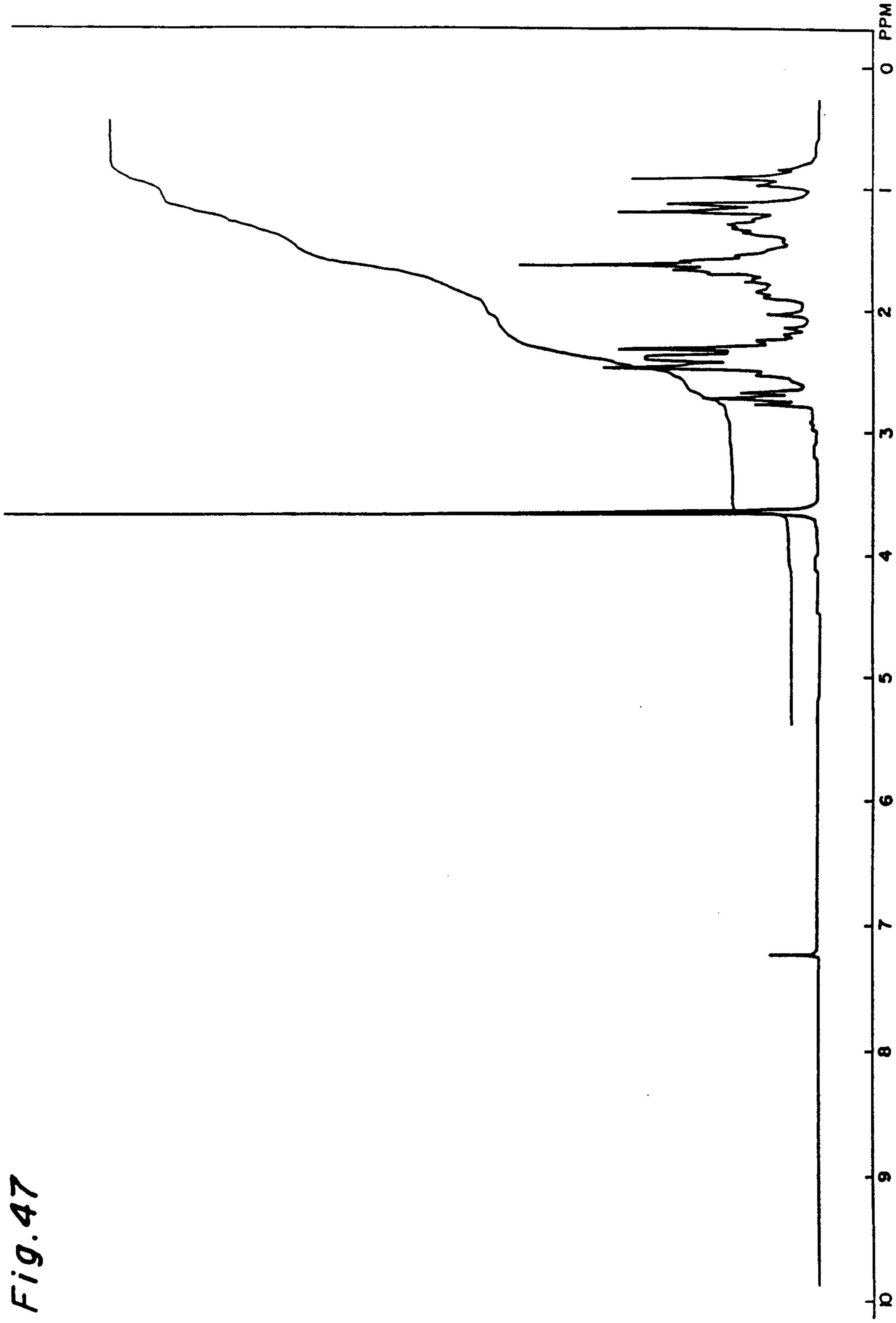


Fig.47

Fig.48

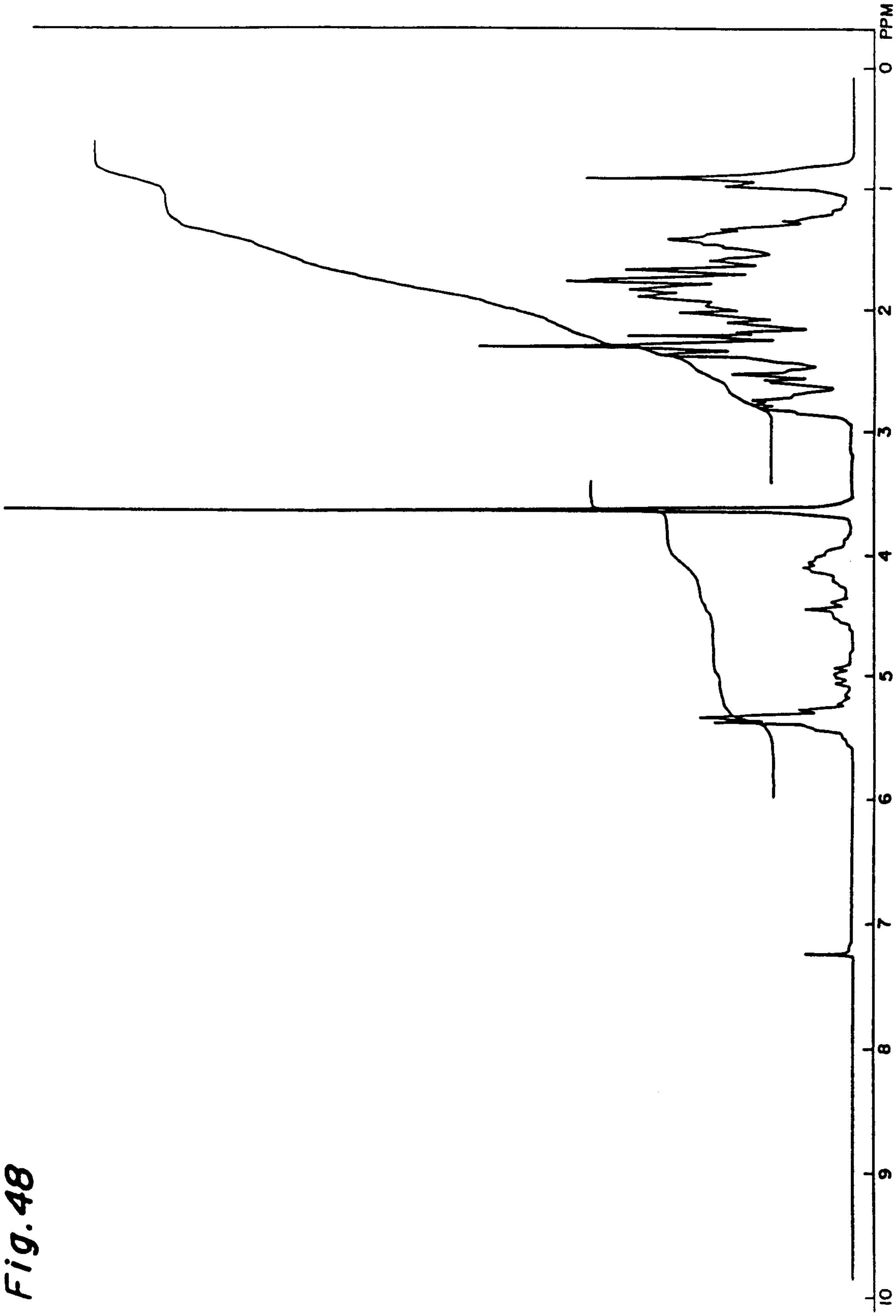

Fig. 49

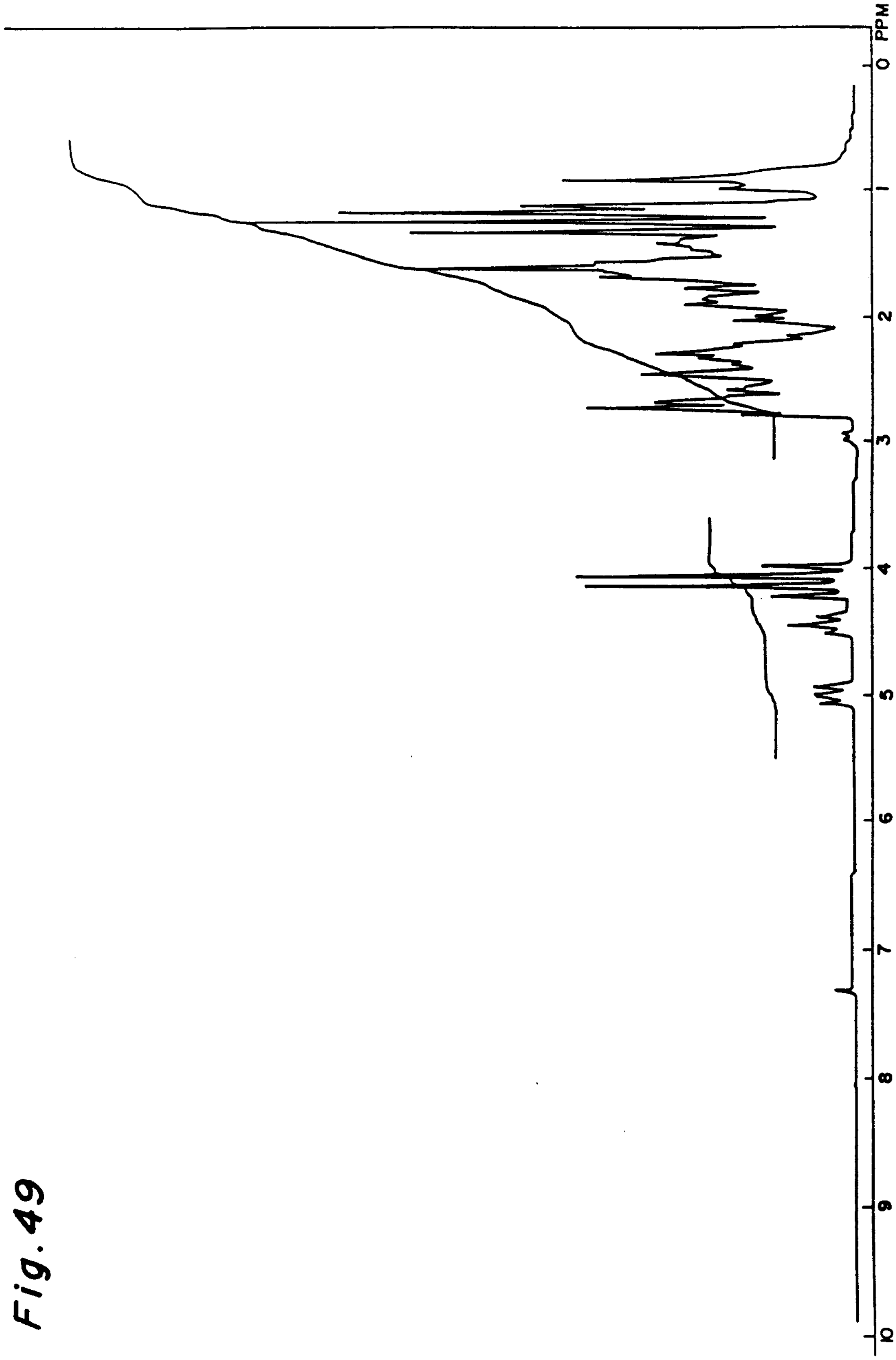

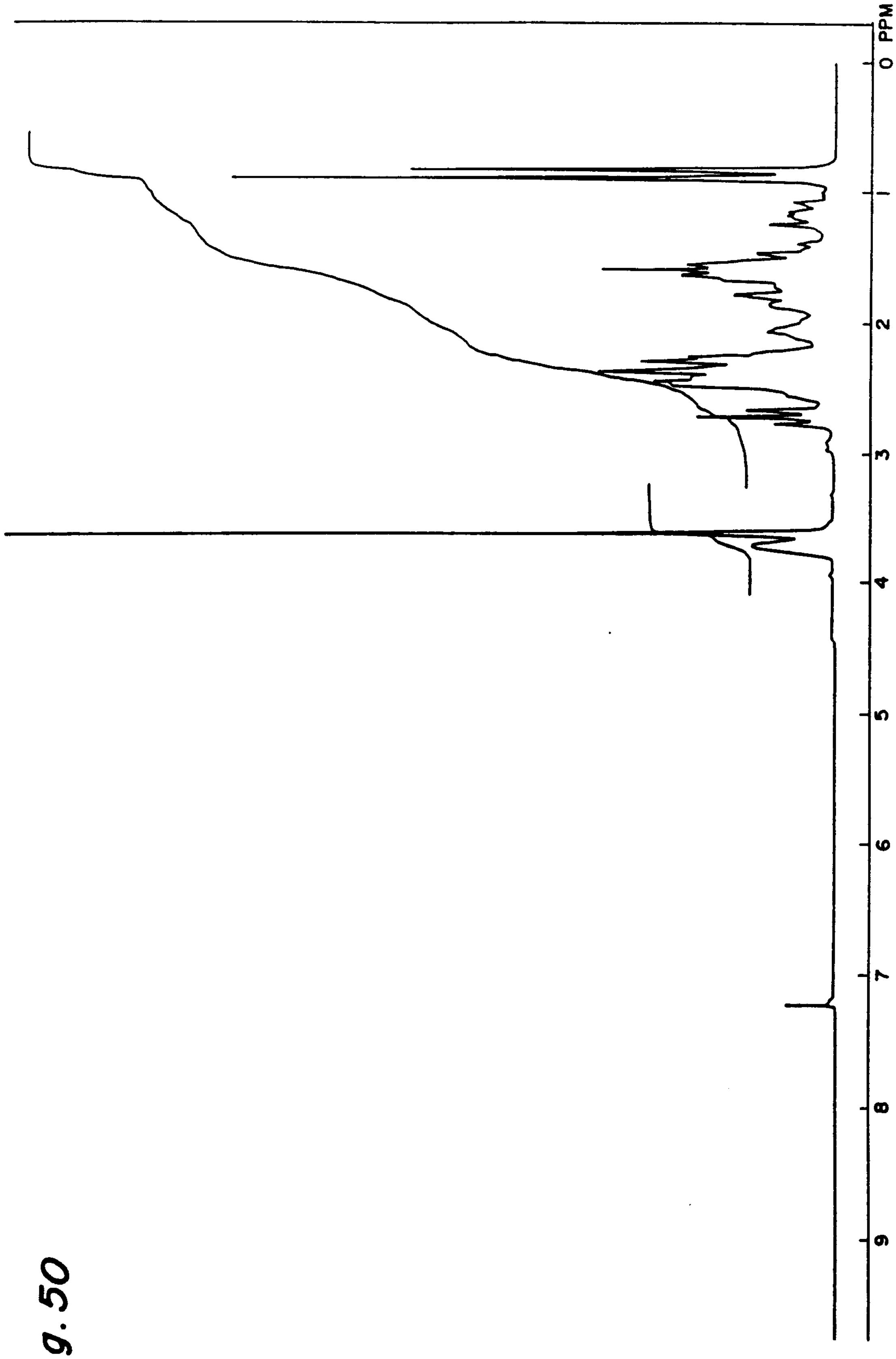


Fig. 50

Fig.5I

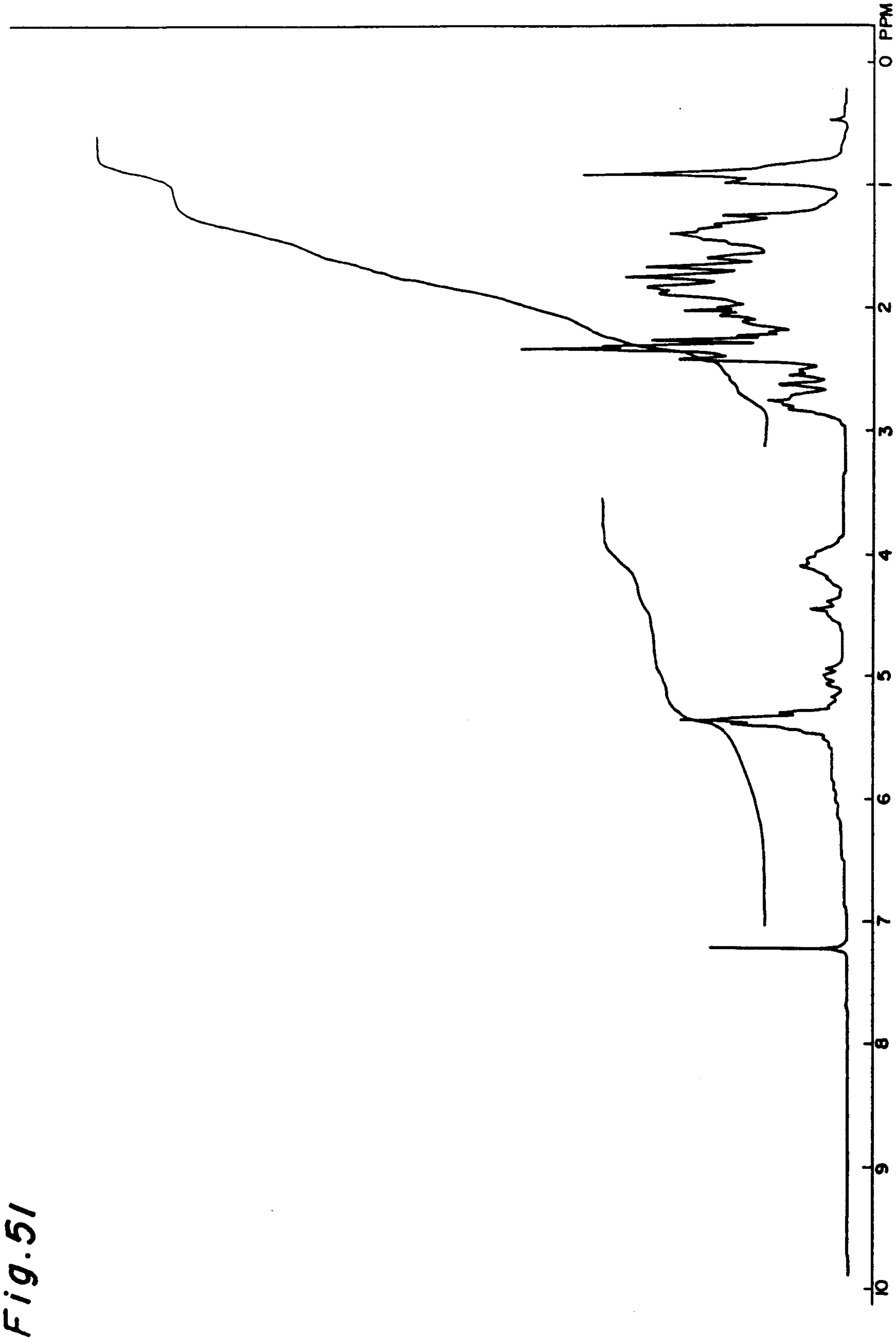

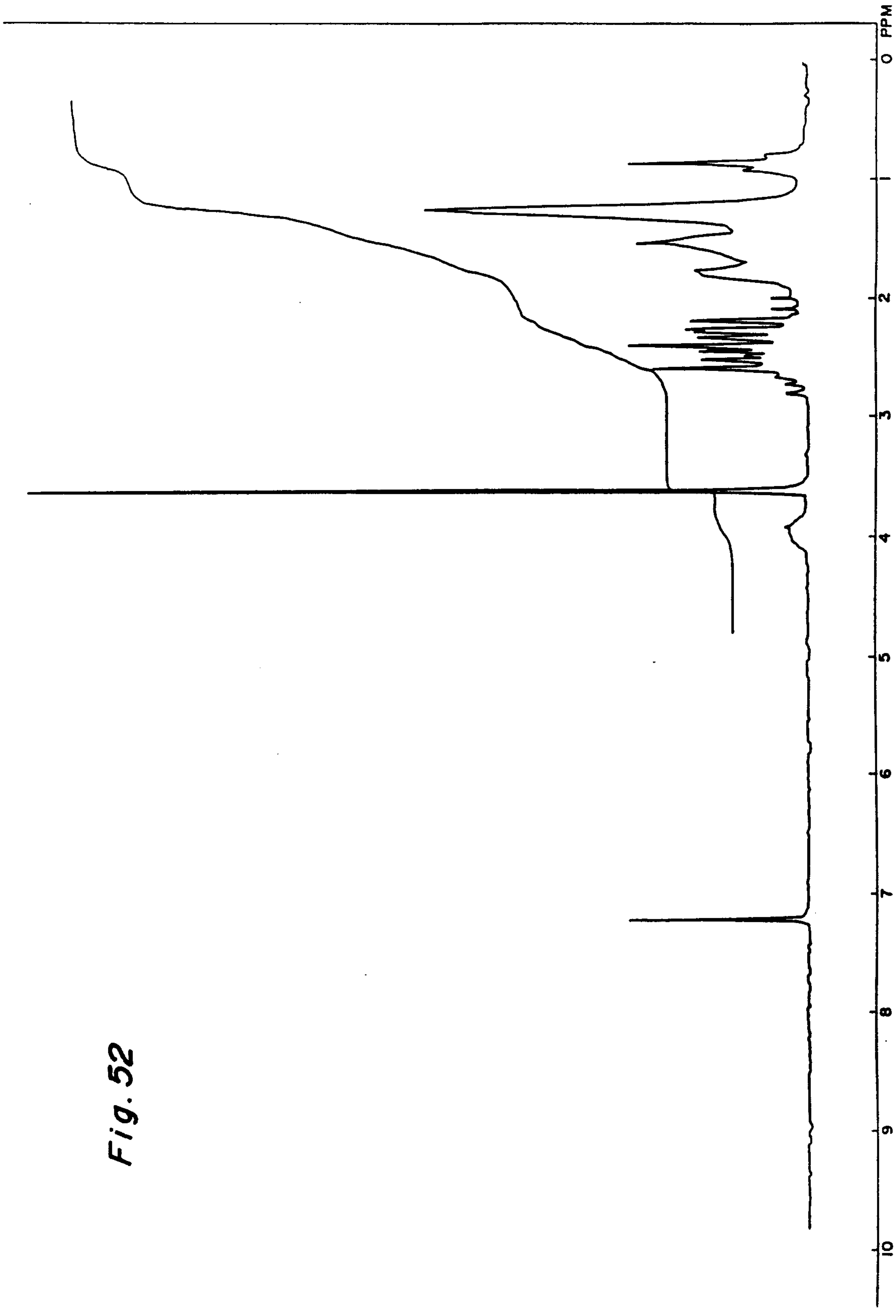


Fig. 52

Fig.53

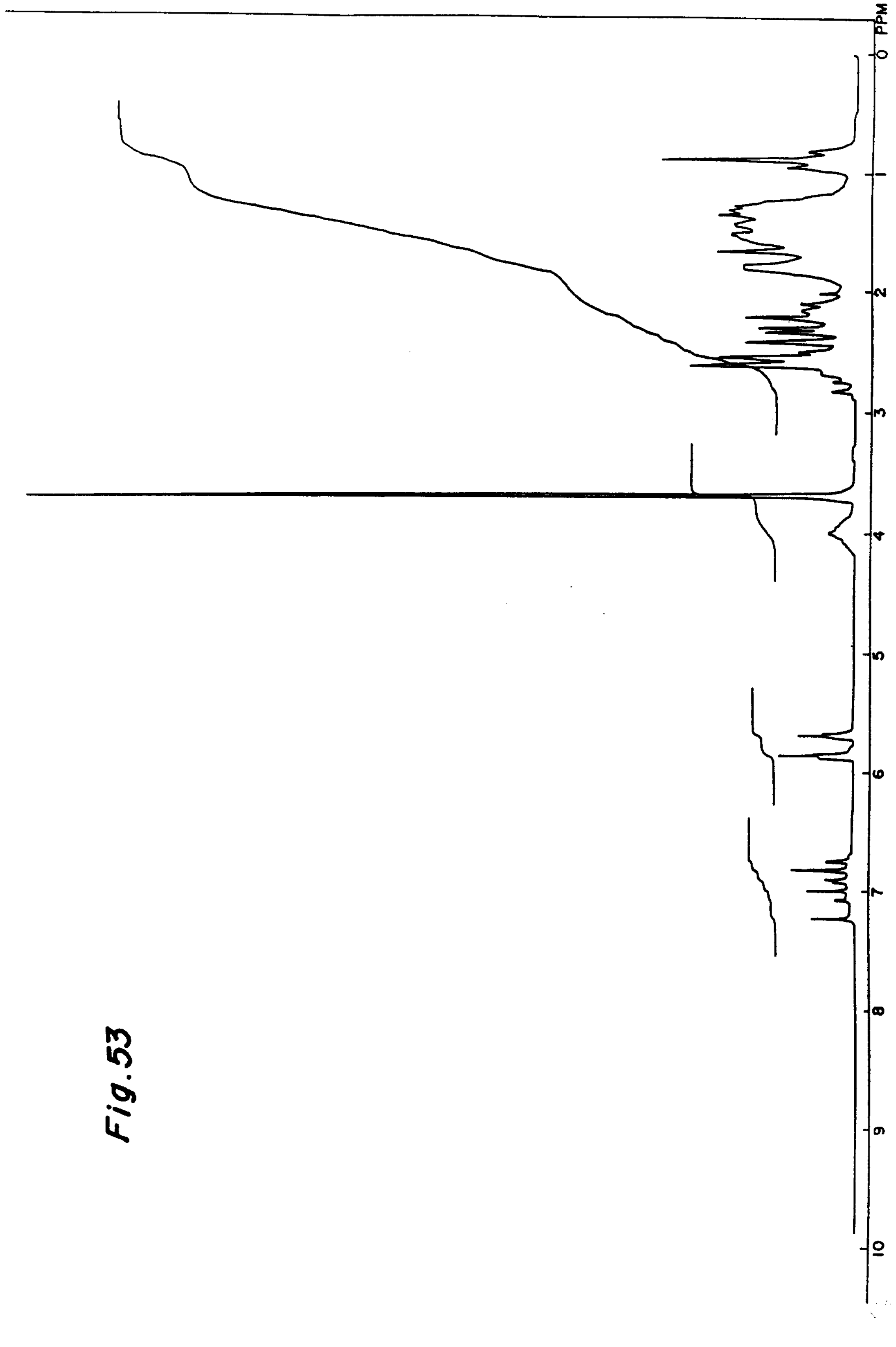

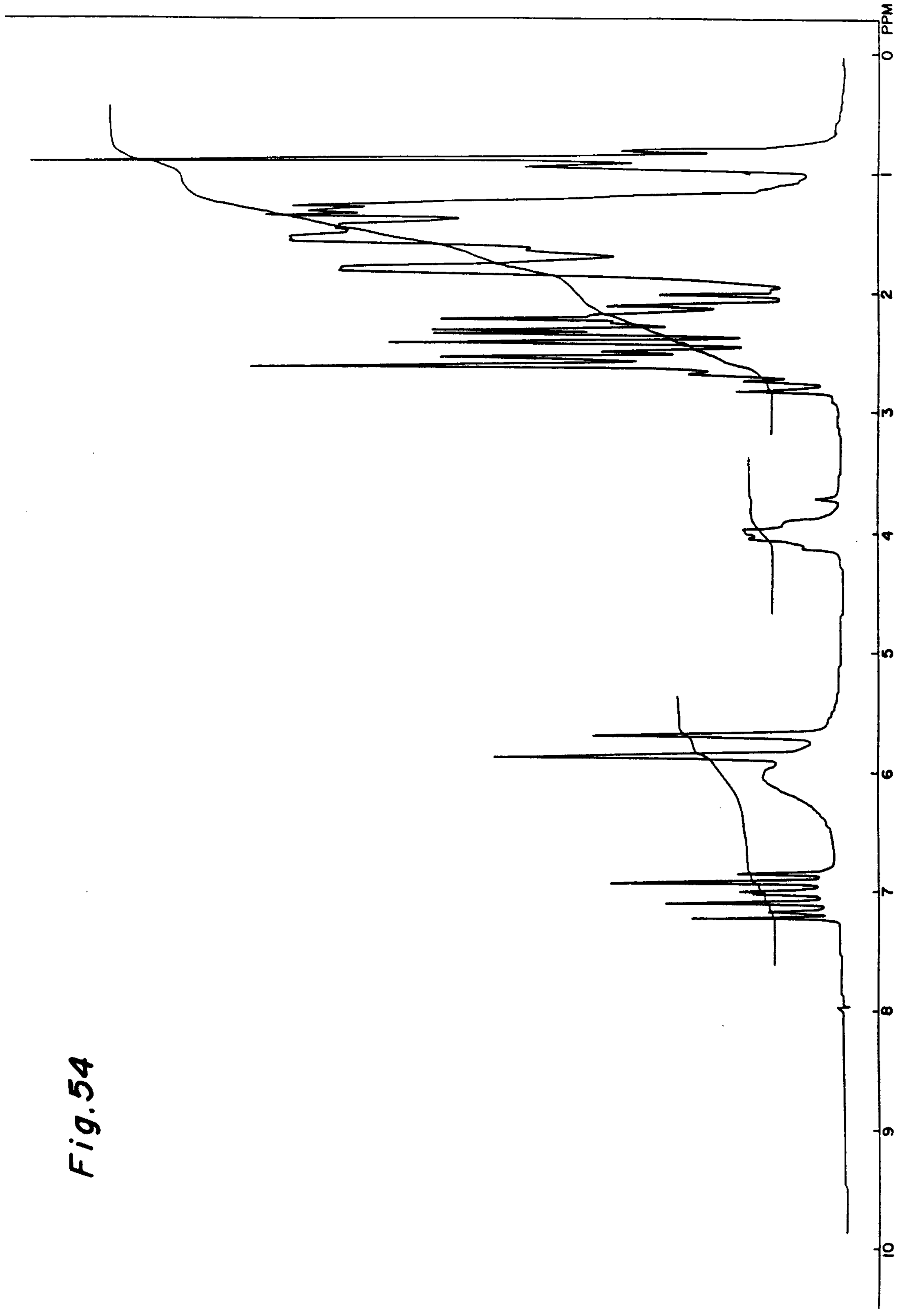


Fig.54

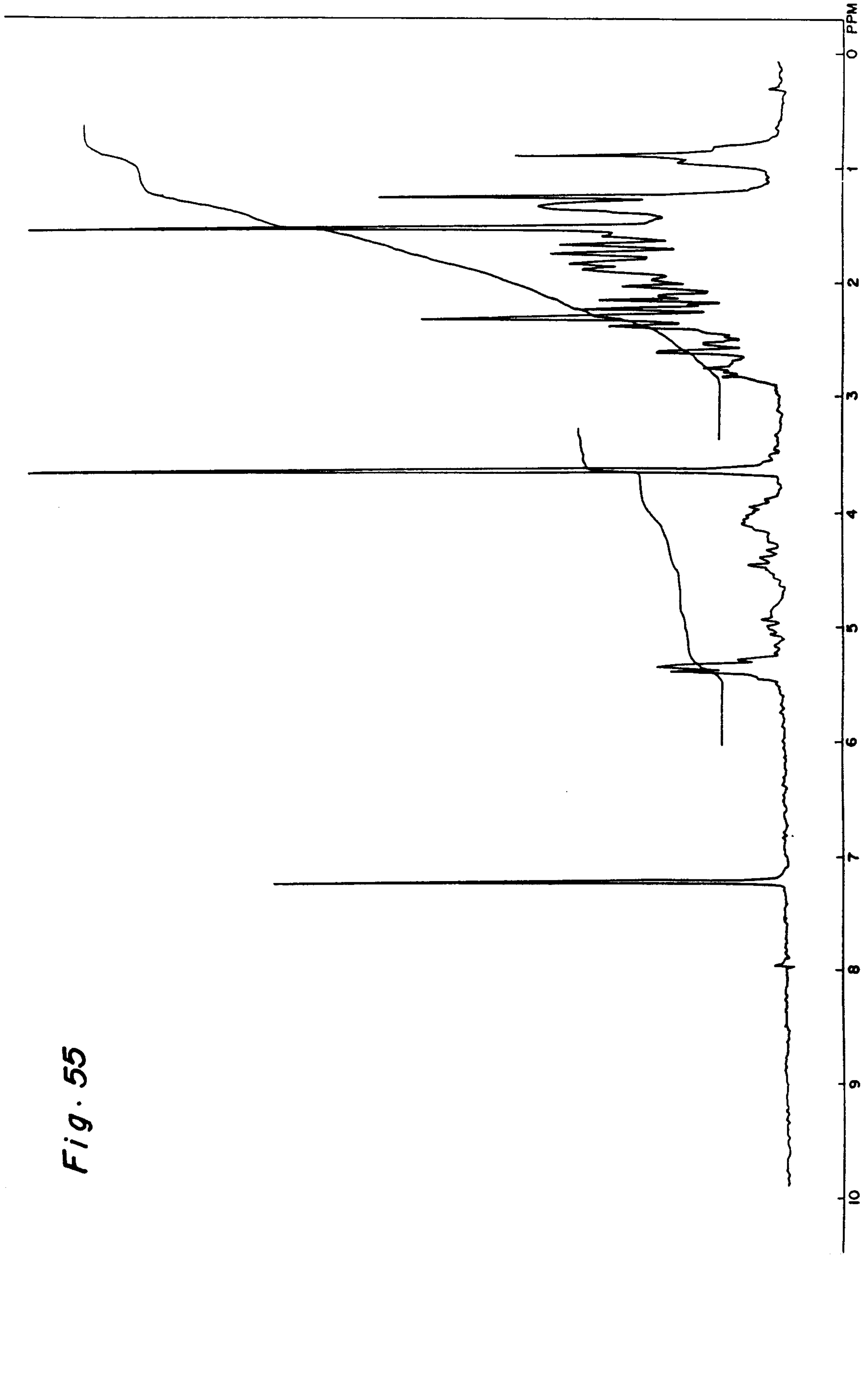


Fig. 55

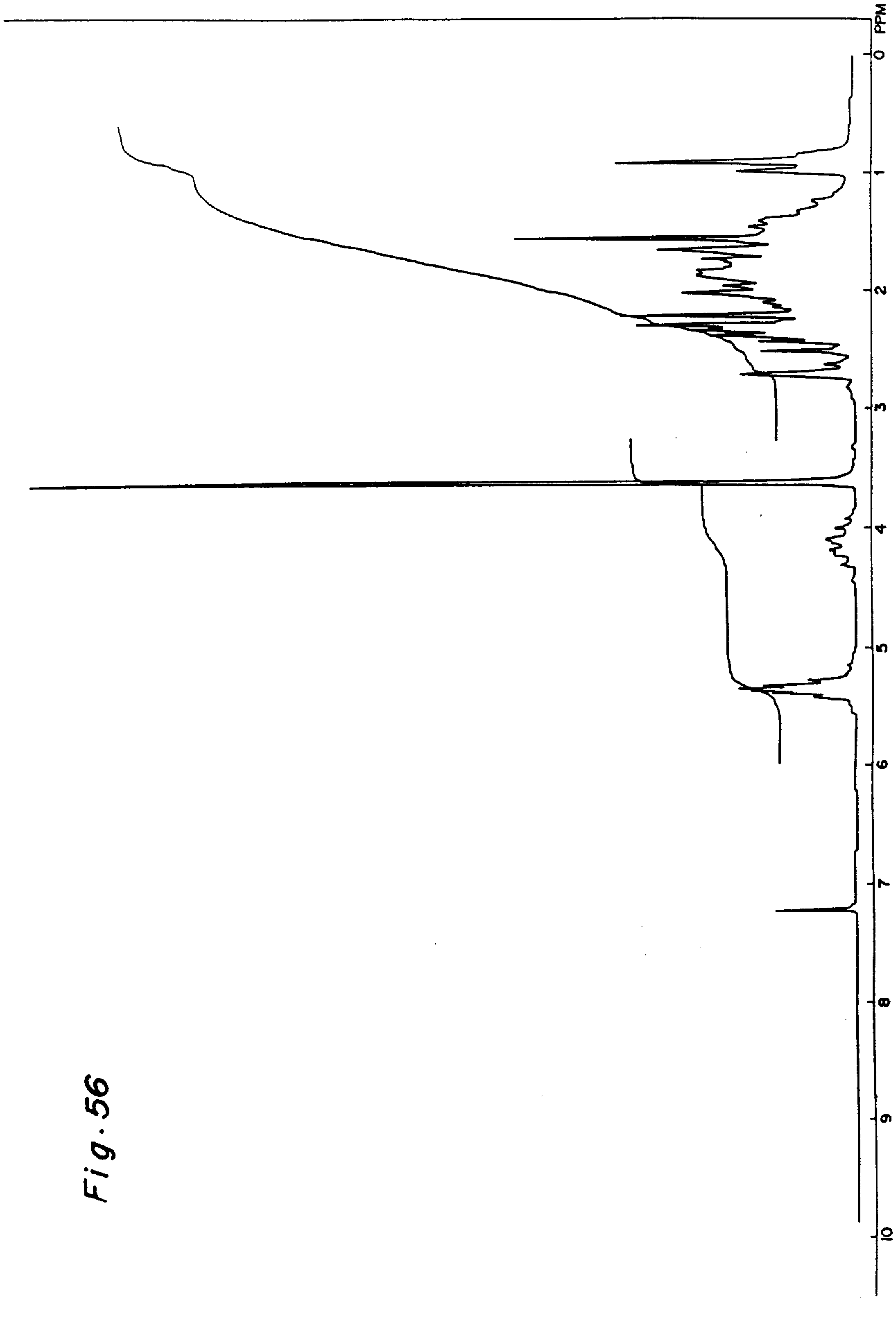


Fig. 56

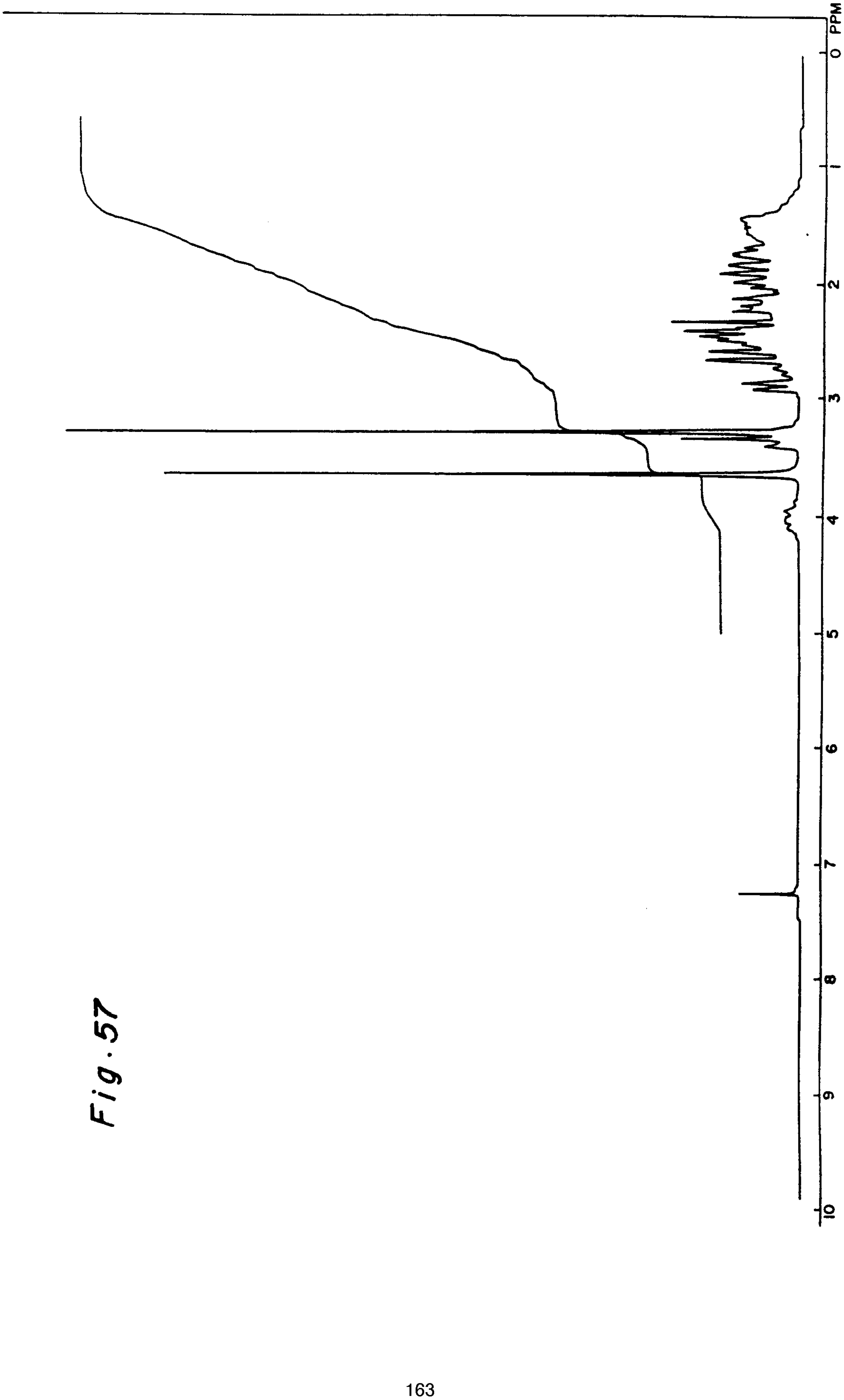


Fig. 57